(19) Europäisches Patentamt
European Patent Office
Office européen des brevets

(11) EP 4 268 824 A1

(12) **EUROPEAN PATENT APPLICATION**

(43) Date of publication:
**01.11.2023 Bulletin 2023/44**

(21) Application number: **22305621.9**

(22) Date of filing: **26.04.2022**

(51) International Patent Classification (IPC):
***A61K 31/53*** (2006.01) ***A61K 31/4745*** (2006.01)
***A61K 31/5025*** (2006.01) ***A61K 31/519*** (2006.01)
***A61K 31/52*** (2006.01) ***A61K 31/5377*** (2006.01)
***A61K 45/06*** (2006.01) ***A61P 43/00*** (2006.01)
***A61K 31/404*** (2006.01) ***A61K 31/4439*** (2006.01)
***A61K 31/47*** (2006.01) ***C07D 403/14*** (2006.01)

(52) Cooperative Patent Classification (CPC):
(C-Sets available)
**A61P 43/00; A61K 31/404; A61K 31/4439; A61K 31/47; A61K 31/4745; A61K 31/5025; A61K 31/519; A61K 31/52; A61K 31/53; A61K 31/5377; A61K 45/06; C07D 487/04** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Manros Therapeutics**
**29680 Roscoff (FR)**

(72) Inventors:
- **PREVELLE, Marion.**
  **45130 Le Bardon (FR)**
- **ELIE, Jonathan**
  **29680 Roscoff (FR)**
- **MIEGE, Frédéric.**
  **69008 Lyon (FR)**
- **BROUDIC, Nathan.**
  **76000 Rouen (FR)**
- **BESSON, Thierry.**
  **76000 Rouen (FR)**
- **BECQ, Frédéric.**
  **86073 Poitiers (FR)**
- **MEIJER, Laurent.**
  **29680 Roscoff (FR)**

(74) Representative: **Cabinet Nony**
**11 rue Saint-Georges**
**75009 Paris (FR)**

(54) **IMIDAZO[2,1-F][1,2,4]TRIAZINE DERIVATIVES USEFUL AS A MEDICAMENT**

(57) The present invention relates to a combination of a compound of formula (I) or any of its pharmaceutically acceptable salt with an ABC transporter modulator, including a CFTR modulator or a combination of CFTR modulators, for use in the prevention and/or treatment of genetic diseases associated with ABC transporters trafficking defects, in particular cystic fibrosis

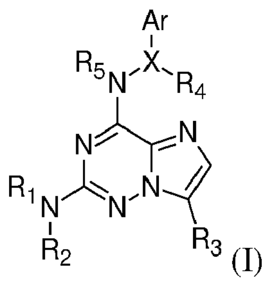
(I)

wherein **Ar** represents a phenyl group or a 6-membered heteroaryl group comprising one to three nitrogen atoms, **-X-** represents a -CH-group, a bond, a -CHCH2-group or a -CH2CH group-, **$R_1$** represents a ($C_1$-$C_6$)alkyl group, a **-R-L-** group, wherein **R** represents a ($C_3$-$C_8$)cycloalkyl group, a ($C_3$-$C_8$)heterocycloalkyl group, a ($C_5$-$C_6$)heteroaryl group or a phenyl group **$R_2$** represents a hydrogen atom or a ($C_1$-$C_3$)alkyl group, or alternatively **$R_1$** and **$R_2$** together form with the nitrogen atom to which they are attached, a ($C_3$-$C_8$)heterocycloalkyl group or a spiro($C_5$-$C_{11}$)heterobicycloalkyl group.

The present invention further relates to a compound of formula (I) as defined above.

The present invention further relates to synthetic methods for obtaining them and to pharmaceutical compositions containing them.

EP 4 268 824 A1

(52) Cooperative Patent Classification (CPC): (Cont.)

C-Sets
**A61K 31/404, A61K 2300/00;**
**A61K 31/4439, A61K 2300/00;**
**A61K 31/47, A61K 2300/00;**
**A61K 31/4745, A61K 2300/00;**
**A61K 31/5025, A61K 2300/00;**
**A61K 31/519, A61K 2300/00;**
**A61K 31/52, A61K 2300/00;**
**A61K 31/53, A61K 2300/00;**
**A61K 31/5377, A61K 2300/00**

## Description

## FIELD OF THE INVENTION

The present invention relates to imidazo[2,1-f][1,2,4]triazine derivatives useful in improving the treatment of genetic diseases associated with ABC transporters trafficking defects, in particular cystic fibrosis. It further relates to their manufacturing process and to pharmaceutical compositions containing them, in particular in combination with an ABC transporter modulator, including a CFTR modulator or a combination of CFTR modulators.

## BACKGROUND

Defects in the superfamily of 44 ABC (ATP-Binding Cassette) transporters is the source of more than 15 different genetic diseases, and among all: Tangier Disease, Familial hypoapoproteinemia (ABCA1), Stargardt/fundus flavimaculatis, Retinitis pigmentosa & Cone-rod dystrophy & Age-related macular degeneration (ABCA4), Stargardt Macular disease (ABCA4), Harlequin ichthyosis (ABCA12), biliary diseases (such as Progressive familial intrahepatic cholestasis type 2 or 3 (PFIC2, PIFC3) (ABCB4, ABCB11), Dubin-Johnson Syndrome (ABCC2), Pseudoxanthoma elasticum (ABCC6), Cystic Fibrosis (ABCC7 = CFTR), idiopathic chronic cholestasis (ABCC12), Adrenoleukodystrophy (ABCD1), low-phospholipid associated cholelithiasis syndrome, intrahepatic cholestasis of pregnancy), Sitosteroliemia and Familial Hypercholesterolemia (ABCG5, ABCG8).

Cystic fibrosis (CF) is the most common recessive autosomal lethal genetic disease. 162,428 people are estimated to be living with CF across 94 countries in the world, among which an estimated 105,352 are diagnosed [Guo et al., 2022. Worldwide rates of diagnosis and effective treatment for cystic fibrosis. J. Cystic Fibrosis, in press. Doi.org/10.1016/j.jcf.2022.01.009]. There are 47,650 estimated diagnosed patients in Europe, 37,002 in North America, 10,034 in South America, 3,652 in Australasia, 5,349 in Asia, 1,665 in Africa. CF is caused by mutations in a single gene (Cystic Fibrosis Transmembrane Regulator (CFTR), also called ABCC7).

The CF gene (locus 7q31) encodes said protein called Cystic Fibrosis Transmembrane Conductance Regulator. Mutations of the CF gene cause abnormal transport of water and electrolytes through the cell membrane of various organs such as the lungs, sudoriparous glands, the intestine and the exocrine pancreas. CFTR controls the transport of various ions responsible for proper hydration throughout the body and anti-inflammatory and/or antibacterial defenses on the mucosal surface. The CFTR protein is a glycoprotein of 1,480 amino acids, belonging to the ABC superfamily of membrane transporters. CFTR is a chloride ion channel notably localized in the apical plasma membrane of lung epithelial cells in healthy individuals.

CFTR is responsible for trans-epithelial transport of water and electrolytes, thereby allowing hydration of lung airways in healthy individuals. Loss of CFTR function leads to the accumulation of viscous secretions and repeated lung infections. Chronic infection and inflammation are some of the major issues faced by patients with CF (PWCF), with most PWCF experiencing multiple bacterial infections throughout their life. In the initial stage, common bacteria such as *Staphylococcus aureus* colonize and infect the lungs. Their incidence declines later in life and infections with *Pseudomonas aeruginosa* become more prominent. In the USA, chronic MRSA is also common and can outcompete *P. aeruginosa.* The appearance of antibiotic-resistant strains (such as those of *Mycobacterium abscessus*), a consequence of chronic use of antibiotics, poses another challenge in treating chronic infections. As a result, chronic respiratory infections are the main cause of morbidity and mortality in PWCF [reviews in Kidd, T.J. et al., 2018. Defining antimicrobial resistance in cystic fibrosis. J. Cyst. Fibros. 17, 696-704. doi:10.1016/j.jcf.2018.08.014; Waters, V.J. et al., 2019. Reconciling antimicrobial susceptibility testing and clinical response in antimicrobial treatment of chronic cystic fibrosis lung infections. Clin. Infect. Dis. 69, 1812-1816. doi:10.1093/cid/ciz364]. Alternatives to conventional antibiotics are thus essential.

Although there are over 2,000 mutations of the CFTR protein, the most common mutation (70% of patients) leads to the deletion of a phenylalanine in the NBD1 domain at position 508 (F508del-CFTR). In CF patients homozygous for F508del-CFTR mutation, and more generally for class-II mutations (i.e. producing a protein that is absent from the cell membrane), CFTR is absent from the plasma membrane due to faulty addressing of this protein, which remains in the endoplasmic reticulum (ER) and undergoes degradation. In such cases, hydration of lung airways is no longer functional. The F508 deletion alters the folding of the NBD1 domain and prevents the full maturation of the protein, which is therefore degraded very early during biosynthesis.

The main cause of mortality in CF patients is thus linked to this deletion and leads to infections or pulmonary insufficiency due to an increase in mucus viscosity. Such viscosity causes occlusion of respiratory airways and promotes infections by opportunistic bacteria, in particular by microorganisms such as *P. aeruginosa,* leading to multi-resistance to antibiotics. Furthermore, an aggravation is observed at the level of the digestive apparatus and the pancreas particularly (patient with pancreatic insufficiency).

There are few effective drugs to prevent disease progression associated with most CF mutations. Standard

CF therapies rely heavily on repeated use of antibiotics, which ultimately fail to eradicate lung infections and lead to the emergence of multiple drug-resistant pathogens. The decline of lung function in CF patients can be seen even in early childhood, and this decline leads to the need for lung transplantation or to premature death due to extensive infection and inflammation and respiratory failure.

Some treatments have been commercialized recently by Vertex, namely Kalydeco®, Orkambi®, Symedeko® and more recently Trikafta®.

Trikafta® is a triple combination precision medicine (ivacaftor, tezacaftor and elexacaftor) and belongs to a class of drugs called CFTR modulators. It is designed for PWCF who have at least one of 178 different mutations in their CFTR (cystic fibrosis transmembrane conductance regulator) gene, in particular the F508del-CFTR mutation.

A compound

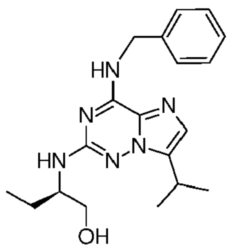

(N & N 2), named compound (6) in the following description, is known from K. Bettayeb et al., 2008, "N-&-N, a new class of cell death-inducing kinase inhibitors derived from the purine roscovitine ", Mol. Cancer Ther. 7, 2713-2724.and from F. Popowycz et al., 2009, "Pyrazolo[1,5-a]-1,3,5-triazine as purine bioisostere: access to potent CDK inhibitor (R)-roscovitine analogue ", J. Med. Chem. 52, 655-663. Said compound is disclosed as mainly showing modest kinase inhibitory activity and modest antitumor properties.

It is additionally known from C. Norez et al. 2014, "Roscovitine is a proteostasis regulator that corrects the trafficking defect of F508del-CFTR by a CDK-independent mechanism", Br. J. Pharmacol. 171, 4831-4849., that the purine roscovitine is able to partially correct the defective function of F508del-CFTR by preventing the ability of the ERQC (endoplasmic reticulum quality control) to interact with and degrade F508del-CFTR *via* two synergistic but CDK-independent mechanisms.

It is additionally known from Riazanski et al., 2015. TRPC6 channel translocation into phagosomal membrane augments phagosomal function. Proc. Natl. Acad. Sci. USA 112, E6486-6495, that roscovitine is able to reduce the intra-phagolysosomal pH of alveolar macrophages, thereby favoring their bactericidal activity which is compromised when CFTR is mutated or absent.

There is a continued need to find new therapeutic agents to improve the treatment of genetic diseases associated with ABC transporters trafficking defects, and more particularly to improve the treatment of PWCF, in particular to improve the treatment of PWCF already treated with an ABC transporters modulator, including a CFTR modulator. There is also a need to reduce the dosing of the current CFTR modulators in order to reduce their side effects.

## SUMMARY OF THE INVENTION

It has now been found that the compounds as defined in formula (I) hereinafter are useful in the treatment and/or prevention of genetic diseases associated with ABC transporters trafficking defects, and more particularly to improve the treatment of CF patients.

## FIGURES

**FIG 1.** represents the conductance measurement as a function of time for assessing the activity of F508del-CFTR in CFBE cells using the Ussing chamber as set forth in example **13.** Cells are incubated for 24 hours with either Trikafta® alone or Trikafta® + a compound of formula (I) as defined herein after. Conductance is measured as a function of time. At time zero, 1 μM forskolin is added, leading to the activation (◄━━━━► ) of a conductance, the nature of which (F508del-CFTR) is confirmed by the addition of 10 μM of a specific CFTR inhibitor, CFTR-172 (◄▪▪▪▪▪▪▪► ). Both activation and inhibition values represent a measure of the channel activity of F508del-CFTR which is increased by Trikafta®, and even more by the combination of Trikafta® + a compound of formula (I) as defined herein after. The 'inhibition shift" induced by CFTR-172 (◄▪▪▪▪▪▪▪► in Fig. 1) represents best the CFTR

activity which was activated by Trikafta® + a compound of formula (I) as defined herein after. This is the measure which are reported in all Tables shown below.

**FIG 2.** The correcting effect of Trikafta® on F508del-CFTR in a pulmonary epithelial cell line derived from a F508del-CFTR CF patient was tested as set forth in **example 13** herein after in the presence of increasing concentrations of compound (44) as defined herein after and compound (107) as defined herein after. Dose-response curves were built from the activation and inhibition data (see FIG. 1). 100% represents the activity of Trikafta® alone.

**FIG 3.** Compound (107) as defined herein after compensates the dilution of Trikafta® in terms of F508del-CFTR activation. CFE cells were treated with Trikafta® 100%, 33%, or 11%, in the absence or presence of 1 $\mu$M of compound (107). F508del-CFTR activity was monitored as described in the Experimental section and above. Activity is reported as percentage of $\delta$F508del-CFTR activity in the presence of Trikafta® alone (100%). It was calculated on the basis of the inhibitory effect of the CFTR inhibitor CFTR-172, as shown in Fig. 1. The fold change over the activity of Trikafta® alone is indicated in parentheses.

**FIG 4.** The correcting effect of further CFTR modulators than Trikafta®, i.e. Galapagos compounds as defined herein after (GLPG 222 (**G1**), GLPG 1837 **(G2),** GLPG 2451 **(G3))** (1 $\mu$M each) on F508del-CFTR in a pulmonary epithelial cell line derived from a F508del-CFTR CF patient was tested as set forth in **example 13** herein after in the presence of increasing concentrations of compound (107) as defined herein after. Dose-response curves were built from the activation and inhibition data (see FIG. 1). 100% represents the activity of each CFTR modulator alone.

## DEFINITIONS

As used herein, the term *"patient"* refers to either an animal, such as a valuable animal for breeding, company or preservation purposes, or preferably a human or a human child, which is afflicted with, or has the potential to be afflicted with one or more diseases and conditions described herein.

In particular, as used in the present application, the term *"patient"* refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term *"treating"* or *"treatment",* as used herein, means preventing, reversing, alleviating, inhibiting the progress of, or preventing the disease of genetic diseases associated with ABC transporters trafficking defects and more particularly in connection to the diseases as described herein after in the paragraph "PATHOLOGIES", in particular cystic fibrosis.

Therefore, the term *"treating"* or *"treatment"* encompasses within the framework of the present invention the improvement of medical conditions of patients suffering from the diseases as described herein after in the paragraph "PATHOLOGIES", related to trafficking defects in the ABC transporters.

As used herein, an *"ABC transporters trafficking modulator"* or "ABC transporters modulator" is a modulator of ATP-Binding Cassette ("ABC") transporters or fragments thereof, including Cystic Fibrosis Transmembrane Conductance Regulator ("CFTR"), and in particular mutated forms thereof, such as F508del-CFTR. There are potentiators (for example ivacaftor) that increase the ability of CFTR to open its chloride channel, and correctors (for example elexacaftor, lumacaftor and tezacaftor) that promote the membrane integration of CFTR. The potentiators and correctors form the modulators.

As used herein, an *"activator"* or *"booster"* means a compound that allows an improvement in terms of correction of the ABC transporters trafficking defects, including the CFTR, in a patient simultaneously, separately or sequentially treated with another ABC transporters modulator, including another CFTR modulator.

As used herein, an *"effective amount"* refers to an amount of a compound of the present invention which is effective in preventing, reducing, eliminating, treating or controlling the symptoms of the herein-described diseases and conditions. An *"effective amount"* also refers to an amount of a compound of the present invention which is effective in reducing the usual dosing of the current CFTR modulators, in order to reduce its side effects.

The term *"controlling"* is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term *"effective amount"* includes *"prophylaxis-effective amount"* as well as "treatment-effective amount".

The term *"preventing",* as used herein, means reducing the risk of onset or slowing the occurrence of a given phenomenon, namely in the present invention, a genetic disease associated with ABC transporters trafficking defects,

in particular CF due to mutations on the CFTR genes leading to abnormal protein (such as F508del-CFTR).

As used herein, *"preventing"* also encompasses *"reducing the likelihood of occurrence"*or *"reducing the likelihood of reoccurrence".*

The term *"prophylaxis-effective amount"* refers to a concentration of compound of this invention that is effective in inhibiting, preventing, decreasing the likelihood of anyone of the hereabove described diseases.

Likewise, the term *"treatment-effective amount"* refers to a concentration of compound that is effective in treating the hereabove described diseases, e.g. leads to improvement of the activity of the ABC transporter, and in particular of F508del-CFTR, following examination when administered after disease has occurred.

As used herein, the term *"pharmaceutically acceptable"* refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

## DETAILED DESCRIPTION OF THE INVENTION

The inventors have surprisingly found that the compounds of formula (I) as disclosed herein after, improve the activity of F508del-CFTR, on immortalized cell line created from bronchial epithelium of a CF patient homozygous for F508del-CFTR (CFBE cells) when added to known CFTR modulators or combinations thereof, such as Trikafta®. In other words, it may be administered to a patient simultaneously, separately or sequentially treated with another ABC transporters modulator, including other CFTR modulators, alone or in combination, such as Trikafta®.

This assertion is based on data as illustrated in the following examples and more detailed herein after. As it will be apparent from the examples, the compounds of formula (I) allow an activation of the activity of the CFTR Cl channel on cells expressing the F508del-CFTR mutation, said cells being already in presence of Trikafta® as well as other CFTR modulators or combination thereof.

The activity of F508del-CFTR was indeed estimated according to the method described below in example 13. This method makes use of CFBE cells, an immortalized cell line generated from bronchial epithelium of a CF patient homozygous for F508del-CFTR [Kunzelmann, K. et al., 1993. An immortalized cystic fibrosis tracheal epithelial cell line homozygous for the F508del CFTR mutation. Am. J. Respir. Cell Mol. Biol. 8, 522-529.]. Cells are exposed for 24 hours to a combination of Trikafta® and a compound of formula (I) as defined herein after to be evaluated, and the activity of F508del-CFTR is measured by conductance in a Ussing chamber. The activation is initiated by addition of Forskolin (1 μM). The nature of the increased conductance is confirmed as due to CFTR activity by the addition of a specific CFTR inhibitor, CFTR-172 (10 μM), at the end of the experiment (FIG. 1). All compounds tested were evaluated in combination with Trikafta®. In most instances, compounds were tested in triplicates at 0.1, 1 and 10 μM. The 'inhibition shift" induced by CFTR-172 (◄·······► in FIG. 1) represents best the CFTR activity which was activated by Trikafta® + a compound of formula (I) as defined herein after.

Wild-type CFBE cells (containing full-length, unmutated CFTR) are used as a reference. The inhibition values are the ones reported in **Table 4** of said **Example 13,** which is the significant value in connection to the claimed activity.

According to a first aspect, a subject-matter of the present invention relates to a combination of a compound of formula (I) or any of its pharmaceutically acceptable salt with an ABC transporter modulator, including a CFTR modulator or a combination of CFTR modulators, for use in the prevention and/or treatment of genetic diseases associated with ABC transporters trafficking defects, in particular cystic fibrosis

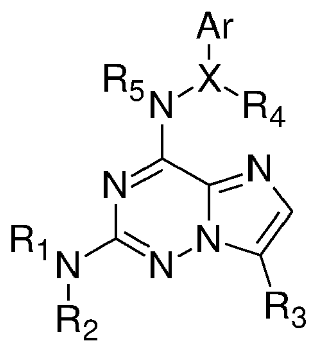

(I)

wherein

**Ar** represents

- a phenyl group, optionally substituted by one to three groups selected from a $(C_1-C_3)$alkyl group, a $(C_1-C_3)$alkoxy group, a $(C_1-C_3)$fluoroalkyl group and a halogen atom, or
- a 6-membered heteroaryl group comprising one to three nitrogen atoms, optionally substituted by one to three groups selected from a $(C_1-C_3)$alkyl group, a $(C_1-C_3)$alkoxy group and a halogen atom,

**-X-** represents a -CH-group, a bond, a -CHCH2- group or a -CH2CH group-, $\mathbf{R_1}$ represents

- a $(C_1-C_6)$alkyl group, optionally substituted by a group selected from a hydroxy group, a halogen atom, a $NR_6R_7$ group, a $(C_1-C_4)$alkylthio group, a phenoxy group and a $(C_1-C_3)$alkoxy group,
- a **-R-L-** group, wherein

  **L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a $-SO_2-$ group, and
  **R** represents:

  - a $(C_3-C_8)$cycloalkyl group, optionally substituted by one to three groups selected from a hydroxy group, a halogen atom, a $(C_1-C_4)$alkyl group and a $(C_1-C_3)$alkoxy group,
  - a $(C_3-C_8)$heterocycloalkyl group, optionally substituted by one or two groups selected from a hydroxy group, a halogen atom, an oxo group, a $(C_1-C_4)$alkyl group and a $(C_1-C_3)$alkoxy group,
  - a $(C_5-C_6)$heteroaryl group, optionally substituted by one to three groups selected from a $(C_1\ C_4)$alkyl group, a hydroxy group, a halogen atom and a $(C_1-C_3)$alkoxy group,
  - a phenyl group, optionally substituted by one or two groups selected from a hydroxy group, a halogen atom, and a $(C_1-C_3)$alkoxy group,
    **R2** represents a hydrogen atom or a $(C_1-C_3)$alkyl group,

or alternatively $\mathbf{R_1}$ and $\mathbf{R_2}$ together form with the nitrogen atom to which they are attached, a $(C_3-C_8)$heterocycloalkyl group or a spiro$(C_5-C_{11})$heterobicycloalkyl group, said groups being optionally substituted by one or two groups selected from a hydroxy group, a $(C_1-C_3)$alkoxy group, a hydroxy$(C_1-C_4)$alkyl group, an oxo group and a $(C_1-C_4)$alkyl group,
$\mathbf{R_3}$ represents

- a hydrogen atom,
  a $(C_1-C_6)$alkyl group,
- a $(C_3-C_8)$cycloalkyl group,
- a halogen atom, or
- a phenyl group optionally substituted by one to three groups selected from a $(C_1-C_3)$alkyl group, a $(C_1-C_3)$alkoxy group, a $(C_1-C_3)$fluoroalkyl group and a halogen atom.

$\mathbf{R_4}$ represents a hydrogen atom or a $(C_1-C_4)$alkyl group or is absent when **-X-** is a bond,
$\mathbf{R_5}$ represents a hydrogen atom or a $(C_1-C_4)$alkyl group, and
$\mathbf{R_6}$ and $\mathbf{R_7}$ independently represent a hydrogen atom or a $(C_1-C_4)$alkyl group.

The inventors have surprisingly discovered that compounds of formula (I) demonstrated an activating or boosting activity on a human lung epithelium cell line derived from a CF patient with F508del-CFTR mutation when administered in combination with another compound improving of the activity of the ABC transporter, and in particular of F508del-CFTR, and even more particularly in combination with the triple combination of ivacaftor, tezacaftor and elexacaftor, as in particular commercialized under the trademark Trikafta®. Compounds of formula (I) also boost the F508del-CFTR activation triggered by single components of Trikafta®, by other CFTR modulators as demonstrated in example 14, and by a temperature shift.

The inventors have further surprisingly discovered that compounds having the following scaffolds of formula **(A)** to **(E)** displayed much reduced activating or boosting activity on a human lung epithelium cell line derived from a CF patient with F508del-CFTR mutation compared to the imidazo[2,1-f][1,2,4]triazine derivatives of formula (I) according to the present invention when administered in combination with another compound improving of the activity of the ABC transporter, and in particular of F508del-CFTR, and even more particularly in combination with the triple combination of ivacaftor, tezacaftor and elexacaftor, as in particular commercialized under the trademark Trikafta®.

**Table 1:** Various molecules which differ by their central scaffold but share the same substituents on homologous positions were synthesized and tested for their ability to boost the F508del-CFTR activity stimulated by Trikafta® in CFBE cells. Activity of these molecules were measured by the 'inhibition shift" induced by CFTR-172 on the F508del-CFTR channel activity stimulated by Trikafta® by the method much more detailed in example 13 herein after. Activities are reported in reference to the CFTR activity induced by Trikafta® alone (value=100).

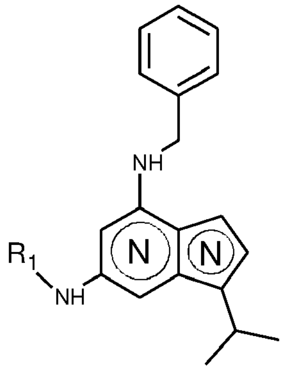

| 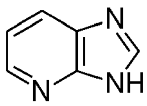 | 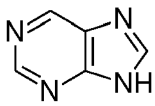 | 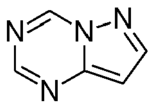 | 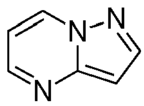 | 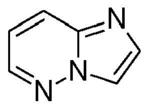 | 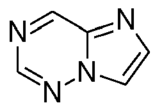 |
| --- | --- | --- | --- | --- | --- |
| **A** | **B** | **C** | **D** | **E** | Compound (I) |

| $R_1$ = | 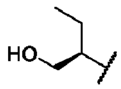 | | | 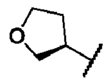 | | |
| --- | --- | --- | --- | --- | --- | --- |
| C (µM) | 0.1 | 1 | 10 | 0.1 | 1 | 10 |
| (A) | - | - | - | 82.7 | 107.8 | 114.5 |
| (B) | 104.0 | 106.7 | 105.6 | 108.3 | 107.1 | 133.2 |
| (C) | - | - | - | 109.7 | 99.5 | 116.1 |
| (D) | - | - | - | 98.0 | 110.1 | 98.7 |
| (E) | 106.5 | 101.6 | 85.7 | 92.5 | 115.5 | 137.3 |
| compound of formula (I) | **94.5** | **124.9** | **121.9** | **112.0** | **126.7** | **155.6** |

Thus, at 0.1, 1 or 10 µM of a compound of the present invention, the activity of Trikafta® is, respectively, boosted by >40%, >70% or >80% (according to the following formula:

[(F508del-CFTR activity value in the presence of Trikafta® AND 0.1, 1 or 10 µM of compound of formula (I)) / (F508del-CFTR activity value in the presence of Trikafta®)] * 100).

Moereover, the inventors have further established that roscovitine displayed no activating or boosting activity on a human lung epithelium cell line derived from a CF patient with F508del-CFTR mutation when administered in combination with another compound improving of the activity of the ABC transporter, and in particular of F508del-CFTR, and even more particularly in combination with the triple combination of ivacaftor, tezacaftor and elexacaftor, as in particular commercialized under the trademark Trikafta®.

According to a particular embodiment, the present invention relates to a compound of formula (I) as defined herein above for use in the prevention and/or treatment of genetic diseases associated with ABC transporters trafficking

defects selected from Tangier Disease, Familial hypoapoproteinemia (ABCA1), Stargardt/fundus flavimaculatis, Retinitis pigmentosa & Cone-rod dystrophy & Age-related macular degeneration (ABCA4), Stargardt Macular disease (ABCA4), Harlequin ichthyosis (ABCA12), biliary diseases (such as Progressive familial intrahepatic cholestasis type 2 or 3 (PFIC2, PIFC3) (ABCB4, ABCB11), Dubin-Johnson Syndrome (ABCC2), Pseudoxanthoma elasticum (ABCC6), Cystic Fibrosis (ABCC7 = CFTR), idiopathic chronic cholestasis (ABCC12), Adrenoleukodystrophy (ABCD1), low-phospholipid associated cholelithiasis syndrome, intrahepatic cholestasis of pregnancy), Sitosteroliemia and Familial Hypercholesterolemia (ABCG5, ABCG8), and in particular Cystic Fibrosis.

According to another particular embodiment, herein is further provided a compound of formula (I) as defined herein above for use wherein **Ar** represents

- a phenyl group, optionally substituted by one to three groups selected from a $(C_1-C_3)$alkyl group, a $(C_1-C_3)$alkoxy group, a $(C_1-C_3)$fluoroalkyl group and a halogen atom,
- a pyridyl group, optionally substituted by one to three groups selected from a $(C_1-C_3)$alkyl group, a (C1-C3)alkoxy group and a halogen atom,
- a pyrimidinyl group, optionally substituted by one to three groups selected from a $(C_1-C_3)$alkyl group, a (C1-C3)alkoxy group and a halogen atom, or
- a pyrazinyl group, optionally substituted by one to three groups selected from a $(C_1-C_3)$alkyl group, a (C1-C3)alkoxy group and a halogen atom,

  and more particularly represents
- a phenyl group, optionally substituted by one to three groups selected from a $(C_1-C_3)$alkyl group, a $(C_1-C_3)$alkoxy group, a $(C_1-C_3)$fluoroalkyl group and a halogen atom,
- a pyridyl group, or
- a pyrazinyl group.

According to another particular embodiment, herein is further provided a compound of formula (I) as defined herein above for use wherein $\mathbf{R_1}$ represents

- a $(C_1-C_6)$alkyl group, optionally substituted by a group selected from a hydroxy group, a NR6R7 group, a $(C_1-C_4)$alkylthio group, a phenoxy group and a $(C_1-C_3)$alkoxy group,
- a **-R-L-** group, wherein

  **L** is either a single bond, a -CH2- group, a -CH(CH3)- group, a -CH2-CH2- group, or a $-SO_2-$ group, and
  **R** represents:

  - a $(C_3-C_8)$cycloalkyl group, optionally substituted by one to three groups selected from a hydroxy group, a halogen atom, and a $(C_1-C_3)$alkoxy group,
  - a $(C_3-C_8)$heterocycloalkyl group, optionally substituted by one or two groups selected from a hydroxy group, a $(C_1-C_4)$alkyl group and a $(C_1-C_3)$alkoxy group,
  - a $(C_5-C_6)$heteroaryl group, optionally substituted by one to three groups selected from a $(C_1\ C_4)$alkyl group and a halogen atom, or
  - a phenyl group, optionally substituted by one or two halogen atoms,

    and

  **R2** represents a hydrogen atom or a $(C_1-C_3)$alkyl group, or alternatively $\mathbf{R_1}$ and $\mathbf{R_2}$ together form with the nitrogen atom to which they are attached, a $(C_3-C_8)$heterocycloalkyl group, optionally substituted by one or two groups selected from a hydroxy group, a $(C_1-C_3)$alkoxy group, a hydroxy$(C_1-C_4)$alkyl group or a spiro$(C_5-C_{11})$heterobicycloalkyl group, and
  $\mathbf{R_6}$ and $\mathbf{R_7}$ independently represent a hydrogen atom or a methyl group.

According to another particular embodiment, herein is further provided a compound of formula (I) as defined herein above for use wherein **R3** represents

- a hydrogen atom,
- a methyl group, an ethyl group or an isopropyl group,
- a cyclopropyl group, a cyclopentyl group or a cyclohexyl group,
- a bromine atom, or
- a phenyl group.

According to another particular embodiment, herein is further provided a compound of formula (I) as defined herein above for use wherein $\mathbf{R_4}$ represents a hydrogen atom or a methyl group or is absent when **-X-** is a bond.

According to another particular embodiment, herein is further provided a compound of formula (I) as defined herein above for use wherein **Rs** is a hydrogen atom.

According to another particular embodiment, herein is further provided a compound of formula (I) as defined herein above for use wherein $\mathbf{R_5}$ is a ($C_1$-$C_4$)alkyl group, in particular a methyl group.

As apparent from example 14 herein after, compounds of formula (I) with a $\mathbf{R_5}$ radical different from a hydrogen atom may present the advantage to loose kinase inhibitory activity but maintain essentially all their ability to enhance Trikafta®'s activity on F508del-CFTR, allowing to remove side effects that might be linked to kinase inhibition.

According to another particular embodiment, herein is further provided a compound of formula (I) as defined herein above for use wherein the ABC transporter modulator is selected from GLPG 2222, GLPG 1837, GLPG 2451, their combinations such as GLPG 2222 and GLPG 1837, GLPG 2222 and GLPG 2451 and GLPG 2222, GLPG 1837 and GLPG 2451, lumacaftor, galicaftor, navocaftor, dirocaftor, nesolicaftor, posenacaftor, ivacaftor, tezacaftor, elexacaftor, and their combinations, in particular is selected from ivacaftor, tezacaftor and elexacaftor and more particularly is a mixture of ivacaftor, tezacaftor and elexacaftor.

According to another particular embodiment, herein is further provided a compound of formula (I) or any of its pharmaceutically acceptable salt,

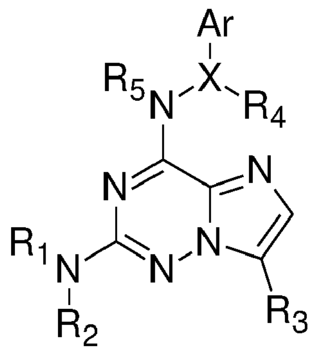

(I)

wherein

**Ar** represents

- a phenyl group, optionally substituted by one to three groups selected from a ($C_1$-$C_3$)alkyl group, a ($C_1$-$C_3$)alkoxy group, a ($C_1$-$C_3$)fluoroalkyl group and a halogen atom,
- a 6-membered heteroaryl group comprising one to three nitrogen atoms, optionally substituted by one to three groups selected from a ($C_1$-$C_3$)alkyl group, a ($C_1$-$C_3$)alkoxy group and a halogen atom, and in particular a pyridyl, a pyrimidinyl or a pyrazinyl group, more particularly a pyridyl or a pyrazinyl group, optionally substituted by one to three groups selected from a ($C_1$-$C_3$)alkyl group, a ($C_1$-$C_3$)alkoxy group and a halogen atom,

**-X-** represents a -CH-group, a bond, a -CHCH2- group or a -CH2CH group-,

$\mathbf{R_1}$ represents

- a ($C_1$-$C_6$)alkyl group, optionally substituted by a group selected from a hydroxy group, a halogen atom, a NR6R7 group, a ($C_1$-$C_4$)alkylthio group, a phenoxy group and a ($C_1$-$C_3$)alkoxy group,
- a **-R-L-** group, wherein

**L** is either a single bond, a ($C_1$-$C_3$)alkanediyl group, a -O-($C_1$-$C_3$)alkanediyl group, said ($C_1$-$C_3$)alkanediyl group being optionally substituted by a group selected from a hydroxy group and a ($C_1$-$C_3$)alkoxy group or a -$SO_2$- group, and **R** represents:

- a ($C_3$-$C_8$)cycloalkyl group, optionally substituted by one to three groups selected from a hydroxy group, a halogen atom, a ($C_1$-$C_4$)alkyl group and a ($C_1$-$C_3$)alkoxy group,
- a ($C_3$-$C_8$)heterocycloalkyl group, optionally substituted by one or two groups selected from a hydroxy group, a halogen atom, an oxo group, a ($C_1$-$C_4$)alkyl group and a ($C_1$-$C_3$)alkoxy group,
- a ($C_5$-$C_6$)heteroaryl group, optionally substituted by one to three groups selected from a ($C_1$ C4)alkyl group, a

hydroxy group, a halogen atom and a $(C_1-C_3)$alkoxy group,

- a phenyl group, optionally substituted by one or two groups selected from a hydroxy group, a halogen atom, and a $(C_1-C_3)$alkoxy group,**R2** represents a hydrogen atom or a $(C_1-C_3)$alkyl group, or alternatively **$R_1$** and **$R_2$** together form with the nitrogen atom to which they are attached, a $(C_3-C_8)$heterocycloalkyl group or a spiro$(C_5-C_{11})$heterobicycloalkyl group, said groups being optionally substituted by one or two groups selected from a hydroxy group, a $(C_1-C_3)$alkoxy group, a hydroxy$(C_1-C_4)$alkyl group, an oxo group and a $(C_1-C_4)$alkyl group,

**$R_3$** represents

- a hydrogen atom, a $(C_1-C_6)$alkyl group,
- a $(C_3-C_8)$cycloalkyl group,
- a halogen atom, or
- a phenyl group.

**$R_4$** represents a hydrogen atom or a $(C_1-C_4)$alkyl group or is absent when **-X-** is a bond,
**$R_5$** represents a hydrogen atom or a $(C_1-C_4)$alkyl group, and
**$R_6$** and **$R_7$** independently represent a hydrogen atom or a $(C_1-C_4)$alkyl group, wherein when **Ar** is a phenyl group and **$R_3$** is an isopropyl group, then **$R_1$** is different from a $(C_1-C_6)$alkyl group substituted by a hydroxy group.

According to another particular embodiment, herein is further provided a compound of formula (I) or any of its pharmaceutically acceptable salt, as defined above, wherein
**$R_1$** represents a $(C_1-C_6)$alkyl group, optionally substituted by a group selected from a hydroxy group, a halogen atom, a NR6R7 group, a $(C_1-C_4)$alkylthio group, a phenoxy group and a $(C_1-C_3)$alkoxy group, and
**R2** represents a hydrogen atom or a $(C_1-C_3)$alkyl group.
Said sub-group of compounds are gathered under the "A1" class within the herein after **Table 2.**
According to another particular embodiment, herein is further provided a compound of formula (I) or any of its pharmaceutically acceptable salt, as defined above, wherein
**$R_1$** represents a **-R-L-** group, wherein

**L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a -$SO_2$- group, and
**R** represents a $(C_3-C_8)$cycloalkyl group, optionally substituted by one to three groups selected from a hydroxy group, a halogen atom, a $(C_1-C_4)$alkyl group and a $(C_1-C_3)$alkoxy group, and
**R2** represents a hydrogen atom or a $(C_1-C_3)$alkyl group.

Said sub-group of compounds are gathered under the "A2" class within the herein after **Table 2.**
According to another particular embodiment, herein is further provided a compound of formula (I) or any of its pharmaceutically acceptable salt, as defined above, wherein **$R_1$** and **$R_2$** together form with the nitrogen atom to which they are attached, a $(C_3-C_8)$heterocycloalkyl group or a spiro$(C_5-C_{11})$heterobicycloalkyl group, said groups being optionally substituted by one or two groups selected from a hydroxy group, a $(C_1-C_3)$alkoxy group, a hydroxy$(C_1-C_4)$alkyl group, an oxo group and a $(C_1-C_4)$alkyl group.
Said sub-group of compounds are gathered under the "A3" class within the herein after **Table 2.**
According to another particular embodiment, the present invention relates to a compound of formula (I) or any of its pharmaceutically acceptable salt, as defined above, wherein
**$R_1$** represents a **-R-L-** group, wherein

**L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a -$SO_2$- group, and
**R** represents a phenyl group, optionally substituted by one or two groups selected from a hydroxy group, a halogen atom, and a $(C_1-C_3)$alkoxy group, and
**R2** represents a hydrogen atom or a $(C_1-C_3)$alkyl group.

Said sub-group of compounds are gathered under the "A4" class within the herein after **Table 2.**
According to another particular embodiment, herein is further provided a compound of formula (I) or any of its pharmaceutically acceptable salt, as defined above, wherein
**$R_1$** represents a **-R-L-** group, wherein

**L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a $-SO_2-$ group, and **R** represents a $(C_3-C_8)$heterocycloalkyl group, optionally substituted by one or two groups selected from a hydroxy group, a halogen atom, an oxo group, a $(C_1-C_4)$alkyl group and a $(C_1-C_3)$alkoxy group, and
**R2** represents a hydrogen atom or a $(C_1-C_3)$alkyl group.

Said sub-group of compounds are gathered under the "A5" class within the herein after **Table 2.**
According to another particular embodiment, herein is further provided a compound of formula (I) or any of its pharmaceutically acceptable salt, as defined above, wherein
**$R_1$** represents a **-R-L-** group, wherein

**L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a $-SO_2-$ group, and
**R** represents a $(C_5-C_6)$heteroaryl group, such as a pyridyl, a pyrazinyl, a pyrimidyl, a pyrazolyl or an imidazolyl group, optionally substituted by one to three groups selected from a ($C_1$ C4)alkyl group, a hydroxy group, a halogen atom and a $(C_1-C_3)$alkoxy group, and
**R2** represents a hydrogen atom or a $(C_1-C_3)$alkyl group

Said sub-group of compounds are gathered under the "A6" class within the herein after **Table 2.**
Herein is further provided a pharmaceutical combination of a compound of formula (I) as defined herein after, or at least any of compounds (1) to (5) and (7) to (133) as defined hereinafter or a pharmaceutically acceptable salt thereof with an ABC modulator, including a CFTR modulator, or a combination of CFTR modulators, or a pharmaceutically acceptable salt thereof.
In the context of the present invention, the term:

- "halogen" is understood to mean chlorine, fluorine, bromine, or iodine, and in particular denotes chlorine, fluorine or bromine,
- "$(C_1-C_x)$alkyl", as used herein, respectively refers to a $C_1-C_x$ normal, secondary or tertiary monovalent saturated hydrocarbon radical, for example (Ci-C6)alkyl. Examples are, but are not limited to, methyl, ethyl, propyl, *n*-propyl, isopropyl, butyl, isobutyl, *sec*-butyl, *tert*-butyl, pentyl, isopentyl, hexyl and isohexyl groups, and the like.
- "$(C_1-C_3)$alkanediyl", as used herein, refers to a divalent saturated hydrocarbon radical which is branched or linear, comprises from 1 to 3 carbon atoms, and more particularly a methylene, ethylene or propylene, such as linear propylene or isopropylene, said alkanediyl may be substituted as it is apparent from the following description.
- a $(C_1-C_4)$alkylthio group also named a $(C_1-C_4)$alkylsulfanyl group: a -S-alkyl group where the alkyl group is as previously defined. By way of examples, mention may be made of, but not limited to: methylthio, ethylthio, propylthio, isopropylthio, linear, secondary or tertiary butylthio, isobutylthio, and the like;
- "$(C_3-C_8)$cycloalkyl", as used herein, refers to a cyclic saturated hydrocarbon, from 3 to 8 carbon atoms, saturated or partially unsaturated and unsubstituted or substituted. Examples are, but are not limited to, cyclopropyl, cyclobutyl, cyclopentyl, cyclohexyl, cycloheptyl and cyclooctyl.
- "$(C_3-C_8)$heterocycloalkyl group", as used herein, refers to a $(C_3-C_8)$cycloalkyl group wherein one or two of the carbon atoms are replaced with a heteroatom such as oxygen, nitrogen or sulphur, and more particularly such as an oxygen or a nitrogen atom. Such heterocycloalkyl group may be saturated or partially saturated and unsubstituted or substituted. Examples are, but are not limited to piperazinyl, piperidinyl, pyrrolidinyl, aziridinyl, oxanyl, oxetanyl, tetrahydropyranyl, morpholinyl, tetrahydrofuranyl, oxazolidinyl, oxepanyl, diazepanyl, dioxanyl and tetrahydrothiopyranyl, and more particularly piperidinyl and piperazinyl, and even more particularly piperazinyl.
- "spiro$(C_5-C_{11})$heterobicycloalkyl ring" refers to two rings connected through a defining single common atom. Such spiro$(C_5-C_{11})$heterobicycloalkyl ring generally comprises 5 to 11 carbon atoms referring to a "spiro$(C_5-C_{11})$bicyclic alkyl group" where one or more carbon atoms of the rings are replaced by heteroatom(s) such as oxygen, nitrogen or sulphur, and more particularly such as a nitrogen atom. Such spiro$(C_5-C_{11})$heterobicycloalkyl ring may be unsubstituted or substituted, in particular by at least one $(C_1-C_3)$alkyl group such as methyl. Examples are, but are not limited to 7-azaspiro[3.5]nonanyl and 2-oxa-6-azaspiro [3.3] heptan- 6-yl.
- "$(C_1-C_x)$alkoxy", as used herein, refers to a -O-$(C_1-C_x)$alkyl or -O-$(C_3-C_x)$cycloalkyl moiety, wherein alkyl and cycloalkyl are as defined above, for example $(C_1-C_6)$alkoxy. Examples are, but are not limited to, methoxy, ethoxy, 1-propoxy, 2-propoxy, cyclopropoxy, butoxy, tert-butoxy and pentoxy.
- a "$(C_1-C_x)$fluoroalkyl group", as used herein, refers to a $(C_1-C_x)$alkyl as defined herein above in which one or more hydrogens have been substituted by fluorine atoms. In one embodiment all the hydrogen atoms are replaced by fluorine atoms, forming perfluoroalkyl groups, such as trifluoromethyl.
- a "hydroxy$(C_1-C_x)$alkyl group" , as used herein, refers to a $(C_1-C_x)$alkyl as defined herein above in which one or

more hydrogens have been substituted by hydroxy group(s). Examples are, but are not limited to 2-hydroxyethyl.

- a ($C_5$-$C_6$)heteroaryl group, as used herein, refers to a monocyclic aromatic group wherein one to three carbon atom is replaced by a heteroatom, such as nitrogen, oxygen or sulphur. By way of examples of heteroaryl groups, mention may be made of, but not limited to: oxazole, isoxazole, pyridine, pyrimidine, pyridazine, triazine, pyrazine, oxadiazole, furane, pyrazole, thiazole, isothiazole, thiadiazole, imidazole, triazole and the like. In the framework of the present invention, the heteroaryl is advantageously pyridine, imidazole, pyrazine, furane, thiazole, pyrazole, thiadiazole, pyridazine and pyrimidine, and in particular pyrazole, imidazole, pyridine, pyrimidine and pyrazine, and more particularly pyrazole, imidazole, pyridine and pyrazine.
- an aromatic ring means, according to Hückel's rule, that a molecule has $4n + 2$ $\pi$-electrons.

In the context of the present invention, the terms "aromatic ring", and "heteroaryl" include all the positional isomers.

The nomenclature of the following compounds (1) to (133) was generated according to the principles of the International Union of Pure and Applied Chemistry, using Accelrys Draw 4.1 SP1. To avoid any confusion, the "(±)" symbol added to designate a racemic mixture; *"cis"* and *"trans"* prefixes were also used to assign the relative stereochemistry of two adjacent chiral centers.

Specific compounds of formula (I) of the present invention are listed herein after:

**Nom IUPAC** *N4*-Benzyl-7-isopropyl-*N2*-(2-methoxyethyl)imidazo[2,1-

(1) *f*][1,2,4]triazine-2,4-diamine *N4*-Benzyl-7-isopropyl-*N2*-(2-phenoxyethyl)imidazo[2,1-
(2) *f*][1,2,4]triazine-2,4-diamine *N4*-Benzyl-7-isopropyl-*N2*-(2-methylsulfanylethyl)imidazo[2,1-
(3) *f*][1,2,4]triazine-2,4-diamine *N4*-Benzyl-7-isopropyl-*N2*-(3-methoxypropyl)imidazo[2,1-
(4) *f*][1,2,4]triazine-2,4-diamine *N4*-Benzyl-*N2*-[3-(dimethylamino)propyl]-7-isopropyl-imidazo[2,1-
(5) *f*][1,2,4]triazine-2,4-diamine (*2R*)-2-[[4-(Benzylamino)-7-isopropyl-imidazo[2,1-*f*][1,2,4]triazin-2-
(6) yl]amino]butan-1-ol *N4*-Benzyl-*N2*-(1-ethylpropyl)-7-isopropyl-imidazo[2,1-*f*[1,2,4]triazine-
(7) 2,4-diamine *N2*-(1-ethylpropyl)-7-isopropyl-*N4*-(2-pyridylmethyl)imidazo[2,1-
(8) *f*][1,2,4]triazine-2,4-diamine *N2*-(1-ethylpropyl)-7-isopropyl-*N4*-(3-pyridylmethyl)imidazo[2,1-
(9) *f*][1,2,4]triazine-2,4-diamine *N2*-(1-ethylpropyl)-7-isopropyl-*N4*-(4-pyridylmethyl)imidazo[2,1-
(10) *f*][1,2,4]triazine-2,4-diamine *N2*-(1-ethylpropyl)-7-isopropyl-*N4*-phenyl-imidazo[2,1-*f*[1,2,4]triazine-
(11) 2,4-diamine *N2*-(1-ethylpropyl)-7-isopropyl-*N4*-(2-phenylethyl)imidazo[2,1-
(12) *f*][1,2,4]triazine-2,4-diamine *N2*-(1-ethylpropyl)-7-isopropyl-*N4*-[2-(2-pyridyl)ethyl]imidazo[2,1-
(13) *f*][1,2,4]triazine-2,4-diamine *N2*-(1-ethylpropyl)-7-isopropyl-*N4*-[2-(3-pyridyl)ethyl]imidazo[2,1-
(14) *f*][1,2,4]triazine-2,4-diamine *N2*-(1-ethylpropyl)-7-isopropyl-*N4*-[2-(4-pyridyl)ethyl]imidazo[2,1-
(15) *f*][1,2,4]triazine-2,4-diamine 7-cyclopropyl-*N2*-(1-ethylpropyl)-*N4*-[2-(3-pyridyl)ethyl]imidazo[2,1-
(16) *f*][1,2,4]triazine-2,4-diamine 7-cyclopropyl-*N2*-(1-ethylpropyl)-*N4*-[2-(4-pyridyl)ethyl]imidazo[2,1-
(17) *f*][1,2,4]triazine-2,4-diamine 2-[[4-(Benzylamino)-7-isopropyl-imidazo[2,1-*f*][1,2,4]triazin-2-
(18) yl]amino]-2-methyl-propan-1-ol *N4*-Benzyl-7-isopropyl-*N2*-[(*3R*)-tetrahydrofuran-3-yl]imidazo[2,1-
(19) *f*][1,2,4]triazine-2,4-diamine *N4*-[(4-Chlorophenyl)methyl]-7-isopropyl-*N2*-[(*3R*)-tetrahydrofuran-3-
(20) yl]imidazo[2,1-*f*][1,2,4]triazine-2,4-diamine 7-Isopropyl-*N4*-(p-tolylmethyl)-*N2*-[(*3R*)-tetrahydrofuran-3-
(21) yl]imidazo[2,1-*f*[1,2,4]triazine-2,4-diamine 7-Isopropyl-*N4*-[(4-methoxyphenyl)methyl]-*N2*-[(*3R*)-tetrahydrofuran-3-
(22) yl]imidazo[2,1-*f*][1,2,4]triazine-2,4-diamine *N4*-[(3,4-Dichlorophenyl)methyl]-7-isopropyl-*N2*-[(*3R*)-tetrahydrofuran-
(23) 3-yl]imidazo[2,1-*f*[1,2,4]triazine-2,4-diamine *N4*-[(4-Fluorophenyl)methyl]-7-isopropyl-*N2*-[(*3R*)-tetrahydrofuran-3-
(24) yl]imidazo[2,1-*f*][1,2,4]triazine-2,4-diamine 7-Isopropyl-*N2*-[(*3R*)-tetrahydrofuran-3-yl]-*N4*-[[4-
(25) (trifluoromethyl)phenyl] methyl]imidazo[2,1-*f*[1,2,4]triazine-2,4-diamine 7-Isopropyl-*N4*-(2-pyridylmethyl)-*N2*-[(*3R*)-tetrahydrofuran-3-
(26) yl]imidazo[2,1-*f*][1,2,4]triazine-2,4-diamine 7-Isopropyl-*N4*-(3-pyridylmethyl)-*N2*-[(*3R*)-tetrahydrofuran-3-
(27) yl]imidazo[2,1-*f*][1,2,4]triazine-2,4-diamine 7-Isopropyl-*N4*-(4-pyridylmethyl)-*N2*-[(*3R*)-tetrahydrofuran-3-
(28) yl]imidazo[2,1-*f*][1,2,4]triazine-2,4-diamine 7-Isopropyl-*N4*-(pyrazin-2-ylmethyl)-*N2*-[(*3R*)-tetrahydrofuran-3-
(29) yl]imidazo[2,1-*f*][1,2,4]triazine-2,4-diamine 7-Isopropyl-*N4*-[(*1R*)-1-phenylethyl]-*N2*-[(*3R*)-tetrahydrofuran-3-
(30) yl]imidazo[2,1-*f*][1,2,4]triazine-2,4-diamine 7-Isopropyl-*N4*-[(*1S*)-1-phenylethyl]-*N2*-[(*3R*)-tetrahydrofuran-3-
(31) yl]imidazo[2,1-*f*][1,2,4]triazine-2,4-diamine *N4*-Benzyl-7-isopropyl-*N4*-methyl-*N2*-[(*3R*)-tetrahydrofuran-3-
(32) yl]imidazo[2,1-*f*][1,2,4]triazine-2,4-diamine *N4*-Benzyl-*N2*-[(*3R*)-tetrahydrofuran-3-yl]imidazo[2,1-*f*][1,2,4]triazine-
(33) 2,4-diamine *N4*-Benzyl-7-bromo-*N2*-[(*3R*)-tetrahydrofuran-3-yl]imidazo[2,1-
(34) *f*][1,2,4]triazine-2,4-diamine *N4*-Benzyl-7-methyl-*N2*-[(*3R*)-tetrahydrofuran-3-yl]imidazo[2,1-

(35) *f*][1,2,4]triazine-2,4-diamine *N4*-Benzyl-7-ethyl-*N2*-[(*3R*)-tetrahydrofuran-3-yl]imidazo[2,1-
(36) *f*][1,2,4]triazine-2,4-diamine *N4*-Benzyl-7-cyclopropyl-*N2*-[(*3R*)-tetrahydrofuran-3-yl]imidazo[2,1-
(37) *f*][1,2,4]triazine-2,4-diamine N4-B enzyl-7-cyclopentyl-*N2*-[(*3R*)-tetrahydrofuran-3-yl]imidazo[2,1-
(38) *f*][1,2,4]triazine-2,4-diamine *N4*-Benzyl-7-cyclohexyl-*N2*-[(*3R*)-tetrahydrofuran-3-yl]imidazo[2,1-
(39) *f*][1,2,4]triazine-2,4-diamine *N4*-Benzyl-7-phenyl-*N2*-[(*3R*)-tetrahydrofuran-3-yl]imidazo[2,1-
(40) *f*][1,2,4]triazine-2,4-diamine N4-Benzyl-7-isopropyl-*N2*-[(*3R*)-tetrahydropyran-3-yl]imidazo[2,1-
(41) *f*][1,2,4]triazine-2,4-diamine 7-Isopropyl-*N4*-(3-pyridylmethyl)-*N2*-[(*3R*)-tetrahydropyran-3-
(42) yl]imidazo[2,1-*f*][1,2,4]triazine-2,4-diamine 7-Isopropyl-*N4*-(4-pyridylmethyl)-*N2*-[(*3R*)-tetrahydropyran-3-
(43) yl]imidazo[2,1-*f*][1,2,4]triazine-2,4-diamine *N4*-Benzyl-7-isorpopyl-*N2*-tetrahydropyran-4-yl-imidazo[2,1-
(44) *f*)[1,2,4]diamine *N4*-[(4-Fluorophenyl)methyl]-7-isopropyl-*N2*-tetrahydropyran-4-yl-
(45) imidazo[2,1-*f*][1,2,4]triazine-2,4-diamine 7-Isopropyl-*N2*-tetrahydropyran-4-yl-*N4*-[[4-
(46) (trifluoromethyl)phenyl] methyl]imidazo[2,1-*f*][1,2,4]triazine-2,4-diamine 7-Isopropyl-*N4*-[(*1R*)-1-phenylethyl]-*N2*-tetrahydropyran-4-yl-
(47) imidazo[2,1-*f*][1,2,4]triazine-2,4-diamine 7-Isopropyl-*N4*-[(*1S*)-1-phenylethyll-*N2*-tetrahydropyran-4-yl-
(48) imidazo[2,1-*f*][1,2,4]triazine-2,4-diamine *N4*-Benzyl-7-isopropyl-*N4*-methyl-*N2*-tetrahydropyran-4-yl-imidazo[2,1-
(49) *f*][1,2,4]triazine-2,4-diamine 7-Isopropyl-*N4*-(2-pyridylmethyl)-*N2*-tetrahydropyran-4-yl-imidazo[2,1-
(50) *f*][1,2,4]triazine-2,4-diamine 7-Isopropyl-*N4*-(3-pyridylmethyl)-*N2*-tetrahydropyran-4-yl-imidazo[2,1-
(51) *f*][1,2,4]triazine-2,4-diamine 7-Isopropyl-*N4*-(4-pyridylmethyl)-*N2*-tetrahydropyran-4-yl-imidazo[2,1-
(52) *f*][1,2,4]triazine-2,4-diamine 7-Isopropyl-*N4*-(pyrazin-2-ylmethyl)-*N2*-tetrahydropyran-4-yl-
(53) imidazo[2,*1-f*][1,2,4]triazine-2,4-diamine *N4*-Benzyl-7-methyl-*N2*-tetrahydropyran-4-yl-imidazo[2,1-
(54) *f*][1,2,4]triazine-2,4-diamine *N4*-Benzyl-7-ethyl-*N2*-tetrahydropyran-4-yl-imidazo[2,1-
(55) *f*][1,2,4]triazine-2,4-diamine *N4*-Benzyl-7-cyclopropyl-*N2*-tetrahydropyran-4-yl-imidazo[2,1-
(56) *f*][1,2,4]triazine-2,4-diamine *N4*-Benzyl-7-cyclohexyl-*N2*-tetrahydropyran-4-yl-imidazo[2,1-
(57) *f*][1,2,4]triazine-2,4-diamine *N4*-Benzyl-7-isopropyl-*N2*-(oxepan-4-yl)imidazo[2,1-*f*][1,2,4]triazine-
(58) 2,4-diamine *N4*-Benzyl-*N2*-cyclohexyl-7-isopropyl-imidazo[2,1-*f*][1,2,4]triazine-2,4-
(59) diamine *N4*-Benzyl-*N2*-(4,4-difluorocyclohexyl)-7-isopropyl-imidazo[2,1-
(60) *f*][1,2,4]triazine-2,4-diamine *N4*-Benzyl-7-isopropyl-*N2*-(1-methyl-4-piperidyl)imidazo[2,1-
(61) *f*][1,2,4]triazine-2,4-diamine (*1S*)-2-[[4-(Benzylamino)-7-isopropyl-imidazo[2,1-*f*][1,2,4]triazin-2-
(62) yl]amino]cyclohexanol 4-[[4-(Benzylamino)-7-isopropyl-imidazo[2,1-*f*][1,2,4]triazin-2-
(63) yl]amino]cyclohexanol *N4*-Benzyl-7-isopropyl-*N2*-(4-methoxycyclohexyl)imidazo[2,1-
(64) *f*][1,2,4]triazine-2,4-diamine (*1S*)-2-[[4-(Benzylamino)-7-isopropyl-imidazo[2,1-*f*][1,2,4]triazin-2-
(65) yl]amino]cyclopentanol (*1S*)-2-[[7-Isopropyl-4-(3-pyridylmethylamino)imidazo[2,1-
(66) *f*][1,2,4]triazin-2-yl]amino]cyclopentanol *N4*-Benzyl-7-isopropyl-*N2*-[(*2S*)-2-methoxycyclopentyl]imidazo[2,1-
(67) *f*][1,2,4]triazine-2,4-diamine 7-Isopropyl-*N2*-[(*2S*)-2-methoxycyclopentyl]-*N4*-(3-
(68) pyridylmethyl)imidazo[2,1-*f*][1,2,4]triazine-2,4-diamine *N4*-Benzyl-7-isopropyl-*N2*-methyl-*N2*-[(*3R*)-tetrahydrofuran-3-
(69) yl]imidazo[2,1-*f*][1,2,4]triazine-2,4-diamine *N4*-Benzyl-7-isopropyl-*N2*-methyl-*N2*-tetrahydropyran-4-yl-imidazo[2,1-
(70) *f*][1,2,4]triazine-2,4-diamine 3-Isopropyl-N6-methyl-N8-(4-pyridylmethyl)-N6-tetrahydropyran-4-yl-
(71) [1,2,4]triazolo[4,3-*b*]pyridazine-6,8-diamine *N*-Benzyl-7-isopropyl-2-oxazolidin-3-yl-imidazo[2,1-*f*][1,2,4]triazin-4-
(72) amine
(73) *N*-Benzyl-7-isopropyl-2-morpholino-imidazo[2,1-*f*][1,2,4]triazin-4-amine 7-Isopropyl-2-morpholino-*N*-(3-pyridylmethyl)imidazo[2,1-
(74) *f*][1,2,4]triazin-4-amine 7-Isopropyl-2-morpholino-*N*-(4-pyridylmethyl)imidazo[2,1-
(75) *f*][1,2,4]triazin-4-amine 1-[4-(Benzylamino)-7-isopropyl-imidazo[2,1-*f*][1,2,4]triazin-2-
(76) yl]pyrrolidin-3-ol 1-[7-Isopropyl-4-(3-pyridylmethylamino)imidazo[2,1-*f*][1,2,4]triazin-2-
(77) yl]pyrrolidin-3-ol *N*-Benzyl-7-isopropyl-2-(2-oxa-6-azaspiro[3.3]heptan-6-yl)imidazo[2,1-
(78) *f*][1,2,4]triazin-4-amine *N*-Benzyl-7-isopropyl-2-(3-methoxypyrrolidin-1-yl)imidazo[2,1-
(79) *f*][1,2,4]triazin-4-amine 7-Isopropyl-2-(3-methoxypyrrolidin-1-yl)-*N*-(3-
(80) pyridylmethyl)imidazo[2,1*f*][1,2,4]triazin-4-amine *N*-Benzyl-7-isopropyl-2-(4-methoxy-1-piperidyl)imidazo[2,1-
(81) *f*][1,2,4]triazin-4-amine 7-Isopropyl-2-(4-methoxy-1-piperidyl)-N-(3-pyridylmethyl)imidazo[2,1-
(82) *f*][1,2,4]triazin-4-amine *N*-Benzyl-7-isopropyl-2-thiomorpholino-imidazo[2,1-*f*][1,2,4]triazin-4-
(83) amine 7-Isopropyl-*N*-(3-pyridylmethyl)-2-thiomorpholino-imidazo[2,1-
(84) *f*][1,2,4]triazin-4-amine 1-[4-(Benzylamino)-7-isopropyl-imidazo[2,1-*f*][1,2,4]triazin-2-
(85) yl]piperidin-4-ol 1-[7-Isopropyl-4-(3-pyridylmethylamino)imidazo[2,1-*f*][1,2,4]triazin-2-
(86) yl]piperidin-4-ol 2-[1-[4-(Benzylamino)-7-isopropyl-imidazo[2,1-*f*][1,2,4]triazin-2-yl]-2-

(87) piperidyl]ethanol *N4*-Benzyl-7-isopropyl-*N2*-(tetrahydrofuran-3-ylmethyl)imidazo[2,1-
(88) *f*][1,2,4]triazine-2,4-diamine *N4*-Benzyl-7-isopropyl-*N2*-(tetrahydrofuran-2-ylmethyl)imidazo[2,1-
(89) *f*][1,2,4]triazine-2,4-diamine *N4*-Benzyl-7-isopropyl-*N2*-(tetrahydropyran-4-ylmethyl)imidazo[2,1-
(90) *f*][1,2,4]triazine-2,4-amine *N4*-Benzyl-7-isopropyl-*N2*-(tetrahydropyran-3-ylmethyl)imidazo[2,1-
(91) *f*][1,2,4]triazine-2,4-amine 7-Isopropyl-*N4*-(2-pyridylmethyl)-*N2*-(tetrahydropyran-3-
(92) ylmethyl)imidazo[2,1-*f*][1,2,4]triazine-2,4-diamine 7-Isopropyl-*N4*-(3-pyridylmethyl)-*N2*-(tetrahydropyran-3-
(93) ylmethyl)imidazo[2,1-*f*][1,2,4]triazine-2,4-diamine 7-Isopropyl-*N4*-(4-pyridylmethyl)-*N2*-(tetrahydropyran-3-
(94) ylmethyl)imidazo[2,1-*f*][1,2,4]triazine-2,4-diamine *N4*-Benzyl-7-isopropyl-*N2*-[[(*3S*)-tetrahydropyran-3-
(95) yl]methyl]imidazo[2,1-*f*][1,2,4]triazine-2,4-diamine 7-Isopropyl-*N4*-(3-pyridylmethyl)-*N2*-[[(*3S*)-tetrahydropyran-3-
(96) yl]methyl]imidazo[2,1-*f*][1,2,4]triazine-2,4-diamine *N4*-Benzyl-7-isopropyl-*N2*-[[(*3R*)-tetrahydropyran-3-
(97) yl]methyl]imidazo[2,1-*f*][1,2,4]triazine-2,4-diamine 7-Isopropyl-*N4*-(3-pyridylmethyl)-*N2*-[[(*3R*)-tetrahydropyran-3-
(98) yl]methyl]imidazo[2,1-*f*][1,2,4]triazine-2,4-diamine *N4*-Benzyl-7-isopropyl-*N2*-(tetrahydropyran-2-ylmethyl)imidazo[2,1-
(99) *f*][1,2,4]triazine-2,4-amine 7-Isopropyl-*N4*-(3-pyridylmethyl)-*N2*-(tetrahydropyran-2-
(100) ylmethyl)imidazo[2,1-*f*][1,2,4]triazine-2,4-diamine 7-Isopropyl-*N4*-(4-pyridylmethyl)-*N2*-(tetrahydropyran-2-
(101) ylmethyl)imidazo[2,1-*f*][1,2,4]triazine-2,4-diamine *N4*-Benzyl-7-isopropyl-*N2*-(1-tetrahydropyran-3-ylethyl)imidazo[2,1-
(102) *f*][1,2,4]triazine-2,4-diamine *N4*-Benzyl-7-isopropyl-*N2*-methyl-*N2*-(tetrahydropyran-3-
(103) ylmethyl)imidazo[2,1-*f*][1,2,4]triazine-2,4-diamine *N4*-Benzyl-7-isopropyl-*N2*-[(1-methyl-4-piperidyl)methyllimidazo[2,1-
(104) *f*][1,2,4]triazine-2,4-diamine *N4*-Benzyl-*N2*-(cyclohexylmethyl)-7-isopropyl-imidazo[2,1-
(105) *f*][1,2,4]triazine-2,4-diamine *N4*-Benzyl-7-isopropyl-*N2*-[2-(1-methylpyrrolidin-2-
(106) yl)ethyl]imidazo[2,1-*f*][1,2,4]triazine-2,4-diamine *N4*-Benzyl-7-isopropyl-*N2*-(2-methylpyrazol-3-yl)imidazo[2,1-
(107) *f*][1,2,4]triazine-2,4-diamine *N4*-Benzyl-7-isopropyl-*N4*-methyl-*N2*-(2-methylpyrazol-3-
(108) yl)imidazo[2,1-*f*][1,2,4]triazine-2,4-diamine *N4*-Benzyl-7-isopropyl-*N2*-(1-methylimidazol-2-yl)imidazo[2,1-
(109) *f*][1,2,4]triazine-2,4-diamine *N4*-Benzyl-7-isopropyl-*N2*-(1-methylpyrazol-4-yl)imidazo[2,1-
(110) *f*][1,2,4]triazine-2,4-diamine *N4*-Benzyl-7-isopropyl-*N2*-(1-methylpyrazol-3-yl)imidazo[2,1-
(111) *f*][1,2,4]triazine-2,4-diamine *N4*-Benzyl-*N2*-(1,3-dimethylpyrazol-4-yl)-7-isopropyl-imidazo[2,1-
(112) *f*][1,2,4]triazine-2,4-diamine *N4*-Benzyl-*N2*-(2,5-dimethylpyrazol-3-yl)-7-isopropyl-imidazo[2,1-
(113) *f*][1,2,4]triazine-2,4-diamine *N4*-Benzyl-7-isopropyl-*N2*-phenyl-imidazo[2,1-*f*][1,2,4]triazine-2,4-
(114) diamine *N4*-Benzyl-7-isopropyl-*N2*-[(1-methylpyrazol-4-yl)methyl]imidazo[2,1-
(115) *f*][1,2,4]triazine-2,4-diamine *N4*-Benzyl-7-isopropyl-*N2*-[(1-methylimidazol-2-yl)methyl]imidazo[2,1-
(116) *f*][1,2,4]triazine-2,4-diamine *N4*-Benzyl-7-isopropyl-*N2*-[(2-methylpyrazol-3-yl)methyl]imidazo[2,1-
(117) *f*][1,2,4]triazine-2,4-diamine *N4*-Benzyl-7-isopropyl-*N2*-[(1-methylpyrazol-3-yl)methyl]imidazo[2,1-
(118) *f*][1,2,4]triazine-2,4-diamine *N4*-Benzyl-7-isopropyl-*N2*- [(3-methylimidazol-4-yl)methyl]imidazo[2,1-
(119) *f*][1,2,4]triazine-2,4-diamine *N4*-Benzyl-7-isopropyl-*N2*-[(1-methylimidazol-4-yl)methyl]imidazo[2,1-
(120) *f*][1,2,4]triazine-2,4-diamine
(121) *N2*,*N4*-Dibenzyl-7-isopropyl-imidazo[2,1-*f*][1,2,4]triazine-2,4-diamine *N4*-Benzyl-*N2*-[(4-Fluorophenyl)methyl]-7-isopropyl-imidazo[2,1-
(122) *f*][1,2,4]triazine-2,4-diamine *N4*-Benzyl-7-isopropyl-*N2*-(2-pyridylmethyl)imidazo[2,1-
(123) *f*][1,2,4]triazine-2,4-diamine *N4*-Benzyl-7-isopropyl-*N2*-(3-pyridylmethyl)imidazo[2,1-
(124) *f*][1,2,4 ]triazine-2,4-diamine *N4*-Benzyl-7-isopropyl-*N2*-(4-pyridylmethyl)imidazo[2,1-
(125) *f*][1,2,4 ]triazine-2,4-diamine *N4*-Benzyl-7-isopropyl-*N2*-[(5-methylpyrazin-2-yl)methyllimidazo[2,1-
(126) *f*][1,2,4]triazine-2,4-diamine *N4*-Benzyl-*N2*-[(4,6-dimethylpyrimidin-2-yl)methyl]-7-isopropyl-
(127) imidazo[2,1-*f*][1,2,4]triazine-2,4-diamine *N*-[4-(benzylamino)-7-isopropyl-imidazo[2,1-*f*][1,2,4]triazin-2-yl]-2-
(128) methyl-pyrazole-3 - sulfonamide *N*-[4-(benzylamino)-7-isopropyl-imidazo[2,1-*f*][1,2,4]triazin-2-yl]-1,3-
(129) dimethyl-pyrazole-4-sulfonamide *N*-[4-(benzylamino)-7-isopropyl-imidazo[2,1-*f*][1,2,4 ]triazin-2-yl]-2,5-
(130) dimethyl-pyrazole-3-sulfonamide *N*-[4-(Benzylamino)-7-isopropyl-imidazo[2,1-*f*][1,2,4]triazin-2-yl]-2,4-
(131) dimethyl-pyrazole-3-sulfonamide *N4*-benzyl-7-isopropyl-*N2*-[(*3S*)-tetrahydrofuran-3-yl]imidazo[2,1-
(132) *f*][1,2,4]triazine-2,4-diamine *N4*-benzyl-*N2*-(2,4-dimethylpyrazol-3-yl)-7-isopropyl-imidazo[2,1-
(133) *f*][1,2,4]triazine-2,4-diamine

and their pharmaceutically acceptable salts.

Compound (6) is the N&N-2 compound as previously described in the literature as explained in the preamble.

Thus, herein is provided anyone of the compounds (1) to (5) and (7) to (133) as defined above, or any of their

pharmaceutically acceptable salts.

According to an even more preferred embodiment of the present invention, the compound of formula (I) is chosen from the group consisting of compounds (19), (20), (22), (23), (25), (27), (28), (37), (39), (41), (42), (43), (44), (45), (46), (48), (49), (51), (52), (56), (57), (58), (63), (64), (74), (91), (93), (94), (95), (96), (97), (98), (100), (101), (102), (103), (107), (110), (112), (113), (115), (117), (119), (120), (121), (122), (123), (124), (125), (133) and their pharmaceutically acceptable salts.

According to an even more preferred embodiment of the present invention, the compound of formula (I) is chosen from the group consisting of compounds (25), (28), (42), (46), (48), (51), (52), (57), (58), (63), (93), (94), (96), (97), (102), (107), (113), (117), (133) and their pharmaceutically acceptable salts.

According to an even more preferred embodiment of the present invention, the compound of formula (I) is chosen from the group consisting of compounds (25), (93), (96), (107), (113), (133) and their pharmaceutically acceptable salts.

According to another aspect, a subject-matter of the present invention relates to a compound of formula (I) as defined above, wherein when **Ar** is a phenyl group and **R3** is an isopropyl group, then **R1** is different from a $(C_1\text{-}C_6)$alkyl group substituted by a hydroxy group, or any of its pharmaceutically acceptable salts, or at least any of compounds (1) to (133) or any of its pharmaceutically acceptable salts, for use as a medicament.

"Pharmaceutically acceptable salt thereof" refers to salts which are formed from acid addition salts formed with inorganic acids (e.g. hydrochloric acid, hydrobromic acid,), as well as salts formed with organic acids such as acetic acid, tartaric acid, succinic acid.

Suitable physiologically acceptable acid addition salts of compounds of formula (I) include hydrobromide, tartrate, hydrochloride, mesylate, succinate and acetate.

The compounds of formula (I), and any of compounds (1) to (5) and (7) to (133) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, *i.e.* combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

**List of abbreviations:**

| Abbreviation/acronym | Name |
|---|---|
| **ACN** | Acetonitrile |
| **AcOH** | Acetic acid |
| **Alk** | Alkyl |
| **ATP** | Adenosine triphosphate |
| **br s** | Broad singlet |
| **C** | Molar concentration |
| **cHex** | Cyclohexane |
| **Cpd N°** | Compound number |
| **d** | Doublet |
| **DCM** | Methylene chloride |
| **dd** | Doublet of doublets |
| **DIPEA** | *N*,*N*-Diisopropylethylamine |
| **DMF** | Dimethylformamide |
| **DMSO** | Dimethylsulfoxide |
| **eq** | Equivalent |

(continued)

| Abbreviation/acronym | Name |
|---|---|
| **ESI** | Electrospray ionization |
| **Et** | Ethyl |
| **GP** | General protocol |
| **Hal** | Halogen |
| **HPLC** | High pressure liquid chromatography |
| **ICso** | Half maximal inhibitory concentration |
| **iPr** | isopropyl |
| **m** | Multiplet |
| **M** | Molarity |
| **Me** | Methyl |
| **MS** | Mass spectroscopy |
| **MW** | Molecular weight |
| **NBS** | N-Bromosuccinimide |
| **NMP** | *N*-Methyl-2-pyrrolidone |
| **NMR** | Nuclear magnetic resonance |
| **N/A** | Not applicable |
| **n.t.** | Not tested |
| **Phe** | phenyl |
| **$POCl_3$** | phosphoryl chloride |
| **PTLC** | Preparative thin layer chromatography |
| **Rac** | Racemic |
| **r.t.** | Room temperature |
| **s** | Singlet |
| **t** | Triplet |
| **TEA** | Triethylamine |
| **TFA** | Trifluoroacetic acid |
| **THF** | Tetrahydrofuran |
| **TLC** | Thin layer chromatography |
| **T °C** | Temperature in degrees Celsius |
| **UV** | Ultraviolet |
| **v/v** | Volume per volume |
| **w/v** | Weight per volume |
| Δ | chemical shift |
| **μw** | Microwave irradiation |

The compounds of general formula (I) can be prepared according to Route 1 depicted in scheme 1 below.

**Scheme 1**

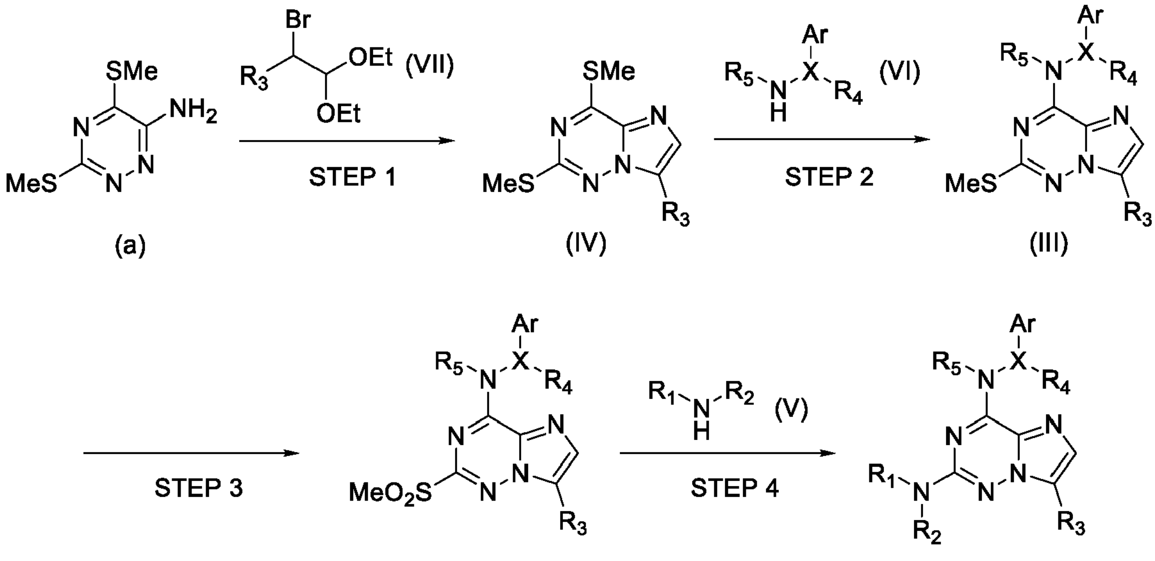

(a) (IV) (III) (II) (I)

According to Route 1, the synthesis of compounds according to the invention is based on a functionalization of a compound of formula (II), wherein $R_3$, $R_4$ and $R_5$ are as defined above by an amine of formula (V) wherein $R_1$ and $R_2$ are as defined above. The compound of formula (II) may be placed in STEP 4 in an aprotic solvent such as Et3N, THF, 2-MeTHF, dioxane, cineol or NMP or a mixture of both or without solvent. The amine of formula (V) may be added, for example in a molar ratio ranging from 1 to 20 eq, in particular of 10 eq, with respect to the compound of formula (II). The reaction mixture may be heated at a temperature between 90 to 180°C. Upon completion of the reaction, the mixture may be brought back to room temperature.

A compound of formula (II) as defined above may be obtained in STEP 3 by reaction of a compound of formula (III) wherein $R_3$, $R_4$ and $R_5$ are as defined above, in a polar aprotic solvent such as a mixture of methanol and water for example oxone®, in particular in a molar ratio ranging from 2 and 6 eq, for example at a molar ratio of 4 eq, with respect to the compound of formula (III). The resulting mixture may then be stirred, for example from 10 to 24 hours, in particular for 16 hours at room temperature.

A compound of formula (III) as defined above may be obtained in STEP 2 by reaction of a compound of formula (IV) wherein $R_3$ is as defined above, with a compound of formula (VI) wherein $R_4$, $R_5$ and Ar are as defined above, in an aprotic solvent such as THF, 2-MeTHF or dioxane, or a mixture thereof. The reaction mixture may be stirred at reflux temperature for a duration ranging from 10 hours to 24 hours, for example for 16 hours.

A compound of formula (IV) as defined above may be obtained in STEP 1 by reacting a compound of formula (a) with a compound of formula (VII) wherein $R_3$ is as defined above, in the presence of acidic reagent in particular CSA, for example in a molar ratio ranging from 0.05 to 1M, in particular of 0.1, with respect to the compound of formula (VII) in a polar aprotic solvent such as acetonitrile.

Accordingly, the present invention further relates to the synthesis process for manufacturing new compounds of formula (I) as defined above, comprising at least a step of substituting a compound of formula (II) with a primary amine.

Thus, herein is further provided a synthesis process for manufacturing a compound of formula (I) as defined above or any of its pharmaceutically acceptable salts, or at least any of compounds (1) to (5) and (7) to (133) as defined above, comprising at least a step of reacting a compound of formula (II)

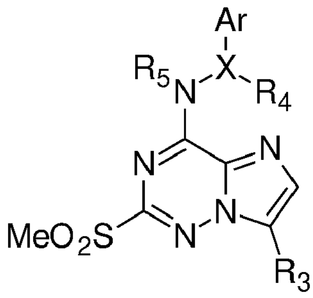
(II)

wherein X, $R_3$, $R_4$ and $R_5$ are as defined above with an amine of formula (V) $R_{1NH}R_2$ (V) wherein $R_1$ and $R_2$ are as defined above, for example in a molar ratio ranging from 1 to 20, in particular of 10, with respect to the compound of formula (II), in an aprotic solvent such as Et3N, THF 2-MeTHF, dioxane, cineol or NMP or a mixture thereof or without solvent.

The compounds of general formula (I) can alternatively be prepared according to Routes 2A, 2B and 2C depicted in schemes 2A, 2B and 2C below.

## Scheme 2A

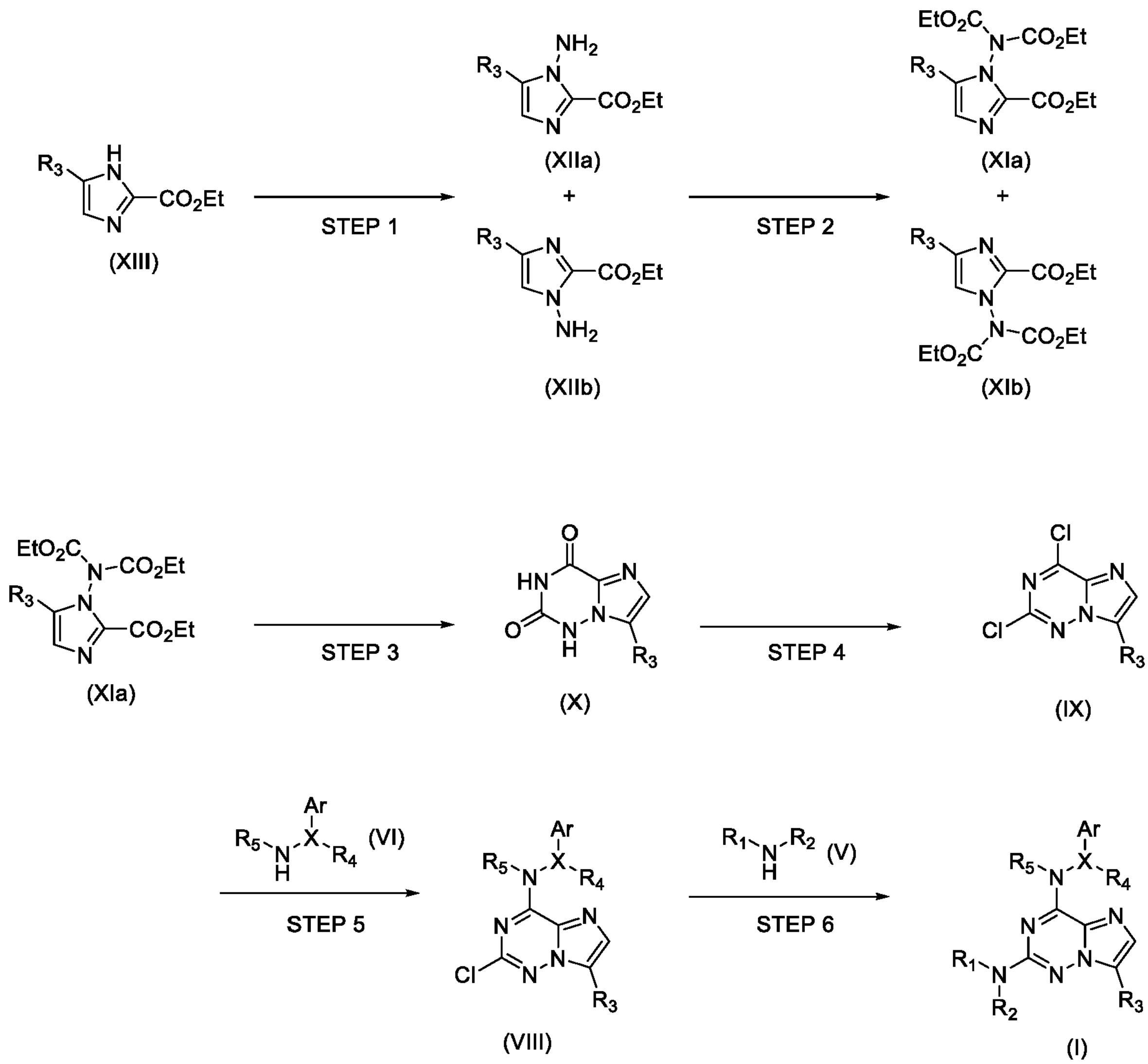


According to Route 2A, the synthesis of compounds according to the invention is based on a functionalization of a compound of formula (VIII), wherein $R_3$, $R_4$, $R_5$ and Ar are as defined above by an amine of formula (V) as defined above. The compound of formula (VIII) may be placed in STEP 6 an aprotic solvent such as Et3N, THF, dioxane, cineol or NMP or a mixture thereof, or without solvent. The amine of formula (V) may be added, for example in a molar ratio ranging from 1 to 20 eq, in particular of 15, with respect to the compound of formula (VIII). The reaction mixture may be heated at a temperature above 120°C, for example at 160°C. Upon completion of the reaction, the mixture may be brought back to room temperature.

A compound of formula (VIII) may be obtained in STEP 5 by reacting a compound of formula (IX) wherein $R_3$ is as defined above with an amine of formula (VI) wherein $R_4$, $R_5$ and Ar are as defined above, for example in a molar ratio ranging from 1 to 5 eq, in particular of 3.5 eq, with respect to the compound of formula (IX) in an aprotic solvent such as THF, 2-MeTHF or dioxane, or a mixture thereof. The reaction mixture may be heated at a temperature between 60 to 110°C, for example at 70°C.

A compound of formula (IX) may be obtained in STEP 4 by reacting a compound of formula (X) wherein $R_3$ is

as defined above, in presence of phosphoryl chloride, for example in a molar ratio ranging from 1 to 15eq, in particular of 11 eq, with respect to the compound of formula (X) and in presence of *N*,*N*-dimethylaniline, for example in a molar ratio ranging from 1 to 5eq, in particular of 2, with respect to the compound of formula (X). The reaction mixture may be heated at a temperature above 100°C, for example at 120°C, in particular during 1 hour.

A compound of formula (X) may be obtained in STEP 3 by reacting a compound of formula (XIa) wherein $R_3$ is as defined above, in presence of ammonium hydroxide, for example in a concentration ranging from 0.05 to 1M, in particular of 0.1 to 0.2M, in a protic solvent, such as isopropyl alcohol. Heating process may be performed in sealed vessel or in microwave reactor. The reaction mixture may be heated at a temperature between 100 to 150°C, for example at 120°C, in particular during 4 hours.

A mixture of compound (XIa) and compound (XIb) wherein $R_3$ is as defined above may be obtained in STEP 2 by reacting a mixture of a compound of formula (XIIa) and a compound of formula (XIIb) wherein $R_3$ is as defined above in presence of ethyl chloroformate, for example in a molar ratio ranging from 1 to 10eq, in particular of 7 eq, with respect to the each of the compounds of formulae (XIa) and (XIb) and in presence of sodium bicarbonate, for example in a molar ratio ranging from 1 to 10eq, in particular of 7, with respect to the each of the compounds of formulae (XIa) and (XIb), in a an aprotic solvent such as THF, water or dioxane, or a mixture of them, such as a mixture of THF and water, for example in a volume ratio 1:1. The resulting mixture may then be stirred, for example from 10 to 140 hours, in particular for 16 to 120 hours at room temperature.

A mixture of a compound of formula (XIIa) and a compound of formula (XIIb) may be obtained in STEP 1 by reacting a compound of formula (XIII) wherein $R_3$ is as defined above with potassium tert-butoxide, for example in a molar ratio ranging from 0.5 to 2M, in particular of 1.05 eq, with respect to the compound of formula (XIII), for example of 1M, in a an aprotic solvent such as THF, DMF or dioxane, or a mixture of them. The resulting mixture may then be stirred, for example during less than 30 minutes at room temperature. The resulting mixture may then be reacted with *O*-(4-nitrobenzoyl)hydroxylamine, for example in a molar ratio ranging from 1 to 2eq, in particular of 1.05, with respect to the compound of formula (XIII). The resulting mixture may then be stirred, for example during more than 2 hours, for example 3 hours at room temperature.

## Scheme 2B

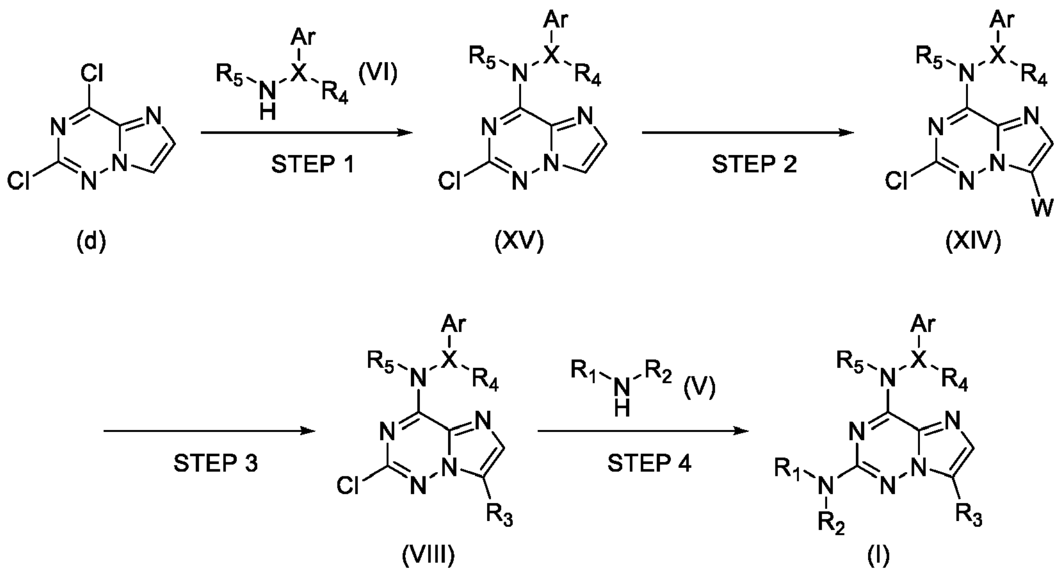

(d) (XV) (XIV)
(VIII) (I)

According to Route 2B, the synthesis of compounds according to the invention is based on a functionalization of a compound of formula (VIII), wherein $R_3$, $R_4$, $R_5$ and Ar are as defined above by an amine of formula (V) as defined above. The compound of formula (VIII) may be placed in STEP 4 in an aprotic solvent such as Et3N, THF, dioxane, cineol or NMP or a mixture thereof. The amine of formula (V) may be added, for example in a molar ratio ranging from 1 to 20 eq., in particular of 15 eq, with respect to the compound of formula (VIII). The reaction mixture may be heated at a temperature above 120°C, for example at 160°C. Upon completion of the reaction, the mixture may be brought back to room temperature.

A compound of formula (VIII) may be obtained in STEP 3 by reacting a compound of formula (XIV), wherein $R_5$ is as defined above, in palladium-catalyzed cross-coupling conditions, optionally followed by a hydrogenation reaction step.

A compound of formula (XIV) may be obtained in STEP 2 by reacting a compound of formula (XV) wherein $R_4$,

$R_5$ and Ar are as defined above in presence of N-Bromosuccinimide or *N*-Iodosuccinimide, in an aprotic solvent such as THF, 2-MeTHF or dioxane, or a mixture thereof. The reaction mixture may be stirred at room temperature for example during 2 hours.

A compound of formula (V) may be obtained in STEP 1 by reacting a compound (d) with an amine of formula (VI) as defined above, for example in a molar ratio ranging from 1 to 5 eq, in particular of 3.5 eq, with respect to the compound of formula (d) in an aprotic solvent such as THF, 2-MeTHF or dioxane, or a mixture thereof.The reaction mixture may be heated at a temperature between 60 to 110°C, for example at 70°C, for example during more than 10 hours, for example 16 hours. Upon completion of the reaction, the mixture may be brought back to room temperature.

Accordingly, the present invention further relates to the synthesis process for manufacturing new compounds of formula (I) as defined above, comprising at least a step of substituting a compound of formula (VIII) with a primary amine.

Thus, herein is further provided a synthesis process for manufacturing a compound of formula (I) as defined above or any of its pharmaceutically acceptable salts, or at least any of compounds (1) to (5) and (7) to (133) as defined above, comprising at least a step of reacting a compound of formula (VIII)

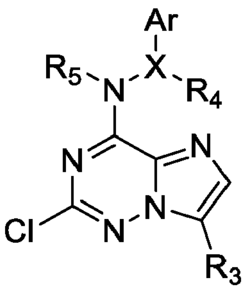
(VIII)

wherein Ar, X, $R_3$, $R_4$, $R_5$ and Ar are as defined in claim 10, with an amine of formula (V) $R_{1_{NH}}R_2$ (V) wherein $R_1$ and $R_2$ are as defined in anyone of claims 8 to 14,

for example in a molar ratio ranging from 1 to 20, in particular of 15, with respect to the compound of formula (VIII), in an aprotic solvent such as Et3N, THF, dioxane, cineol or NMP or a mixture thereof, or without solvent.

The present invention further relates to a compound of formula (XIV), in particular as in intermediate compound

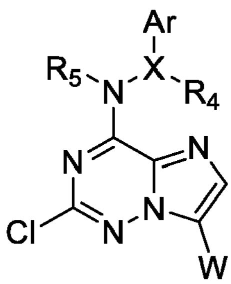
(XIV)

wherein

**Ar, X** and $\mathbf{R_4}$ are as defined above and $\mathbf{R_5}$ represents a $(C_1-C_4)$alkyl group and **W** represents a iodine or bromine atom, or alternatively

**X, W,** $\mathbf{R_4}$ and $\mathbf{R_5}$ are as defined above and **Ar** represents

- a 6-membered heteroaryl group comprising one to three nitrogen atoms, optionally substituted by one to three groups selected from a $(C_1-C_3)$alkyl group, a $(C_1-C_3)$alkoxy group and a halogen atom, and in particular a pyridyl, a pyrimidinyl or a pyrazinyl group, more particularly a pyridyl or a pyrazinyl group, optionally substituted by one to three groups selected from a $(C_1-C_3)$alkyl group, a $(C_1-C_3)$alkoxy group and a halogen atom.

The present invention further relates to a compound of formula (VIII), in particular as in intermediate compound

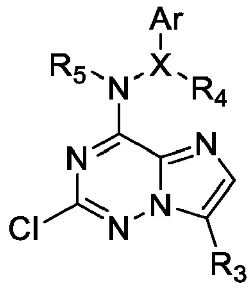

(VIII)

wherein **Ar**, **X, $R_3$ $R_4$** and **$R_5$** are as defined above.

The intermediate products may be obtained commercially or according to methods known to the man skilled in the art.

As far as intermediate compound (a) as mentioned above in scheme 1, it may be obtained according to scheme 3 below.

**Scheme 3**

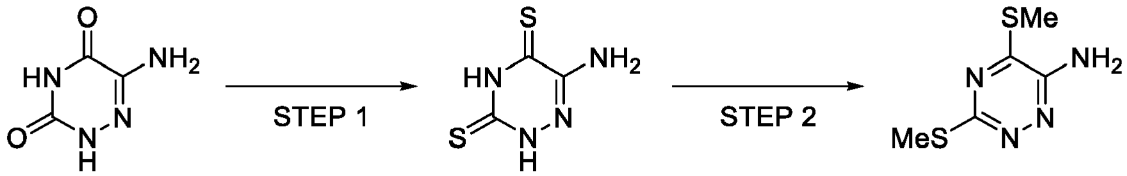


(c) (b) (a)

Compound (a) may be obtained in STEP 2 by reacting compound (b) in presence of iodomethane for example in a molar ratio of 2.2 with respect to compound (b), in presence of *N*,*N*-diisopropylethylamine, for example in a molar ratio of 6 with respect to compound (b), in dichloromethane, at room temperature. The reaction mixture may be stirred at room temperature for example during 16 hours.

Compound (b) may be obtained in STEP 1 by reacting compound (c) with phosphorus(V) sulfide for example in a molar ratio of 4 with respect to compound (c), in presence of pyridine at reflux temperature, for example during 5 hours. To enhance the precipitation, the reaction mixture may be placed in presence of $S_8$ at room temperature and stirred during for example 16 hours.

As far as intermediate compound (d) as mentioned above in scheme 2B, it may be obtained according to scheme 4 below.

**Scheme 4**

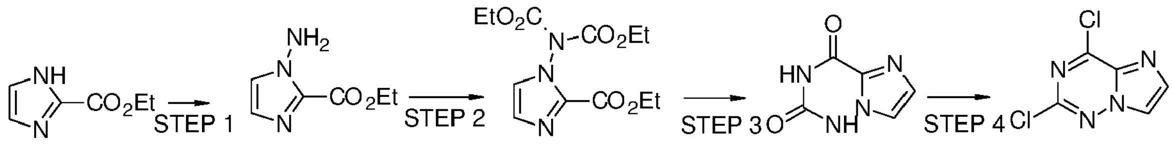


(h) (g) (f) (e) (d)

Compound (d) may be obtained in STEP 4 by reacting compound (e) in presence of phosphoryl chloride, for example in a molar ratio of 11 with respect to compound (e), in presence of *N*,*N*-dimethylaniline, for example in a molar ratio of 2 with respect to compound (e), for example at a temperature of 120°C, in particular during 1 to 3 hours.

Compound (e) may be obtained in STEP 3 by reacting compound (f), in presence of ammonium hydroxide, for example in a concentration ranging from 0.05 to 2 M, in particular of 0.1 to 0.2M, in a protic solvent, such as isopropyl alcohol. Heating process may be performed in sealed vessel or in microwave reactor. The reaction mixture may be heated at a temperature above 100°C, for example at 120°C, in particular during 4 hours.

Compound (f) may be obtained in STEP 2 by reacting compound (g) in presence of ethyl chloroformate, for example in a molar ratio ranging from 2 to 5 eq, in particular of 3.6 eq, with respect to compound (g) and in presence of

sodium bicarbonate, for example in a molar ratio ranging from 2 to 10eq., in particular of 7, with respect to compound (g), in a an aprotic solvent such as THF, water or dioxane, or a mixture of them, such as a mixture of THF and water, for example in a volume ratio 1:1. The resulting mixture may then be stirred, for example from 10 to 140 hours, in particular for 16 to 120 hours at room temperature.

Compound (g) may be obtained in STEP 1 by reacting compound (h) with potassium tert-butoxide, for example in a molar ratio ranging from 0.5 to 2M, in particular of 1.05M, with respect to compound (h), for example of 1M, in a an aprotic solvent such as THF, DMF or dioxane, or a mixture of them. The resulting mixture may then be stirred, for example during less than 30 minutes at room temperature. The resulting mixture may then be reacted with *O*-(4-nitrobenzoyl)hydroxylamine, for example in a molar ratio ranging from 1 to 2 eq, in particular of 1.05 eq, with respect to compound (h). The resulting mixture may then be stirred, for example during more than 2 hours, for example 3 hours at room temperature.

**Scheme 2C**

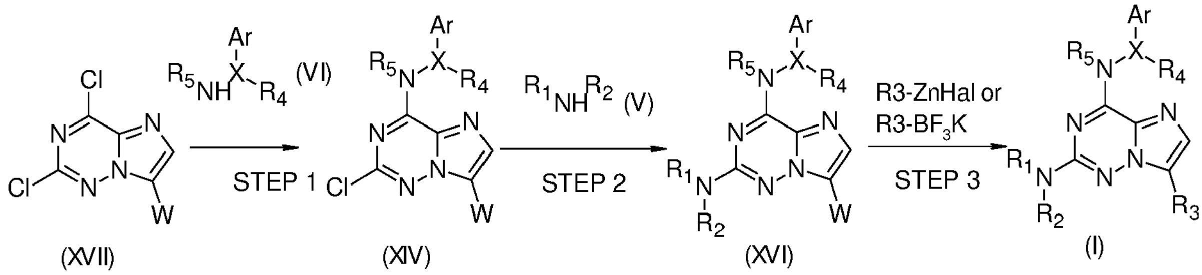


(XVII) (XIV) (XVI) (I)

According to Route 2C, the synthesis of compounds according to the invention, wherein $R_1$, $R_2$, $R_4$, $R_5$, X and Ar are as defined above may be obtained in STEP 3 by reacting a compound of formula (XVI), wherein $R_1$, $R_2$, $R_4$, $R_5$, X, W and Ar are as defined above, in palladium-catalyzed cross-coupling conditions with a compound of formula R3-ZnHal compound, wherein $R_3$ is as defined above and Hal is a chlorine or bromine atom, or with a compound of formula $R_3$-$BF_3K$ followed by a hydrogenation reaction step.

A compound of formula (XVI) is based on a functionalization of a compound of formula (XIV), wherein $R_4$, $R_5$, X, W and Ar are as defined above by an amine of formula (V) as defined above. The compound of formula (XIV) may be placed in STEP 2 in an aprotic solvent such as Et3N, THF, dioxane, cineol or NMP or a mixture thereof, or without solvent. The amine of formula (V) may be added, for example in a molar ratio ranging from 1 to 20 eq, in particular of 15, with respect to the compound of formula (VIII). The reaction mixture may be heated at a temperature above 120°C, for example at 160°C. Upon completion of the reaction, the mixture may be brought back to room temperature.

A compound of formula (XIV) may be obtained in STEP 1 by reacting a compound (XVII), wherein W is as defined above, with an amine of formula (VI) as defined above, for example in a molar ratio ranging from 1 to 5 eq, in particular of 1.5 eq, with respect to the compound of formula (XVII) in an aprotic solvent such as THF, 2-MeTHF or dioxane, or a mixture thereof. The reaction mixture may be heated at a temperature between 50 to 110°C, for example at 70°C, during 1 to 24 hours, for example 1.5 hours. Upon completion of the reaction, the mixture may be brought back to room temperature.

Accordingly, the present invention further relates to the synthesis process for manufacturing new compounds of formula (I) as defined above, comprising at least a step of functionalizing a compound of formula (XVI) with a palladium-catalyzed cross-coupling followed by a hydrogenation reaction step.

Thus, herein is further provided a synthesis process for manufacturing a compound of formula (I) as defined above 15 or any of its pharmaceutically acceptable salts, or at least any of compounds (1) to (5) and (7) to (133) as defined in claim 16, comprising at least a step of reacting a compound of formula (XVI)

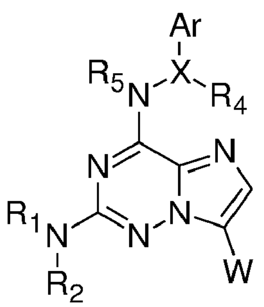

(XVI), wherein **$R_1$**, **$R_2$**, **$R_4$**, **$R_5$**, **X**, **W** and **Ar** are as defined above, in palladium-catalyzed cross-coupling conditions with a compound of formula $R_3$ZnHal compound, wherein $R_3$ is as defined above and Hal is a chlorine or bromine atom, or with a compound of formula R3BF3K followed by a hydrogenation reaction step.

The present invention further relates to a compound of formula (XVI), in particular as in intermediate compound

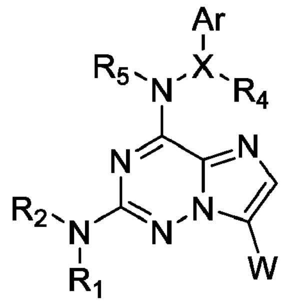

(XVI)

wherein **$R_1$**, **$R_2$**, **Ar**, **X**, **$R_5$** and **$R_4$** are as defined above, and **W** represents an iodine or bromine atom.

The present invention further relates to a compound of formula (XVI), in particular as in intermediate compound

**Scheme 5**

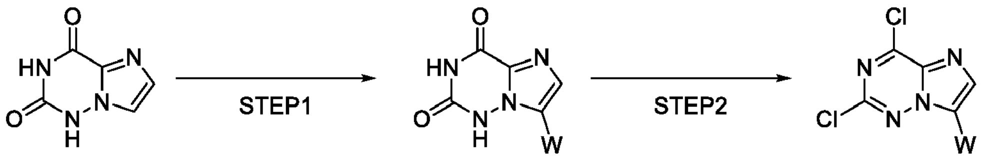


(e) (XVIII) (XVII)

Compound (XVII) may be obtained in STEP 2 by reacting compound (XVIII), wherein W is as defined above, in presence of phosphoryl chloride, for example in a molar ratio between 10 to 20 eq with respect to compound (XVIII), in presence of *N*,*N*-dimethylaniline, triethylamine or triethylamine hydrochloride, for example in a molar ratio of 2 with respect to compound (XVIII), for example at a temperature of 120°C, during 1 to 24 hours, for example 3 hours.

Compound (XVIII) may be obtained in STEP 1 by reacting compound (e), in presence of N-Bromosuccinimide or *N*-Iodosuccinimide, in a protic solvent such as water, methanol, ethanol or AcOH, or in an aprotic solvent such as DCM, THF, 2-MeTHF or dioxane, or a mixture thereof, for example in a molar ratio of 1 with respect to compound (e), for example at room temperature, during 1 to 24 hours, for example 1 hours.

The chemical structures, the analytical and spectroscopic data of some compounds of formula (I) of the invention are illustrated respectively in the following **Table 2** and **Table 3.**

Reactions were performed using glassware or oven-dried glassware under inert atmosphere of argon or not. Unless otherwise noted, all reagent-grade chemicals and solvents were obtained from commercial suppliers and were used as received. Reactions were monitored by thin-layer chromatography with silica gel 60 F254 pre-coated aluminum plates (0.25 mm). Visualization was performed under UV light and 254 or 365 nm, or with appropriate TLC stains including, but not limited to: phosphomolybdic acid, $KMnO_4$, ninhydrin, CAM, vanillin, *p*-anisaldehyde.

Chromatographic purifications of compounds were achieved on an automated Interchim Puriflash XS420 equipped with 30 $\mu$m spherical silica-filled prepacked columns as stationary phase (normal phase) or with C18 silica prepacked columns as stationary phase (reversed phase). Nevertheless, a second purification could be achieved with preparative thin layer chromatography with standard silica. Purifications work with various solvent as pur or mixed such as ethyl acetate, cyclohexane, methanol, methanol with ammonia 7N, dichloromethane, triethylamine and tetrahydrofuran on a normal phase then ACN, MeOH, $H_2O$ and $NH_4OH$ on a reversed phase.

Mixtures possibilities: cHex/EtOAc, cHex/DCM, cHex/DCM/EtOAc, DCM/EtOAc, DCM/MeOH, DCM/MeOH $NH_3$ 7N, DCM/MeOH/$Et_3N$, DCM/MeOH/THF, EtOAc/MeOH, EtOAc/MeOH $NH_3$ 7N, EtOAc/THF, ACN/$H_2O$, ACN/$NH_4OH$, MeOH/$H_2O$, MeOH/$NH_4OH$.

Some compounds of the invention are described with their structure in the below **Table 2,** which is merely illustrative and does not limit the scope of the present invention.

**Table 2**: Structure of compounds (1) to (133). Formulas and molecular weights were generated using Perkin Elmer's Chemdraw® version 17.1.0.105.

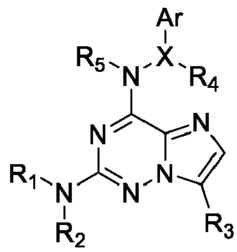

(I)

| N° | R1R2N- | Ar | R3 | X | R4 | R5 | Class | MW |
|---|---|---|---|---|---|---|---|---|
| 1 | 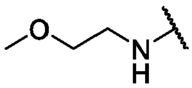 | 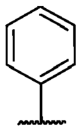 | iPr | CH | H | H | A1 | 340.43 |
| 2 | 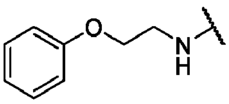 | 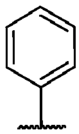 | iPr | CH | H | H | A1 | 402.50 |
| 3 | 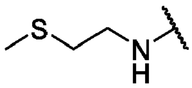 | 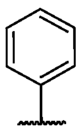 | iPr | CH | H | H | A1 | 356.49 |
| 4 | 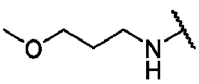 | 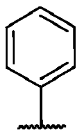 | iPr | CH | H | H | A1 | 354.46 |
| 5 | 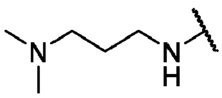 | 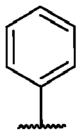 | iPr | CH | H | H | A1 | 367.50 |
| 6 | 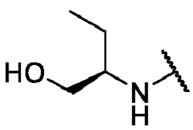 | 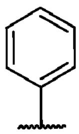 | iPr | CH | H | H | A1 | 354.46 |
| 7 | 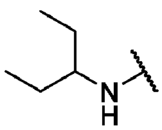 | 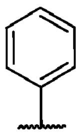 | iPr | CH | H | H | A1 | 352.49 |
| 8 | 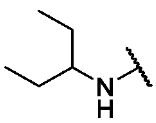 | 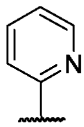 | iPr | CH | H | H | A1 | 353.47 |

(continued)

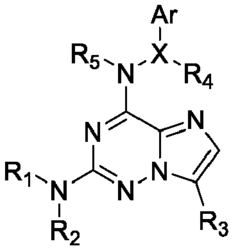 (I)

| N° | R1R2N- | Ar | R3 | X | R4 | R5 | Class | MW |
|---|---|---|---|---|---|---|---|---|
| 9 | 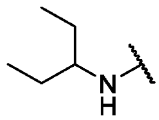 | 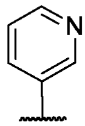 | iPr | CH | H | H | A1 | 353.47 |
| 10 | 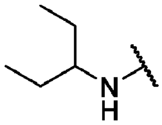 | 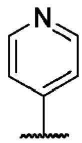 | iPr | CH | H | H | A1 | 353.47 |
| 11 | 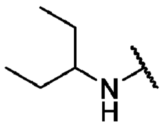 | 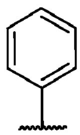 | iPr | Bond | - | H | A1 | 338.46 |
| 12 | 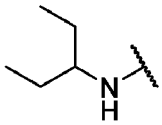 | 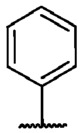 | iPr | $CHCH_2$ | H | H | A1 | 366.51 |
| 13 | 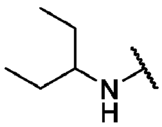 | 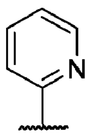 | iPr | $CHCH_2$ | H | H | A1 | 367.50 |
| 14 | 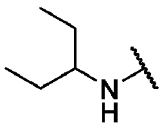 | 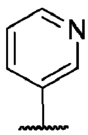 | iPr | $CHCH_2$ | H | H | A1 | 367.50 |
| 15 | 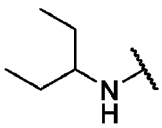 | 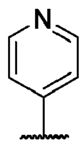 | iPr | $CHCH_2$ | H | H | A1 | 367.50 |
| 16 | 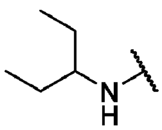 | 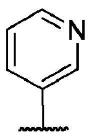 | 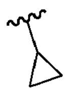 | $CHCH_2$ | H | H | A1 | 365.49 |

(continued)

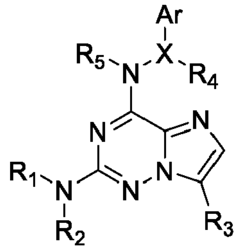 (I)

| N° | R1R2N- | Ar | R3 | X | R4 | R5 | Class | MW |
|---|---|---|---|---|---|---|---|---|
| 17 | 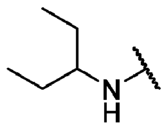 | 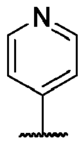 | 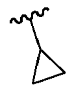 | $CHCH_2$ | H | H | A1 | 365.49 |
| 18 | 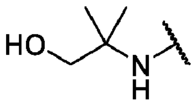 | 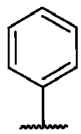 | iPr | CH | H | H | A1 | 354.46 |
| 19 | 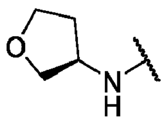 | 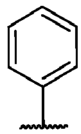 | iPr | CH | H | H | A5 | 352.44 |
| 20 | 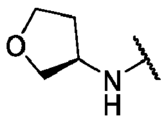 | 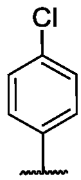 | iPr | CH | H | H | A5 | 386.88 |
| 21 | 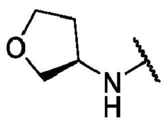 | 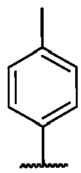 | iPr | CH | H | H | A5 | 366.47 |
| 22 | 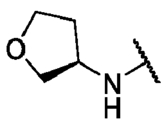 | 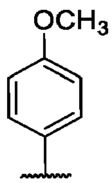 | iPr | CH | H | H | A5 | 382.47 |
| 23 | 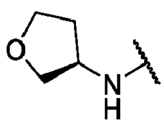 | 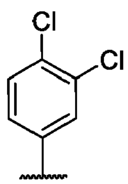 | iPr | CH | H | H | A5 | 421.33 |

(continued)

| 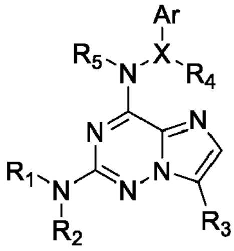 (I) | | | | | | | | |
|---|---|---|---|---|---|---|---|---|
| N° | R1R2N- | Ar | R3 | X | R4 | R5 | Class | MW |
| 24 | 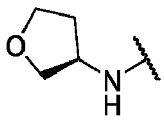 | 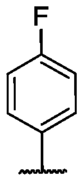 | iPr | CH | H | H | A5 | 370.43 |
| 25 | 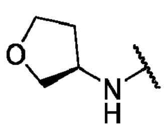 | 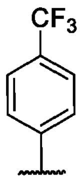 | iPr | CH | H | H | A5 | 420.44 |
| 26 | 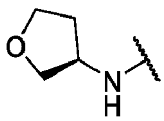 | 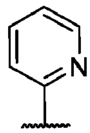 | iPr | CH | H | H | A5 | 353.43 |
| 27 | 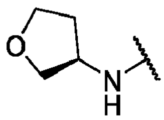 | 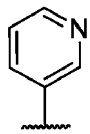 | iPr | CH | H | H | A5 | 353.43 |
| 28 | 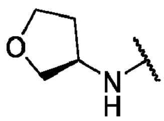 | 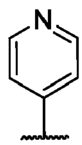 | iPr | CH | H | H | A5 | 353.43 |
| 29 | 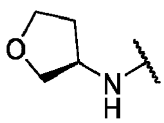 | 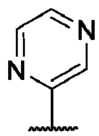 | iPr | CH | H | H | A5 | 354.42 |
| 30 | 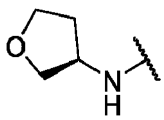 | 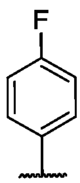 | iPr | CH | $CH_3$ (R) | H | A5 | 366.47 |
| 31 | 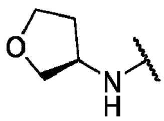 | 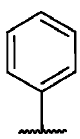 | iPr | CH | $CH_3$ (S) | H | A5 | 366.47 |

(continued)

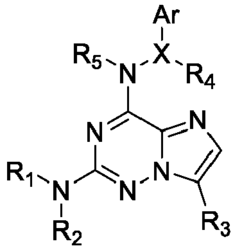
(I)

| N° | R1R2N- | Ar | R3 | X | R4 | R5 | Class | MW |
|---|---|---|---|---|---|---|---|---|
| 32 | 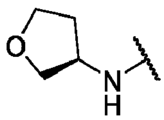 | 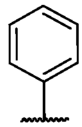 | iPr | CH | H | $CH_3$ | A5 | 366.47 |
| 33 | 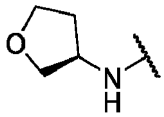 | 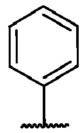 | H | CH | H | H | A5 | 310.36 |
| 34 | 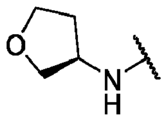 | 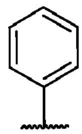 | Br | CH | H | H | A5 | 389.26 |
| 35 | 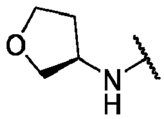 | 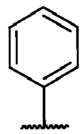 | Me | CH | H | H | A5 | 324.39 |
| 36 | 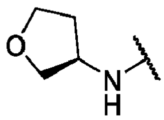 | 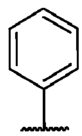 | Et | CH | H | H | A5 | 388.42 |
| 37 | 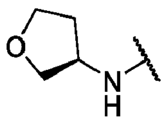 | 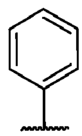 | 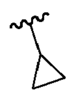 | CH | H | H | A5 | 350.43 |
| 38 | 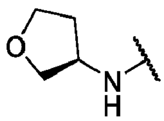 | 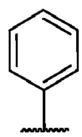 | 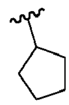 | CH | H | H | A5 | 378.48 |
| 39 | 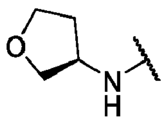 | 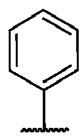 | 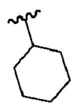 | CH | H | H | A5 | 392.51 |
| 40 | 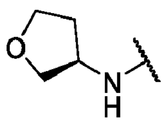 | 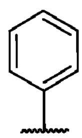 | Ph | CH | H | H | A5 | 386.46 |

(continued)

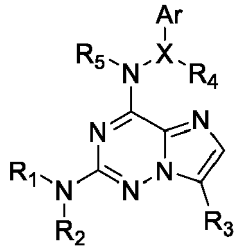 (I)

| N° | R1R2N- | Ar | R3 | X | R4 | R5 | Class | MW |
|---|---|---|---|---|---|---|---|---|
| 41 | 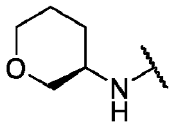 | 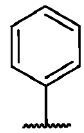 | iPr | CH | H | H | A5 | 366.47 |
| 42 | 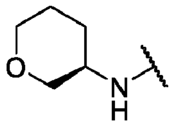 | 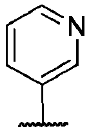 | iPr | CH | H | H | A5 | 367.46 |
| 43 | 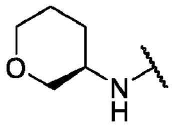 | 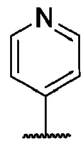 | iPr | CH | H | H | A5 | 367.46 |
| 44 | 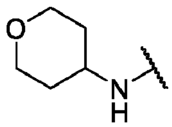 | 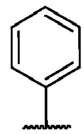 | iPr | CH | H | H | A5 | 366.47 |
| 45 | 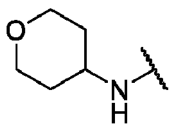 | 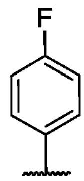 | iPr | CH | H | H | A5 | 384.46 |
| 46 | 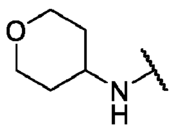 | 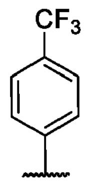 | iPr | CH | H | H | A5 | 434.47 |
| 47 | 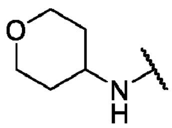 | 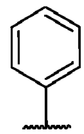 | iPr | CH | $CH_3$ (R) | H | A5 | 380.50 |
| 48 | 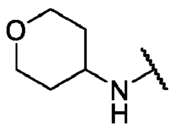 | 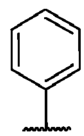 | iPr | CH | $CH_3$ (S) | H | A5 | 380.50 |

(continued)

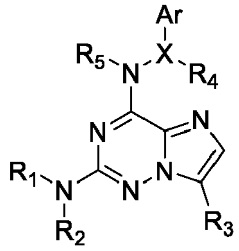 (I)

| N° | R1R2N- | Ar | R3 | X | R4 | R5 | Class | MW |
|---|---|---|---|---|---|---|---|---|
| 49 | 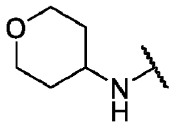 | 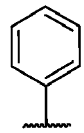 | iPr | CH | H | $CH_3$ | A5 | 380.50 |
| 50 | 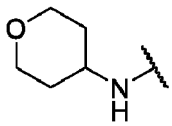 | 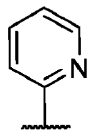 | iPr | CH | H | H | A5 | 367.46 |
| 51 | 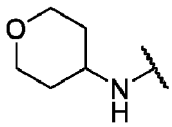 | 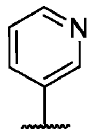 | iPr | CH | H | H | A5 | 367.46 |
| 52 | 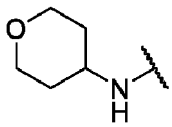 | 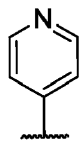 | iPr | CH | H | H | A5 | 367.46 |
| 53 | 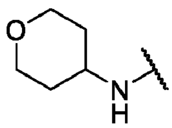 | 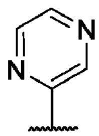 | iPr | CH | H | H | A5 | 368.45 |
| 54 | 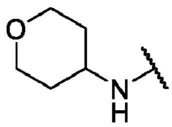 | 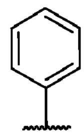 | Me | CH | H | H | A5 | 338.42 |
| 55 | 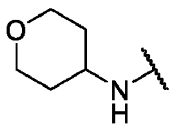 | 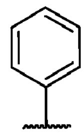 | Et | CH | H | H | A5 | 352.44 |
| 56 | 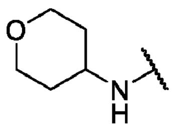 | 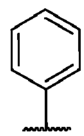 | 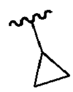 | CH | H | H | A5 | 364.45 |

(continued)

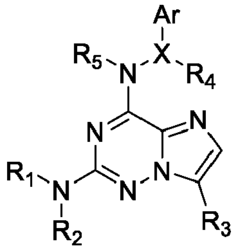
(I)

| N° | R1R2N- | Ar | R3 | X | R4 | R5 | Class | MW |
|---|---|---|---|---|---|---|---|---|
| 57 | 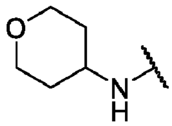 | 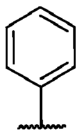 | 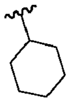 | CH | H | H | A5 | 406.53 |
| 58 | 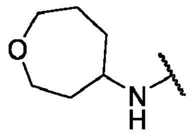 | 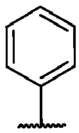 | iPr | CH | H | H | A5 | 380.50 |
| 59 | 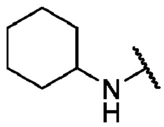 | 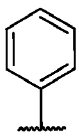 | iPr | CH | H | H | A2 | 364.50 |
| 60 | 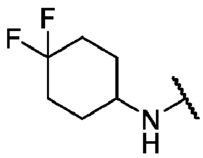 | 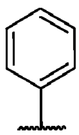 | iPr | CH | H | H | A2 | 400.48 |
| 61 | 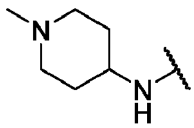 | 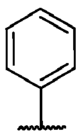 | iPr | CH | H | H | A5 | 379.51 |
| 62 | 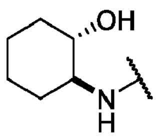 | 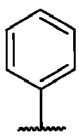 | iPr | CH | H | H | A2 | 380.50 |
| 63 | 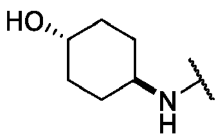 | 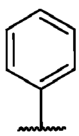 | iPr | CH | H | H | A2 | 380.50 |
| 64 | 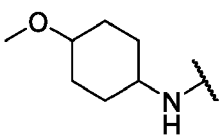 | 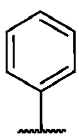 | iPr | CH | H | H | A2 | 394.52 |

(continued)

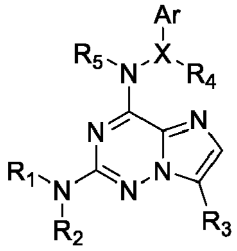
(I)

| N° | R1R2N- | Ar | R3 | X | R4 | R5 | Class | MW |
|---|---|---|---|---|---|---|---|---|
| 65 | 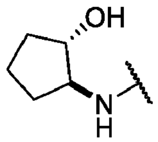 | 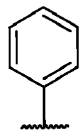 | iPr | CH | H | H | A2 | 366.47 |
| 66 | 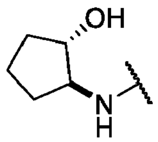 | 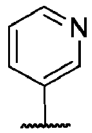 | iPr | CH | H | H | A2 | 367.46 |
| 67 | 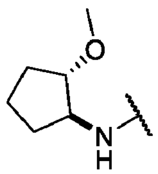 | 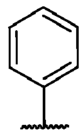 | iPr | CH | H | H | A2 | 380.50 |
| 68 | 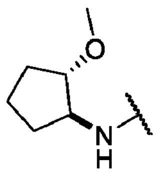 | 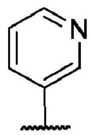 | iPr | CH | H | H | A2 | 381.48 |
| 69 | 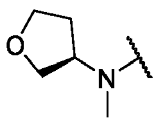 | 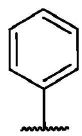 | iPr | CH | H | H | A5 | 366.47 |
| 70 | 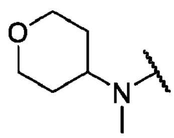 | 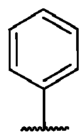 | iPr | CH | H | H | A5 | 380.50 |
| 71 | 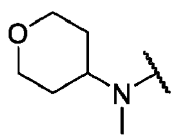 | 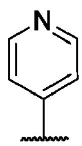 | iPr | CH | H | H | A5 | 381.48 |
| 72 | 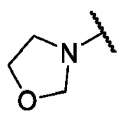 | 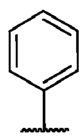 | iPr | CH | H | H | A3 | 338.42 |

(continued)

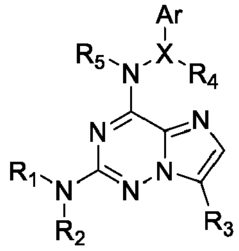 (I)

| N° | R1R2N- | Ar | R3 | X | R4 | R5 | Class | MW |
|---|---|---|---|---|---|---|---|---|
| 73 | 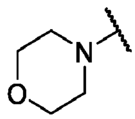 | 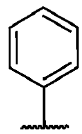 | iPr | CH | H | H | A3 | 352.44 |
| 74 | 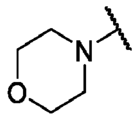 | 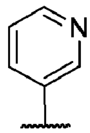 | iPr | CH | H | H | A3 | 353.43 |
| 75 | 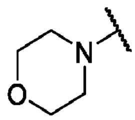 | 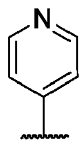 | iPr | CH | H | H | A3 | 353.43 |
| 76 | 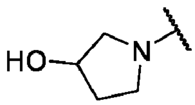 | 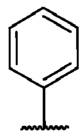 | iPr | CH | H | H | A3 | 352.44 |
| 77 | 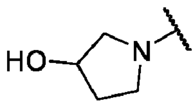 | 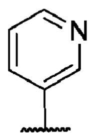 | iPr | CH | H | H | A3 | 353.43 |
| 78 | 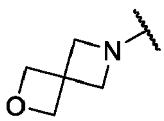 | 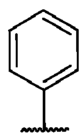 | iPr | CH | H | H | A3 | 364.45 |
| 79 | 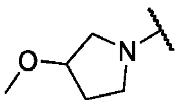 | 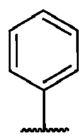 | iPr | CH | H | H | A3 | 366.47 |
| 80 | 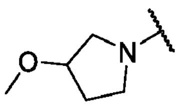 | 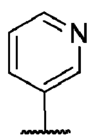 | iPr | CH | H | H | A3 | 367.46 |

(continued)

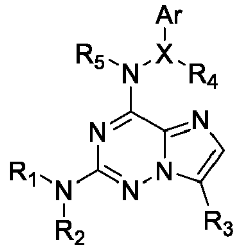 (I)

| N° | R1R2N- | Ar | R3 | X | R4 | R5 | Class | MW |
|---|---|---|---|---|---|---|---|---|
| 81 | 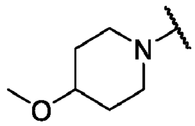 | 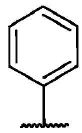 | iPr | CH | H | H | A3 | 380.50 |
| 82 | 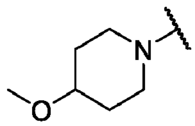 | 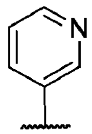 | iPr | CH | H | H | A3 | 381.48 |
| 83 | 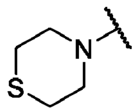 | 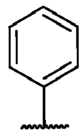 | iPr | CH | H | H | A3 | 368.50 |
| 84 | 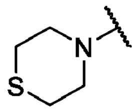 | 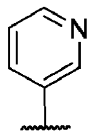 | iPr | CH | H | H | A3 | 369.49 |
| 85 | 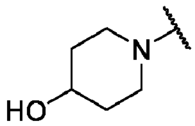 | 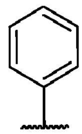 | iPr | CH | H | H | A3 | 366.47 |
| 86 | 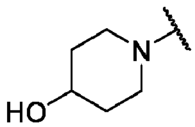 | 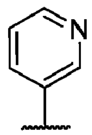 | iPr | CH | H | H | A3 | 367.46 |
| 87 | 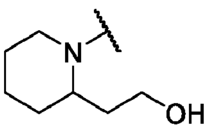 | 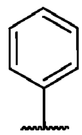 | iPr | CH | H | H | A3 | 394.52 |
| 88 | 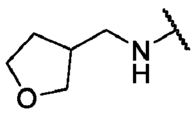 | 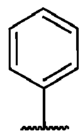 | iPr | CH | H | H | A5 | 366.47 |
| 89 | 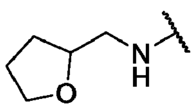 | 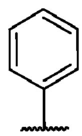 | iPr | CH | H | H | A5 | 366.47 |

(continued)

| 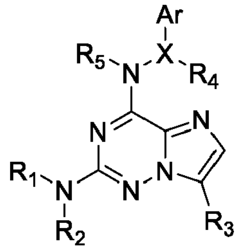 (I) | | | | | | | | |
|---|---|---|---|---|---|---|---|---|
| N° | R1R2N- | Ar | R3 | X | R4 | R5 | Class | MW |
| 90 | 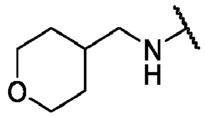 | 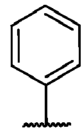 | iPr | CH | H | H | A5 | 380.50 |
| 91 | 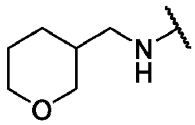 | 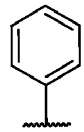 | iPr | CH | H | H | A5 | 380.50 |
| 92 | 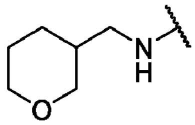 | 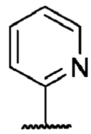 | iPr | CH | H | H | A5 | 381.48 |
| 93 | 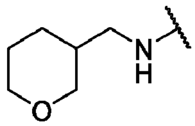 | 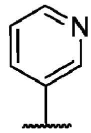 | iPr | CH | H | H | A5 | 381.48 |
| 94 | 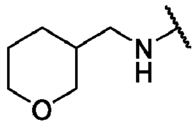 | 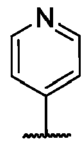 | iPr | CH | H | H | A5 | 381.48 |
| 95 | 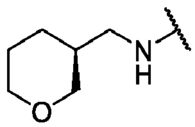 | 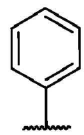 | iPr | CH | H | H | A5 | 380.50 |
| 96 | 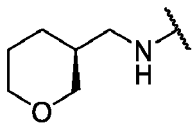 | 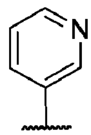 | iPr | CH | H | H | A5 | 381.48 |
| 97 | 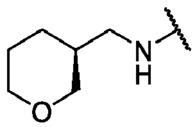 | 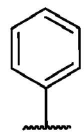 | iPr | CH | H | H | A5 | 380.50 |

(continued)

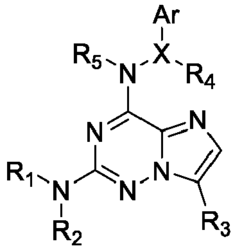 (I)

| N° | R1R2N- | Ar | R3 | X | R4 | R5 | Class | MW |
|---|---|---|---|---|---|---|---|---|
| 98 | 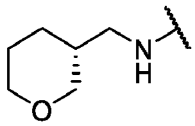 | 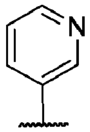 | iPr | CH | H | H | A5 | 381.48 |
| 99 | 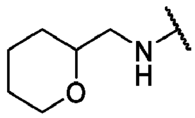 | 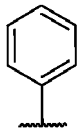 | iPr | CH | H | H | A5 | 380.50 |
| 100 | 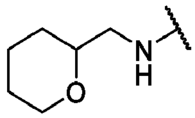 | 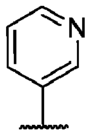 | iPr | CH | H | H | A5 | 381.48 |
| 101 | 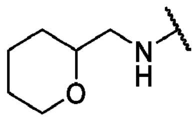 | 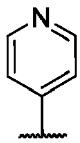 | iPr | CH | H | H | A5 | 381.48 |
| 102 | 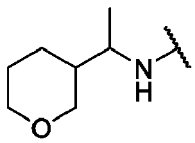 | 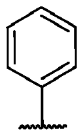 | iPr | CH | H | H | A5 | 394.52 |
| 103 | 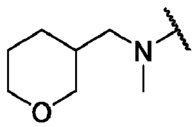 | 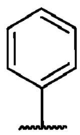 | iPr | CH | H | H | A5 | 394.52 |
| 104 | 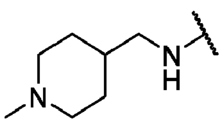 | 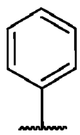 | iPr | CH | H | H | A5 | 393.54 |
| 105 | 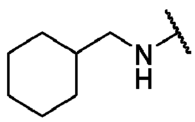 | 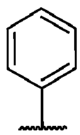 | iPr | CH | H | H | A2 | 378.52 |

(continued)

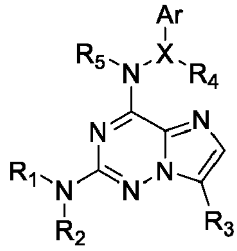 (I)

| N° | R1R2N- | Ar | R3 | X | R4 | R5 | Class | MW |
|---|---|---|---|---|---|---|---|---|
| 106 | 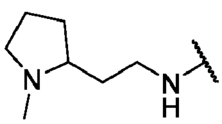 | 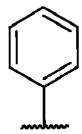 | iPr | CH | H | H | A5 | 393.54 |
| 107 | 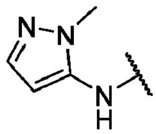 | 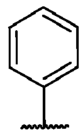 | iPr | CH | H | H | A6 | 362.44 |
| 108 | 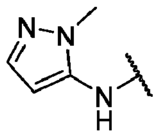 | 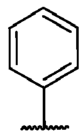 | iPr | CH | H | $CH_3$ | A6 | 376.47 |
| 109 | 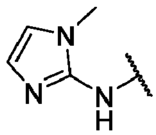 | 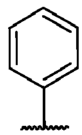 | iPr | CH | H | H | A6 | 362.44 |
| 110 | 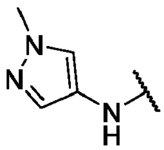 | 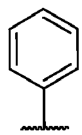 | iPr | CH | H | H | A6 | 362.44 |
| 111 | 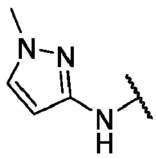 | 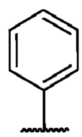 | iPr | CH | H | H | A6 | 362.44 |
| 112 | 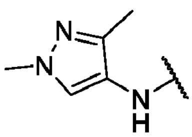 | 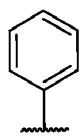 | iPr | CH | H | H | A6 | 376.47 |
| 113 | 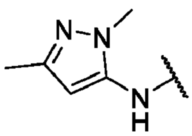 | 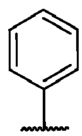 | iPr | CH | H | H | A6 | 376.47 |

(continued)

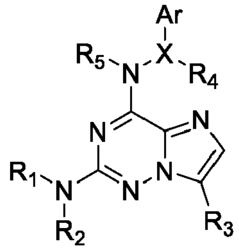 (I)

| N° | R1R2N- | Ar | R3 | X | R4 | R5 | Class | MW |
|---|---|---|---|---|---|---|---|---|
| 114 | 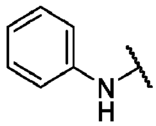 | 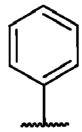 | iPr | CH | H | H | A4 | 358.45 |
| 115 | 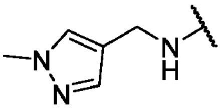 | 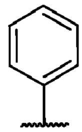 | iPr | CH | H | H | A6 | 376.47 |
| 116 | 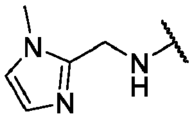 | 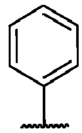 | iPr | CH | H | H | A6 | 376.47 |
| 117 | 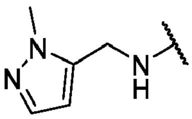 | 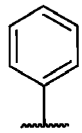 | iPr | CH | H | H | A6 | 376.47 |
| 118 | 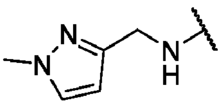 | 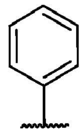 | iPr | CH | H | H | A6 | 376.47 |
| 119 | 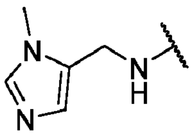 | 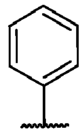 | iPr | CH | H | H | A6 | 376.47 |
| 120 | 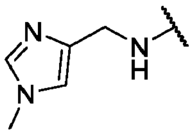 | 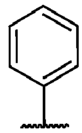 | iPr | CH | H | H | A6 | 376.47 |
| 121 | 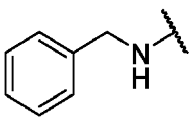 | 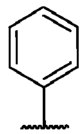 | iPr | CH | H | H | A4 | 372.48 |
| 122 | 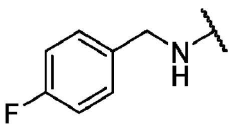 | 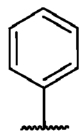 | iPr | CH | H | H | A4 | 390.47 |

(continued)

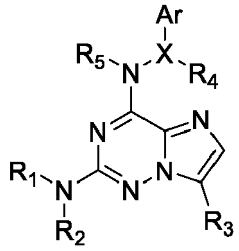 (I)

| N° | R1R2N- | Ar | R3 | X | R4 | R5 | Class | MW |
|---|---|---|---|---|---|---|---|---|
| 123 | 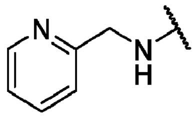 | 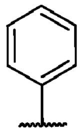 | iPr | CH | H | H | A6 | 373.46 |
| 124 | 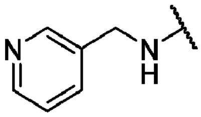 | 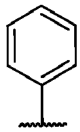 | iPr | CH | H | H | A6 | 373.46 |
| 125 | 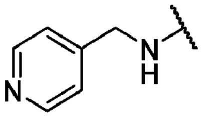 | 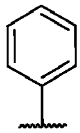 | iPr | CH | H | H | A6 | 373.46 |
| 126 | 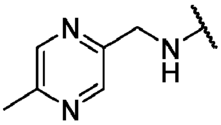 | 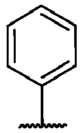 | iPr | CH | H | H | A6 | 388.48 |
| 127 | 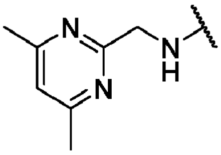 | 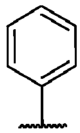 | iPr | CH | H | H | A6 | 402.51 |
| 128 | 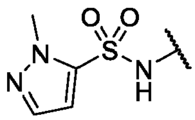 | 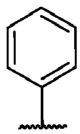 | iPr | CH | H | H | A6 | 426.50 |
| 129 | 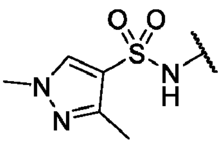 | 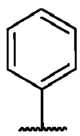 | iPr | CH | H | H | A6 | 440.53 |
| 130 | 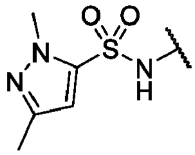 | 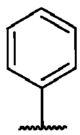 | iPr | CH | H | H | A6 | 440.53 |

(continued)

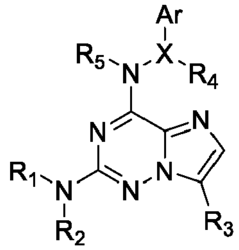

(I)

| N° | R1R2N- | Ar | R3 | X | R4 | R5 | Class | MW |
|---|---|---|---|---|---|---|---|---|
| 131 | 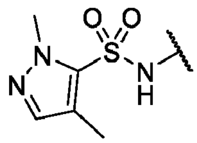 | 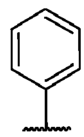 | iPr | CH | H | H | A6 | 440.53 |
| 132 | 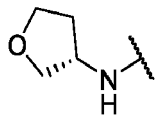 | 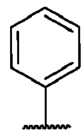 | iPr | CH | H | H | A5 | 352.44 |
| 133 | 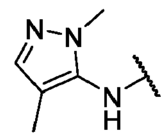 | 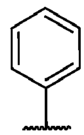 | iPr | CH | H | H | A6 | 376.47 |

The below **Table 3** describes the analytical and spectroscopic data of the compounds introduced in **Table 2**. $^{1}H$ NMR analyses (400 or 500 MHz) and $^{13}C$ NMR spectra (101 MHz) were recorded with a Bruker ULTRASHIELD 500 or 400 spectrometer. Processing and analyses of the spectra were performed with MestReNova. Data appear in the following order: chemical shifts in ppm which were referenced to the internal solvent signal, multiplicity, number of protons, and coupling constant J in Hertz.

Synthetic intermediates: reversed-phase UPLC/MS analyses were carried out with a UPLC Acquity (Waters) with an UV-DAD detector and a mass detector (SQD2). Compounds (0.2 to 0.6 mg) were solubilized in a mixture of DMSO/$H_2O$ (1/1) and filtered on syringe filter 0.2 $\mu$m.

- Acidic conditions:

Acquity BEH C18 column, 2.1 × 50 mm, 1.7 $\mu$m. Flow rate: 0.65 mL/min. Gradient: (H2O + 0.1% HCOOH v/v)/(CAN + 0.1% HCOOH v/v) from 95/5 to 5/95 in 4.0 min.

Final compounds: Reversed-phase HPLC/MS analyses were carried out with a HPLC Ultimate 3000 (Thermo-Scientific) with an UV-DAD detector. Mass detection processing with direct infusion in mass detector (SQD2) from UPLC Acquity (Waters). Compounds (0.2 to 0.6 mg) were solubilized in a mixture of DMSO/$H_2O$ (1/1) and filtered on syringe filter 0.2 $\mu$m.

- Acidic conditions:

Thermo Scientific Syncronis C18 column, 150 x 4.6 mm, 5 $\mu$m. Flow rate: 1 mL/min. Gradient: ($H_2O$ + 0.1% HCOOH v/v)/(ACN + 0.1% HCOOH v/v) from 95/5 to 5/95 in 19.0 min.

- Alkaline conditions:

Thermo Scientific Syncronis C18 column, 150 × 4.6 mm, 5 $\mu$m. Flow rate: 1 mL/min. Gradient: ($HCOONH_4$ 10mM + $NH_4OH$ 25% aq. to ajust pH = 10)/ACN from 95/5 to 5/95 in 19.0 min.

**Table 3:** Spectroscopic and analytical characterization of compounds (1) to (133)

| N° | Formula | HPLC | Description |
|---|---|---|---|
| 1 | $C_{18}H_{24}N_6O$ | >98% [a] | White solid. $^1$H NMR (400 MHz, DMSO-$d_6$) δ 8.73 (t, *J* = 6.3 Hz, 1H), 7.39 - 7.27 (m, 4H), 7.25 - 7.18 (m, 1H), 7.11 (s, 1H), 6.28 (t, *J* = 5.8 Hz, 1H), 4.63 (d, *J* = 6.3 Hz, 2H), 3.45 (t, *J* = 6.0 Hz, 2H), 3.33 (q, *J* = 5.8 Hz, 2H), 3.24 (s, 3H), 3.17 (h, *J* = 6.9 Hz, 1H), 1.29 (d, *J* = 6.9 Hz, 6H). $^{13}$C NMR (101 MHz, DMSO-$d_6$) δ 156.4, 151.4, 139.3, 134.4, 128.2 (2C), 127.3 (2C), 126.7, 126.4, 125.3, 70.3, 57.8, 42.6, 40.4, 23.7, 20.6 (2C). MS (ESI$^+$): [M+H]$^+$ 341.2. LogD (HPLC): 3.50 |
| 2 | $C_{23}H_{26}N_6O$ | > 98 % [a] | White solid. $^1$H NMR (400 MHz, DMSO-$d_6$) δ 8.79 (t, *J* = 6.3 Hz, 1H), 7.40 - 7.18 (m, 7H), 7.13 (s, 1H), 7.00 - 6.88 (m, 3H), 6.54 (t, *J* = 5.8 Hz, 1H), 4.64 (d, *J* = 6.3 Hz, 2H), 4.10 (t, *J* = 6.2Hz, 2H), 3.55 (m, 2H), 3.20 (h, *J* = 6.9 Hz, 1H), 1.30 (d, *J* = 7.0 Hz, 6H). $^{13}$C NMR (101 MHz, DMSO) δ 158.9, 156.9, 152.0, 139.8, 134.9, 129.9 (2C), 128.7 (2C), 127.8 (2C), 127.2, 126.9, 125.9, 121.0, 114.9 (2C), 66.2, 43.2, 40.8, 24.1, 21.2 (2C). MS (ESI$^+$): [M+H]$^+$ 403.2. LogD (HPLC): 4.74 |
| 3 | $C_{18}H_{24}N_6S$ | 99% [a] | Yellow oil. $^1$H NMR (400 MHz, DMSO-$d_6$) δ 8.75 (t, *J* = 5.5 Hz, 1H), 7.38 - 7.26 (m, 4H), 7.22 (m, 1H), 7.11 (s, 1H), 6.50 (t, *J* = 5.4 Hz, 1H), 4.63 (d, *J* = 6.2 Hz, 2H), 3.38 - 3.33 (m, 2H), 3.17 (h, *J* = 6.9 Hz, 1H), 2.63 (t, *J* = 7.4 Hz, 2H), 2.07 (s, 3H), 1.29 (d, *J* = 6.9 Hz, 6H). $^{13}$C NMR (101 MHz, DMSO-$d_6$) δ 156.7, 152.0, 139.7, 134.9, 128.7 (2C), 127.8 (2C), 127.2, 126.9, 125.8, 43.1, 41.0, 32.8, 24.2, 21.1 (2C), 15.0. MS (ESI$^+$): [M+H]$^+$357.2. LogD (HPLC): 4.13 |
| 4 | $C_{19}H_{26}N_6O$ | > 98% [b] | White solid. NMR $^1$H (400 MHz, DMSO-$d_6$) : δ (ppm) = 8.69 (t, *J* = 6.3 Hz, 1H), 7.35 (d, *J* = 7.2 Hz, 2H), 7.30 (t, *J* = 7.4 Hz, 2H), 7.26 - 7.18 (m, 1H), 7.10 (s, 1H), 6.34 (t, *J* = 5.7 Hz, 1H), 4.63 (d, *J* = 6.3 Hz, 2H), 3.36 (t, *J* = 6.3 Hz, 2H), 3.25 - 3.11 (m, 6H), 1.76 (p, *J* = 6.7 Hz, 2H), 1.29 (d, *J* = 6.9 Hz, 6H). NMR $^{13}$C (101 MHz, DMSO-$d_6$) : δ (ppm) = 156.51, 151.44, 139.36, 134.38, 128.19 (2C), 127.36 (2C), 126.72, 126.41, 125.26, 70.05, 57.82, 42.57, 38.34, 28.90, 23.69, 20.65 (2C). MS (ESI+, m/z) : 355.31 [M+H+]. M.p. 115 - 117 °C. |
| 5 | $C_{20}H_{29}N_7$ | 95% [a] | White solid. $^1$H NMR (400 MHz, DMSO-$d_6$) δ 8.68 (t, *J* = 6.1 Hz, 1H), 7.38 - 7.26 (m, 4H), 7.26 - 7.18 (m, 1H), 7.10 (s, 1H), 6.35 (t, *J* = 5.8 Hz, 1H), 4.63 (d, *J* = 6.2 Hz, 2H), 3.16 (m, 3H), 2.23 (t, *J* = 7.1 Hz, 2H), 2.10 (s, 6H), 1.66 (p, *J* = 7.1 Hz, 2H), 1.29 (d, *J* = 6.9 Hz, 6H). $^{13}$C NMR (101 MHz, DMSO-$d_6$) δ 157.0, 151.9, 139.8, 134.8, 128.6 (2C), 127.8 (2C), 127.2, 126.9, 125.7, 57.6, 45.7 (2C), 43.1, 27.3, 24.2, 21.2 (2C). 1 carbon signal overlap with solvent. MS (ESI$^+$): [M+H]$^+$ 403.3. LogD (HPLC): 2.10 |
| 6 | $C_{19}H_{26}N_6O$ | > 98% [b] | White solid. NMR $^1$H (400 MHz, DMSO-$d_6$) : δ (ppm) = 8.69 (t, *J* = 6.5 Hz, 1H), 7.36 (d, *J* = 7.6 Hz, 2H), 7.30 (t, *J* = 7.4 Hz, 2H), 7.22 (t, *J* = 7.2 Hz, 1H), 7.10 (s, 1H), 5.88 (d, *J* = 8.3 Hz, 1H), 4.65 (tt, *J* = 15.1, 7.5 Hz, 2H), 4.54 (t, *J* = 5.6 Hz, 1H), 3.71 - 3.58 (m, 1H), 3.51 - 3.43 (m, 1H), 3.42 - 3.36 (m, 1H), 3.15 (hept, *J* = 6.9 Hz, 1H), 1.67-1.55 (m, 1H), 1.51 - 1.39 (m, 1H), 1.29 (dd, *J* = 6.9, 2.5 Hz, 6H), 0.85 (t, *J* = 7.3 Hz, 3H). NMR $^{13}$C (101 MHz, DMSO-$d_6$) : δ (ppm) = 156.49, 151.45, 139.39, 134.31, 128.21 (2C), 127.38 (2C), 126.75, 126.43, 125.31, 62.58, 54.17, 42.57, 23.73, 23.69, 20.68, 20.62, 10.65. MS (ESI+, m/z) : [M+H+] 355.31. M.p. 108 - 110 °C. |
| 7 | $C_{20}H_{28}N_6$ | > 91 % [a] | Beige solid. NMR $^1$H (400 MHz, DMSO-$d_6$) : δ (ppm) = 8.64 (t, *J* = 6.2 Hz, 1H), 7.36 (d, *J* = 7.3 Hz, 2H), 7.30 (t, *J* = 7.5 Hz, 2H), 7.21 (t, *J* = 7.4 Hz, 1H), 7.08 (s, 1H), 6.04 (d, *J* = 8.6 Hz, 1H), 4.64 (d, *J* = 6.2 Hz, 2H), 3.65 - 3.50 (m, 1H), 3.15 (hept, *J* = 6.9 Hz, 1H), 1.54 - 1.40 (m, *J* = 6.7 Hz, 4H), 1.29 (d, *J* = 6.9 Hz, 6H), 0.83 (t, *J* = 7.3 Hz, 6H). NMR $^{13}$C (101 MHz, DMSO-$d_6$) : δ (ppm) = 156.72, 151.40, 139.40, 134.20, 128.16 (2C), 127.37 (2C), 126.71, 126.41, 125.21, 53.44, 42.58, 26.56 (2C), 23.73, 20.59 (2C), 10.63 (2C). MS (ES+, m/z) : [M+H+] 353.42. M.p. 82 - 84 °C. |

(continued)

| N° | Formula | HPLC | Description |
|---|---|---|---|
| 8 | $C_{19}H_{27}N_7$ | >98% [a] | White solid. NMR $^1$H (400 MHz, $CD_3OD$) δ 8.53-8.48 (m, 1H), 7.79 (td, ***J*** = 7.8 Hz, ***J*** = 1.8 Hz, 1H), 7.45 (d, ***J*** = 7.9 Hz, 1H), 7.34-7.26 (m, 1H), 3.64 (td, ***J*** = 7.5 Hz, *J* = 3.8 Hz, 1H), 3.29-3.21 (m, 1H), 1.64-1.53 (m, 2H), 1.53-1.42 (m, 2H), 1.36 (d, ***J*** = 6.9 Hz, 6H), 0.90 (t, ***J*** = 7.4 Hz, 6H). NMR $^{13}$C (101 MHz, $CD_3OD$) δ 158.5, 152.8, 151.4, 149.9 (2C), 136.7, 127.9, 126.1 (2C), 125.9, 55.3, 41.3, 35.6, 28.3 (2C), 25.4, 21.0 (2C), 11.0 (2C). MS (ESI$^+$): [M+H]$^+$ 368.3. |
| 9 | $C_{19}H_{27}N_7$ | >95% [a] | White solid. $^1$H NMR (400 MHz, $CDCl_3$) δ 8.64 (d, ***J*** = 2.2 Hz, 1H), 8.53 (dd, ***J*** = 4.9, ***J*** = 1.7 Hz, 1H), 7.67 (dt, ***J*** = 7.9, ***J*** = 2.0 Hz, 1H), 7.26-7.22 (m, 1H), 7.05 (s, 1H), 6.62 (br s, 1H), 4.75 (d, ***J*** = 5.3 Hz, 2H), 4.25 (d, ***J*** = 8.7 Hz, 1H), 3.77-3.67 (m, 1H), 3.32-3.20 (m, 1H), 1.69-1.57 (m, 2H), 1.57-1.45 (m, 2H), 1.35 (d, ***J*** = 6.9 Hz, 6H), 0.94 (t, ***J*** = 7.4 Hz, 6H). $^{13}$C NMR (100 MHz, $CDCl_3$) δ 156.7, 151.9, 149.6, 149.2, 136.0, 135.5, 133.8, 127.0, 126.0, 123.6, 54.1, 41.7, 27.3 (2C), 24.4, 20.9 (2C), 10.5 (2C). MS (ESI$^+$): [M+H]$^+$ 354.2. |
| 10 | $C_{19}H_{27}N_7$ | >98% [a] | Off-white solid. NMR $^1$H (400 MHz, $CD_3OD$) δ 8.49-8.43 (m, 2H), 7.45-7.41 (m, 2H), 7.12 (s, 1H), 4.82 (s, 2H), 3.69-3.56 (m, 1H), 3.29-3.19 (m, 1H), 1.64-1.51 (m, 2H), 1.52-1.42 (m, 2H), 1.37 (d, ***J*** = 6.9 Hz, 6H), 0.89 (t, ***J*** = 7.4 Hz, 6H). NMR $^{13}$C (101 MHz, DMSO-*d*$_6$) δ 158.5, 152.9, 151.1, 150.6, 136.8, 127.9, 126.3, 123.9, 55.3, 43.6, 28.3 (2C), 25.5, 21.0 (2C), 10.9 (2C). MS (ESI$^+$): [M+H]$^+$ 354.2. |
| 11 | $C_{19}H_{26}N_6$ | >95% [a] | White solid. $^1$H NMR (400 MHz, $CD_3OD$) δ 7.93-7.88 (m, 2H), 7.40-7.34 (m, 2H), 7.17 (s, 1H), 7.14-7.08 (m, 2H), 3.77-3.68 (m, ***1***H), 3.33-3.23 (m, 1H), 1.72-1.61 (m, ***2***H), 1.61-1.51 (m, 2H), 1.39 (d, ***J*** = 7.0 Hz, 6H), 0.96 (t, ***J*** = 7.4 Hz, 6H). $^{13}$C NMR (100 MHz, $CD_3OD$) δ 158.5, 152.8, 151.4, 149.9 (2C), 136.7, 127.9, 126.1 (2C), 125.9, 55.3, 41.3, 35.6, 28.3 (2C), 25.4, 21.0 (2C), 11.0 (2C). MS (ESI$^+$): [M+H]$^+$ 339.2. |
| 12 | $C_{21}H_{30}N_6$ | >98% [a] | White solid. $^1$H NMR (400 MHz, $CD_3OD$) δ 7.31-7.23 (m, 4H), 7.22-7.14 (m, 1H), 7.04 (s, 1H), 3.79 (t, ***J*** = 7.3 Hz, 2H), 3.75-3.65 (m, 1H), 3.29-3.16 (m, 1H), 2.98 (t, ***J*** = 7.3 Hz, 2H), 1.73-1.60 (m, 2H), 1.59-1.48 (m, 2H), 1.35 (d, ***J*** = 6.9 Hz, 6H), 0.97 (t, ***J*** = 7.4 Hz, 6H). $^{13}$C NMR (100 MHz, $CD_3OD$) δ 158.6, 152.8, 140.5, 136.6, 129.9 (2C), 129.5 (2C), 128.0, 127.4, 125.8, 55.3, 42.7, 36.3, 28.3 (2C), 25.4, 21.1 (2C), 11.0 (2C). MS (ESI$^+$): [M+H]$^+$ 367.2. |
| 13 | $C_{20}H_{29}N_7$ | >98% [a] | White solid. NMR $^1$H (400 MHz, $CD_3OD$) δ 8.49 (dt, ***J*** = 4.7 Hz, ***J*** = 1.4 Hz, 1H), 7.74 (td, ***J*** = 7.7 Hz, ***J*** = 1.8 Hz, 1H), 7.36 (d, ***J*** = 7.8 Hz, 1H), 7.29-7.24 (m, 1H), 7.04 (s, 1H), 3.92 (t, ***J*** = 7.0 Hz, 2H), 3.75-3.66 (m, 1H), 3.29-3.20 (m, 1H), 3.17 (t, ***J*** = 7.0 Hz, 2H), 1.70-1.58 (m, 2H), 1.58-1.49 (m, 2H), 1.35 (d, ***J*** = 6.9 Hz, 6H), 0.97 (t, ***J*** = 7.4 Hz, 6H). NMR $^{13}$C (101 MHz, DMSO-*d*$_6$) δ 160.4, 158.5, 152.7, 149.9, 138.7, 136.7, 125.7, 125.3, 123.2, 55.2, 41.1, 38.0, 28.3 (2C), 25.4, 21.0 (2C), 11.0 (2C). MS (ESI$^+$): [M+H]$^+$ 368.2. |
| 14 | $C_{20}H_{29}N_7$ | >98% [a] | White solid. $^1$H NMR (400 MHz, $CD_3OD$) δ 8.45 (d, ***J*** = 2.2 Hz, 1H), 8.37 (dd, ***J*** = 4.9, ***J*** = 1.6 Hz, 1H), 7.79 (dt, ***J*** = 7.9, ***J*** = 1.9 Hz, 1H), 7.36 (ddd, ***J*** = 7.9, ***J*** = 4.9, ***J*** = 0.9 Hz, 1H), 3.83 (t, ***J*** = 7.0 Hz, 2H), 3.70 (ddd, ***J*** = 7.6, ***J*** = 5.5, ***J*** = 2.1 Hz, 1H), 3.28-3.18 (m, 1H), 3.05 (t, ***J*** = 7.0 Hz, 2H), 1.69-1.59 (m, 2H), 1.58-1.47 (m, 2H), 1.35 (d, ***J*** = 6.9 Hz, 6H), 0.96 (t, ***J*** = 7.4 Hz, 6H). $^{13}$C NMR (100 MHz, $CD_3OD$) δ 158.5, 152.8, 150.5, 148.0, 138.8, 137.1, 136.7, 127.9, 125.9, 125.2, 55.2, 42.0, 33.4, 28.3 (2C), 25.4, 21.0 (2C), 11.0 (2C). MS (ESI$^+$): [M+H]$^+$ 368.2. |

(continued)

| N° | Formula | HPLC | Description |
|---|---|---|---|
| 15 | $C_{20}H_{29}N_7$ | 96% [a] | Beige solid. NMR $^1H$ (400 MHz, $CD_3OD$) δ 8.46-8.38 (m, 2H), 7.40-7.35 (m, 2H), 7.05 (s, 1H), 3.87 (t, ***J*** = 7.1 Hz, 2H), 3.75-366 (m, 1H), 3.24 (p, ***J*** = 6.9 Hz, 1H), 3.06 (t, ***J*** = 7.0 Hz, 2H), 1.71-1.59 (m, 2H), 1.59-1.48 (m, 2H), 1.35 (d, ***J*** = 6.9 Hz, 6H), 0.96 (t, ***J*** = 7.4 Hz, 6H).NMR $^{13}C$ (101 MHz, $CD_3OD$) δ 158.5, 152.8, 151.4, 149.9 (2C), 136.7, 127.9, 126.1 (2C), 125.9, 55.3, 41.3, 35.6, 28.3 (2C), 25.4, 21.0 (2C), 11.0 (2C).MS ($ESI^+$): $[M+H]^+$ 368.3. |
| 16 | $C_{20}H_{27}N_7$ | 88% | White solid. NMR 1H N.D. MS ($ESI^+$): $[M+H]^+$ 366.2 |
| 17 | $C_{20}H_{27}N_7$ | 96% [a] | White solid. NMR $^1H$ (400 MHz, $CD_3OD$) δ 8.44-8.39 (m, 2H), 7.40-7.30 (m, 2H), 6.94 (s, 1H), 3.87 (t, ***J*** = 7.1 Hz, 2H), 3.79-3.67 (m, 1H), 3.06 (t, ***J*** = 7.0 Hz, 2H), 2.12-1.96 (m, 1H), 1.70-1.59 (m, 2H), 1.59-1.48 (m, 2H), 1.03-0.94 (m, 2H), 0.97 (t, ***J*** = 7.4 Hz, 6H), 0.87-0.76 (m, 2H). NMR $^{13}C$ (101 MHz, $CD_3OD$) δ 152.7, 151.4, 150.0 (2C), 137.3, 133.2, 126.3, 126.1 (2C), 55.2, 41.3, 35.6, 28.4 (2C), 11.0 (2C), 6.4 (2C), 5.6. MS ($ESI^+$): $[M+H]^+$ 366.2. |
| 18 | $C_{19}H_{26}N_6O$ | 94% [a] | White solid. $^1H$ NMR (400 MHz, DMSO-*d*$_6$) δ 8.66 (t, ***J*** = 6.4 Hz, 1H), 7.37 - 7.27 (m, 4H), 7.26 - 7.19 (m, 1H), 7.11 (s, 1H), 5.40 (s, 1H), 4.85 (t, *J* = 5.8 Hz, 1H), 4.64 (d, *J* = 6.4 Hz, 2H), 3.45 (d, *J* = 5.8 Hz, 2H), 3.21 - 3.12 (m, 1H), 1.31 (d, *J* = 6.8 Hz, 6H), 1.30 (s, 6H). $^{13}C$ NMR (101 MHz, DMSO-*d*$_6$) δ 156.2, 151.8, 139.8, 134.8, 128.7 (2C), 127.6 (2C), 127.2, 126.7, 125.8, 68.5, 54.2, 42.9, 24.3, 23.8 (2C), 21.1 (2C). MS ($ESI^+$): $[M+H]^+$ 355.7. LogD (HPLC): 3.32 |
| 19 | $C_{19}H_{24}N_6O$ | 97 % [a] | White solid. NMR $^1H$ (400 MHz, DMSO-*d*$_6$) : δ (ppm) = 8.75 (t, *J* = 6.4 Hz, 1H), 7.38 - 7.26 (m, 4H), 7.25 - 7.19 (m, 1H), 7.12 (s, 1H), 6.59 (d, *J* = 5.6 Hz, 1H), 4.65 (d, *J* = 6.4 Hz, 2H), 4.23 - 4.13 (m, 1H), 3.86 (dd, *J* = 8.8, 6.1 Hz, 1H), 3.79 (q, *J* = 7.5 Hz, 1H), 3.71 - 3.64 (m, 1H), 3.54 (dd, *J* = 8.8, 4.5 Hz, 1H), 3.17 (hept, *J* = 6.9 Hz, 1H), 2.15 - 2.03 (m, 1H), 1.94 - 1.83 (m, 1H), 1.30 (dd, *J* = 6.9, 2.8 Hz, 6H). NMR $^{13}C$ (101 MHz, DMSO-*d*$_6$) : δ (ppm) = 156.19, 151.52, 139.31, 134.45, 128.23 (2C), 127.27 (2C), 126.74, 126.38, 125.38, 72.78, 66.42, 51.76, 42.58, 31.86, 23.75, 20.63, 20.58. MS (ES+, m/z) : [M+H+] 353.26. M.p. 111 - 113 °C. |
| 20 | $C_{19}H_{23}ClN_6O$ | 94 % [a] | White solid. NMR $^1H$ (400 MHz, DMSO-*d*$_6$) : δ (ppm) = 8.79 (t, *J* = 6.3 Hz, 1H), 7.36 (s, 4H), 7.12 (s, 1H), 6.61 (d, *J* = 5.6 Hz, 1H), 4.62 (d, *J* = 6.3 Hz, 2H), 4.24 - 4.11 (m, 1H), 3.85 (dd, *J* = 8.9, 6.1 Hz, 1H), 3.79 (q, *J* = 7.6 Hz, 1H), 3.68 (q, *J* = 7.6 Hz, 1H), 3.54 (dd, *J* = 8.9, 4.5 Hz, 1H), 3.17 (hept, *J* = 6.9 Hz, 1H), 2.09 (dq, *J* = 14.6, 7.6 Hz, 1H), 1.89 (dq, *J* = 12.2, 5.9 Hz, 1H), 1.30 (dd, *J* = 6.9, 2.7 Hz, 6H). NMR $^{13}C$ (101 MHz, DMSO-*d*$_6$) : δ (ppm) = 156.11, 151.45, 138.32, 134.47, 131.27, 129.17 (2C), 128.16 (2C), 126.31, 125.43, 72.74, 66.39, 51.74, 41.98, 31.85, 23.73, 20.59, 20.55. MS (ES+, m/z) : [M+H+] 387.29. M.p. 182 - 184 °C. |
| 21 | $C_{20}H_{26}N_6O$ | 97 % [a] | White solid. NMR $^1H$ (400 MHz, DMSO-*d*$_6$) : δ (ppm) = 8.70 (t, *J* = 6.4 Hz, 1H), 7.23 (d, *J* = 7.8 Hz, 2H), 7.13-7.07 (m, 3H), 6.58 (d, *J* = 5.6 Hz, 1H), 4.59 (d, *J* = 6.4 Hz, 2H), 4.24 - 4.10 (m, 1H), 3.87 (dd, *J* = 8.7, 6.1 Hz, 1H), 3.79 (q, *J* = 7.5 Hz, 1H), 3.68 (td, *J* = 8.0, 5.8 Hz, 1H), 3.54 (dd, *J* = 8.7, 4.5 Hz, 1H), 3.17 (hept, *J* = 6.9 Hz, 1H), 2.25 (s, 3H), 2.16 - 2.04 (m, 1H), 1.95 - 1.84 (m, 1H), 1.29 (dd, *J* = 6.9, 2.8 Hz, 6H). NMR $^{13}C$ (101 MHz, DMSO-*d*$_6$) : δ (ppm) = 156.18, 151.45, 136.23, 135.74, 134.40, 128.73 (2C), 127.28 (2C), 126.39, 125.31, 72.77, 66.40, 51.75, 42.31, 31.86, 23.73, 20.65, 20.60, 20.55. MS (ES+, m/z) : [M+H+] 367.33. M.p. 151 - 153 °C. |

(continued)

| N° | Formula | HPLC | Description |
|---|---|---|---|
| 22 | $C_{20}H_{26}N_6O_2$ | 98 % [a] | Pale yellow solid. NMR $^1$H (400 MHz, DMSO-$d_6$) : δ (ppm) = 8.68 (t, *J* = 6.4 Hz, 1H), 7.29 (d, *J* = 8.2 Hz, 2H), 7.10 (s, 1H), 6.86 (d, *J* = 8.2 Hz, 2H), 6.60 (d, *J* = 5.6 Hz, 1H), 4.56 (d, *J* = 6.4 Hz, 2H), 4.24 - 4.13 (m, 1H), 3.88 (dd, *J* = 8.9, 6.1 Hz, 1H), 3.80 (q, *J* = 7.5 Hz, 1H), 3.73-3.69 (m, 4H), 3.69 (d, *J* = 7.1 Hz, 3H), 3.55 (dd, *J* = 8.8, 4.5 Hz, 1H), 3.16 (hept, *J* = 6.9 Hz, 1H), 2.10 (dt, *J* = 14.7, 7.2 Hz, 1H), 1.91 (tt, *J* = 12.4, 5.4 Hz, 1H), 1.29 (dd, *J* = 6.9, 2.7 Hz, 6H). NMR $^{13}$C (101 MHz, DMSO-$d_6$) : δ (ppm) = 158.19, 156.19, 151.38, 134.39, 131.24, 128.74 (2C), 126.40, 125.30, 113.61 (2C), 72.79, 66.41, 55.02, 51.76, 42.02, 31.88, 23.73, 20.61, 20.56. MS (ES+, m/z) : [M+H+] 383.32. M.p. 142 - 144 °C. |
| 23 | $C_{19}H_{22}Cl_2N_6O$ | 95 % [a] | White solid. NMR $^1$H (400 MHz, DMSO-$d_6$) : δ (ppm) = 8.82 (t, *J* = 6.3 Hz, 1H), 7.60 (s, 1H), 7.58 (d, *J* = 8.3 Hz, 1H), 7.34 (d, *J* = 8.3 Hz, 1H), 7.13 (s, 1H), 6.64 (d, *J* = 5.7 Hz, 1H), 4.62 (d, *J* = 6.3 Hz, 2H), 4.24 - 4.11 (m, 1H), 3.85 (dd, *J* = 8.8, 6.1 Hz, 1H), 3.79 (q, *J* = 7.5 Hz, 1H), 3.68 (td, *J* = 8.0, 5.8 Hz, 1H), 3.53 (dd, *J* = 8.8, 4.5 Hz, 1H), 3.17 (hept, *J* = 6.9 Hz, 1H), 2.09 (dq, *J* = 12.6, 7.5 Hz, 1H), 1.88 (tt, *J* = 12.3, 5.6 Hz, 1H), 1.30 (dd, *J* = 7.0, 2.7 Hz, 6H). NMR $^{13}$C (101 MHz, DMSO-$d_6$) : δ (ppm) = 156.05, 151.41, 140.58, 134.53, 130.77, 130.46, 129.30, 129.28, 127.71, 126.25, 125.54, 72.72, 66.39, 51.74, 41.68, 31.87, 23.72, 20.59, 20.54. MS (ES+, m/z) : [M+H+] 421.25. M.p. 151 - 153 °C. |
| 24 | $C_{19}H_{23}FN_6O$ | 97 % [a] | White solid. NMR $^1$H (400 MHz, DMSO-$d_6$) : δ (ppm) = 8.77 (t, *J* = 6.4 Hz, 1H), 7.39 (dd, *J* = 8.6, 5.7 Hz, 2H), 7.17 - 7.08 (m, 3H), 6.61 (d, *J* = 5.7 Hz, 1H), 4.61 (d, *J* = 6.4 Hz, 2H), 4.24 - 4.11 (m, 1H), 3.86 (dd, *J* = 8.8, 6.1 Hz, 1H), 3.79 (q, *J* = 7.6 Hz, 1H), 3.68 (td, *J* = 8.0, 5.8 Hz, 1H), 3.54 (dd, *J* = 8.8, 4.5 Hz, 1H), 3.17 (hept, *J* = 6.9 Hz, 1H), 2.16 - 2.04 (m, 1H), 1.95 - 1.84 (m, 1H), 1.29 (dd, *J* = 6.9, 2.7 Hz, 6H). NMR $^{13}$C (101 MHz, DMSO-$d_6$) : δ (ppm) = 161.15 (d, *J* = 242.2 Hz), 156.14, 151.42, 135.46 (d, *J* = 3.0 Hz), 134.45, 129.34 (d, *J* = 8.1 Hz) (2C), 126.33, 125.39, 114.92 (d, *J* = 21.3 Hz) (2C), 72.76, 66.40, 51.75, 41.91, 31.87, 23.73, 20.60, 20.55. MS (ES+, m/z) : [M+H+] 371.23. M.p. 159 - 161 °C |
| 25 | $C_{20}H_{23}F_3N_6O$ | 97 % [a] | White solid. NMR $^1$H (400 MHz, DMSO-$d_6$) : δ (ppm) = 8.87 (t, *J* = 6.3 Hz, 1H), 7.68 (d, *J* = 8.1 Hz, 2H), 7.55 (d, *J* = 8.1 Hz, 2H), 7.14 (s, 1H), 6.60 (d, *J* = 5.6 Hz, 1H), 4.72 (d, *J* = 6.3 Hz, 2H), 4.22 - 4.07 (m, 1H), 3.84 (dd, *J* = 8.8, 6.1 Hz, 1H), 3.78 (q, *J* = 7.6 Hz, 1H), 3.67 (td, *J* = 8.0, 5.8 Hz, 1H), 3.52 (dd, *J* = 8.8, 4.5 Hz, 1H), 3.17 (hept, *J* = 6.9 Hz, 1H), 2.08 (dq, *J* = 12.7, 7.5 Hz, 1H), 1.93 - 1.81 (m, 1H), 1.30 (dd, *J* = 6.9, 2.7 Hz, 6H). NMR $^{13}$C (101 MHz, DMSO-$d_6$) : δ (ppm) = 156.11, 151.54, 144.18, 134.53, 127.87 (2C), 127.28, 126.65 (d, *J* = 190.1 Hz), 126.30, 125.52 (2C), 125.14 (q, *J* = 3.8 Hz), 72.72, 66.39, 51.75, 42.33, 31.84, 23.75, 20.60, 20.56. MS (ES+, m/z) : [M+H+] 421.35. M.p. 187 - 189 °C. |
| 26 | $C_{18}H_{23}N_7O$ | 91 % [a] | Light brown solid. NMR $^1$H (400 MHz, DMSO-$d_6$) : δ (ppm) = 8.71 (t, *J* = 6.2 Hz, 1H), 8.51 (d, *J* = 4.3 Hz, 1H), 7.74 (td, *J* = 7.7, 1.8 Hz, 1H), 7.31 (d, *J* = 7.9 Hz, 1H), 7.26 (dd, *J* = 7.5, 4.9 Hz, 1H), 7.16 (s, 1H), 6.61 (d, *J* = 5.6 Hz, 1H), 4.76 (d, *J* = 6.2 Hz, 2H), 4.20 - 4.08 (m, 1H), 3.83 (dd, *J* = 8.8, 6.1 Hz, 1H), 3.77 (q, *J* = 7.5 Hz, 1H), 3.66 (td, *J* = 8.0, 5.8 Hz, 1H), 3.51 (dd, *J* = 8.8, 4.5 Hz, 1H), 3.19 (hept, *J* = 6.9 Hz, 1H), 2.07 (dq, *J* = 12.3, 7.5 Hz, 1H), 1.92 - 1.82 (m, 1H), 1.32 (dd, *J* = 6.9, 2.9 Hz, 6H). NMR $^{13}$C (101 MHz, DMSO-$d_6$) : δ (ppm) = 158.18, 156.12, 151.67, 148.81, 136.71, 134.49, 126.40, 125.49, 122.05, 120.69, 72.71, 66.37, 51.73, 44.52, 31.76, 23.75, 20.61, 20.56. MS (ES+, m/z) : [M+H+] 354.30. M.p. 52-54 °C. |

(continued)

| N° | Formula | HPLC | Description |
|---|---|---|---|
| 27 | $C_{18}H_{23}N_7O$ | 97 % [a] | Light brown solid. NMR $^1H$ (400 MHz, DMSO-$d_6$) : δ (ppm) = 8.83 (t, *J* = 6.3 Hz, 1H), 8.61 (d, *J* = 2.3 Hz, 1H), 8.44 (dd, *J* = 4.8, 1.6 Hz, 1H), 7.77 (d, *J* = 7.9 Hz, 1H), 7.34 (dd, *J* = 7.9, 4.8 Hz, 1H), 7.12 (s, 1H), 6.65 (d, *J* = 5.7 Hz, 1H), 4.64 (d, *J* = 6.3 Hz, 2H), 4.23 - 4.12 (m, 1H), 3.86 (dd, *J* = 8.8, 6.1 Hz, 1H), 3.80 (q, *J* = 7.5 Hz, 1H), 3.68 (td, *J* = 8.0, 5.8 Hz, 1H), 3.55 (dd, *J* = 8.8, 4.5 Hz, 1H), 3.17 (hept, *J* = 6.9 Hz, 1H), 2.11 (dq, *J* = 12.4, 7.5 Hz, 1H), 1.94 - 1.83 (m, 1H), 1.29 (dd, *J* = 7.0, 2.7 Hz, 6H). NMR $^{13}C$ (101 MHz, DMSO-$d_6$) : δ (ppm) = 156.06, 151.41, 149.08, 148.08, 135.29, 134.71, 134.48, 126.29, 125.46, 123.43, 72.74, 66.40, 51.75, 40.44, 31.88, 23.72, 20.58, 20.54. MS (ES+, m/z) : [M+H+] 354.33. M.p. 183 - 185 °C. |
| 28 | $C_{18}H_{23}N_7O$ | 97 % [a] | Light brown solid. NMR $^1H$ (400 MHz, DMSO-$d_6$) : δ (ppm) = 8.84 (t, *J* = 6.3 Hz, 1H), 8.48 (d, *J* = 6.1 Hz, 2H), 7.31 (d, *J* = 6.1 Hz, 2H), 7.15 (s, 1H), 6.60 (d, *J* = 5.7 Hz, 1H), 4.66 (d, *J* = 6.3 Hz, 2H), 4.18 - 4.09 (m, 1H), 3.82 (dd, *J* = 8.8, 6.1 Hz, 1H), 3.77 (q, *J* = 7.5 Hz, 1H), 3.66 (td, *J* = 8.0, 5.8 Hz, 1H), 3.51 (dd, *J* = 8.8, 4.5 Hz, 1H), 3.18 (hept, *J* = 6.9 Hz, 1H), 2.07 (dq, *J* = 12.4, 7.5 Hz, 1H), 1.91 - 1.81 (m, 1H), 1.30 (dd, *J* = 6.9, 2.8 Hz, 6H). NMR $^{13}C$ (101 MHz, DMSO-$d_6$) : δ (ppm) = 156.06, 151.62, 149.51 (2C), 148.24, 134.54, 126.27, 125.57, 122.12 (2C), 72.71, 66.39, 51.74, 41.81, 31.81, 23.75, 20.60, 20.56. MS (ES+, m/z) : [M+H+] 354.30. M.p. 192 - 194 °C. |
| 29 | $C_{17}H_{22}N_8O$ | 98 % [a] | Beige powder. NMR $^1H$ (400 MHz, DMSO-$d_6$) : δ (ppm) = 8.80 (t, *J* = 6.1 Hz, 1H), 8.65 (d, *J* = 1.5 Hz, 1H), 8.58 (t, *J* = 2.0 Hz, 1H), 8.53 (d, *J* = 2.5 Hz, 1H), 7.15 (s, 1H), 6.62 (d, *J* = 5.6 Hz, 1H), 4.79 (d, *J* = 6.1 Hz, 2H), 4.18-4.09 (m, 1H), 3.85 - 3.73 (m, 2H), 3.70 - 3.62 (m, 1H), 3.50 (dd, *J* = 8.8, 4.5 Hz, 1H), 3.18 (hept, *J* = 6.9 Hz, 1H), 2.13 - 1.99 (m, 1H), 1.86 (dq, *J* = 12.2, 5.8 Hz, 1H), 1.30 (dd, *J* = 6.9, 2.7 Hz, 6H). NMR $^{13}C$ (101 MHz, DMSO-$d_6$) : δ (ppm) = 156.00, 153.85, 151.62, 143.90, 143.40, 143.15, 134.51, 126.32, 125.58, 72.68, 66.37, 51.72, 42.81, 31.79, 23.73, 20.59, 20.54. MS (ES+, m/z) : [M+H+] 355.31. M.p. 184 - 186 °C. |
| 30 | $C_{20}H_{26}N_6O$ | 99 % [a] | White solid. NMR $^1H$ (400 MHz, DMSO-$d_6$) : δ (ppm) = 8.65 (d, *J* = 8.5 Hz, 1H), 7.44 (d, *J* = 7.3 Hz, 2H), 7.31 (dd, *J* = 8.4, 6.8 Hz, 2H), 7.24 - 7.17 (m, 1H), 7.12 (s, 1H), 6.54 (d, *J* = 5.7 Hz, 1H), 5.43 (p, *J* = 7.2 Hz, 1H), 4.22 - 4.10 (m, 1H), 3.88 (dd, *J* = 8.7, 6.1 Hz, 1H), 3.78 (q, *J* = 7.5 Hz, 1H), 3.68 (td, *J* = 8.0, 5.8 Hz, 1H), 3.54 (dd, *J* = 8.7, 4.6 Hz, 1H), 3.16 (hept, *J* = 6.9 Hz, 1H), 2.06 (dq, *J* = 12.5, 7.5 Hz, 1H), 1.90 - 1.80 (m, 1H), 1.55 (d, *J* = 7.2 Hz, 3H), 1.29 (dd, *J* = 8.3, 6.9 Hz, 6H). NMR $^{13}C$ (101 MHz, DMSO-$d_6$) : δ (ppm) = 156.12, 150.71, 144.36, 134.42, 128.17 (2C), 126.65, 126.29, 126.26 (2C), 125.19, 72.70, 66.39, 51.73, 48.42, 31.91, 23.70, 21.82, 20.63, 20.53. MS (ES+, m/z) : [M+H+] 367.37. M.p. 135 - 137 °C. |
| 31 | $C_{20}H_{26}N_6O$ | 99 % [a] | White solid. NMR $^1H$ (400 MHz, DMSO-$d_6$) : δ (ppm) = 8.65 (d, *J* = 8.5 Hz, 1H), 7.43 (d, *J* = 7.3 Hz, 2H), 7.31 (t, *J* = 7.5 Hz, 2H), 7.21 (t, *J* = 7.3 Hz, 1H), 7.12 (s, 1H), 6.54 (d, *J* = 5.7 Hz, 1H), 5.44 (p, *J* = 7.1 Hz, 1H), 4.22-4.11 (m, 1H), 3.86 - 3.74 (m, 2H), 3.68 (td, *J* = 8.0, 5.8 Hz, 1H), 3.48 (dd, *J* = 8.8, 4.4 Hz, 1H), 3.16 (hept, *J* = 6.9 Hz, 1H), 2.16 - 2.05 (m, 1H), 1.94 - 1.84 (m, 1H), 1.55 (d, *J* = 7.1 Hz, 3H), 1.29 (dd, *J* = 6.9, 3.0 Hz, 6H). NMR $^{13}C$ (101 MHz, DMSO-$d_6$) : δ (ppm) = 156.12, 150.71, 144.35, 134.41, 128.17 (2C), 126.65, 126.27, 126.23 (2C), 125.18, 72.80, 66.38, 51.73, 48.41, 31.83, 23.70, 21.82, 20.58 (2C). MS (ES+, m/z) : [M+H+] 367.37. M.p. 128-130 °C. |

(continued)

| N° | Formula | HPLC | Description |
|---|---|---|---|
| 32 | $C_{20}H_{26}N_6O$ | > 99 % [a] | Pale yellow paste. NMR spectrum shows conformers, described below. NMR $^1$H (400 MHz, DMSO-$d_6$, 70°C) : δ (ppm) = 7.37 - 7.23 (m, 5H), 7.16 (s, 1H), 6.30 (d, *J* = 5.9 Hz, 1H), 5.70 (s, 0.8H), 5.44 (br. s, 1.2H), 4.29 - 4.20 (m, 1H), 3.90 (dd, *J* = 8.8, 6.2 Hz, 1H), 3.83 (q, *J* = 7.6 Hz, 1H), 3.74 - 3.67 (m, 1H), 3.59 (dd, *J* = 8.8, 4.6 Hz, 1H), 3.39 (br. s, 3H), 3.28 - 3.19 (m, 1H), 2.21 - 2.09 (m, 1H), 1.92 (dq, *J* = 12.2, 5.8 Hz, 1H), 1.32 (dd, *J* = 6.9, 2.3 Hz, 6H). NMR $^{13}$C (101 MHz, $CDCl_3$) : δ (ppm) = 155.54, 151.78, 137.19, 135.79, 128.79 (2C), 127.80 (2C), 127.59, 127.35, 125.70, 74.09, 67.19, 54.99 (0.5C), 52.93 (0.5C), 52.46, 37.95 (0.5C), 35.75 (0.5C), 33.42, 24.46, 20.77, 20.74. MS (ES+, m/z) : [M+H+] 367.40. |
| 33 | $C_{16}H_{18}N_6O$ | > 99 % [a] | White solid. NMR $^1$H (400 MHz, DMSO-$d_6$) : δ (ppm) = 8.86 (t, *J* = 6.2 Hz, 1H), 7.71 (d, *J* = 1.0 Hz, 1H), 7.38-7.27 (m, 5H), 7.26 - 7.19 (m, 1H), 6.61 (d, *J* = 6.2 Hz, 1H), 4.65 (d, *J* = 6.4 Hz, 2H), 4.23 - 4.13 (m, 1H), 3.84-3.75 (m, 2H), 3.71 - 3.63 (m, 1H), 3.53 (dd, *J* = 8.8, 4.3 Hz, 1H), 2.08 (dq, *J* = 12.5, 7.6 Hz, 1H), 1.91 - 1.80 (m, 1H). NMR $^{13}$C (101 MHz, DMSO-$d_6$) : δ (ppm) = 156.60, 151.35, 139.18, 129.54, 128.26 (2C), 127.32 (2C), 126.79, 126.56, 117.09, 72.66, 66.39, 51.51, 42.64, 32.02. MS (ES+, m/z) : [M+H+] 311.23. M.p. 172 - 174 °C. |
| 34 | $C_{16}H_{17}BrN_6O$ | 99 % [a] | White solid. NMR $^1$H (400 MHz, DMSO-$d_6$) : δ (ppm) = 9.01 (t, *J* = 6.4 Hz, 1H), 7.45 (s, 1H), 7.38 - 7.28 (m, 4H), 7.26 - 7.20 (m, 1H), 6.91 (d, *J* = 5.9 Hz, 1H), 4.66 (d, *J* = 6.3 Hz, 2H), 4.27 - 4.18 (m, 1H), 3.85 (dd, *J* = 8.7, 6.1 Hz, 1H), 3.79 (q, *J* = 7.5 Hz, 1H), 3.72 - 3.64 (m, 1H), 3.50 (dd, *J* = 8.8, 4.6 Hz, 1H), 2.15 - 2.03 (m, 1H), 1.92-1.82 (m, 1H). NMR $^{13}$C (101 MHz, DMSO-$d_6$) : δ (ppm) = 157.13, 151.19, 138.93, 129.52, 128.25 (2C), 127.34, 127.29 (2C), 126.81, 99.97, 72.65, 66.38, 51.65, 42.83, 31.85. MS (ES+, m/z) : [M+H+] 389.13. M.p. 137 - 139 °C. |
| 35 | $C_{17}H_{20}N_6O$ | > 99 % [a] | White solid. NMR $^1$H (400 MHz, DMSO-$d_6$) : δ (ppm) = 8.74 (t, *J* = 6.4 Hz, 1H), 7.38 - 7.27 (m, 4H), 7.26 - 7.18 (m, 1H), 7.14 (s, 1H), 6.60 (d, *J* = 5.9 Hz, 1H), 4.65 (d, *J* = 6.4 Hz, 2H), 4.27 - 4.17 (m, 1H), 3.85 (dd, *J* = 8.8, 6.2 Hz, 1H), 3.79 (q, *J* = 7.5 Hz, 1H), 3.71 - 3.63 (m, 1H), 3.50 (dd, *J* = 8.8, 4.6 Hz, 1H), 2.29 (s, 3H), 2.14 - 2.03 (m, 1H), 1.92 - 1.81 (m, 1H). NMR $^{13}$C (101 MHz, DMSO-$d_6$) : δ (ppm) = 156.51, 151.37, 139.31, 128.21 (2C), 127.70, 127.24 (2C), 126.71, 126.15, 124.49, 72.76, 66.38, 51.65, 42.61, 31.93, 8.21. MS (ES+, m/z) : [M+H+] 325.35. M.p. 150 - 152 °C. |
| 36 | $C_{18}H_{22}N_6O$ | 99 %[a] | White solid. NMR $^1$H (400 MHz, DMSO-$d_6$) : δ (ppm) = 8.75 (t, *J* = 6.4 Hz, 1H), 7.39 - 7.27 (m, 4H), 7.26 - 7.18 (m, 1H), 7.14 (s, 1H), 6.60 (d, *J* = 5.8 Hz, 1H), 4.65 (d, *J* = 6.4 Hz, 2H), 4.25 - 4.15 (m, 1H), 3.85 (dd, *J* = 8.8, 6.1 Hz, 1H), 3.79 (q, *J* = 7.5 Hz, 1H), 3.72 - 3.63 (m, 1H), 3.52 (dd, *J* = 8.8, 4.6 Hz, 1H), 2.73 (q, *J* = 7.5 Hz, 2H), 2.15 - 2.03 (m, 1H), 1.93 - 1.83 (m, 1H), 1.24 (t, *J* = 7.5 Hz, 3H). NMR $^{13}$C (101 MHz, DMSO-$d_6$): δ (ppm) = 156.36, 151.44, 139.30, 130.22, 128.21 (2C), 127.24 (2C), 126.72, 126.66, 126.22, 72.75, 66.38, 51.69, 42.59, 31.88, 16.41, 11.98. MS (ES+, m/z) : [M+H+] 339.38. M.p. 138 - 140 °C. |
| 37 | $C_{19}H_{22}N_6O$ | > 99 %[a] | White solid. NMR $^1$H (400 MHz, DMSO-$d_6$) : δ (ppm) = 8.74 (t, *J* = 6.3 Hz, 1H), 7.36 - 7.27 (m, 4H), 7.25 - 7.19 (m, 1H), 7.03 (s, 1H), 6.61 (d, *J* = 5.8 Hz, 1H), 4.64 (d, *J* = 6.3 Hz, 2H), 4.27 - 4.13 (m, 1H), 3.86 (dd, *J* = 8.7, 6.1 Hz, 1H), 3.79 (q, *J* = 7.5 Hz, 1H), 3.71 - 3.63 (m, 1H), 3.51 (dd, *J* = 8.7, 4.6 Hz, 1H), 2.15 - 2.05 (m, 1H), 2.05 - 1.96 (m, 1H), 1.92 - 1.82 (m, 1H), 0.96 - 0.90 (m, 2H), 0.84 - 0.78 (m, 2H). NMR $^{13}$C (101 MHz, DMSO-$d_6$) : δ (ppm) = 156.41, 151.33, 139.30, 130.85, 128.20 (2C), 127.24 (2C), 126.71, 126.19, 125.69, 72.79, 66.39, 51.70, 42.60, 31.92, 6.11, 6.02, 4.77. MS (ES+, m/z) : [M+H+] 351.37. M.p. 134 - 136 °C. |

(continued)

| N° | Formula | HPLC | Description |
|---|---|---|---|
| 38 | $C_{21}H_{26}N_6O$ | 98 %[a] | White solid. NMR $^1H$ (400 MHz, DMSO-$d_6$) : δ (ppm) = 8.73 (t, *J* = 6.4 Hz, 1H), 7.39 - 7.26 (m, 4H), 7.26 - 7.18 (m, 1H), 7.14 (d, *J* = 0.7 Hz, 1H), 6.58 (d, *J* = 5.6 Hz, 1H), 4.64 (d, *J* = 6.4 Hz, 2H), 4.22 - 4.12 (m, 1H), 3.85 (dd, *J* = 8.8, 6.1 Hz, 1H), 3.78 (q, *J* = 7.4 Hz, 1H), 3.71 - 3.64 (m, 1H), 3.54 (dd, *J* = 8.7, 4.5 Hz, 1H), 3.30 - 3.20 (m, 1H), 2.15 - 1.99 (m, 3H), 1.94 - 1.84 (m, 1H), 1.80 - 1.59 (m, 6H). NMR $^{13}C$ (101 MHz, DMSO-$d_6$) : δ (ppm) = 156.15, 151.48, 139.32, 132.56, 128.23 (2C), 127.28 (2C), 126.74, 126.48, 125.89, 72.79, 66.42, 51.77, 42.58, 34.49, 31.85, 30.78, 30.70, 24.80 (2C). MS (ES+, m/z) : [M+H+] 379.29. M.p. 181 - 183 °C. |
| 39 | $C_{22}H_{28}N_6O$ | 99 %[a] | White solid. NMR $^1H$ (400 MHz, DMSO-$d_6$) : δ (ppm) = 8.75 (t, *J* = 6.4 Hz, 1H), 7.38 - 7.26 (m, 4H), 7.25 - 7.18 (m, 1H), 7.10 (s, 1H), 6.59 (d, *J* = 5.5 Hz, 1H), 4.64 (d, *J* = 6.4 Hz, 2H), 4.21 - 4.10 (m, 1H), 3.86 (dd, *J* = 8.8, 6.1 Hz, 1H), 3.78 (q, *J* = 7.5 Hz, 1H), 3.72 - 3.63 (m, 1H), 3.53 (dd, *J* = 8.8, 4.6 Hz, 1H), 2.92 - 2.81 (m, 1H), 2.14 - 2.04 (m, 1H), 2.06 - 1.97 (m, 2H), 1.94 - 1.83 (m, 1H), 1.78 (d, *J* = 12.4 Hz, 2H), 1.70 (d, *J* = 12.1 Hz, 1H), 1.53 - 1.31 (m, 4H), 1.31 - 1.18 (m, 1H). NMR $^{13}C$ (101 MHz, DMSO-$d_6$): δ (ppm) = 156.19, 151.51, 139.30, 133.59, 128.20 (2C), 127.26 (2C), 126.71, 126.16, 125.62, 72.86, 66.41, 51.83, 42.60, 33.10, 31.86, 30.64, 30.51, 25.80, 25.77, 25.73. MS (ES+, m/z) : [M+H+] 393.40. M.p. 173 - 175 °C. |
| 40 | $C_{22}H_{22}N_6O$ | 99 %[a] | Beige solid. NMR $^1H$ (400 MHz, DMSO-$d_6$) : δ (ppm) = 8.92 (t, *J* = 6.4 Hz, 1H), 8.17 (d, *J* = 7.8 Hz, 2H), 7.83 (s, 1H), 7.48 (t, *J* = 7.7 Hz, 2H), 7.39 (d, *J* = 7.5 Hz, 2H), 7.33 (t, *J* = 7.5 Hz, 3H), 7.24 (t, *J* = 7.2 Hz, 1H), 6.79 (d, *J* = 5.6 Hz, 1H), 4.70 (d, *J* = 6.4 Hz, 2H), 4.29 - 4.19 (m, 1H), 3.90 (dd, *J* = 8.8, 6.1 Hz, 1H), 3.81 (q, *J* = 7.5 Hz, 1H), 3.71 (q, *J* = 8.1, 1H), 3.63 (dd, *J* = 8.9, 4.2 Hz, 1H), 2.14 (dq, *J* = 12.4, 7.6 Hz, 1H), 2.00 - 1.88 (m, 1H). NMR $^{13}C$ (101 MHz, DMSO-$d_6$) : δ (ppm) = 156.60, 151.67, 139.15, 129.03, 128.59, 128.49 (2C), 128.25 (2C), 127.60, 127.44, 127.29 (2C), 126.77, 126.03 (2C), 72.81, 66.45, 51.85, 42.67, 40.19, 31.95. MS (ES+, m/z) : [M+H+] 387.29. M.p. 168 - 170 °C. |
| 41 | $C_{20}H_{26}N_6O$ | 98 %[a] | White solid. NMR $^1H$ (400 MHz, DMSO-$d_6$) : δ (ppm) = 8.76 (t, *J* = 5.8 Hz, 1H), 7.38 - 7.27 (m, 4H), 7.25 - 7.19 (m, 1H), 7.11 (s, 1H), 6.21 (d, *J* = 7.6 Hz, 1H), 4.63 (d, *J* = 6.4 Hz, 2H), 3.98 - 3.86 (m, 1H), 3.80 - 3.61 (m, 2H), 3.29 - 3.20 (m, 1H), 3.16 (hept, *J* = 6.9 Hz, 1H), 3.05 (t, *J* = 10.0 Hz, 1H), 1.97 - 1.86 (m, 1H), 1.71 - 1.62 (m, 1H), 1.60 - 1.44 (m, 2H), 1.29 (dd, *J* = 6.9, 2.8 Hz, 6H). NMR $^{13}C$ (101 MHz, DMSO-$d_6$) : δ (ppm) = 155.85, 151.54, 139.32, 134.38, 128.21 (2C), 127.34 (2C), 126.74, 126.39, 125.41, 70.62, 67.04, 47.02, 42.62, 29.05, 24.85, 23.68, 20.72, 20.64. MS (ES+, m/z) : [M+H+] 367.40. M.p. 129 - 131 °C. |
| 42 | $C_{19}H_{25}N_7O$ | 94 %[a] | Beige solid. NMR $^1H$ (400 MHz, DMSO-$d_6$) : δ (ppm) = 8.83 (t, *J* = 6.3 Hz, 1H), 8.60 (s, 1H), 8.46 - 8.40 (m, 1H), 7.77 (d, *J* = 7.9 Hz, 1H), 7.33 (dd, *J* = 7.9, 4.8 Hz, 1H), 7.12 (s, 1H), 6.26 (d, *J* = 7.7 Hz, 1H), 4.63 (d, *J* = 6.3 Hz, 2H), 3.92 (dd, *J* = 10.3, 4.2 Hz, 1H), 3.79 - 3.61 (m, 2H), 3.29 - 3.21 (m, 1H), 3.16 (hept, *J* = 6.9 Hz, 1H), 3.07 (t, *J* = 9.9 Hz, 1H), 1.98 - 1.87 (m, 1H), 1.71 - 1.63 (m, 1H), 1.60 - 1.45 (m, 2H), 1.28 (dd, *J* = 6.9, 2.9 Hz, 6H). NMR $^{13}C$ (101 MHz, DMSO-$d_6$) : δ (ppm) = 155.74, 151.45, 149.08, 148.08, 135.32, 134.73, 134.44, 126.30, 125.50, 123.42, 70.57, 67.05, 47.02, 40.47, 29.03, 24.78, 23.67, 20.68, 20.60. MS (ES+, m/z) : [M+H+] 368.41. M.p. 185 - 187 °C. |

(continued)

| N° | Formula | HPLC | Description |
|---|---|---|---|
| 43 | $C_{19}H_{25}N_7O$ | 95 % [a] | Beige solid. NMR $^1$H (400 MHz, DMSO-*d6*) : δ (ppm) = 8.84 (t, *J* = 6.3 Hz, 1H), 8.48 (d, *J* = 6.1 Hz, 2H), 7.31 (d, *J* = 6.1 Hz, 2H), 7.14 (s, 1H), 6.21 (d, *J* = 7.7 Hz, 1H), 4.64 (d, *J* = 6.3 Hz, 2H), 3.93 - 3.82 (m, 1H), 3.76 - 3.69 (m, 1H), 3.68 - 3.58 (m, 1H), 3.28 - 3.10 (m, 2H), 3.03 (t, *J* = 9.9 Hz, 1H), 1.93 - 1.82 (m, 1H), 1.69 - 1.60 (m, 1H), 1.58 - 1.42 (m, 2H), 1.29 (dd, *J* = 6.9, 2.8 Hz, 6H). NMR $^{13}$C (101 MHz, DMSO-*d6*) : δ (ppm) = 155.73, 151.65, 149.49 (2C), 148.24, 134.48, 126.27, 125.59, 122.15 (2C), 70.52, 67.02, 47.00, 41.85, 28.98, 24.78, 23.68, 20.68, 20.61. MS (ES+, m/z) : [M+H+] 368.41. M.p. 169 - 171 °C. |
| 44 | $C_{20}H_{26}N_6O$ | 98 %[a] | White powder. NMR $^1$H (400 MHz, DMSO-*d6*) : δ (ppm) = 8.75 (t, *J* = 6.3 Hz, 1H), 7.38 - 7.26 (m, 4H), 7.21 (t, *J* = 7.1 Hz, 1H), 7.11 (s, 1H), 6.29 (d, *J* = 7.6 Hz, 1H), 4.63 (d, *J* = 6.3 Hz, 2H), 3.84 (d, *J* = 11.8, 2H), 3.77 - 3.66 (m, 1H), 3.16 (hept, *J* = 6.9 Hz, 1H), 1.81 (d, *J* = 12.8 Hz, 2H), 1.45 (qd, *J* = 15.0, 13.3, 5.9 Hz, 3H), 1.29 (d, *J* = 6.9 Hz, 6H). NMR $^{13}$C (101 MHz, DMSO-*d6*) : δ (ppm) = 155.75, 151.52, 139.38, 134.33, 128.19 (2C), 127.22 (2C), 126.69, 126.40, 125.36, 66.21 (2C), 47.17, 42.67, 32.53 (2C), 23.70, 20.67 (2C). MS (ES+, m/z) : [M+H+] 367.33. M.p. 143 - 145 °C. |
| 45 | $C_{20}H_{25}FN_6O$ | 97 %[a] | White solid. NMR $^1$H (400 MHz, DMSO-*d6*) : δ (ppm) = 8.76 (t, *J* = 6.3 Hz, 1H), 7.39 (dd, *J* = 8.5, 5.7 Hz, 2H), 7.17 - 7.04 (m, 3H), 6.31 (d, *J* = 7.6 Hz, 1H), 4.61 (d, *J* = 6.3 Hz, 2H), 3.84 (d, *J* = 11.7 Hz, 2H), 3.78 - 3.64 (m, 1H), 3.38 - 3.27 (m, 2H), 3.15 (hept, *J* = 6.9 Hz, 1H), 1.82 (d, *J* = 11.7 Hz, 2H), 1.46 (qd, *J* = 11.7, 4.4 Hz, 2H), 1.28 (d, *J* = 6.9 Hz, 6H). NMR $^{13}$C (101 MHz, DMSO-*d6*) : δ (ppm) = 161.12 (d, *J* = 242.0 Hz), 155.71, 151.44, 135.53 (d, *J* = 3.0 Hz), 134.34, 129.26 (d, *J* = 8.1 Hz) (2C), 126.36, 125.38, 114.89 (d, *J* = 21.3 Hz) (2C), 66.18 (2C), 47.17, 42.00, 32.53 (2C), 23.68, 20.64 (2C). MS (ES+, m/z) : [M+H+] 385.37. M.p. 152 - 154 °C. |
| 46 | $C_{21}H_{25}F_3N_6O$ | 98 %[a] | White solid. NMR $^1$H (400 MHz, DMSO-*d6*) : δ (ppm) = 8.87 (t, *J* = 6.3 Hz, 1H), 7.68 (d, *J* = 7.9 Hz, 2H), 7.54 (d, *J* = 7.9 Hz, 2H), 7.13 (d, *J* = 1.4 Hz, 1H), 6.31 (d, *J* = 7.5 Hz, 1H), 4.71 (d, *J* = 6.3 Hz, 2H), 3.81 (d, *J* = 11.5 Hz, 2H), 3.72 - 3.60 (m, 1H), 3.32 - 3.23 (m, 2H), 3.16 (hept, *J* = 6.9 Hz, 1H), 1.75 (d, *J* = 12.6 Hz, 2H), 1.49 - 1.36 (m, 2H), 1.29 (dd, *J* = 6.9, 1.5 Hz, 6H). NMR $^{13}$C (101 MHz, DMSO-*d6*) : δ (ppm) = 155.70, 151.55, 144.30, 134.43, 127.74 (2C), 127.21, 126.62 (d, *J* = 182.7 Hz), 126.32, 125.52 (2C), 125.13 (q, *J* = 3.7 Hz), 66.17 (2C), 47.18, 42.48, 32.50 (2C), 23.70, 20.67 (2C). MS (ES+, m/z) : [M+H+] 435.36. M.p. 170 - 172 °C. |
| 47 | $C_{21}H_{28}N_6O$ | 99 %[a] | White solid. NMR $^1$H (400 MHz, DMSO-*d6*) : δ (ppm) = 8.64 (d, *J* = 8.3 Hz, 1H), 7.43 (d, *J* = 7.3 Hz, 2H), 7.30 (dd, *J* = 8.4, 6.8 Hz, 2H), 7.23 - 7.16 (m, 1H), 7.11 (s, 1H), 6.25 (d, *J* = 7.7 Hz, 1H), 5.39 (p, *J* = 7.1 Hz, 1H), 3.91 - 3.77 (m, 2H), 3.77 - 3.64 (m, 1H), 3.42 - 3.28 (m, 2H), 3.14 (hept, *J* = 6.9 Hz, 1H), 1.89 - 1.77 (m, 1H), 1.69 (d, *J* = 12.5 Hz, 1H), 1.54 (d, *J* = 7.1 Hz, 3H), 1.51 - 1.43 (m, 1H), 1.42 - 1.32 (m, 1H), 1.27 (dd, *J* = 6.9, 5.4 Hz, 6H). NMR $^{13}$C (101 MHz, DMSO-*d6*) : δ (ppm) = 155.73, 150.75, 144.61, 134.31, 128.15 (2C), 126.60, 126.30, 126.17 (2C), 125.19, 66.27, 66.19, 48.71, 47.15, 32.65, 32.50, 23.65, 22.01, 20.70, 20.64. MS (ES+, m/z) : [M+H+] 381.41. M.p. 75 - 77 °C. |
| 48 | $C_{21}H_{28}N_6O$ | 98 %[a] | White solid. NMR $^1$H (400 MHz, DMSO-*d6*) : δ (ppm) = 8.64 (d, *J* = 8.3 Hz, 1H), 7.43 (d, *J* = 7.2 Hz, 2H), 7.30 (t, *J* = 7.5 Hz, 2H), 7.20 (t, *J* = 7.3 Hz, 1H), 7.11 (s, 1H), 6.25 (d, *J* = 7.6 Hz, 1H), 5.39 (p, *J* = 7.1 Hz, 1H), 3.90 - 3.77 (m, 2H), 3.76 - 3.64 (m, 1H), 3.40 - 3.27 (m, 2H), 3.14 (hept, *J* = 6.9 Hz, 1H), 1.83 (dq, *J* = 12.8, 2.3 Hz, 1H), 1.75 - 1.61 (m, 1H), 1.54 (d, *J* = 7.1 Hz, 3H), 1.52 - 1.43 (m, 1H), 1.43 - 1.32 (m, 1H), 1.27 (dd, *J* = 6.9, 5.4 Hz, 6H). NMR $^{13}$C (101 MHz, DMSO-*d6*) : δ (ppm) = 155.73, 150.75, 144.61, 134.30, 128.14 (2C), 126.59, 126.30, 126.17 (2C), 125.18, 66.26, 66.18, 48.70, 47.14, 32.65, 32.49, 23.64, 21.99, 20.69, 20.64. MS (ES+, m/z) : [M+H+] 381.37. M.p. 68 - 70 °C. |

(continued)

| N° | Formula | HPLC | Description |
|---|---|---|---|
| 49 | $C_{21}H_{28}N_6O$ | 97 %[a] | Brown paste. NMR spectrum shows conformers, described below. NMR $^1H$ (400 MHz, DMSO-$d_6$) : δ (ppm) = 7.37 - 7.20 (m, 5H), 7.16 (s, 1H), 6.29 (br. s, 1H), 5.74 (br. s, 0.8H), 4.97 (br. s, 1.2H), 3.97 - 3.60 (m, 4.2H), 3.37 (m under $H_2O$, 2H), 3.18 (hept, *J* = 6.9 Hz, 1H), 3.08 (s, 1.8H), 1.87 (br. s, 2H), 1.49 (br. s, 2H), 1.29 (d, *J* = 6.9 Hz, 6H). NMR $^{13}C$ (101 MHz, DMSO): δ (ppm) = 155.20, 151.40, 137.88, 134.19, 128.53 (2C), 127.27 (2C), 127.09, 126.94, 125.48, 66.17 (2C), 53.68, 47.17, 35.06, 32.58 (2C), 23.80, 20.50 (2C). MS (ES+, m/z) : [M+H+] 381.41. |
| 50 | $C_{19}H_{25}N_7O$ | 94 %[a] | Beige solid. NMR $^1H$ (400 MHz, DMSO-$d_6$) : δ (ppm) = 8.69 (t, *J* = 6.2 Hz, 1H), 8.50 (d, *J* = 4.9 Hz, 1H), 7.73 (td, *J* = 7.8, 1.9 Hz, 1H), 7.29 (d, *J* = 7.8 Hz, 1H), 7.25 (dd, *J* = 7.5, 4.9 Hz, 1H), 7.14 (s, 1H), 6.30 (d, *J* = 7.5 Hz, 1H), 4.74 (d, *J* = 6.2 Hz, 2H), 3.84 - 3.76 (m, 2H), 3.71 - 3.59 (m, 1H), 3.23 - 3.25 (m under $H_2O$, 2H), 3.17 (p, *J* = 6.9 Hz, 1H), 1.81 - 1.71 (m, 2H), 1.50 - 1.34 (m, 2H), 1.30 (d, *J* = 6.9 Hz, 6H). NMR $^{13}C$ (101 MHz, DMSO-$d_6$) : δ (ppm) = 158.28, 155.67, 151.69, 148.79, 136.65, 134.38, 126.41, 125.47, 121.99, 120.65, 66.15 (2C), 47.14, 44.61, 32.41 (2C), 23.70, 20.65 (2C). MS (ES+, m/z) : [M+H+] 368.41. M.p. 120 - 122 °C. |
| 51 | $C_{19}H_{25}N_7O$ | 98 %[a] | Beige powder. NMR $^1H$ (400 MHz, DMSO-$d_6$) : δ (ppm) = 8.82 (t, *J* = 6.1 Hz, 1H), 8.60 (d, *J* = 2.2 Hz, 1H), 8.44 (dd, *J* = 4.8, 1.7 Hz, 1H), 7.79 - 7.72 (m, 1H), 7.33 (dd, *J* = 7.9, 4.8 Hz, 1H), 7.12 (s, 1H), 6.35 (d, *J* = 7.6 Hz, 1H), 4.63 (d, *J* = 6.1 Hz, 2H), 3.84 (d, *J* = 11.3 Hz, 2H), 3.77 - 3.66 (m, 1H), 3.39 - 3.31 (m, 2H), 3.15 (hept, *J* = 6.9 Hz, 1H), 1.81 (d, *J* = 12.7 Hz, 2H), 1.46 (qd, *J* = 11.5, 4.4 Hz, 2H), 1.28 (d, *J* = 6.9 Hz, 6H). NMR $^{13}C$ (101 MHz, DMSO-$d_6$) : δ (ppm) = 155.64, 151.44, 149.02, 148.05, 135.19, 134.79, 134.38, 126.31, 125.46, 123.41, 66.17 (2C), 47.16, 40.53, 32.52 (2C), 23.67, 20.64 (2C). MS (ES+, m/z) : [M+H+] 368.37. M.p. 204 - 206 °C. |
| 52 | $C_{19}H_{25}N_7O$ | > 99 % [a] | Pale yellow solid. NMR $^1H$ (400 MHz, DMSO-$d_6$) : δ (ppm) = 8.84 (t, *J* = 6.3 Hz, 1H), 8.49 (d, *J* = 5.0 Hz, 2H), 7.31 (d, *J* = 5.0 Hz, 2H), 7.15 (s, 1H), 6.31 (d, *J* = 7.5 Hz, 1H), 4.65 (d, *J* = 6.3 Hz, 2H), 3.82 (d, *J* = 11.5 Hz, 2H), 3.72 - 3.60 (m, 1H), 3.32 - 3.21 (m, 2H), 3.17 (hept, *J* = 6.9 Hz, 1H), 1.76 (d, *J* = 12.7 Hz, 2H), 1.50- 1.36 (m, 2H), 1.30 (d, *J* = 6.9 Hz, 6H). NMR $^{13}C$ (101 MHz, DMSO-$d_6$) : δ (ppm) = 155.63, 151.61, 149.47 (2C), 148.35, 134.42, 125.55, 122.05 (2C), 66.15 (2C), 47.16, 41.92, 32.47 (2C), 23.68, 20.64 (2C). MS (ES+, m/z) : [M+H+] 368.37. M.p. 197 - 199 °C. |
| 53 | $C_{18}H_{24}N_8O$ | 96 %[a] | Beige powder. NMR $^1H$ (400 MHz, DMSO-$d_6$) : δ (ppm) = 8.79 (t, *J* = 6.1 Hz, 1H), 8.65 (d, *J* = 1.5 Hz, 1H), 8.58 (dd, *J* = 2.5, 1.5 Hz, 1H), 8.52 (d, *J* = 2.5 Hz, 1H), 7.14 (s, 1H), 6.33 (d, *J* = 7.6 Hz, 1H), 4.78 (d, *J* = 6.1 Hz, 2H), 3.81 (d, *J* = 11.3 Hz, 2H), 3.71 - 3.58 (m, 1H), 3.36 - 3.25 (m, 2H), 3.16 (hept, *J* = 6.9 Hz, 1H), 1.74 (d, *J* = 12.6 Hz, 2H), 1.42 (qd, *J* = 11.7, 4.3 Hz, 2H), 1.29 (d, *J* = 6.9 Hz, 6H). NMR $^{13}C$ (101 MHz, DMSO-$d_6$) : δ (ppm) = 155.58, 153.98, 151.63, 143.89, 143.36, 143.09, 134.41, 126.33, 125.58, 66.15 (2C), 47.13, 42.92, 32.45 (2C), 23.68, 20.64 (2C). MS (ES+, m/z) : [M+H+] 369.35. M.p. 173 - 175 °C. |
| 54 | $G_{18}H_{22}N_6O$ | 99 %[a] | White solid. NMR $^1H$ (400 MHz, DMSO-$d_6$) : δ (ppm) = 8.73 (t, *J* = 6.5 Hz, 1H), 7.37 - 7.26 (m, 4H), 7.24 - 7.18 (m, 1H), 7.12 (s, 1H), 6.31 (d, *J* = 7.7 Hz, 1H), 4.64 (d, *J* = 6.3 Hz, 2H), 3.87 - 3.79 (m, 2H), 3.79 - 3.69 (m, 1H), 3.38 - 3.27 (m under $H_2O$,2H), 2.28 (s, 3H), 1.78 (d, *J* = 12.7 Hz, 2H), 1.43 (qd, *J* = 11.8, 4.3 Hz, 2H). NMR $^{13}C$ (101 MHz, DMSO-$d_6$): δ (ppm) = 156.10, 151.39, 139.40, 128.18 (2C), 127.69, 127.20 (2C), 126.68, 126.19, 124.37, 66.21 (2C), 47.04, 42.74, 32.64 (2C), 8.26. MS (ES+, m/z) : [M+H+] 339.37. M.p. 177 - 179 °C. |

(continued)

| N° | Formula | HPLC | Description |
|---|---|---|---|
| 55 | $C_{19}H_{24}N_6O$ | 99 %[a] | White solid. NMR $^1$H (400 MHz, DMSO-$d_6$) : δ (ppm) = 8.75 (t, $J$ = 6.4 Hz, 1H), 7.38 - 7.26 (m, 4H), 7.25 - 7.18 (m, 1H), 7.13 (s, 1H), 6.30 (d, $J$ = 7.7 Hz, 1H), 4.64 (d, $J$ = 6.4 Hz, 2H), 3.83 (d, $J$ = 11.3 Hz, 2H), 3.79 - 3.67 (m 1H), 3.38 - 3.28 (m, 2H), 2.71 (q, $J$ = 7.5 Hz, 2H), 1.79 (d, $J$ = 12.7 Hz, 2H), 1.44 (qd, $J$ = 12.4, 4.2 Hz, 2H), 1.24 (t, $J$ = 7.5 Hz, 3H). NMR $^{13}$C (101 MHz, DMSO-$d_6$): δ (ppm) = 155.94, 151.46, 139.39, 130.10, 128.17 (2C), 127.20 (2C), 126.67, 126.64, 126.26, 66.20 (2C), 47.08, 42.71, 32.58 (2C), 16.41, 11.96. MS (ES+, m/z) : [M+H+] 353.39. M.p. 155 - 158 °C. |
| 56 | $C_{20}H_{24}N_6O$ | 99 %[a] | White solid. NMR $^1$H (400 MHz, DMSO-$d_6$) : δ (ppm) = 8.74 (t, $J$ = 6.3 Hz, 1H), 7.37 - 7.26 (m, 4H), 7.25 - 7.18 (m, 1H), 7.01 (s, 1H), 6.33 (d, $J$ = 7.7 Hz, 1H), 4.62 (d, $J$ = 6.3 Hz, 2H), 3.83 (d, $J$ = 11.3 Hz, 2H), 3.79 - 3.67 (m, 1H), 3.38 - 3.26 (m, 2H), 1.99 (tt, $J$ = 8.6, 5.2 Hz, 1H), 1.78 (d, $J$ = 12.7 Hz, 2H), 1.44 (qd, $J$ = 11.9, 4.3 Hz, 2H), 0.96 - 0.89 (m, 2H), 0.81 - 0.75 (m, 2H). NMR $^{13}$C (101 MHz, DMSO-$d_6$) : δ (ppm) = 156.01, 151.33, 139.40, 130.79, 128.17 (2C), 127.20 (2C), 126.67, 126.23, 125.59, 66.22 (2C), 47.10, 42.73, 32.64 (2C), 6.16 (2C), 4.72. MS (ES+, m/z) : [M+H+] 365.38. M.p. 167 - 169 °C. |
| 57 | $C_{23}H_{30}N_6O$ | 99 %[a] | White solid. NMR $^1$H (400 MHz, DMSO-$d_6$) : δ (ppm) = 8.76 (t, $J$ = 6.4 Hz, 1H), 7.38 - 7.26 (m, 4H), 7.24 - 7.18 (m, 1H), 7.09 (s, 1H), 6.30 (d, $J$ = 7.5 Hz, 1H), 4.62 (d, $J$ = 6.4 Hz, 2H), 3.88 - 3.78 (m, 2H), 3.76 - 3.65 (m, 1H), 3.37 - 3.26 (m, 2H), 2.91 - 2.80 (m, 1H), 2.01 (d, $J$ = 11.1 Hz, 2H), 1.84 - 1.74 (m, 4H), 1.70 (d, $J$ = 12.4 Hz, 1H), 1.52 - 1.13 (m, 7H). NMR $^{13}$C (101 MHz, DMSO-$d_6$): δ (ppm) = 155.77, 151.51, 139.40, 133.44, 128.16 (2C), 127.22 (2C), 126.67, 126.18, 125.63, 66.22 (2C), 47.33, 42.73, 33.08, 32.58 (2C), 30.64 (2C), 25.78 (2C), 25.71. MS (ES+, m/z) : [M+H+] 407.44. M.p. 159 - 161 °C. |
| 58 | $C_{21}H_{28}N_6O$ | 95 %[a] | White solid. NMR $^1$H (400 MHz, DMSO-$d_6$) : δ (ppm) = 8.70 (t, $J$ = 6.4 Hz, 1H), 7.39 - 7.25 (m, 4H), 7.25 - 7.17 (m, 1H), 7.10 (s, 1H), 6.25 (d, $J$ = 7.7 Hz, 1H), 4.65 (d, $J$ = 6.4 Hz, 2H), 3.87 - 3.75 (m, 1H), 3.69 - 3.61 (m, 2H), 3.60 - 3.48 (m, 2H), 3.16 (hept, $J$ = 6.9 Hz, 1H), 2.02 - 1.84 (m, 2H), 1.78 - 1.65 (m, 2H), 1.65 - 1.56 (m, 2H), 1.30 (d, $J$ = 6.9 Hz, 6H). NMR $^{13}$C (101 MHz, DMSO-$d_6$) : δ (ppm) = 155.57, 151.53, 139.36, 134.29, 128.18 (2C), 127.23 (2C), 126.69, 126.38, 125.30, 68.86, 65.57, 50.64, 42.54, 36.27, 32.20, 26.53, 23.76, 20.56 (2C). MS (ES+, m/z) : [M+H+] 381.41. M.p. 118 - 120 °C. |
| 59 | $C_{21}H_{28}N_6$ | > 98 %[a] | Yellow solid. $^1$H NMR (400 MHz, DMSO-$d_6$) δ 8.68 (t, $J$ = 6.3 Hz, 1H), 7.25 - 7.37 (m, 4H), 7.18 - 7.24 (m, 1H), 7.09 (br s, 1H), 6.10 (d, $J$ = 7.8 Hz, 1H), 4.63 (d, $J$ = 6.3 Hz, 2H), 3.52 (m, 1H), 3.15 (h, $J$ = 6.9 Hz, 1H), 1.85 - 1.93 (m, 2H), 1.66 - 1.74 (m, 2H), 1.54 - 1.62 (m, 1H), 1.29 (d, $J$ = 6.9 Hz, 6H), 1.05 - 1.26 (m, 5H). $^{13}$C NMR (101 MHz, DMSO-$d_6$) δ 156.3, 151.9, 139.9, 134.7, 128.6 (2C), 127.8 (2C), 127.2, 126.9, 125.7, 50.2, 43.1, 32.9 (2C), 26.0, 25.4 (2C), 24.2, 21.1 (2C). MS (ESI$^+$): $[M+H]^+$ 365.2. LogD (HPLC): 5.47 |
| 60 | $C_{21}H_{26}F_2N_6$ | > 98 %[a] | Beige solid. $^1$H NMR (400 MHz, DMSO-$d_6$) δ 8.75 (t, $J$ = 6.4 Hz, 1H), 7.40 - 7.27 (m, 4H), 7.26 - 7.18 (m, 1H), 7.12 (s, 1H), 6.37 (d, $J$ = 7.4 Hz, 1H), 4.66 (d, $J$ = 6.3 Hz, 2H), 3.72 (m, 1H), 3.18 (h, $J$ = 6.9 Hz, 1H), 2.15 - 1.75 (m, 6H), 1.57 (m, 2H), 1.30 (d, $J$ = 6.9 Hz, 6H). $^{19}$F NMR (376 MHz, DMSO) δ -92.7 (d, $J$ = 233 Hz), -97.7 (d, $J$ = 233 Hz). $^{13}$C NMR (101 MHz, DMSO) δ 156.4, 152.0, 139.9, 134.8, 128.7 (2C), 127.7 (2C), 127.2, 126.9, 125.9, 121.9, 47.7, 43.1, 32.0 (*t, J* = 23 Hz, 2C), 28.2 (2C), 24.2, 21.2 (2C). MS (ESI$^+$): $[M+H]^+$ 401.2. LogD (HPLC): 4.57 |

(continued)

| N° | Formula | HPLC | Description |
|---|---|---|---|
| 61 | $C_{21}H_{29}N_7$ | 98 %[a] | White solid. $^1$H NMR (400 MHz, DMSO-*d6*) δ 8.72 (t, *J* = 6.3 Hz, 1H), 7.42 - 7.27 (m, 4H), 7.26 - 7.18 (m, 1H), 7.11 (s, 1H), 6.21 (d, *J* = 7.6 Hz, 1H), 4.64 (d, *J* = 6.4 Hz, 2H), 3.52 (m, 1H), 3.16 (h, *J* = 6.9 Hz, 1H), 2.84 - 2.72 (m, 2H), 2.20 (s, 3H), 2.01 (m, 2H), 1.90 - 1.79 (m, 2H), 1.48 (m, 2H), 1.30 (d, *J* = 6.9 Hz, 6H), $^{13}$C NMR (101 MHz, DMSO) δ 156.3, 152.0, 139.9, 134.8, 128.6 (2C), 127.8 (2C), 127.2, 126.9, 125.8, 54.9 (2C), 48.0, 46.2, 43.1, 31.7 (2C), 24.2, 21.1 (2C). MS (ESI$^+$): [M+H]$^+$ 380.2. LogD (HPLC): 2.14 |
| 62 | $C_{21}H_{28}N_6O$ | > 98 %[a] | Beige solid. $^1$H NMR (400 MHz, DMSO-*d6*) δ 8.72 (t, *J* = 6.4 Hz, 1H), 7.40 - 7.28 (m, 4H), 7.26 - 7.20 (m, 1H), 7.11 (s, 1H), 5.93 (d, *J* = 5.6 Hz, 1H), 4.72 - 4.54 (m, 3H), 3.36 (m, 2H), 3.16 (h, *J* = 6.9 Hz, 1H), 2.11 (m, 1H), 1.95 - 1.82 (m, 1H), 1.72 - 1.54 (m, 2H), 1.34 - 1.28 (m, 6H), 1.28 - 1.03 (m, 4H). $^{13}$C NMR (101 MHz, DMSO) δ 157.0, 151.9, 139.8, 134.7, 128.7 (2C), 127.8 (2C), 127.2, 126.9, 125.8, 71.7, 57.6, 43.1, 34.8, 31.1, 24.8, 24.6, 24.2, 21.2, 21.1. MS (ESI$^+$): [M+H]$^+$ 381.2. LogD (HPLC):3.60 |
| 63 | $C_{21}H_{28}N_6O$ | > 98 %[a] | Light brown solid. $^1$H NMR (400 MHz, DMSO-*d6*) δ 8.70 (t, *J* = 6.7 Hz, 1H), 7.39 - 7.27 (m, 4H), 7.26 - 7.19 (m, 1H), 7.10 (s, 1H), 6.10 (d, *J* = 7.8 Hz, 1H), 4.63 (d, *J* = 6.3 Hz, 2H), 4.48 (d, *J* = 4.5 Hz, 1H), 3.56 - 3.42 (m, 1H), 3.42 - 3.31 (m, 1H), 3.16 (h, *J* = 6.9 Hz, 1H), 2.00 - 1.76 (m, 4H), 1.30 (d, *J* = 6.9 Hz, 6H), 1.29 - 1.14 (m, 4H). $^{13}$C NMR (101 MHz, DMSO) δ 151.9, 139.88, 138.86, 134.7, 128.6 (2C), 127.8 (2C), 127.2, 126.9, 125.8, 69.0, 49.9, 43.1, 34.8 (2C), 30.8 (2C), 24.2, 21.1 (2C). MS (ESI$^+$): [M+H]$^+$ 381.2. LogD (HPLC): 2.93 |
| 64 | $C_{22}H_{30}N_6O$ | 96%[a] | Light pink solid. $^1$H NMR (400 MHz, DMSO-$d_6$, mixture of diastereomers) δ 8.75 - 8.60 (m, 1H), 7.42 - 7.17 (m, 5H), 7.15 -7.05 (m, 1H), 6.20 - 6.10 (m, 1H), 4.70 - 4.55 (m, 2H), 3.65 - 3.45 (m, 1H), 3.26 - 3.02 (m, 4H), 2.10 - 1.72 (m, 3H), 1.71 - 1.38 (m, 4H), 1.36 - 1.08 (m, 8H). MS (ESI$^+$): [M+H]$^+$ 395.3. LogD (HPLC): 4.48 |
| 65 | $C_{20}H_{26}N_6O$ | 96%[a] | Colorless oil. $^1$H NMR (400 MHz, DMSO-*d6*) δ 8.68 (t, *J* = 6.4 Hz, 1H), 7.38 - 7.34 (m, 2H), 7.30 (m, 2H), 7.26 - 7.19 (m, 1H), 7.11 (s, 1H), 6.24 (d, *J* = 6.3 Hz, 1H), 4.65 (m, 3H), 3.98 (p, *J* = 5.0 Hz, 1H), 3.72 (p, *J* = 6.3 Hz, 1H), 3.21 - 3.12 (m, 1H), 2.05 (m, 1H), 1.82 (m, 1H), 1.68 - 1.56 (m, 2H), 1.45 (m, 2H), 1.30 (dd, *J* = 7.0, 1.8 Hz, 6H). $^{13}$C NMR (101 MHz, DMSO-*d6*) δ 156.9, 151.9, 139.8, 134.9, 128.7 (2C), 127.8 (2C), 127.2, 126.8, 125.7, 76.6, 60.5, 43.0, 32.9, 30.4, 24.2, 21.2, 21.12, 21.09. MS (ESI$^+$): [M+H]$^+$ 367.7. LogD (HPLC): 3.26 |
| 66 | $C_{19}H_{25}N_7O$ | 93%[a] | Beige solid. $^1$H NMR (400 MHz, DMSO-*d6*) δ 8.77 (t, *J* = 6.2 Hz, 1H), 8.63 (d, *J* = 2.2 Hz, 1H), 8.45 (dd, *J* = 4.8 & 1.6 Hz, 1H), 7.82 - 7.76 (m, 1H), 7.34 (dd, *J* = 7.8 & 4.7 Hz, 1H), 7.11 (s, 1H), 6.31 (d, *J* = 6.4 Hz, 1H), 4.70-4.60 (m, 3H), 4.05 - 3.92 (m, 1H), 3.77 - 3.67 (m, 1H), 3.17 (h, *J* = 6.8 Hz, 1H), 2.12 - 1.98 (m, 1H), 1.92 - 1.77 (m, 1H), 1.70 - 1.55 (m, 2H), 1.52 - 1.39 (m, 2H), 1.33 - 1.24 (m, 6H). $^{13}$C NMR (101 MHz, DMSO-*d6*) δ 156.8, 151.8, 149.6, 148.6, 135.8, 135.3, 134.9, 126.8, 125.8, 123.9, 76.5, 60.5, 40.9, 32.9, 30.4, 24.2, 21.2, 21.1 (2C). MS (ESI$^+$): [M+H]$^+$ 368.7. LogD (HPLC): 2.36 |
| 67 | $C_{21}H_{28}N_6O$ | 97%[a] | Colorless oil. $^1$H NMR (400 MHz, DMSO-*d6*) δ 8.68 (t, *J* = 5.8 Hz, 1H), 7.39 - 7.26 (m, 4H), 7.22 (t, *J* = 7.1 Hz, 1H), 7.11 (s, 1H), 6.34 (d, *J* = 6.9 Hz, 1H), 4.66 (m, 2H), 3.96 (br s, 1H), 3.75 - 3.67 (m, 1H), 3.27 (s, 3H), 3.19 - 3.16 (m, 1H), 2.02 - 1.89 (m, 1H), 1.84 - 1.77 (m, 1H), 1.76 - 1.44 (m, 4H), 1.30 (dd, *J* = 7.0, 2.5 Hz, 6H). $^{13}$C NMR (101 MHz, DMSO-*d6*) δ 156.4, 152.0, 139.8, 134.9, 128.7 (2C), 127.7 (2C), 127.2, 126.9, 125.7, 86.2, 57.1, 56.4, 43.0, 30.5, 30.2, 24.2, 22.0, 21.2, 21.1. MS (ESI$^+$): [M+H]$^+$ 381.7. LogD (HPLC): 4.25 |

(continued)

| N° | Formula | HPLC | Description |
|---|---|---|---|
| 68 | $C_{20}H_{27}N_7O$ | 92%[a] | Beige solid. $^1$H NMR (400 MHz, DMSO-$d_6$) δ 8.77 (t, *J* = 6.2 Hz, 1H), 8.61 (d, *J* = 2.2 Hz, 1H), 8.45 (dd, *J* = 4.8 & 1.6 Hz, 1H), 7.81 - 7.74 (m, 1H), 7.33 (dd, *J* = 7.8 & 4.7 Hz, 1H), 7.12 (s, 1H), 6.40 (d, *J* = 6.9 Hz, 1H), 4.70 - 4.58 (m, 2H), 4.02 - 3.90 (m, 1H), 3.76 - 3.68 (m, 1H), 3.28 (s, 3H), 3.23 - 3.12 (m, 1H), 2.03 - 1.91 (m, 1H), 1.90 - 1.78 (m, 1H), 1.75 - 1.45 (m, 4H), 1.35 - 1.25 (m, 6H). $^{13}$C NMR (101 MHz, DMSO-$d_6$) δ 156.3, 151.9, 149.5, 148.6, 135.8, 135.2, 135.0, 126.8, 125.8, 123.9, 86.2, 57.1, 56.4, 40.9, 30.5, 30.1, 24.2, 22.0, 21.2, 21.1. MS (ESI$^+$): [M+H]$^+$ 382.6. LogD (HPLC): 3.04 |
| 69 | $C_{20}H_{26}N_6O$ | 99 %[a] | White solid. NMR $^1$H (400 MHz, DMSO-$d_6$) : δ (ppm) = 8.93 (t, *J* = 6.2 Hz, 1H), 7.37 (d, *J* = 6.9 Hz, 2H), 7.30 (t, *J* = 7.5 Hz, 2H), 7.25 - 7.18 (m, 1H), 7.14 (s, 1H), 5.26 - 5.14 (m, 1H), 4.62 (d, *J* = 6.2 Hz, 2H), 3.90 (td, *J* = 8.4, 5.0 Hz, 1H), 3.71 - 3.56 (m, 3H), 3.18 (hept, *J* = 6.9 Hz, 1H), 2.89 (s, 3H), 2.05 (dtd, *J* = 13.1, 8.1, 5.2 Hz, 1H), 1.83 (dtd, *J* = 13.1, 8.1, 5.2 Hz, 1H), 1.29 (d, *J* = 6.9 Hz, 6H). NMR $^{13}$C (101 MHz, DMSO-$d_6$) : δ (ppm) = 156.39, 150.73, 139.45, 134.48, 128.17 (2C), 127.48 (2C), 126.73, 125.91, 125.70, 69.33, 67.03, 55.14, 43.22, 29.36, 29.16, 23.75, 20.52 (2C). MS (ES+, m/z) : [M+H+] 367.40. M.p. 105 - 107 °C. |
| 70 | $C_{21}H_{28}N_6O$ | 99 %[a] | NMR $^1$H (400 MHz, DMSO-$d_6$) : δ (ppm) = 8.92 (t, *J* = 6.2 Hz, 1H), 7.37 (d, *J* = 7.1 Hz, 2H), 7.29 (t, *J* = 7.5 Hz, 2H), 7.20 (t, *J* = 7.3 Hz, 1H), 7.13 (s, 1H), 4.61 (d, *J* = 6.2 Hz, 2H), 4.53 (tt, *J* = 11.9, 4.0 Hz, 1H), 3.90 (dd, *J* = 11.2, 4.4 Hz, 2H), 3.31 (t, J = 11.3 Hz, 2H), 3.17 (hept, *J* = 6.9 Hz, 1H), 2.85 (s, 3H), 1.70 (qd, *J* = 12.2, 4.6 Hz, 2H), 1.42 (d, *J* = 10.3 Hz, 2H), 1.29 (d, J= 6.9 Hz, 6H). NMR $^{13}$C (101 MHz, DMSO-$d_6$) : δ (ppm) = 155.94, 150.77, 139.46, 134.40, 128.11 (2C), 127.27 (2C), 126.65, 125.97, 125.63, 66.87 (2C), 51.45, 43.29, 29.70 (2C), 28.59, 23.78, 20.48 (2C). MS (ES+, m/z) : [M+H+] 381.37. 202 M.p. 136 - 138 °C. |
| 71 | $C_{20}H_{27}N_7O$ | > 99 %[a] | Beige powder. NMR $^1$H (400 MHz, DMSO-$d_6$) : δ (ppm) = 8.98 (t, *J* = 6.1 Hz, 1H), 8.47 (d, *J* = 6.1 Hz, 2H), 7.32 (d, *J* = 5.3 Hz, 2H), 7.17 (s, 1H), 4.62 (d, *J* = 6.1 Hz, 2H), 4.38 (tt, *J* = 11.9, 4.0 Hz, 1H), 3.85 (dd, *J* = 11.3, 4.3 Hz, 2H), 3.27 - 3.11 (m, 3H), 2.81 (s, 3H), 1.65 (qd, *J* = 12.2, 4.5 Hz, 2H), 1.39 - 1.32 (m, 2H), 1.30 (d, *J* = 6.9 Hz, 6H). NMR $^{13}$C (101 MHz, DMSO-$d_6$) : δ (ppm) = 155.81, 150.85, 149.43 (2C), 148.43, 134.53, 125.85 (2C), 122.00 (2C), 66.79 (2C), 51.48, 42.58, 29.67 (2C), 28.56, 23.79, 20.47 (2C). MS (ES+, m/z) : [M+H+] 382.38. M.p. 171 - 173 °C. |
| 72 | $C_{18}H_{22}N_6O$ | 98 %[a] | White solid. NMR $^1$H (400 MHz, DMSO-$d_6$) : δ (ppm) = 8.84 (t, *J* = 6.3 Hz, 1H), 7.37 (d, *J* = 7.7 Hz, 2H), 7.30 (t, *J* = 7.5 Hz, 2H), 7.22 (t, *J* = 7.3 Hz, 1H), 7.11 (s, 1H), 4.64 - 4.56 (m, 2H), 3.53 (s, 4H), 3.17 (hept, *J* = 6.9 Hz, 1H), 3.04 (s, 2H), 1.29 (d, *J* = 6.9 Hz, 6H). NMR $^{13}$C (101 MHz, DMSO-$d_6$) : δ (ppm) = 156.17, 150.79, 139.52, 134.34, 128.19 (2C), 127.60 (2C), 126.76, 125.83, 125.52, 59.02, 51.63, 43.08, 36.27, 23.77, 20.53 (2C). MS (ES+, m/z) : [M+H+] 341.40. M.p. 79 - 81 °C. |
| 73 | $C_{19}H_{24}N_6O$ | > 99 %[a] | White solid. NMR $^1$H (400 MHz, DMSO-$d_6$) : δ (ppm) = 8.97 (t, *J* = 6.3 Hz, 1H), 7.43 - 7.26 (m, 4H), 7.22 (m, 1H), 7.17 (s, 1H), 4.63 (d, *J* = 6.3 Hz, 2H), 3.63 (s, 4H), 3.50 (s, 4H), 3.18 (hept, *J* = 6.9 Hz, 1H), 1.29 (d, *J* = 6.9 Hz, 6H). NMR $^{13}$C (101 MHz, DMSO-$d_6$) : δ (ppm) = 156.43, 151.05, 139.31, 134.71, 128.21 (2C), 127.55 (2C), 126.80, 126.10, 125.96, 65.77 (2C), 44.72 (2C), 43.03, 23.71, 20.58 (2C). MS (ES+, m/z) : [M+H+] 353.29. M.p. 149 - 151 °C. |

(continued)

| N° | Formula | HPLC | Description |
|---|---|---|---|
| 74 | $C_{18}H_{23}N_7O$ | > 99 %[a] | Beige solid. NMR $^1H$ (400 MHz, DMSO-$d_6$) : $\delta$ (ppm) = 9.04 (t, *J* = 6.3 Hz, 1H), 8.59 (d, *J* = 2.2 Hz, 1H), 8.44 (dd, *J* = 4.8, 1.7 Hz, 1H), 7.76 (d, *J* = 7.9 Hz, 1H), 7.33 (dd, *J* = 7.8, 4.8 Hz, 1H), 7.18 (s, 1H), 4.64 (d, *J* = 6.3 Hz, 2H), 3.64 (dd, *J* = 5.7, 3.9 Hz, 4H), 3.50 (dd, *J* = 5.7, 3.9 Hz, 4H), 3.18 (hept, *J* = 6.9 Hz, 1H), 1.29 (d, *J* = 6.9 Hz, 6H). NMR $^{13}C$ (101 MHz, DMSO-$d_6$) : $\delta$ (ppm) = 156.35, 151.01, 149.07, 148.13, 135.34, 134.79, 134.77, 126.08, 126.02, 123.46, 65.77 (2C), 44.71 (2C), 40.78, 23.70, 20.56 (2C). MS (ES+, m/z) : [M+H+] 354.38. M.p. 188 - 190 °C. |
| 75 | $C_{18}H_{23}N_7O$ | > 99 %[a] | Beige solid. NMR $^1H$ (400 MHz, DMSO-$d_6$) : $\delta$ (ppm) = 9.05 (t, *J* = 6.2 Hz, 1H), 8.48 (d, *J* = 6.1 Hz, 2H), 7.32 (d, *J* = 6.1 Hz, 2H), 7.20 (s, 1H), 4.64 (d, *J* = 6.2 Hz, 2H), 3.60 (t, *J* = 4.7 Hz, 4H), 3.44 (t, *J* = 4.7 Hz, 4H), 3.19 (hept, *J* = 6.9 Hz, 1H), 1.29 (d, *J* = 6.9 Hz, 6H). NMR $^{13}C$ (101 MHz, DMSO-$d_6$) : $\delta$ (ppm) = 156.29, 151.16, 149.48 (2C), 148.19, 134.81, 126.15, 125.98, 122.31 (2C), 65.70 (2C), 44.65 (2C), 42.24, 23.70, 20.55 (2C). MS (ES+, m/z) : [M+H+] 354.30. M.p. 184 - 186 °C. |
| 76 | $C_{19}H_{24}N_6O$ | 97%[a] | White solid. $^1H$ NMR (400 MHz, DMSO-$d_6$) $\delta$ 8.82 (t, *J* = 6.3 Hz, 1H), 7.39 (d, *J* = 7.1 Hz, 2H), 7.32 - 7.28 (m, 2H), 7.25 - 7.19 (m, 1H), 7.11 (s, 1H), 4.88 (d, *J* = 3.6 Hz, 1H), 4.62 (d, *J* = 6.3 Hz, 2H), 4.32 (br s, 1H), 3.47 (m, 3H), 3.34 (d, *J* = 12.0 Hz, 1H), 3.18 (h, *J* = 6.9 Hz, 1H), 2.00 - 1.91 (m, 1H), 1.87 - 1.78 (m, 1H), 1.30 (d, *J* = 6.9 Hz, 6H). $^{13}C$ NMR (101 MHz, DMSO-$d_6$) $\delta$ 155.6, 151.4, 140.0, 134.7, 128.6 (2C), 128.2 (2C), 127.2, 126.5, 125.8, 69.6, 55.2, 44.8, 43.5, 33.9, 24.2, 21.0 (2C). MS (ESI$^+$): $[M+H]^+$ 353.2. LogD (HPLC): 3.16 |
| 77 | $C_{18}H_{23}N_7O$ | 94% [a] | Beige solid. $^1H$ NMR (400 MHz, DMSO-$d_6$) $\delta$ 8.90 (t, *J* = 6.2 Hz, 1H), 8.63 (d, *J* = 2.2 Hz, 1H), 8.44 (dd, *J* = 4.8 & 1.6 Hz, 1H), 7.82 - 7.76 (m, 1H), 7.33 (dd, *J* = 7.8 & 4.7 Hz, 1H), 7.12 (s, 1H), 4.89 (d, *J* = 3.6 Hz, 1H), 4.63 (d, *J* = 6.2 Hz, 2H), 4.37 - 4.29 (m, 1H), 3.55 - 3.40 (m, 3H), 3.39 - 3.30 (m, 1H), 3.27 - 3.12 (m, 1H), 2.03 - 1.91 (m, 1H), 1.88 - 1.77 (m, 1H), 1.29 (d, *J* = 6.9 Hz, 6H). $^{13}C$ NMR (101 MHz, DMSO-$d_6$) $\delta$ 155.5, 151.4, 149.8, 148.6, 136.0, 135.4, 134.8, 126.4, 126.0, 123.9, 69.6, 55.2, 44.8, 41.3, 34.0, 24.2, 21.0, 20.9. MS (ESI$^+$): $[M+H]^+$ 354.6. LogD (HPLC): 2.29 |
| 78 | $C_{20}H_{24}N_6O$ | > 99 %[a] | Colorless oil. $^1H$ NMR (400 MHz, DMSO-$d_6$) $\delta$ 8.94 (t, *J* = 6.3 Hz, 1H), 7.38 (m, 2H), 7.31 (t, *J* = 7.5 Hz, 2H), 7.24 (m, 1H), 7.15 (s, 1H), 4.69 (s, 4H), 4.60 (d, *J* = 6.3 Hz, 2H), 4.08 (s, 4H), 3.16 (m, 1H), 1.28 (d, J= 6.9 Hz, 6H). MS (ESI$^+$): $[M+H]^+$ 365.6. LogD (HPLC): 3.42 |
| 79 | $C_{20}H_{26}N_6O$ | 97%[a] | White solid. $^1H$ NMR (400 MHz, DMSO-$d_6$) $\delta$ 8.86 (t, *J* = 6.3 Hz, 1H), 7.40 (d, *J* = 7.0 Hz, 2H), 7.30 (t, *J* = 7.5 Hz, 2H), 7.26 - 7.18 (m, 1H), 7.12 (s, 1H), 4.62 (d, *J* = 6.3 Hz, 2H), 4.01 (br s, 1H), 3.55 - 3.34 (m, 4H), 3.23 (s, 3H), 3.18 (p, *J* = 6.9 Hz, 1H), 1.99 (td, *J* = 7.4, 4.2 Hz, 2H), 1.30 (d, *J* = 6.9 Hz, 6H). $^{13}C$ NMR (101 MHz, DMSO-$d_6$) $\delta$ 155.6, 151.4, 140.0, 134.7, 128.6 (2C), 128.2 (2C), 127.2, 126.5, 125.9, 79.5, 56.2, 51.7, 44.8, 43.6, 30.6, 24.2, 21.0 (2C). MS (ESI$^+$): $[M+H]^+$ 367.2. LogD (HPLC): 4.42 |
| 80 | $C_{19}H_{25}N_7O$ | >98% [a] | Beige solid. $^1H$ NMR (400 MHz, DMSO-$d_6$) $\delta$ 8.93 (t, *J* = 6.2 Hz, 1H), 8.63 (d, *J* = 2.2 Hz, 1H), 8.44 (dd, *J* = 4.8 & 1.6 Hz, 1H), 7.83 - 7.75 (m, 1H), 7.34 (dd, *J* = 7.8 & 4.7 Hz, 1H), 7.13 (s, 1H), 4.63 (d, *J* = 6.2 Hz, 2H), 4.05 - 3.98 (m, 1H), 3.56 - 3.42 (m, 3H), 3.42 - 3.30 (m, 1H), 3.24 (s, 3H), 3.24 - 3.13 (m, 1H), 2.06 - 1.94 (m, 2H), 1.35 - 1.25 (m, 6H). $^{13}C$ NMR (101 MHz, DMSO-$d_6$) $\delta$ 155.5, 151.4, 149.8, 148.5, 135.9, 135.4, 134.8, 126.4, 126.0, 123.9, 79.5, 56.2, 51.8, 44.8, 41.3, 30.5, 24.2, 21.0 (2C). MS (ESI$^+$): $[M+H]^+$ 368.7. LogD (HPLC): 3.06 |

(continued)

| N° | Formula | HPLC | Description |
|---|---|---|---|
| 81 | $C_{21}H_{28}N_6O$ | 97%[a] | White solid. $^1H$ NMR (400 MHz, DMSO-$d_6$) δ 8.91 (t, $J$ = 6.2 Hz, 1H), 7.37 (d, $J$ = 7.5 Hz, 2H), 7.30 (t, $J$ = 8.3, 6.6 Hz, 2H), 7.22 (t, $J$ = 7.1 Hz, 1H), 7.15 (s, 1H), 4.61 (d, $J$ = 6.2 Hz, 2H), 4.05 - 3.96 (m, 2H), 3.37 (m, 1H), 3.25 (s, 3H), 3.23 - 3.12 (m, 3H), 1.82 (m, 2H), 1.29 (m, 8H). $^{13}C$ NMR (101 MHz, DMSO-$d_6$) δ 156.6, 151.5, 139.8, 135.0, 128.6 (2C), 128.0 (2C), 127.2, 126.4, 126.3, 76.2, 55.3, 43.6, 42.3 (2C), 30.3 (2C), 24.2, 21.0 (2C). MS (ESI$^+$): [M+H]$^+$ 381.7. LogD (HPLC): 4.60 |
| 82 | $C_{20}H_{27}N_7O$ | >98% [a] | Beige solid. $^1H$ NMR (400 MHz, DMSO-$d_6$) δ 8.97 (t, $J$ = 6.2 Hz, 1H), 8.60 (d, $J$ = 2.2 Hz, 1H), 8.44 (dd, $J$ = 4.8 & 1.6 Hz, 1H), 7.80 - 7.73 (m, 1H), 7.33 (dd, $J$ = 7.8 & 4.7 Hz, 1H), 7.16 (s, 1H), 4.63 (d, $J$ = 6.1 Hz, 2H), 4.05 - 3.94 (m, 2H), 3.42 - 3.33 (m, 1H), 3.26 (s, 3H), 3.23 - 3.13 (m, 3H), 1.88 - 1.76 (m, 2H), 1.40 - 1.23 (m, 8H). $^{13}C$ NMR (101 MHz, DMSO-$d_6$) δ 156.5, 151.5, 149.5, 148.6, 135.7, 135.3, 135.1, 126.4, 126.3, 123.9, 76.2, 55.3, 42.3 (2C), 41.3, 30.3 (2C), 24.2, 21.0 (2C). MS (ESI$^+$): [M+H]$^+$ 382.7. LogD (HPLC): 3.15 |
| 83 | $C_{19}H_{24}N_6S$ | 97%[a] | White solid. $^1H$ NMR (400 MHz, DMSO-$d_6$) δ 8.99 (t, $J$ = 6.3 Hz, 1H), 7.37 (m, 2H), 7.31 (m, 2H), 7.26 - 7.20 (m, 1H), 7.17 (s, 1H), 4.60 (d, $J$ = 6.2 Hz, 2H), 3.96 - 3.84 (m, 4H), 3.17 (h, $J$ = 6.8 Hz, 1H), 1.29 (d, $J$ = 6.9 Hz, 6H). 4H missing, overlap with the DMSO signal. $^{13}C$ NMR (101 MHz, DMSO-$d_6$) δ 156.2, 151.6, 139.8, 135.1, 128.7 (2C), 128.0 (2C), 127.2, 126.4, 47.1 (2C), 43.7, 25.5 (2C), 24.2, 21.0 (2C). MS (ESI$^+$): [M+H]$^+$ 369.6. LogD (HPLC): 5.05 |
| 84 | $C_{18}H_{23}N_7S$ | 97%[a] | Beige solid. $^1H$ NMR (400 MHz, DMSO-$d_6$) δ 9.05 (t, $J$ = 6.2 Hz, 1H), 8.60 (d, $J$ = 2.2 Hz, 1H), 8.44 (dd, $J$ = 4.8 & 1.6 Hz, 1H), 7.79 - 7.73 (m, 1H), 7.33 (dd, $J$ = 7.8 & 4.7 Hz, 1H), 7.18 (s, 1H), 4.63 (d, $J$ = 6.1 Hz, 2H), 3.95 - 3.85 (m, 4H), 3.17 (h, $J$ = 6.8 Hz, 1H), 2.57 - 2.47 (m, 4H), 1.29 (d, $J$ = 6.8 Hz, 6H). $^{13}C$ NMR (101 MHz, DMSO-$d_6$) δ 155.8, 151.5, 149.5, 148.6, 135.7, 135.24, 135.17, 126.5, 126.3, 123.9, 47.2 (2C), 41.4, 25.5 (2C), 24.2, 21.0 (2C). MS (ESI$^+$): [M+H]$^+$ 370.6. LogD (HPLC): 3.50 |
| 85 | $C_{20}H_{26}N_6O$ | 97%[a] | White solid. $^1H$ NMR (400 MHz, DMSO-$d_6$) δ 8.89 (t, $J$ = 6.2 Hz, 1H), 7.37 (d, $J$ = 7.2 Hz, 2H), 7.30 (t, $J$ = 7.5 Hz, 2H), 7.27 - 7.18 (m, 1H), 7.14 (s, 1H), 4.62 (m, 3H), 4.09 (dt, $J$ = 13.2, 4.5 Hz, 2H), 3.65 (m, 1H), 3.23 - 3.12 (m, 1H), 3.08 (ddd, $J$ = 13.2, 10.0, 3.0 Hz, 2H), 1.72 (m, 2H), 1.30 (m, 8H). $^{13}C$ NMR (101 MHz, DMSO-$d_6$) δ 156.6, 151.5, 139.9, 135.0, 128.6 (2C), 128.0 (2C), 127.2, 126.4, 126.2, 66.8, 43.6, 42.6 (2C), 34.1 (2C), 24.2, 21.0 (2C). MS (ESI$^+$): [M+H]$^+$ 367.6. LogD (HPLC): 3.19 |
| 86 | $C_{19}H_{25}N_7O$ | 96%[a] | Beige solid. $^1H$ NMR (400 MHz, DMSO-$d_6$) δ 8.95 (t, $J$ = 6.2 Hz, 1H), 8.60 (d, $J$ = 2.2 Hz, 1H), 8.44 (dd, $J$ = 4.8 & 1.6 Hz, 1H), 7.80 - 7.73 (m, 1H), 7.33 (dd, $J$ = 7.8 & 4.7 Hz, 1H), 7.16 (s, 1H), 4.70 - 4.58 (m, 3H), 4.15 - 4.03 (m, 2H), 3.72 - 3.60 (m, 1H), 3.24 - 3.02 (m, 3H), 1.79 - 1.65 (m, 2H), 1.35 - 1.20 (m, 8H). $^{13}C$ NMR (101 MHz, DMSO-$d_6$) δ 156.5, 151.4, 149.5, 148.6, 135.7, 135.3, 135.0, 126.4, 126.3, 123.9, 66.7, 42.6 (2C), 41.3, 34.0 (2C), 24.2, 21.0 (2C). MS (ESI$^+$): [M+H]$^+$ 368.7. LogD (HPLC): 2.30 |
| 87 | $C_{22}H_{30}N_6O$ | 96%[a] | Yellow oil. $^1H$ NMR (400 MHz, DMSO-$d_6$) δ 8.83 (t, $J$ = 6.3 Hz, 1H), 7.42 - 7.36 (m, 2H), 7.34 - 7.27 (m, 2H), 7.26 - 7.19 (m, 1H), 7.13 (s, 1H), 4.79 - 4.70 (m, 1H), 4.61 (d, $J$ = 6.3 Hz, 2H), 4.38 - 4.28 (m, 2H), 3.41 - 3.34 (m, 2H), 3.16 (h, $J$ = 6.9 Hz, 1H), 2.84 (td, $J$ = 13.1, 2.3 Hz, 1H), 1.75 (m, 2H), 1.65 - 1.48 (m, 5H), 1.29 (m, 7H). $^{13}C$ NMR (101 MHz, DMSO-$d_6$) δ 156.5, 151.4, 139.9, 134.9, 128.6 (2C), 128.1 (2C), 127.2, 126.4, 126.1, 59.3, 47.9, 43.6, 39.1, 32.6, 28.2, 25.4, 24.2, 21.1, 21.0, 19.4. MS (ESI$^+$): [M+H]$^+$ 395.6. LogD (HPLC): 2.71 |

(continued)

| N° | Formula | HPLC | Description |
|---|---|---|---|
| 88 | $C_{20}H_{26}N_6O$ | 98 %[a] | White solid. NMR $^1$H (400 MHz, DMSO-$d_6$) : δ (ppm) = 8.73 (t, $J$ = 6.4 Hz, 1H), 7.39 - 7.26 (m, 4H), 7.25 - 7.19 (m, 1H), 7.11 (s, 1H), 6.54 (t, $J$ = 5.8 Hz, 1H), 4.64 (d, $J$ = 6.3 Hz, 2H), 3.75 - 3.63 (m, 2H), 3.58 (q, $J$ = 7.6 Hz, 1H), 3.44 (dd, $J$ = 8.5, 5.4 Hz, 1H), 3.22 - 3.07 (m, 3H), 2.50 (m under DMSO, 1H), 1.95 - 1.84 (m, 1H), 1.57 (dq, $J$ = 12.7, 6.8 Hz, 1H), 1.29 (d, $J$ = 6.9 Hz, 6H). NMR $^{13}$C (101 MHz, DMSO-$d_6$) : δ (ppm) = 156.58, 151.45, 139.32, 134.38, 128.18 (2C), 127.28 (2C), 126.70, 126.40, 125.30, 70.79, 66.78, 43.91, 42.62, 38.55, 29.67, 23.67, 20.64 (2C). MS (ES+, m/z) : [M+H+] 367.33. M.p. 98 - 100 °C. |
| 89 | $C_{20}H_{26}N_6O$ | 96 %[a] | White powder. NMR $^1$H (400 MHz, DMSO-$d_6$) : δ (ppm) = 8.74 (t, $J$ = 6.4 Hz, 1H), 7.35 (d, $J$ = 7.2 Hz, 2H), 7.30 (t, $J$ = 7.5 Hz, 2H), 7.22 (t, $J$ = 7.2 Hz, 1H), 7.11 (s, 1H), 6.29 (t, $J$ = 6.1 Hz, 1H), 4.63 (d, $J$ = 6.4 Hz, 2H), 4.00 (p, $J$ = 6.0 Hz, 1H), 3.74 (q, $J$ = 7.2 Hz, 1H), 3.59 (q, $J$ = 7.0 Hz, 1H), 3.28 - 3.20 (m, 1H), 3.16 (p, $J$ = 6.3 Hz, 2H), 1.91 - 1.70 (m, 3H), 1.64 - 1.53 (m, 1H), 1.29 (d, $J$ = 7.0 Hz, 6H). NMR $^{13}$C (101 MHz, DMSO-$d_6$) : δ (ppm) = 156.51, 151.43, 139.32, 134.39, 128.18 (2C), 127.36 (2C), 126.71, 126.40, 125.34, 76.88, 66.97, 45.15, 42.62, 28.75, 25.02, 23.67, 20.64 (2C). MS (ES+, m/z) : [M+H+] 367.30. M.p. 111 - 113 °C. |
| 90 | $C_{21}H_{28}N_6O$ | 97 %[a] | White solid. NMR $^1$H (400 MHz, DMSO-$d_6$) : δ (ppm) = 8.70 (t, $J$ = 6.3 Hz, 1H), 7.38 - 7.26 (m, 4H), 7.25 - 7.18 (m, 1H), 7.10 (s, 1H), 6.45 (t, $J$ = 6.0 Hz, 1H), 4.63 (d, $J$ = 6.3 Hz, 2H), 3.81 (dd, $J$ = 11.3, 2.6 Hz, 2H), 3.24 - 3.11 (m, 3H), 3.05 (t, $J$ = 6.4 Hz, 2H), 1.80 (br. s, 1H), 1.56 (d, $J$ = 12.4 Hz, 2H), 1.29 (d, $J$ = 6.9 Hz, 6H), 1.14 (qd, $J$ = 12.4, 4.5 Hz, 2H). NMR $^{13}$C (101 MHz, DMSO-$d_6$) : δ (ppm) = 156.73, 151.42, 139.36, 134.30, 128.18 (2C), 127.27 (2C), 126.69, 126.39, 125.29, 66.88 (2C), 46.91, 42.60, 34.60, 30.74 (2C), 23.70, 20.61 (2C). MS (ES+, m/z) : [M+H+] 381.27. M.p. 138 - 140 °C. |
| 91 | $C_{21}H_{28}N_6O$ | 94 %[a] | White solid. NMR $^1$H (400 MHz, DMSO-$d_6$) : δ (ppm) = 8.71 (t, $J$ = 6.4 Hz, 1H), 7.39 - 7.26 (m, 4H), 7.22 (t, $J$ = 7.1 Hz, 1H), 7.10 (s, 1H), 6.44 (t, $J$ = 6.0 Hz, 1H), 4.63 (d, $J$ = 6.4 Hz, 2H), 3.77 (dd, $J$ = 11.3, 3.9 Hz, 1H), 3.69 (dt, $J$ = 11.3, 3.9 Hz, 1H), 3.27 (dt, $J$ = 10.8, 2.8 Hz, 1H), 3.16 (hept, $J$ = 6.9 Hz, 1H), 3.11 - 2.96 (m, 3H), 1.84 (br. s, 1H), 1.74 (dd, $J$ = 12.9, 4.0 Hz, 1H), 1.55 (dt, $J$ = 12.9, 4.0 Hz, 1H), 1.48 - 1.35 (m, 1H), 1.29 (d, $J$ = 6.9 Hz, 6H), 1.25 - 1.13 (m, 1H). NMR $^{13}$C (101 MHz, DMSO-$d_6$) : δ (ppm) = 156.67, 151.41, 139.34, 134.33, 128.18 (2C), 127.31 (2C), 126.70, 126.38, 125.29, 71.00, 67.43, 43.29, 42.61, 35.67, 27.22, 24.98, 23.70, 20.60 (2C). MS (ES+, m/z) : [M+H+] 381.31. M.p. 150 - 152 °C. |
| 92 | $C_{20}H_{27}N_7O$ | 97 %[a] | Brown solid. NMR $^1$H (400 MHz, DMSO-$d_6$) : δ (ppm) = 8.65 (t, $J$ = 6.1 Hz, 1H), 8.50 (d, $J$ = 4.2 Hz, 1H), 7.73 (td, $J$ = 7.8, 1.8 Hz, 1H), 7.30 (d, $J$ = 7.8 Hz, 1H), 7.28 - 7.22 (m, 1H), 7.13 (s, 1H), 6.43 (t, $J$ = 5.9 Hz, 1H), 4.74 (d, $J$ = 6.1 Hz, 2H), 3.73 (d, $J$ = 11.2 Hz, 1H), 3.70 - 3.64 (m, 1H), 3.32 - 3.21 (m, 1H), 3.17 (hept, $J$ = 6.9 Hz, 1H), 3.07 - 2.91 (m, 3H), 1.79 (s, 1H), 1.70 (d, $J$ = 12.5 Hz, 1H), 1.57 - 1.48 (m, 1H), 1.44 - 1.35 (m, 1H), 1.30 (d, $J$ = 6.9 Hz, 6H), 1.21 - 1.09 (m, 1H). NMR $^{13}$C (101 MHz, DMSO-$d_6$) : δ (ppm) = 158.23, 156.64, 151.60, 148.81, 136.69, 134.42, 126.42, 125.44, 122.05, 120.74, 70.99, 67.43, 44.57, 43.27, 35.64, 27.20, 24.97, 23.73, 20.62 (2C). MS (ES+, m/z) : [M+H+] 382.41. M.p. 156 - 158 °C. |

(continued)

| N° | Formula | HPLC | Description |
|---|---|---|---|
| 93 | $C_{20}H_{27}N_7O$ | 97 %[a] | Beige powder. NMR $^1$H (400 MHz, DMSO-$d_6$) : δ (ppm) = 8.79 (t, *J* = 6.3 Hz, 1H), 8.60 (s, 1H), 8.44 (d, *J* = 4.8 Hz, 1H), 7.77 (d, *J* = 7.9 Hz, 1H), 7.33 (dd, J = 7.9, 4.8 Hz, 1H), 7.11 (s, 1H), 6.49 (t, *J* = 6.0 Hz, 1H), 4.63 (d, *J* = 6.3 Hz, 2H), 3.77 (dd, *J* = 11.4, 3.8 Hz, 1H), 3.69 (dt, *J* = 11.4, 3.8 Hz, 1H), 3.34 - 3.22 (m, 1H), 3.16 (hept, *J* = 6.9 Hz, 1H), 3.11 - 2.94 (m, 3H), 1.84 (br. s, 1H), 1.75 (d, *J* = 11.9 Hz, 1H), 1.60 - 1.50 (m, 1H), 1.48 - 1.35 (m, 1H), 1.29 (d, *J* = 6.9 Hz, 6H), 1.26 - 1.13 (m, 1H). NMR $^{13}$C (101 MHz, DMSO-$d_6$) : δ (ppm) = 156.57, 151.33, 149.10, 148.07, 135.31, 134.76, 134.40, 126.31, 125.41, 123.41, 70.98, 67.44, 43.28, 40.49, 35.67, 27.22, 24.97, 23.70, 20.59 (2C). MS (ES+, m/z) : [M+H+] 382.38. M.p. 158 - 160 °C. |
| 94 | $C_{20}H_{27}N_7O$ | 97 %[a] | Beige powder. NMR $^1$H (400 MHz, DMSO-$d_6$) : δ (ppm) = 8.80 (t, *J* = 6.3 Hz, 1H), 8.48 (d, *J* = 5.1 Hz, 2H), 7.31 (d, *J* = 5.1 Hz, 2H), 7.13 (s, 1H), 6.44 (t, *J* = 6.0 Hz, 1H), 4.64 (d, *J* = 6.3 Hz, 2H), 3.73 (dd, *J* = 10.7, 3.4 Hz, 1H), 3.70 - 3.63 (m, 1H), 3.32 - 3.21 (m, 1H), 3.17 (hept, *J* = 6.9 Hz, 1H), 3.07 - 2.91 (m, 3H), 1.79 (br. s, 1H), 1.70 (d, *J* = 11.0 Hz, 1H), 1.58 - 1.48 (m, 1H), 1.45 - 1.32 (m, 1H), 1.30 (d, *J* = 6.9 Hz, 6H), 1.21 - 1.06 (m, 1H). NMR $^{13}$C (101 MHz, DMSO-$d_6$) : δ (ppm) = 156.57, 151.53, 149.48 (2C), 148.29, 134.44, 126.27, 125.50, 122.14 (2C), 70.96, 67.43, 43.25, 41.87, 35.65, 27.19, 24.95, 23.72, 20.60 (2C). MS (ES+, m/z) : [M+H+] 382.38. M.p. 168 - 170 °C. |
| 95 | $C_{21}H_{28}N_6O$ | 98 %[a] | White solid. NMR $^1$H (400 MHz, DMSO-$d_6$) : δ (ppm) = 8.70 (t, *J* = 6.4 Hz, 1H), 7.39 - 7.25 (m, 4H), 7.22 (t, *J* = 7.2 Hz, 1H), 7.10 (s, 1H), 6.43 (t, *J* = 5.9 Hz, 1H), 4.63 (d, *J* = 6.4 Hz, 2H), 3.82 - 3.72 (m, 1H), 3.72 - 3.64 (m, 1H), 3.31 - 3.22 (m, 1H), 3.16 (hept, *J* = 7.2 Hz, 1H), 3.10 - 2.95 (m, 3H), 1.84 (br. s, 1H), 1.74 (d, J= 12.7 Hz, 1H), 1.59 - 1.50 (m, 1H), 1.48 - 1.34 (m, 1H), 1.29 (d, *J* = 6.9 Hz, 6H), 1.22 - 1.11 (m, 1H). NMR $^{13}$C (101 MHz, DMSO-$d_6$) : δ (ppm) = 156.71, 151.44, 139.36, 134.39, 128.22 (2C), 127.35 (2C), 126.75, 126.41, 125.33, 71.03, 67.47, 43.32, 42.65, 35.71, 27.25, 25.01, 23.74, 20.63(2C). MS (ES+, m/z) : [M+H+] 381.41. M.p. 140 - 142 °C. |
| 96 | $C_{20}H_{27}N_7O$ | 98 %[a] | White solid. NMR $^1$H (400 MHz, DMSO-$d_6$) : δ (ppm) = 8.79 (t, *J* = 6.3 Hz, 1H), 8.60 (s, 1H), 8.44 (dd, *J* = 4.8, 1.7 Hz, 1H), 7.77 (dt, *J* = 7.9, 2.0 Hz, 1H), 7.33 (dd, *J* = 7.8, 4.8 Hz, 1H), 7.11 (s, 1H), 6.49 (t, *J* = 5.9 Hz, 1H), 4.63 (d, *J* = 6.3 Hz, 2H), 3.77 (dd, *J* = 11.0, 3.1 Hz, 1H), 3.69 (dt, *J* = 11.0, 3.9 Hz, 1H), 3.28 (td, *J* = 10.9, 2.7 Hz, 1H), 3.16 (hept, *J* = 6.9 Hz, 1H), 3.10 - 2.95 (m, 3H), 1.84 (br. s, 1H), 1.78 - 1.69 (m, 1H), 1.55 (dt, *J* = 13.1, 3.7 Hz, 1H), 1.48 - 1.36 (m, 1H), 1.29 (d, *J* = 6.9 Hz, 6H), 1.26 - 1.12 (m, 1H). NMR $^{13}$C (101 MHz, DMSO-$d_6$) : δ (ppm) = 156.57, 151.33, 149.10, 148.07, 135.31, 134.77, 134.40, 126.31, 125.40, 123.41, 70.98, 67.45, 43.29, 40.49, 35.67, 27.22, 24.97, 23.70, 20.59 (2C). MS (ES+, m/z) : [M+H+] 382.38. M.p. 147 - 149 °C. |
| 97 | $C_{21}H_{28}N_6O$ | 99 %[a] | White solid. NMR $^1$H (400 MHz, DMSO-$d_6$) : δ (ppm) = 8.71 (t, *J* = 6.3 Hz, 1H), 7.39 - 7.26 (m, 4H), 7.26 - 7.18 (m, 1H), 7.10 (s, 1H), 6.43 (t, *J* = 6.0 Hz, 1H), 4.63 (d, *J* = 6.3 Hz, 2H), 3.81 - 3.73 (m, 1H), 3.69 (dt, *J* = 11.0, 3.8 Hz, 1H), 3.27 (td, *J* = 10.9, 2.7 Hz, 1H), 3.16 (hept, *J* = 6.9 Hz, 1H), 3.10 - 2.95 (m, 3H), 1.84 (br. s, 1H), 1.74 (d, *J* = 12.9 Hz, 1H), 1.55 (dt, *J* = 13.5, 3.6 Hz, 1H), 1.48 - 1.35 (m, 1H), 1.29 (d, *J* = 6.9 Hz, 6H), 1.24 - 1.11 (m, 1H). NMR $^{13}$C (101 MHz, DMSO-$d_6$) : δ (ppm) = 156.68, 151.42, 139.35, 134.34, 128.18 (2C), 127.31 (2C), 126.71, 126.39, 125.29, 71.00, 67.44, 43.30, 42.62, 35.68, 27.23, 24.98, 23.71, 20.61 (2C). MS (ES+, m/z) : [M+H+] 381.41. M.p. 139 - 141 °C. |

(continued)

| N° | Formula | HPLC | Description |
|---|---|---|---|
| 98 | $C_{20}H_{27}N_7O$ | 96 %[a] | White solid. NMR $^{1}$H (400 MHz, DMSO-$d_6$) : δ (ppm) = 8.79 (t, *J* = 6.3 Hz, 1H), 8.60 (s, 1H), 8.44 (dd, *J* = 4.8, 1.7 Hz, 1H), 7.77 (dt, *J* = 7.9, 2.0 Hz, 1H), 7.33 (dd, *J* = 7.8, 4.8 Hz, 1H), 7.11 (s, 1H), 6.49 (t, *J* = 5.9 Hz, 1H), 4.63 (d, *J* = 6.3 Hz, 2H), 3.77 (dd, *J* = 10.8, 3.2 Hz, 1H), 3.69 (dt, *J* = 11.0, 3.9 Hz, 1H), 3.28 (td, *J* = 10.9, 2.7 Hz, 1H), 3.16 (hept, *J* = 6.9 Hz, 1H), 3.10 - 2.94 (m, 3H), 1.84 (br. s, 1H), 1.79 - 1.69 (m, 1H), 1.55 (dt, *J* = 12.7, 3.4 Hz, 1H), 1.48 - 1.35 (m, 1H), 1.29 (d, *J* = 6.9 Hz, 6H), 1.26 - 1.12 (m, 1H). NMR $^{13}$C (101 MHz, DMSO-$d_6$) : δ (ppm) = 156.56, 151.33, 149.09, 148.07, 135.31, 134.76, 134.40, 126.30, 125.40, 123.41, 70.98, 67.44, 43.28, 40.49, 35.67, 27.22, 24.97, 23.70, 20.59 (2C). MS (ES+, m/z) : [M+H+] 382.38. M.p. 159 - 161 °C. |
| 99 | $C_{21}H_{28}N_6O$ | 97 %[a] | White solid. NMR $^{1}$H (400 MHz, DMSO-$d_6$) : δ (ppm) = 8.74 (t, *J* = 6.4 Hz, 1H), 7.38 - 7.26 (m, 4H), 7.26 - 7.19 (m, 1H), 7.11 (s, 1H), 6.23 (t, *J* = 6.0 Hz, 1H), 4.63 (d, *J* = 6.4 Hz, 2H), 3.88 - 3.80 (m, 1H), 3.51 - 3.40 (m, 1H), 3.31 - 3.23 (m, 1H), 3.22 - 3.10 (m, 3H), 1.74 (d, *J* = 10.3 Hz, 1H), 1.58 (d, *J* = 13.0 Hz, 1H), 1.47 - 1.36 (m, 3H), 1.29 (dd, *J* = 6.9, 1.2 Hz, 6H), 1.20 - 1.07 (m, 1H). NMR $^{13}$C (101 MHz, DMSO-$d_6$) : δ (ppm) = 156.48, 151.43, 139.32, 134.39, 128.21 (2C), 127.35 (2C), 126.73, 126.38, 125.30, 75.66, 67.31, 46.29, 42.62, 29.32, 25.72, 23.71, 22.78, 20.59 (2C). MS (ES+, m/z) : [M+H+] 381.37. M.p. 113 - 115 °C. |
| 100 | $C_{20}H_{27}N_7O$ | > 99 %[a] | Yellow solid. NMR $^{1}$H (400 MHz, DMSO-$d_6$) : δ (ppm) = 8.81 (t, *J* = 6.3 Hz, 1H), 8.60 (d, *J* = 2.3 Hz, 1H), 8.44 (d, *J* = 3.5 Hz, 1H), 7.77 (d, *J* = 7.9 Hz, 1H), 7.33 (dd, *J* = 7.9, 4.8 Hz, 1H), 7.11 (s, 1H), 6.31 (t, *J* = 6.0 Hz, 1H), 4.62 (d, *J* = 6.3 Hz, 2H), 3.85 (dd, *J* = 11.4, 3.0 Hz, 1H), 3.49 - 3.40 (m, 1H), 3.32 - 3.24 (m, 1H), 3.21 - 3.09 (m, 3H), 1.75 (d, *J* = 9.6 Hz, 1H), 1.59 (d, *J* = 13.0 Hz, 1H), 1.48 - 1.34 (m, 3H), 1.29 (dd, *J* = 6.9, 1.3 Hz, 6H), 1.20 - 1.07 (m, 1H). NMR $^{13}$C (101 MHz, DMSO-$d_6$) : δ (ppm) = 156.36, 151.35, 149.12, 148.07, 135.34, 134.75, 134.42, 126.31, 125.44, 123.41, 75.64, 67.30, 46.28, 40.46, 29.31, 25.70, 23.69, 22.77, 20.58, 20.55. MS (ES+, m/z) : [M+H+] 382.41. M.p. 119 - 121 °C. |
| 101 | $C_{20}H_{27}N_7O$ | > 99 %[a] | White solid. NMR $^{1}$H (400 MHz, DMSO-$d_6$) : δ (ppm) = 8.82 (t, *J* = 6.3 Hz, 1H), 8.48 (d, *J* = 6.1 Hz, 2H), 7.31 (d, *J* = 6.1 Hz, 2H), 7.14 (s, 1H), 6.24 (t, *J* = 6.0 Hz, 1H), 4.64 (d, *J* = 6.3 Hz, 2H), 3.83 (d, *J* = 11.2 Hz, 1H), 3.45 - 3.36 (m, 1H), 3.28 - 3.09 (m, 4H), 1.72 (d, *J* = 11.0 Hz, 1H), 1.53 (d, *J* = 13.0 Hz, 1H), 1.47 - 1.31 (m, 3H), 1.30 (dd, *J* = 6.9, 1.3 Hz, 6H), 1.16 - 1.03 (m, 1H). NMR $^{13}$C (101 MHz, DMSO-$d_6$) : δ (ppm) = 156.34, 151.54, 149.46 (2C), 148.26, 134.45, 126.27, 125.53, 122.14 (2C), 75.59, 67.26, 46.24, 41.83, 29.27, 25.67, 23.70, 22.73, 20.59, 20.57. MS (ES+, m/z) : [M+H+] 382.41. M.p. 136 - 138 °C. |
| 102 | $C_{22}H_{30}N_6O$ | 96 %[a] | White solid. NMR $^{1}$H (400 MHz, DMSO-$d_6$) : δ (ppm) = 8.69 (d, *J* = 6.5 Hz, 1H), 7.38 - 7.26 (m, 4H), 7.25 - 7.18 (m, 1H), 7.10 (s, 1H), 6.19 (t, *J* = 8.4 Hz, 1H), 4.64 (qd, J = 15.1, 6.5 Hz, 2H), 3.82 (t, *J* = 9.8 Hz, 1H), 3.76 - 3.65 (m, 2H), 3.25 - 3.06 (m, 3H), 1.77 (d, *J* = 12.8 Hz, 1H), 1.72 - 1.61 (m, 1H), 1.58 - 1.48 (m, 1H), 1.47 - 1.32 (m, 1H), 1.28 (d, *J* = 6.9 Hz, 6H), 1.23 - 1.13 (m, 1H), 1.06 (dd, *J* = 6.7, 2.9 Hz, 3H). $^{13}$C NMR spectrum shows a mixture of diasteroisomers, estimated to a 1:1 mixture for description. NMR $^{13}$C (101 MHz, DMSO-$d_6$) : δ (ppm) = 156.30 (0.5C), 156.24 (0.5C), 151.45 (0.5C), 151.43 (0.5C), 139.37, 134.28, 128.17 (2C), 127.33, 127.26, 126.70, 126.39, 125.28, 70.55 (0.5C), 70.07 (0.5C), 67.37 (0.5C), 67.26 (0.5 C), 47.77 (0.5C), 47.54 (0.5C), 42.63 (0.5C), 42.59 (0.5C), 40.97 (0.5C), 40.94 (0.5C), 26.34 (0.5C), 26.07 (0.5 C), 25.48 (0.5 C), 25.43 (0.5C), 23.69, 20.67, 20.64 (0.5C), 20.60 (0.5C), 17.79 (0.5 C), 17.75 (0.5C). MS (ES+, m/z) : [M+H+] 395.45. M.p. 119 - 121 °C. |

(continued)

| N° | Formula | HPLC | Description |
|---|---|---|---|
| 103 | $C_{22}H_{30}N_6O$ | 98 %[a] | White solid. NMR $^1$H (400 MHz, DMSO-$d_6$) : δ (ppm) = 8.88 (t, *J* = 6.3 Hz, 1H), 7.38 - 7.27 (m, 4H), 7.25 - 7.18 (m, 1H), 7.13 (s, 1H), 4.69 - 4.54 (m, 2H), 3.66 (d, *J* = 11.2 Hz, 1H), 3.60 (dd, *J* = 11.4, 3.7 Hz, 1H), 3.26 - 3.38 (m, 2H), 3.25 - 3.12 (m, 2H), 2.96 (s, 3H), 2.92 - 3.02 (m, 1H), 1.88 (br. s, 1H), 1.64 - 1.55 (m, 1H), 1.54 - 1.44 (m, 1H), 1.30 (d, *J* = 6.9 Hz, 6H), 1.43 - 1.22 (m, 1H), 1.13 - 0.99 (m, 1H). NMR $^{13}$C (101 MHz, DMSO-$d_6$) : δ (ppm) = 156.41, 150.73, 139.48, 134.37, 128.20 (2C), 127.12 (2C), 126.69, 125.78, 125.54, 70.60, 67.35, 51.02, 43.07, 35.66, 35.09, 26.92, 24.93, 23.77, 20.49 (2C). MS (ES+, m/z) : [M+H+] 395.38. M.p. 159 - 161 °C. |
| 104 | $C_{22}H_{31}N_7$ | 98%[a] | Off-white solid. $^1$H NMR (500 MHz, DMSO-$d_6$) δ 8.71 (m, 1H), 7.37 - 7.27 (m, 4H), 7.25 - 7.19 (m, 1H), 7.10 (s, 1H), 6.45 (m, 1H), 4.63 (d, *J* = 6.4 Hz, 2H), 3.15 (h, *J* = 6.8 Hz, 1H), 3.04 (t, *J* = 6.3 Hz, 2H), 2.81 (m, 2H), 2.21 (br s, 3H), 1.99 - 1.83 (m, 2H), 1.70 - 1.51 (m, 3H), 1.29 (d, *J* = 6.9 Hz, 6H), 1.20 - 1.15 (m, 2H). MS (ESI$^+$): $[M+H]^+$ 394.3. LogD (HPLC): 2.20 |
| 105 | $C_{22}H_{30}N_6$ | 98 %[a] | White solid. $^1$H NMR (400 MHz, DMSO-$d_6$) δ 8.67 (m, 1H), 7.45 - 7.18 (m, 5H), 7.10 (s, 1H), 6.37 (m, 1H), 4.64 (d, *J* = 6.3 Hz, 2H), 3.16 (h, *J* = 6.8 Hz, 1H), 3.01 (t, *J* = 6.4 Hz, 2H), 1.80 - 1.50 (m, 6H), 1.30 (d, *J* = 6.9 Hz, 6H), 1.25 - 1.05 (m, 3H), 1.00 - 0.75 (m, 2H). $^{13}$C NMR (101 MHz, DMSO) δ 151.94, 151.86, 139.9, 134.7, 128.6 (2C), 127.8 (2C), 127.2, 125.7, 117.2, 47.9, 43.1, 37.7, 31.2 (2C), 26.6, 26.0 (2C), 24.2, 21.1 (2C). MS (ESI$^+$): $[M+H]^+$ 379.3. LogD (HPLC): 5.90 |
| 106 | $C_{22}H_{31}N_7$ | 98% [a] | White solid. $^1$H NMR (400 MHz, DMSO-$d_6$) δ 8.67 (t, *J* = 6.2 Hz, 1H), 7.37 - 7.26 (m, 4H), 7.22 (t, *J* = 7.2 Hz, 1H), 7.10 (s, 1H), 6.35 (t, *J* = 5.7 Hz, 1H), 4.63 (d, *J* = 6.2 Hz, 2H), 3.24 - 3.08 (m, 3H), 2.93 - 2.86 (m, 1H), 2.17 (s, 3H), 2.03 - 1.96 (m, 2H), 1.93 - 1.82 (m, 2H), 1.62 - 1.56 (m, 2H), 1.49 - 1.38 (m, 2H), 1.30 (d, *J* = 6.8 Hz, 6H). $^{13}$C NMR (101 MHz, DMSO-$d_6$) δ 156.9, 151.9, 139.8, 134.8, 128.6 (2C), 127.8 (2C), 127.2, 125.8, 125.7, 64.3, 57.1, 43.0, 38.8, 32.9, 30.5, 24.2, 22.1, 21.1 (2C). 1 carbon signal overlap with solvent. MS (ESI$^+$): $[M+H]^+$ 394.3. LogD (HPLC): 2.16 |
| 107 | $C_{19}H_{22}N_8$ | 96% [a] | Orange powder. $^1$H NMR (400 MHz, DMSO-$d_6$) δ 9.04 (t, *J* = 6.4 Hz, 1H), 8.75 (s, 1H), 7.37 - 7.20 (m, 7H), 6.20 (d, *J* = 1.9 Hz, 1H), 4.66 (d, *J* = 6.3 Hz, 2H), 3.67 (s, 3H), 3.18 (h, *J* = 6.9 Hz, 1H), 1.30 (d, *J* = 6.9 Hz, 6H). $^{13}$C NMR (101 MHz, DMSO-$d_6$) δ 154.6, 152.2, 139.4, 138.9, 137.6, 135.5, 128.7 (2C), 128.0 (2C), 127.3, 126.9, 126.5, 98.5, 43.3, 35.9, 24.1, 21.2 (2C). MS (ESI$^+$): $[M+H]^+$ 363.2. LogD (HPLC): 2.98 |
| 108 | $C_{20}H_{24}N_8$ | >97% [b] | Yellow solid. NMR 1H N.D. MS (ES+, m/z) : [M+H+] 377.31 |
| 109 | $C_{19}H_{22}N_8$ | 90% [a] | Colorless oil. $^1$H NMR (400 MHz, DMSO-$d_6$) δ 9.77 (t, *J* = 6.2 Hz, 1H), 7.43 - 7.31 (m, 5H), 7.31 - 7.22 (m, 2H), 6.78 (q, *J* = 5.0 Hz, 1H), 6.56 (d, *J* = 1.8 Hz, 1H), 4.74 (d, *J* = 6.2 Hz, 2H), 3.38 - 3.32 (m, 1H), 2.80 (d, *J* = 5.0 Hz, 3H), 1.34 (d, *J* = 6.9 Hz, 6H). $^{13}$C NMR (101 MHz, DMSO-$d_6$) δ 152.2, 150.8, 150.1, 138.6, 136.5, 128.9 (2C), 128.0, 127.5, 127.4 (2C), 126.8, 125.8, 111.9, 44.2, 30.0, 24.1, 21.0 (2C). MS (ESI$^+$): $[M+H]^+$ 363.2. LogD (HPLC): 3.12 |
| 110 | $C_{19}H_{22}N_8$ | 98% [a] | White solid. $^1$H NMR (400 MHz, DMSO-$d_6$) δ 8.87 (t, *J* = 6.3 Hz, 1H), 8.80 (s, 1H), 7.70 (s, 1H), 7.48 (s, 1H), 7.39 - 7.36 (m, 2H), 7.34 - 7.30 (m, 2H), 7.20 - 7.25 (m, 1H), 7.18 (s, 1H), 4.69 (d, *J* = 6.4 Hz, 2H), 3.78 (s, 3H), 3.31 - 3.27 (m, 1H), 1.35 (d, *J* = 6.9 Hz, 6H). MS (ESI$^+$): $[M+H]^+$ 403.3. LogD (HPLC): 2.89 |

(continued)

| N° | Formula | HPLC | Description |
|---|---|---|---|
| 111 | $C_{19}H_{22}N_8$ | >98% [a] | Yellow solid. $^1$H NMR (400 MHz, DMSO-$d_6$) δ 9.04 (s, 1H), 8.90 (t, *J* = 6.4 Hz, 1H), 7.52 (s, 1H), 7.48 - 7.40 (m, 2H), 7.35 - 7.15 (m, 5H), 6.54 (s, 1H), 4.66 (d, *J* = 6.3 Hz, 2H), 3.73 (s, 3H), 3.23 (h, *J* = 6.9 Hz, 1H), 1.33 (d, *J* = 6.9 Hz, 6H).$^{13}$C NMR (101 MHz, DMSO-$d_6$) δ 153.9, 152.0, 148.8, 139.7, 135.3, 131.0, 128.6 (2C), 128.3 (2C), 127.3, 126.8, 126.2, 96.5, 43.2, 38.7, 24.3, 21.1 (2C). MS (ESI$^+$): [M+H]$^+$ 363.2. |
| 112 | $C_{20}H_{24}N_8$ | 97% [a] | Yellow solid. $^1$H NMR (400 MHz, DMSO-$d_6$) δ 9.01 (t, *J* = 6.4 Hz, 1H), 8.35 (s, 1H), 7.32 - 7.15 (m, 7H), 4.52 (d, *J* = 6.2 Hz, 2H), 3.56 (s, 3H), 3.11 (h, *J* = 6.9 Hz, 1H), 1.83 (s, 3H), 1.25 (d, *J* = 6.9 Hz, 6H).$^{13}$C NMR (101 MHz, DMSO-$d_6$) δ 155.9, 152.2, 139.3, 137.8, 136.1, 135.3, 128.6 (2C), 128.4 (2C), 127.3, 126.9, 126.4, 110.0, 43.3, 35.7, 24.0, 21.3 (2C), 8.8. MS (ESI$^+$): [M+H]$^+$ 377.2. |
| 113 | $C_{20}H_{24}N_8$ | >98% [a] | Yellow solid. $^1$H NMR (400 MHz, DMSO-$d_6$) δ 9.04 (t, *J* = 6.4 Hz, 1H), 8.68 (s, 1H), 7.35 - 7.18 (m, 6H), 5.94 (s, 1H), 4.64 (d, *J* = 6.3 Hz, 2H), 3.58 (s, 3H), 3.17 (h, *J* = 6.9 Hz, 1H), 2.09 (s, 3H), 1.30 (d, *J* = 6.9 Hz, 6H).$^{13}$C NMR (101 MHz, DMSO-$d_6$) δ 154.6, 152.2, 145.3, 139.4, 139.2, 135.5, 128.7 (2C), 127.9 (2C), 127.3, 126.9, 126.5, 98.0, 43.3, 35.5, 24.1, 21.2 (2C), 14.3. MS (ESI$^+$): [M+H]$^+$ 377.2. |
| 114 | $C_{21}H_{22}N_6$ | >98% [a] | Offwhite solid. $^1$H NMR (400 MHz, DMSO-$d_6$) δ .99 (t, *J* = 6.4 Hz, 1H), 8.94 (s, 1H), 7.73 (d, *J* = 8.8 Hz, 2H), 7.44 - 7.38 (m, 2H), 1.37 - 7.30 (m, 2H), 7.28 - 7.20 (m, 4H), 6.89 (t, *J* = 7.4 Hz, 1H), 4.74 (d, *J* = 6.2 Hz, 2H), 3.30 (m, 1H), 1.37 (d, *J* = 6.9 Hz, 6H). MS (ESI$^+$) : [M+H]$^+$ 359.2. LogD (HPLC): 4.64 |
| 115 | $C_{20}H_{24}N_8$ | >98% [a] | Orange oil. $^1$H NMR (400 MHz, DMSO-$d_6$) δ 8.71 (t, *J* = 6.3 Hz, 1H), 7.47 (s, 1H), 7.37 - 7.26 (m, 5H), 7.25 - 7.19 (m, 1H), 7.11 (s, 1H), 6.60 (t, *J* = 6.1 Hz, 1H), 4.63 (d, *J* = 6.3 Hz, 2H), 4.17 (d, *J* = 6.0 Hz, 2H), 3.73 (s, 3H), 3.20 (h, *J* = 6.9 Hz, 1H), 1.30 (d, *J* = 6.9 Hz, 6H). $^{13}$C NMR (101 MHz, DMSO-$d_6$) δ 156.2, 151.5, 139.3, 138.1, 134.5, 129.3, 128.2 (2C), 127.3 (2C), 126.7, 126.4, 125.3, 119.8, 42.6, 38.3, 35.1, 23.6, 20.7 (2C). MS (ESI$^+$): [M+H]$^+$ 377.2. LogD (HPLC): 2.98 |
| 116 | $C_{20}H_{24}N_8$ | 96% [a] | Colorless oil. $^1$H NMR (400 MHz, DMSO-$d_6$) δ 8.77 (t, *J* = 6.3 Hz, 1H), 7.37 (d, *J* = 7.1 Hz, 2H), 7.33 - 7.25 (m, 2H), 7.25 - 7.19 (m, 1H), 7.11 (s, 1H), 7.01 (br s, 1H), 6.74 (d, *J* = 1.2 Hz, 1H), 6.71 (t, *J* = 5.7 Hz, 1H), 4.63 (d, *J* = 6.4 Hz, 2H), 4.40 (d, *J* = 5.7 Hz, 2H), 3.62 (s, 3H), 3.22 - 3.11 (m, 1H), 1.27 (d, *J* = 7.0 Hz, 6H). $^{13}$C NMR (101 MHz, DMSO-$d_6$) δ 156.5, 151.9, 145.8, 139.8, 135.0, 128.6 (2C), 128.0 (2C), 127.2, 126.9, 126.6, 125.8, 121.8, 43.1, 37.7, 32.7, 24.1, 21.2 (2C). MS (ESI$^+$): [M+H]$^+$ 377.2. LogD (HPLC): 2.49 |
| 117 | $C_{20}H_{24}N_8$ | >98% [a] | White solid. $^1$H NMR (400 MHz, DMSO-$d_6$) δ 8.78 (t, *J* = 6.4 Hz, 1H), 7.38 - 7.26 -m, 4H), 7.26 - 7.18 (m, 2H), 7.13 (s, 1H), 6.96 (t, *J* = 6.0 Hz, 1H), 6.12 (s, 1H), 4.65 (d, *J* = 6.3 Hz, 2H), 4.39 (d, *J* = 6.0 Hz, 2H), 3.81 (s, 3H), 3.18 (h, *J* = 6.8 Hz, 1H), 1.28 (d, *J* = 6.9 Hz, 6H).$^{13}$C NMR (101 MHz, DMSO-$d_6$) δ 155.6, 152.0, 141.2, 139.7, 137.5, 135.0, 128.7 (2C), 127.8 (2C), 127.2, 126.9, 125.9, 105.7, 43.1, 36.6, 36.1, 24.1, 21.2 (2C). MS (ESI$^+$): [M+H]$^+$ 377.1. |
| 118 | $C_{20}H_{24}N_8$ | >98% [a] | White solid. $^1$H NMR (400 MHz, DMSO-$d_6$) δ 8.72 (t, *J* = 6.4 Hz, 1H), 7.52 (d, *J* = 2.2 Hz, 1H), 7.39 - 7.18 (m, 5H), 7.11 (s, 1H), 6.60 (t, *J* = 6.1 Hz, 1H), 6.12 (d, *J* = 2.1 Hz, 1H), 4.64 (d, *J* = 6.4 Hz, 2H), 4.31 (d, *J* = 6.0 Hz, 2H), 3.76 (s, 3H), 3.18 (h, *J* = 6.8 Hz, 1H), 1.29 (d, *J* = 6.9 Hz, 6H).$^{13}$C NMR (101 MHz, DMSO-$d_6$) δ 156.8, 151.9, 150.9, 139.8, 134.9, 131.4, 128.6 (2C), 128.0 (2C), 127.2, 126.9, 125.8, 104.3, 43.1, 39.2, 38.6, 24.2, 21.1 (2C). MS (ESI$^+$): [M+H]$^+$ 377.1. |

(continued)

| N° | Formula | HPLC | Description |
|---|---|---|---|
| 119 | $C_{20}H_{24}N_8$ | >98% [a] | White solid. $^1$H NMR (400 MHz, DMSO-$d_6$) δ 8.76 (t, *J* = 6.4 Hz, 1H), 7.47 (s, 1H), 7.39 - 7.26 (m, 4H), 7.26 - 7.18 (m, 1H), 7.13 (s, 1H), 6.86 - 6.76 (m, 2H), 4.65 (d, *J* = 6.3 Hz, 2H), 4.39 (d, *J* = 5.8 Hz, 2H), 3.60 (s, 3H), 3.21 (h, *J* = 6.9 Hz, 1H), 1.31 (d, *J* = 6.9 Hz, 6H). $^{13}$C NMR (101 MHz, DMSO-$d_6$) δ 156.5, 152.0, 139.7, 138.3, 135.0, 130.2, 128.7 (2C), 128.1, 127.9 (2C), 127.2, 126.9, 125.9, 43.1, 34.6, 31.4, 24.2, 21.2 (2C). MS (ESI$^+$): [M+H]$^+$ 377.1. |
| 120 | $C_{20}H_{24}N_8$ | >98% [a] | White solid. $^1$H NMR (400 MHz, DMSO-$d_6$) δ 8.72 (t, *J* = 6.4 Hz, 1H), 7.44 (s, 1H), 7.39 - 7.33 (m, 2H), 7.32 - 7.26 (m, 2H), 7.25 - 7.19 (m, 1H), 7.11 (s, 1H), 6.86 (s, 1H), 6.40 (t, *J* = 5.8 Hz, 1H), 4.63 (d, *J* = 6.3 Hz, 2H), 4.23 (d, *J* = 5.8 Hz, 2H), 3.56 (s, 3H), 3.19 (h, *J* = 6.9 Hz, 1H), 1.29 (d, *J* = 6.9 Hz, 6H). $^{13}$C NMR (101 MHz, DMSO-$d_6$) δ 156.8, 151.9, 140.5, 139.9, 137.4, 134.9, 128.6 (2C), 128.0 (2C), 127.2, 126.9, 125.78, 117.8, 43.1, 33.2, 24.1, 21.2 (2C), one peak overlaps with solvent. MS (ESI$^+$): [M+H]$^+$ 377.1. |
| 121 | $C_{22}H_{24}N_6$ | >98% [a] | Glass-like solid. $^1$H NMR (400 MHz, DMSO-$d_6$) δ 8.72 (t, *J* = 6.3 Hz, 1H), 7.40 - 7.15 (m, 10H), 7.10 (s, 1H), 7.00 (t, *J* = 6.4 Hz, 1H), 4.63 (d, *J* = 6.4 Hz, 2H), 4.36 (d, *J* = 6.3 Hz, 2H), 3.13 (h, *J* = 6.9 Hz, 1H), 1.24 (d, *J* = 7.0 Hz, 6H). $^{13}$C NMR (101 MHz, DMSO) δ 156.8, 152.0, 141.2, 139.8, 134.9, 128.7 (2C), 128.4 (2C), 127.93 (2C), 127.89 (2C), 127.2, 126.9, 126.8, 125.8, 45.0, 43.1, 24.1, 21.1 (2C). MS (ESI$^+$): [M+H]$^+$ 379.3. LogD (HPLC): 4.60 |
| 122 | $C_{22}H_{23}FN_6$ | 99% [a] | Colorless oil. $^1$H NMR (400 MHz, DMSO-$d_6$) δ 8.74 (t, *J* = 6.3 Hz, 1H), 7.38 - 7.26 (m, 6H), 7.25 - 7.20 (m, 1H), 7.12 - 6.99 (m, 4H), 4.63 (d, *J* = 6.3 Hz, 2H), 4.33 (d, *J* = 6.3 Hz, 2H), 3.13 (h, *J* = 6.9 Hz, 1H), 1.24 (d, *J* = 6.9 Hz, 6H). $^{13}$C NMR (101 MHz, DMSO-$d_6$) δ 162.6, 160.2, 156.7, 152.0, 139.7, 134.9, 129.8, 129.7, 128.7 (2C), 127.8 (2C), 127.2, 126.8, 125.8, 115.2, 115.0, 44.3, 43.1, 24.1, 21.1 (2C). MS (ESI$^+$): [M+H]$^+$ 391.2. LogD (HPLC): 4.64 |
| 123 | $C_{21}H_{23}N_7$ | 95% [a] | Yellow oil. $^1$H NMR (400 MHz, DMSO-$d_6$) δ 8.76 (t, *J* = 6.4 Hz, 1H), 8.47 (dt, *J* = 4.7 &1.4 Hz, 1H), 7.67 (td, *J* = 7.7 & 1.8 Hz, 1H), 7.37 - 7.17 (m, 7H), 7.08 (s, 1H), 7.00 (t, *J* = 6.1 Hz, 1H), 4.61 (d, *J* = 6.4 Hz, 2H), 4.47 (d, *J* = 6.1 Hz, 2H), 3.06 (h, *J* = 6.9 Hz, 1H), 1.17 (d, *J* = 6.9 Hz, 6H). $^{13}$C NMR (101 MHz, DMSO-$d_6$) δ 160.3, 156.8, 152.0, 149.0, 139.7, 136.8, 134.9, 128.6 (2C), 127.9 (2C), 127.2, 126.8, 125.8, 122.1, 121.2, 47.0, 43.1, 24.1, 20.9 (2C). MS (ESI$^+$): [M+H]$^+$ 374.1. LogD (HPLC): 3.26 |
| 124 | $C_{21}H_{23}N_7$ | 97% [a] | Yellow powder. $^1$H NMR (400 MHz, DMSO-$d_6$) δ 8.77 (t, *J* = 6.3 Hz, 1H), 8.56 (d, *J* = 2.1 Hz, 1H), 8.40 (dd, *J* = 4.8, 1.6 Hz, 1H), 7.71 (d, *J* = 7.8 Hz, 1H), 7.36 - 7.26 (m, 5H), 7.26 - 7.19 (m, 1H), 7.10 - 7.08 (m, 2H), 4.63 (d, *J* = 6.3 Hz, 2H), 4.36 (d, *J* = 6.2 Hz, 2H), 3.12 (h, *J* = 6.9 Hz, 1H), 1.23 (d, *J* = 6.9 Hz, 6H). $^{13}$C NMR (101 MHz, DMSO-$d_6$) δ 156.6, 152.0, 149.5, 148.1, 139.7, 136.5, 135.6, 134.9, 128.7 (2C), 127.8 (2C), 127.2, 126.8, 125.8, 123.7, 43.1, 42.7, 24.1, 21.1 (2C). MS (ESI$^+$): [M+H]$^+$ 374.2. LogD (HPLC): 3.12 |
| 125 | $C_{21}H_{23}N_7$ | 99% [a] | White powder. $^1$H NMR (400 MHz, DMSO-$d_6$) δ 8.78 (t, *J* = 6.4 Hz, 1H), 8.47 - 8.40 (m, 2H), 7.35 - 7.18 (m, 7H), 7.13 (t, *J* = 6.3 Hz, 1H), 7.09 (s, 1H), 4.62 (d, *J* = 6.3 Hz, 2H), 4.37 (d, *J* = 6.2 Hz, 2H), 3.06 (h, *J* = 6.9 Hz, 1H), 1.18 (d, *J* = 6.9 Hz, 6H). $^{13}$C NMR (101 MHz, DMSO-$d_6$) δ 156.7, 152.0, 150.4, 149.7 (2C), 139.7, 134.9, 128.7 (2C), 127.9 (2C), 127.2, 126.8, 125.9, 122.7 (2C), 44.2, 43.1, 24.1, 20.9 (2C). MS (ESI$^+$): [M+H]$^+$ 374.2. LogD (HPLC): 3.07 |
| 126 | $C_{21}H_{24}N_8$ | > 98 % [a] | Light yellow solid. $^1$H NMR (400 MHz, DMSO-$d_6$) δ 8.79 (t, *J* = 6.4 Hz, 1H), 8.46 (s, 1H), 8.42 (s, 1H), 7.37 - 7.17 (m, 5H), 7.15 - 7.03 (s, 2H), 4.61 (d, *J* = 6.3 Hz, 2H), 4.46 (d, *J* = 6.0 Hz, 2H), 3.06 (h, *J* = 6.9 Hz, 1H), 2.43 (s, 3H), 1.18 (d, *J* = 6.9 Hz, 6H). $^{13}$C NMR (101 MHz, DMSO) δ 156.6, 152.6, 152.0, 151.6, 143.5, 142.5, 139.7, 134.9, 128.6 (2C), 127.9 (2C), 127.2, 126.8, 125.9, 44.9, 43.1, 24.1, 21.1, 20.9 (2C). MS (ESI$^+$): [M+H]$^+$ 389.3. LogD (HPLC): 3.00 |

(continued)

| N° | Formula | HPLC | Description |
|---|---|---|---|
| 127 | $C_{22}H_{26}N_8$ | 99% [a] | White solid. $^1$H NMR (400 MHz, DMSO-$d_6$) δ 8.74 (t, *J* = 6.4 Hz, 1H), 7.36 - 7.24 (m, 4H), 7.24 - 7.19 (m, 1H), 7.07 (d, *J* = 7.4 Hz, 2H), 6.64 (t, *J* = 5.8 Hz, 1H), 4.62 (d, *J* = 6.2 Hz, 2H), 4.46 (d, *J* = 5.7 Hz, 2H), 3.08 (h, *J* = 6.9 Hz, 1H), 2.36 (s, 6H), 1.20 (d, *J* = 6.9 Hz, 6H). $^{13}$C NMR (101 MHz, DMSO-$d_6$) δ 167.4, 166.6 (2C), 156.6, 151.9, 139.8, 134.8, 128.6 (2C), 127.9 (2C), 127.2, 126.8, 125.9, 118.3, 47.7, 43.1, 24.1, 23.9 (2C), 20.9 (2C). MS (ESI$^+$): [M+H]$^+$ 403.3. LogD (HPLC): 3.28 |
| 128 | $C_{19}H_{22}N_8O_2S$ | >98% [a] | White solid. $^1$H NMR (400 MHz, DMSO-$d_6$) δ 11.80 (br s, 1H), 9.25 (br s, 1H), 7.48 (d, *J* = 2.0 Hz, 1H), 7.40 - 7.20 (m, 6H), 6.79 (s, 1H), 4.61 (d, *J* = 6.4 Hz, 2H), 4.01 (s, 3H), 3.09 (h, *J* = 6.9 Hz, 1H), 1.25 (d, *J* = 6.9 Hz, 6H).$^{13}$C NMR (101 MHz, DMSO-$d_6$) δ 152.2, 139.1, 137.6, 136.0, 128.7 (2C), 128.1 (2C), 127.4, 127.2, 126.9, 112.0, 43.3, 38.5, 24.1, 21.1 (2C) Two peaks are too weak or overlap. MS (ESI$^+$): [M+H]$^+$ 427.1. |
| 129 | $C_{20}H_{24}N_8O_2S$ | >98% [a] | White solid. $^1$H NMR (400 MHz, DMSO-$d_6$) δ 11.06 (br s, 1H), 9.22 (t, *J* = 6.4 Hz,, 1H), 8.17 (s, 1H), 7.45 - 7.20 (m, 6H), 4.63 (d, *J* = 6.3 Hz, 2H), 3.68 (s, 3H), 3.13 (h, *J* = 6.9 Hz, 1H), 2.28 (s, 3H), 1.25 (d, *J* = 6.9 Hz, 6H).$^{13}$C NMR (101 MHz, DMSO-$d_6$) δ 152.2, 151.2, 146.5, 139.2, 136.0 (2C), 128.7 (2C), 128.3 (2C), 127.4, 127.1, 126.8, 119.7, 43.5, 38.9, 24.1, 21.1 (2C), 12.3. MS (ESI$^+$): [M+H]$^+$ 441.1. |
| 130 | $C_{20}H_{24}N_8O_2S$ | >98% [a] | White solid. $^1$H NMR (400 MHz, DMSO-$d_6$) δ 11.73 (br s, 1H), 9.26 (br s, 1H), 7.37 - 7.20 (m, 6H), 6.59 (s, 1H), 4.62 (d, *J* = 6.3 Hz, 2H), 3.93 (s, 3H), 3.11 (h, *J* = 6.9 Hz, 1H), 2.06 (s, 3H), 1.25 (d, *J* = 7.0 Hz, 6H).$^{13}$C NMR (101 MHz, DMSO-$d_6$) δ 152.3, 146.0, 139.1, 136.0, 128.7 (2C), 128.1 (2C), 127.4, 127.3, 126.9, 111.2, 43.3, 38.1, 24.1, 21.1 (2C), 13.3. Two peaks are too weak or overlap. MS (ESI$^+$): [M+H]$^+$ 441.1. |
| 131 | $C_{20}H_{24}N_8O_2S$ | >98% [a] (but 90% by NMR) | White solid. $^1$H NMR (400 MHz, DMSO-$d_6$) δ 11.75 (br s, 1H), 9.28 (br s, 1H), 7.40 - 7.15 (m, 7H), 4.61 (d, *J* = 6.4 Hz, 2H), 4.00 (s, 3H), 3.07 (h, *J* = 6.9 Hz, 1H), 2.19 (s, 3H), 1.21 (d, *J* = 6.9 Hz, 6H).$^{13}$C NMR (101 MHz, DMSO-$d_6$) δ 152.3, 139.0, 138.8, 136.1, 128.7 (2C), 128.2 (2C), 127.5, 127.2, 126.8, 43.3, 39.3, 24.0, 21.2 (2C), 10.0 Three peaks are too weak or overlap. MS (ESI$^+$): [M+H]$^+$ 441.1. |
| 132 | $C_{19}H_{24}N_6O$ | > 97% [a] | White solid. $^1$H NMR (400 MHz, DMSO-$d_6$) δ : 8.75 (t, *J* = 6.4 Hz, 1H), 7.38 - 7.26 (m, 4H), 7.26 - 7.17 (m, 1H), 7.12 (s, 1H), 6.59 (d, *J* = 5.6 Hz, 1H), 4.65 (d, *J* = 6.4 Hz, 2H), 4.18 (h, *J* = 5.0 Hz, 1H), 3.86 (dd, *J* = 8.8, 6.1 Hz, 1H), 3.79 (q, *J* = 7.5 Hz, 1H), 3.68 (td, *J* = 8.0, 5.8 Hz, 1H), 3.54 (dd, *J* = 8.8, 4.5 Hz, 1H), 3.17 (hept, *J* = 7.0 Hz, 1H), 2.17 - 1.83 (m, 2H), 1.30 (dd, *J* = 6.9, 2.8 Hz, 6H). $^{13}$C NMR (101 MHz, DMSO-$d_6$) δ : 156.2, 151.5, 139.3, 134.4, 128.2, 127.3, 126.7, 126.4, 125.4, 72.8, 66.4, 51.7, 42.6, 31.8, 23.7, 20.6, 20.6.. MS (ES+, m/z) : [M+H+] 353.32. |
| 133 | $C_{20}H_{24}N_8$ | > 97% [a] | Yellow solid. $^1$H NMR (400 MHz, DMSO-$d_6$) δ 9.01 (t, *J* = 6.4 Hz, 1H), 8.35 (s, 1H), 7.32 - 7.15 (m, 7H), 4.52 (d, *J* = 6.2 Hz, 2H), 3.56 (s, 3H), 3.11 (h, *J* = 6.9 Hz, 1H), 1.83 (s, 3H), 1.25 (d, *J* = 6.9 Hz, 6H).$^{13}$C NMR (101 MHz, DMSO-$d_6$) δ 155.9, 152.2, 139.3, 137.8, 136.1, 135.3, 128.6 (2C), 128.4 (2C), 127.3, 126.9, 126.4, 110.0, 43.3, 35.7, 24.0, 21.3 (2C), 8.8. MS (ESI$^+$): [M+H]+ 377.2. |
| [a]Acidic conditions, [b]Alkaline conditions | | | |

## PATHOLOGIES

Examples of diseases whose symptoms may be affected by ABC Transporter e.g. CFTR include, but are not limited to, Hereditary emphysema, Hereditary hemochromatosis, Coagulation-Fibrinolysis deficiencies, such as Protein C deficiency, Type 1 hereditary angioedema, Lipid processing deficiencies, Tangier Disease, Familial hypoapoproteinemia (ABCA1), Stargardt/fundus flavimaculatis, Retinitis pigmentosa & Cone-rod dystrophy & Age-related macular degeneration (ABCA4), Stargardt Macular disease (ABCA4), Harlequin ichthyosis (ABCA12), biliary diseases (such as Progressive familial intrahepatic cholestasis type 2 or 3 (PFIC2, PIFC3) (ABCB4, ABCB11), Dubin-Johnson Syndrome (ABCC2), Pseudoxanthoma elasticum (ABCC6), Cystic Fibrosis (ABCC7 = CFTR), idiopathic chronic cholestasis (ABCC12), Adrenoleukodystrophy (ABCD1), low-phospholipid associated cholelithiasis syndrome, intrahepatic cholestasis of pregnancy), Sitosteroliemia and Familial Hypercholesterolemia (ABCG5, ABCG8), Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophysiol DI, Nephrogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, several polyglutamine neurological disorders such as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler-Scheinker syndrome, COPD, dry-eye disease, and Sjogren's disease, (review in Borst P, Elferink RO, 2002. Mammalian ABC transporters in health and disease. Annu Rev Biochem. 71, 537->592. doi: 10.1146/annurev.biochem.71.102301.093055).

The compounds of formula (I) may in particular be useful in the treatment and/or in the prevention of Tangier Disease, Familial hypoapoproteinemia (ABCA1), Stargardt/fundus flavimaculatis, Retinitis pigmentosa & Cone-rod dystrophy & Age-related macular degeneration (ABCA4), Stargardt Macular disease (ABCA4), Harlequin ichthyosis (ABCA12), biliary diseases (such as Progressive familial intrahepatic cholestasis type 2 or 3 (PFIC2, PIFC3) (ABCB4, ABCB11), Dubin-Johnson Syndrome (ABCC2), Pseudoxanthoma elasticum (ABCC6), Cystic Fibrosis (ABCC7 = CFTR), idiopathic chronic cholestasis (ABCC12), Adrenoleukodystrophy (ABCD1), low-phospholipid associated cholelithiasis syndrome, intrahepatic cholestasis of pregnancy), Sitosteroliemia and Familial Hypercholesterolemia (ABCG5, ABCG8), and in particular Cystic Fibrosis, particularly in association with another ABC transporter modulator, including a CFTR modulator or a combination of said CFTR modulators.

ABC modulator, including a CFTR modulator

The ABC transporter modulator, in particular the CFTR modulator, may be selected from:

- Abbvie/Galapagos products GLPG 2222, GLPG 1837, GLPG 2451, their combinations such as GLPG 2222 and GLPG 1837, GLPG 2222 and GLPG 2451 and GLPG 2222, GLPG 1837 and GLPG 2451, galicaftor and navocaftor;
- products developed by Yumanity Therapeutics dirocaftor, nesolicaftor and posenacaftor; and
- products developed by Vertex lumacaftor, ivacaftor, tezacaftor and elexacaftor, and their combinations, in particular is selected from ivacaftor, tezacaftor and elexacaftor and more particularly is a mixture of ivacaftor, tezacaftor and elexacaftor, commercialized under the name Trikafta®.

Further CFTR modulators may be used in the framework of the present invention, as the one described in [Lopes-Pacheco M, 2020 CFTR Modulators: The Changing Face of Cystic Fibrosis in the Era of Precision Medicine., Front. Pharmacol. 10:1662 doi: 10.3389/fphar.2019.01662], as for example in figure 6 of such article.

Examples of ABC modulator, including a CFTR modulator include, but are not limited to ivacaftor, tezacaftor and/or elexacaftor, even more particularly from a mixture of ivacaftor, tezacaftor and elexacaftor such as commercialized under the trademark Trikafta®, lumacaftor, compounds developed by Galapagos (Abbvie) such as GLPG 2222 (**G1**), GLPG 1837 (**G2**), and GLPG 2451 (**G3**).

GLPG 1837, which is known as a CFTR potentiator, presents the following structure:

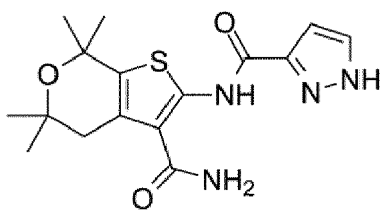

**[0137]** ,

GLPG 2451, which is known as a CFTR potentiator, presents the following structure:

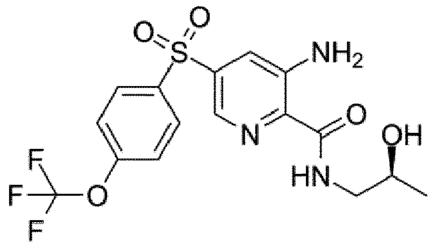

, and

GLPG 2222, which is known as CFTR potentiator, presents the following structure:

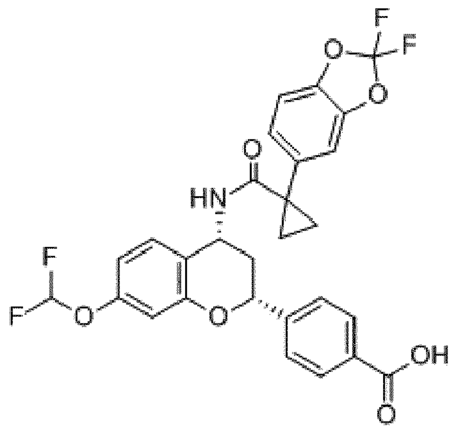

.

## COMBINATION

Herein is further provided a compound of formula (I) or any of its pharmaceutically acceptable salt or any compound of formula (1) to (133) as defined above, or any of its pharmaceutically acceptable salt, for use to reduce the dose of an ABC modulator, including a CFTR modulator or a combination of CFTR modulators, while preserving or increasing the said efficacy and/or safety to treat said variety of diseases as detailed above of said ABC modulator, including a CFTR modulator or a combination of said CFTR modulators.

It is more particularly herein provided a pharmaceutical combination of a compound of formula (I) as defined herein above, a pharmaceutically acceptable salt thereof with an ABC modulator, including a CFTR modulator or a combination of CFTR modulators or a pharmaceutically acceptable salt thereof.

Herein is also provided a pharmaceutical combination of a compound of formula (I) as defined herein above, a pharmaceutically acceptable salt thereof with an ABC modulator, including a CFTR modulator or a combination of CFTR modulators or a pharmaceutically acceptable salt thereof for use in the treatment of at least a disease as detailed above.

The compound of formula (I) as defined herein above or a pharmaceutically acceptable salt thereof and the ABC modulator or a combination of CFTR modulators, including a CFTR modulator or a pharmaceutically acceptable salt thereof may be used simultaneously, separately or may be spread out over time, and preferably simultaneously.

Accordingly, herein is provided a pharmaceutical combination of a compound of formula (I) as defined herein above or a pharmaceutically acceptable salt thereof and the ABC modulator, including a CFTR modulator or a combination of CFTR modulators or a pharmaceutically acceptable salt thereof for separate administration, administration spread out over time or simultaneous administration to patients suffering from at least on disease as detailed herein above, including CF.

Herein is further provided a pharmaceutical composition comprising a compound of formula (I) as defined herein above or a pharmaceutically acceptable salt thereof and the ABC modulator, including a CFTR modulator or a combination of CFTR modulators or a pharmaceutically acceptable salt thereof and at least one pharmaceutically acceptable excipient.

Herein is further provided a pharmaceutical kit, in particular intended for treating at least one of the herein above listed diseases, including CF, comprising:

(i) a first galenical formulation comprising a compound of formula (I) as defined herein above or a pharmaceutically acceptable salt thereof, and

(ii) a second galenical formulation comprising a ABC modulator or a combination of CFTR modulators, including a

CFTR modulator or a pharmaceutically acceptable salt thereof.

The separate administration, simultaneous administration or administration spread out over time of a medicinal combination means that the elementary constituents of the combination, can be administered at the same time, each in one go at distinct moments, or repeatedly, or else at different moments, in particular during cycles. The elementary constituents can, in order to do this, be formulated as mixtures, only if they are administered simultaneously, or else formulated separately for the other administration schemes.

The following examples are provided as illustrations and in no way limit the scope of this invention.

The following examples illustrate in detail the preparation of some compounds according to the invention. The structures of the products obtained have been confirmed by NMR analyses and mass spectroscopy.

**Example 1: Synthesis of compound (a) and compounds of formula (VII) for synthetic route 1**

**1.1.Intermediate 1: 2-Bromo-1,1-diethoxy-3-methyl-butane**

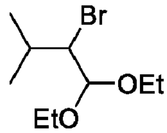

To a solution of freshly distilled 3-methylbutanal (1.0 eq, 383 mmol, 33.0 g) in abs. EtOH (190 mL), was added $Br_2$ (1.1 eq, 421 mmol, 21.6 mL). The mixture was heated to reflux for 4 h. $K_2CO_3$ (0.5 eq, 192 mmol, 26.5 g) was added to the cooled mixture and it was stirred at r.t. for 16 h. The mixture was diluted into pentane (150 mL) and sat. $Na_2S_2O_3$ (150 mL). The aqueous layer was extracted with pentane (2 x 150 mL). The combined organic layers were washed with brine, dried over $MgSO_4$, filtered and concentrated. The crude residue was purified by distillation to afford **Intermediate 1** as a colorless liquid (65.5 g, 274 mmol, 72 % yield).

NMR $^1H$ (400 MHz, DMSO-$d_6$) : δ (ppm) = 4.58 (d, *J* = 7.3 Hz, 1H), 4.11 (dd, *J* = 7.3, 3.0 Hz, 1H), 3.70 - 3.58 (m, 2H), 3.58 - 3.48 (m, 2H), 2.02 (ddp, *J* = 9.6, 6.6, 3.0 Hz, 1H), 1.13 (td, *J* = 7.1, 2.4 Hz, 6H), 0.95 (d, *J* = 6.8 Hz, 3H), 0.89 (d, *J* = 6.5 Hz, 3H).

**1.2. Intermediate 2: (1-Bromo-2,2-diethoxy-ethyl)cyclopentane**

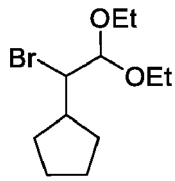

To a solution of 2-cyclopentylacetaldehyde (1.0 eq, 7.85 mmol, 0.880 g) in EtOH (4.5 mL), was added $Br_2$ (1.1 eq, 8.63 mmol, 0.44 mL). The mixture was heated to reflux for 5 h. $K_2CO_3$ (0.5 eq, 3.92 mmol, 0.542 g) was added to the cooled mixture and it was stirred for 16 h. The mixture was diluted into pentane (20 mL) and sat. $Na_2S_2O_3$ (20 mL). The aqueous layer was extracted with pentane (2 x 20 mL). The combined organic layers were washed with brine, dried over $MgSO_4$, filtered and concentrated to afford **Intermediate 2** as a crude yellow oil (1.35 g, 85 % pure, 4.31 mmol, 55 % crude yield). It was used without further purification.

**1.3. Compound (b): 6-Amino-2*H*-1,2,4-triazine-3,5-dithione**

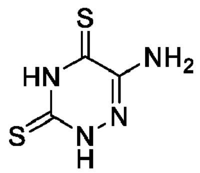

To a solution of 6-amino-2*H*-1,2,4-triazine-3,5-dione (1.0 eq, 15.6 mmol, 2.00 g) in pyridine (100 mL), was added $P_2S_5$ (4.0 eq, 31.2 mmol, 13.9 g). The mixture was heated to reflux for 5 h. The mixture was cooled down to rt and $S_8$ was added. It was stirred at r.t. for 16 h. The mixture was filtered. The filtrate was concentrated. Toluene (3 x 60 mL) was added to the residue for coevaporation. The crude residue was recrystallized in $H_2O$ (50 mL) to afford **compound** (**b**) as a brown solid (4.97 g, quant.).

**1.4. Compound (a): 3,5-Bis(methylsulfanyl)-1,2,4-triazin-6-amine**

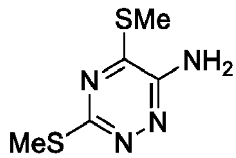

To a solution of **Compound (b)** (15.6 mmol) in DCM (100 mL), were added iodomethane (2.2 eq, 34.4 mmol, 2.1 mL) and DIPEA (6.0 eq, 93.7 mmol, 15.1 mL). The mixture was stirred at r.t. for 16 h. The mixture was concentrated and purified by flash chromatography on silica gel with cHex/EtOAc (1:0 to 2:8 v/v) to afford **Compound (a)** as a yellow solid (1.28 g, 6.79 mmol, 44 % yield over 2 steps).

NMR $^1H$ (400 MHz, CD6CN): δ (ppm) = 5.11 (br. s, 2H), 2.57 (s, 3H), 2.55 (s, 3H). MS (ESI+, m/z): [M+H+] 189.02

**Example 2: Synthesis of compounds of formula (IV) for synthetic route 1**

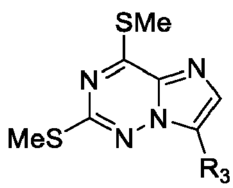
(IV)

**GP1:** To a solution of **Compound (a)** (1.0 eq) and camphorsulfonic acid (0.1 eq) in dry MeCN (0.2 M) under inert atmosphere, was added the corresponding (2-bromo-1,1-diethoxy-ethyl)alkane (2.0 eq). The mixture was heated to reflux for t. Most of MeCN was removed in *vaccuo.* The mixture was diluted into DCM (50 mL) and sat. $NaHCO_3$ (50 mL). The aqueous layer was extracted with DCM (2 x 50 mL). The combined organic layers were washed with brine, dried over $MgSO_4$, filtered and concentrated. The crude residue was purified by flash chromatography on silica gel with cHex/EtOAc.

**2.1 Intermediate 3: 7-Isopropyl-2,4-bis(methylsulfanyl)imidazo[2,1-*f*][1,2,4]triazine**

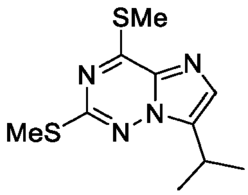

Red solid (0.902 g, 90 % pure, 3.19 mmol, 47 % yield) obtained from **Compound (a)** (6.82 mmol, 1.28 g) and **Intermediate 1** (13.6 mmol, 3.30 g) according to the **GP1**, with t = 64 h, purified by flash chromatography on silica gel with cHex/EtOAc (1:0 to 1:1 v/v).

NMR $^1H$ (400 MHz, DMSO-$d_6$) : δ (ppm) = 7.54 (s, 1H), 3.40 - 3.28 (hept, *J* = 6.9 Hz, 1H), 2.62 (s, 3H), 2.58 (s, 3H), 1.35 (d, *J* = 6.9 Hz, 6H).

MS (ESI+, m/z) : [M+H+] 255.07

**2.2. Intermediate 4: 2,4-Bis(methylsulfanyl)imidazo[2,1-*f*][1,2,4]triazine**

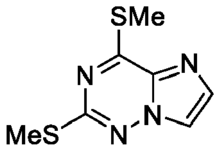

Beige solid (0.488 g, 2.30 mmol, 72 % yield) obtained from **Compound (a)** (3.19 mmol, 0.600 g) and 2-chloro-1,1-dimethoxy-ethane (6.37 mmol, 0.96 mL), according to the **GP1**, with t = 20 h, purified by flash column chromatography on silica gel with cHex/EtOAc (1:0 to 7:3 v/v).

NMR $^1H$ (400 MHz, DMSO-$d_6$): δ (ppm) = 8.26 (s, 1H), 7.71 (s, 1H), 2.63 (d, *J* = 1.5 Hz, 3H), 2.57 (d, *J* = 1.5 Hz, 3H).

**2.3. Intermediate 5: 7-Cyclopentyl-2,4-bis(methylsulfanyl)imidazo[2,1-*f*][1,2,4]triazine**

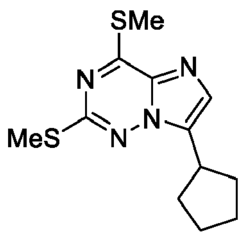

Brown solid (0.145 g, 0.517 mmol, 24 % yield) obtained from **Compound (a)** (2.16 mmol, 0.406 g) and **Intermediate 2** (4.31 mmol, 1.35 g) according to the general procedure, with unknown t as heating stopped during the weekend, purified by flash column chromatography on silica gel with cHex/EtOAc (1:0 to 7:3 v/v).

NMR $^1H$ (400 MHz, DMSO-$d_6$): δ (ppm) = 7.57 (s, 1H), 3.42 (p, *J* = 7.6 Hz, 1H), 2.62 (s, 3H), 2.58 (s, 3H), 2.18 - 2.06 (m, 2H), 1.84 - 1.62 (m, 6H).

**Example 3: Synthesis of compounds of formula (III) for synthetic route 1**

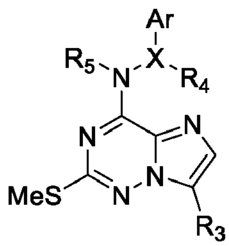

(III)

**GP2:** To a solution of a compound of formula (IV) as obtained in **Example 2** above (1.0 eq) in THF (0.2 M), was added benzylamine (3.5 eq). The mixture was heated to reflux for 16 h. The mixture was diluted into DCM and HCl 1 M or sat. $NH_4Cl$. The aqueous layer was extracted with DCM (2 x). The combined organic layers were dried over $MgSO_4$, filtered and concentrated. The crude residue was purified either by trituration or by flash chromatography on silica gel to give compound of formula (III).

**3.1. Intermediate 6: *N*-Benzyl-7-isopropyl-2-methylsulfanyl-imidazo[2,1-*f*][1,2,4]triazin-4-amine**

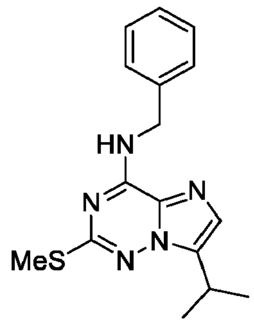

White solid (0.476 g, 1.52 mmol, 86 % yield) obtained from **Intermediate 3** (1.76 mmol, 0.448 g) according to the **GP2,** purified by trituration into cHex.

NMR $^1$H (400 MHz, DMSO-*d*$_6$): δ (ppm) = 9.31 (t, *J* = 6.3 Hz, 1H), 7.38 - 7.28 (m, 5H), 7.27 - 7.20 (m, 1H), 4.66 (d, *J* = 6.4 Hz, 2H), 3.25 (p, *J* = 6.9 Hz, 1H), 2.46 (s, 3H), 1.32 (d, *J* = 6.9 Hz, 6H).

MS (ESI+, m/z) : [M+H+] 314.14

**3.2. Intermediate 7: *N*-Benzyl-2-methylsulfnayl-imidazo[2,1-*f*][1,2,4]triazin-4-amine**

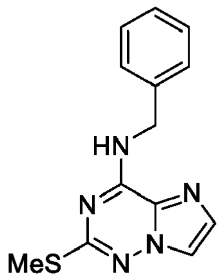

Fluffy light yellow solid (0.520 g, 1.92 mmol, 83 % yield) obtained from **Intermediate 4** (2.30 mmol, 0.488 g) according to the **GP2,** purified by flash column chromatography on silica gel with cHex/EtOAc (1:0 to 4:6 v/v).

NMR $^1$H (400 MHz, DMSO-*d*$_6$): δ (ppm) = 9.42 (t, *J* = 6.3 Hz, 1H), 7.97 (d, *J* = 1.1 Hz, 1H), 7.51 (d, *J* = 1.1 Hz, 1H), 7.40 - 7.28 (m, 4H), 7.28 - 7.19 (m, 1H), 4.67 (d, *J* = 6.3 Hz, 2H), 2.44 (s, 3H).

**3.3. Intermediate 8: *N*-Benzyl-7-cyclopentyl-2-methylsulfanyl-imidazo[2,1-*f*][1,2,4]triazin-4-amine**

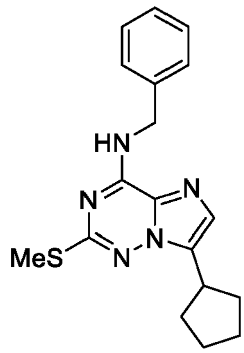

Fluffy light brown solid (0.139 g, 0.409 mmol, 84 % yield) obtained from **Intermediate 5** (0.489 mmol, 0.137 g) according to the **GP2,** purified by flash column chromatography on silica gel with cHex/EtOAc (1:0 to 7:3 v/v).

NMR $^1$H (400 MHz, DMSO-$d_6$): δ (ppm) = 9.29 (t, *J* = 6.3 Hz, 1H), 7.36 - 7.27 (m, 5H), 7.26 - 7.20 (m, 1H), 4.65 (d, *J* = 6.3 Hz, 2H), 3.39 - 3.26 (m, 1H), 2.45 (s, 3H), 2.18 - 2.00 (m, 2H), 1.84 - 1.59 (m, 6H).

**Example 4: Synthesis of compounds of formula (II) for synthetic route 1**

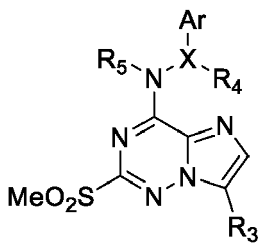

(II)

**GP3:** To a solution of a compound of formula (III) as obtained in **Example 3** above (1.0 eq) in MeOH (0.5 M), was added a solution of Oxone® (4.0 eq) in $H_2O$ (0.5M). The mixture was stirred at r.t. for 20 h. Most of MeOH was removed in *vaccuo.* The residue was diluted into DCM and $H_2O$. The aqueous layer was extracted with DCM (2 x). The combined organic layers were washed with brine, dried over $MgSO_4$, filtered and concentrated. The crude product was directly used or purified by flash chromatography to give pure compound of formula (II).

**4.1. Intermediate 9: *N*-Benzyl-7-isopropyl-2-methylsulfonyl-imidazo[2,1-*f*][1,2,4]triazin-4-amine**

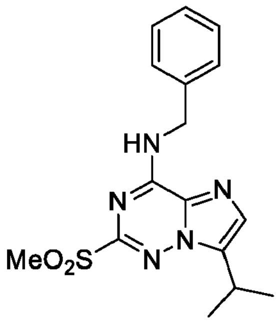

White solid (0.120 g, 0.347 mmol, 75 % yield) obtained from **Intermediate 6** (0.464 mmol, 0.145 g) according to the **GP3,** purified by flash chromatography on silica gel with cHex/EtOAc (1:0 to 6:4 v/v).

NMR $^1$H (400 MHz, DMSO-$d_6$): δ (ppm) = 9.96 (t, *J* = 6.3 Hz, 1H), 7.55 (s, 1H), 7.41 (d, *J* = 7.6 Hz, 2H), 7.32 (t, *J* = 7.6 Hz, 2H), 7.28 - 7.22 (m, 1H), 4.74 (d, *J* = 6.2 Hz, 2H), 3.35 - 3.25 (m, 1H), 2.50 (s under DMSO signal, 3H), 1.34 (d, *J* = 6.9 Hz, 6H). MS (ESI+, m/z): [M+H+] 346.13

**4.2. Intermediate 10: *N*-Benzyl-2-methylsulfonyl-imidazo[2,1-*f*][1,2,4]triazin-4-amine**

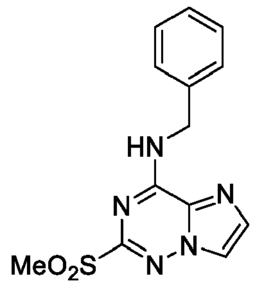

White foam (0.580 g, no isolated yield) obtained from **Intermediate 7** (1.92 mmol, 0.520 g) according to the **GP3** and used without further purification.

**4.3. Intermediate 11: *N*-Benzyl-7-cyclopentyl-2-methylsulfonyl-imidazo[2,1-*f*][1,2,4]triazin-4-amine**

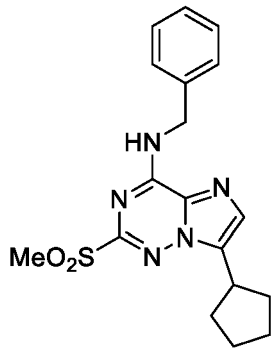

Light yellow foam (0.148 g, no isolated yield) obtained from **Intermediate 8** (0.409 mmol, 0.139 g) according to the **GP3** and used without further purification.

**<u>Example 5</u>: Synthesis of compounds of formula (I) for synthetic route 1**

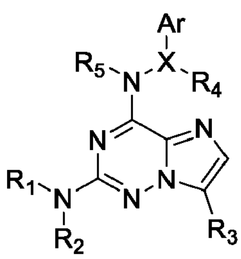

(I)

**GP4:** To a compound as obtained in **Example 4** above was added R-$NH_2$ (with $NEt_3$ in case of a hydrochloride salt of R-$NH_2$). The mixture was heated to T for t in a sealed vial. The mixture was diluted into DCM (15 mL) and sat. $NH_4Cl$ (15 mL). The aqueous layer was extracted with DCM (2 x 15 mL). The combined organic layers were washed with brine, dried over $MgSO_4$, filtered and concentrated. The crude product was purified by flash chromatography on silica gel and a second time with TLC prep if necessary to afford the expected compound of formula (**I**). When needed, the product was triturated into a mixture of $Et_2O$ and pentane or pentane alone.

Purifications conditions established following TLC reaction analysis with pure solvent or mixture thereof define before **Table 2.** All analytical data are in **Table 3** for each final compound.

**5.1. (2*R*)-2-[[4-(Benzylamino)-7-isopropyl-imidazo[2,1-*f*][1,2,4]triazin-2-yl]amino]butan-1-ol (6)**

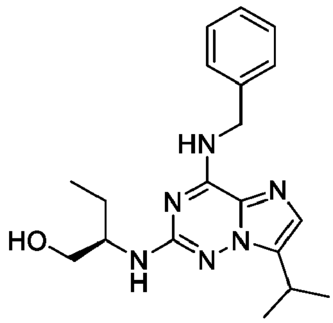

White solid (0.030 g, 0.0846 mmol, 30 % yield) obtained from **Intermediate 9** (1.0 eq, 0.278 mmol, 0.096 g) and (*R*)-2-aminobutan-1-ol (10.0 eq, 2.78 mmol, 0.26 mL) according to the **GP4,** with T = 170 °C and t = 11 h, purified by flash chromatography on silica gel with cHex/EtOAc (1:0 to 2:8 v/v).

**5.2. *N4*-Benzyl-7-isopropyl-*N2*-[(3*R*)-tetrahydrofuran-3-yl]imidazo[2,1-*f*][1,2,4]triazine-2,4-diamine (19)**

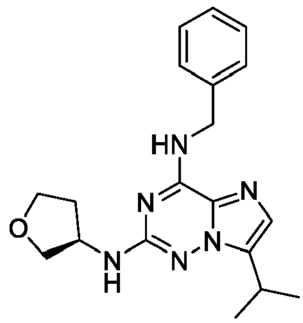

White solid (0.047 g, 0.133 mmol, 18 % yield) obtained from **Intermediate 9** (1.0 eq, 0.724 mmol, 0.250 g) and (*R*)-3-aminotetrahydrofuran (3.5 eq, 2.53 mmol, 0.22 mL) according to the **GP4,** with T = 160 °C and t = 64 h, purified by flash chromatography on silica gel with cHex/EtOAc (1:0 to 6:4 v/v).

**5.3. *N4*-Benzyl-7-isopropyl-*N2*-(3-methoxypropyl)imidazo[2,1-*f*][1,2,4]triazine-2,4-diamine (4)**

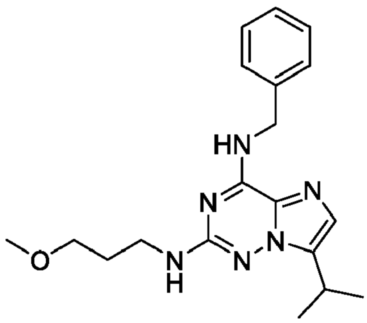

White solid (0.034 g, 0.0959 mmol, 24 % yield) obtained from **Intermediate 9** (1.0 eq, 0.405 mmol, 0.140 g) and 3-methoxypropylamine (10.0 eq, 4.053 mmol, 0.42 mL) according to the **GP4,** with T = 90 °C and t = 96 h, purified by flash chromatography on silica gel with cHex/EtOAc (1:0 to 1:1 v/v).

**5.4. *N4*-Benzyl-7-isopropyl-*N2*-[(3*R*)-tetrahydropyran-3-yl]imidazo[2,1-*f*][1,2,4]triazine-2,4-diamine (44)**

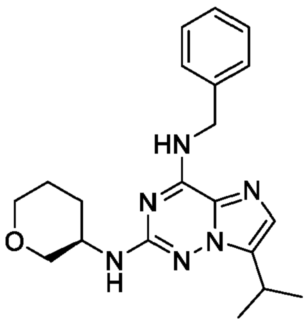

White solid (0.055 g, 0.150 mmol, 21 % yield) obtained from **Intermediate 9** (1.0 eq, 0.724 mmol, 0.250 g) and (*R*)-3-aminotetrahydro-2H-pyran hydrochloride (3.5 eq, 2.53 mmol, 0.349 g) according to the **GP4,** with T = 160 °C and t = 168 h, purified by flash chromatography on silica gel with cHex/EtOAc (1:0 to 6:4 v/v).

HPLC: $t_R$ = 12.55 min ; 98 %

**5.5. *N4*-Benzyl-*N2*-[(3*R*)-tetrahydrofuran-3-yl]imidazo[2,1-*f*][1,2,4]triazine-2,4-diamine (33)**

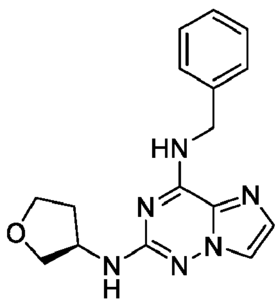

White solid (0.022 g, 0.0709 mmol, 12 % yield over 2 steps) obtained from **Intermediate 10** (1.0 eq, 0.595 mmol, 0.180 g) and (*R*)-3-aminotetrahydrofuran (10.0 eq, 5.95 mmol, 0.51 mL) according to the **GP4,** with T = 120 °C and t = 16 h, purified by flash chromatography with DCM/MeOH (1:0 to 98:2 v/v).

HPLC: $t_R$ = 10.28 min ; > 99 %

### 5.6. *N4*-Benzyl-7-cyclopentyl-*N2*-[(3*R*)-tetrahydrofuran-3-yl]imidazo[2,1-*f*][1,2,4]triazine-2,4-diamine (38)

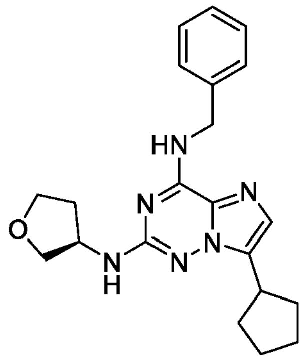

White solid (0.016 g, 0.0423 mmol, 10 % yield over 2 steps) obtained from **Intermediate 11** (1.0 eq, 0.398 mmol, 0.148 g) and (*R*)-3-aminotetrahydrofuran (10.0 eq, 3.98 mmol, 0.34 mL) according to the **GP4,** with T = 120 °C and t = 24 h, purified by flash chromatography with cHex/EtOAc (1:0 to 75:25 v/v).

HPLC : $t_R$ = 13.37 min; 98 %

### Example 6: Synthesis of compound (d), (e), (f), (g), (h) and compounds of formula (IX), (X), (XIa), (XIb), (XIIa), (XIIb), (XIII), (XVII), (XVIII) for synthetic route 2

### 6.1. Intermediate 12: 2-(3-Methyl-2-oxo-butyl)isoindoline-1,3-dione

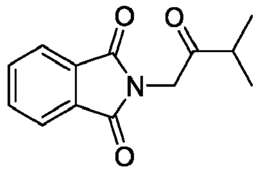

To a solution of 1-bromo-3-methyl-butan-2-one (1.0 eq, 283 mmol, 46.8 g) in DMF (200 mL), was added potassium phthalimide (1.0 eq, 283 mmol, 52.5 g). The mixture was stirred at r.t. for 4 h. The mixture was diluted into EtOAc (400 mL), washed with HCl 1 M (800 mL) and brine, dried over $MgSO_4$, filtered and concentrated. The residue was triturated in cHex (100 mL) to afford Intermediate 12 as a white solid (62.1 g, 269 mmol, 95 % yield). NMR $^1H$ (400 MHz, DMSO-$d_6$): δ (ppm) = 7.89 (m, 4H), 4.63 (s, 2H), 2.86 (hept, *J* = 6.9

Hz, 1H), 1.09 (d, *J* = 6.9 Hz, 6H).

HPLC : $t_R$ = 2.49 min ; 86 %

MS (ES+, m/z) : [M+H+] 232.1

### 6.2. Intermediate 13: 1-Amino-3-methyl-butan-2-one hydrochloride

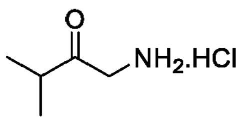

**Intermediate 12** (1.0 eq, 40.9 mmol, 9.45 g) was dissolved into AcOH (102 mL) and HCl 6 M (102 mL). The mixture was heated to 120 °C for 24 h. The mixture was cooled down to 0 °C and let stand at 0 °C overnight for precipitation. The white solid was filtered and the filtrate was concentrated to dryness to afford **Intermediate 13** as a light brown solid (8.27 g, quant.).

NMR $^1H$ (400 MHz, DMSO-$d_6$): δ (ppm) = 8.29 (br. S, 3H), 3.98 (q, *J* = 5.7 Hz, 2H), 2.73 (hept, *J* = 6.9 Hz, 1H), 1.05 (d, *J* = 6.9 Hz, 6H).
HPLC : $t_R$ = 1.22 min ; 83 %
MS (ES+, m/z) : [M+2Na+] 148.9

### 6.3. Intermediate 14: (2-Ethoxy-1-methylsulfanyl-2-oxo-ethyldiene)ammonium tetrafluoroborate

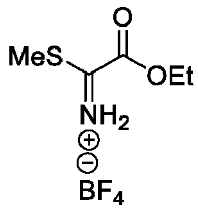

To a solution of **intermediate 13** (1.0 eq, 120 mmol, 16.0 g) in DCM (400 mL), was added trimethyloxonium tetrafluoroborate (1.2 eq, 144 mmol, 21.3 g). The mixture was stirred at r.t. for 16 h. The mixture was concentrated to dryness and used in next step with no further work-up nor purification.

### 6.4. Intermediate 15: Ethyl 5-isopropyl-1H-imidazole-2-carboxylate

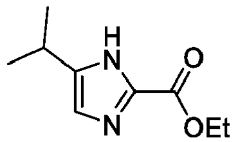

To a solution of **Intermediate 14** (1.0 eq, 120 mmol) in AcOH (240 mL), were added NaOAc.3$H_2O$ (1.25 eq, 150 mmol, 20.4 g) and **Intermediate 13** (0.78 eq, 94.8 mmol, 90 %, 14.5 g). The mixture was heated at 100 °C for 4 h. The mixture was cooled down and most of AcOH was removed in *vaccuo.* Sat. $NaHCO_3$ was added until pH 8. EtOAc (250 mL) was added. The aqueous layer was extracted with EtOAc (2 x 200 mL). The combined organic layers were washed with brine, dried over $MgSO_4$, filtered and concentrated. The crude residue was purified by flash chromatography on silica gel with cHex/EtOAc (1:0 to 2:8 v/v) to afford **Intermediate 15** as a white solid (15.4 g, 84.7 mmol, 89 % yield).
NMR $^1H$ (400 MHz, DMSO-$d_6$) : δ (ppm) = 12.99 (br. s, 1H), 7.06 (s, 0.5 H), 6.85 (s, 0.5H), 4.28 (q, *J* = 7.1 Hz, 2H), 2.93 (hept, *J* = 6.9 Hz, 0.5H), 2.81 (hept, *J* = 6.9 Hz, 0.5H), 1.30 (t, *J* = 7.1 Hz, 3H), 1.21 (d, *J* = 6.9 Hz, 3H), 1.18 (d, *J* = 6.9 Hz, 3H).
HPLC : $t_R$ = 1.30 min ; > 99 %
MS (ES+, m/z) : [M+H+] 183.1

### 6.5. Intermediates 16 & 17: Ethyl 1-amino-5-isopropyl-imidazole-2-carboxylate & Ethyl 1-amino-4-isopropyl-imidazole-2-carboxylate

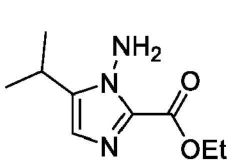

+

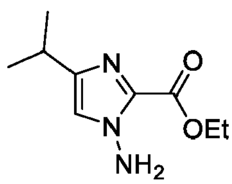

To a solution of **Intermediate 15** (1.0 eq, 6.12 mmol, 1.12 g) in DMF (30 mL), was added tBuOK 1 M in THF (1.05 eq, 6.43 mmol, 6.4 mL). The mixture was stirred at r.t. for 20 min. *O*-(4-nitrobenzoyl)hydroxylamine **79** (1.05 eq, 6.43 mmol, 1.17 g) was then added to the mixture. It was stirred at r.t. for 3 h. The mixture was filtered off and the solid

was washed with MeCN (2 x). The filtrate was diluted into DCM (80 mL) and $H_2O$ (300 mL). The aqueous layer was extracted with DCM (2 x 80 mL). The combined organic layers were washed with brine, dried over $MgSO_4$, filtered and concentrated (coevap. with hexanes) to afford a mixture of **Intermediate 16** and **17** as a yellow oil (1.19 g, 6.02 mmol, 98 % yield). **71** : NMR $^1H$ (400 MHz, DMSO-$d_6$) : δ (ppm) = 6.80 (s, 1H), 6.26 (s, 2H), 4.28 (q, J = 7.1 Hz, 2H), 3.05 (hept, J = 6.9 Hz, 1H), 1.30 (t, J = 7.1, 3H), 1.21 (d, J = 6.9 Hz, 6H). **78** : NMR $^1H$ (400 MHz, DMSO-$d_6$) : δ (ppm) = 7.12 (s, 1H), 6.51 (s, 2H), 4.28 (q, *J* = 7.1 Hz, 2H), 2.75 (hept, *J* = 6.9 Hz, 1H), 1.30 (t, *J* = 7.1, 3H), 1.15 (d, *J* = 6.9 Hz, 6H).

**6.6. Compound (g): Ethyl 1-aminoimidazole-2-carboxylate**

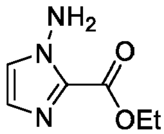

To a solution of commercial **Compound (h)** (1.0 eq, 42.8 mmol, 6.00 g) in DMF (200 mL), was added *t*BuOK 1 M in THF (1.05 eq, 45.0 mmol, 45 mL). The mixture was stirred at r.t. for 20 min. *O*-(4-nitrobenzoyl)hydroxylamine (1.05 eq, 45.0 mmol, 6.19 g) was then added. The mixture was stirred at r.t. for 3 h. The precipitate was filtered and washed with MeCN (2 x 80 mL). The filtrate was diluted into EtOAc (200 mL) and $H_2O$ (800 mL). The aqueous layer was extracted with EtOAc (2 x 200 mL). The combined organic layers were washed with brine, dried over $MgSO_4$, filtered and concentrated to afford **Compound (g)** as an orange oil (5.85 g, 37.7 mmol, 89 % yield).

**6.7. Intermediate 18 & 19: Ethyl 1-[bis(ethoxycarbonyl)amino]-5-isopropyl-imidazole-2-carboxylate & Ethyl 1-[bis(ethoxycarbonyl)amino]-4-isopropyl-imidazole-2-carboxylate**

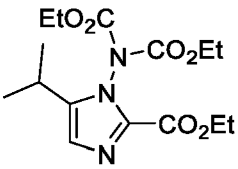

+

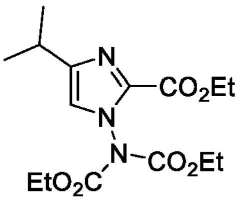

To a solution of **intermediates 16 & 17** (1.0 eq, 6.02 mmol, 1.19 g) in THF/$H_2O$ 1:1 (30.0 mL), were added $NaHCO_3$ (7.0 eq, 42.2 mmol, 3.54 g) and ethyl chloroformate (7.0 eq, 42.2 mmol, 2.74 mL). The mixture was stirred at r.t. until completion (here, 110 h). The mixture was diluted into EtOAc (50 mL) and $H_2O$ (50 mL). The aqueous layer was extracted with EtOAc (2 x 50 mL). The combined organic layers were washed with brine, dried over $MgSO_4$, filtered and concentrated. The two isomers were separated by flash chromatography on silica gel with cHex/EtOAc (1:0 to 6:4 v/v with a long hold at 7:3 v/v). Both isomers are afforded as slightly yellow oils (**Intermediate 18** : 0.662 g, 1.94 mmol, 32 % yield ; **Intermediate 19** : 0.979 g, 2.87 mmol, 48 % yield).

**Intermediate 18:** NMR $^1H$ (400 MHz, DMSO-$d_6$) : δ (ppm) = 7.06 (s, 1H), 4.25 (q, *J* = 7.1 Hz, 6H), 2.72 (hept., *J* = 6.9 Hz, 1H), 1.24 (t, *J* = 7.1 Hz, 3H), 1.18 - 1.13 (m, 12H).

HPLC : $t_R$ = 2.65 min ; 86 %

MS (ES+, m/z) : [M+H+] 342.3

**Intermediate 19:** NMR $^1H$ (400 MHz, DMSO-$d_6$) : δ (ppm) = 7.48 (s, 1H), 4.27 - 4.20 (m, 6H), 2.81 (hept, *J* = 6.9 Hz, 1H), 1.25 (t, *J* = 7.1 Hz, 3H), 1.20 - 1.13 (m, 12H).

HPLC : $t_R$ = 2.67 min ; 97 %

MS (ES+, m/z) : [M+H+] 342.3

**6.8. Compound (f): Ethyl 1-[bis(ethoxycarbonyl)amino]imidazole-2-carboxylate**

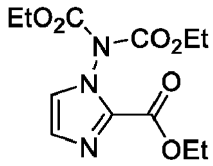

To a solution of **Compound (g)** (1.0 eq, 37.7 mmol, 5.85 g) in THF/$H_2O$ 1:1 (188 mL), were added $NaHCO_3$ (7.0 eq, 264 mmol, 22.2 g) and ethyl chloroformate (3.6 eq, 136 mmol, 12.9 mL). The mixture was stirred at r.t. for 16 h. The mixture was diluted into EtOAc (100 mL) and $H_2O$ (100 mL). The aqueous layer was extracted with EtOAc (2 x 100 mL). The combined organic layers were washed with brine, dried over $MgSO_4$, filtered and concentrated. The crude residue was purified by flash chromatography on silica gel with DCM/MeOH (1:0 to 97:3 v/v) to afford **Compound (f)** as a slightly yellow oil (7.80 g, 26.1 mmol, 70 %).

NMR $^1$H (400 MHz, DMSO-$d_6$): δ (ppm) = 7.77 (d, *J* = 1.3 Hz, 1H), 7.20 (d, *J* = 1.3 Hz, 1H), 4.32 - 4.18 (m, 6H), 1.25 (t, *J* = 7.1 Hz, 3H), 1.16 (t, *J* = 7.1 Hz, 6H).

HPLC : $t_R$ = 2.46 min ; 83 %

MS (ES+, m/z) : [M+H+] 300.2

**6.9. Intermediate 20: 7-Isopropyl-1H-imidazo[2,1-*f*][1,2,4]triazine-2,4-dione**

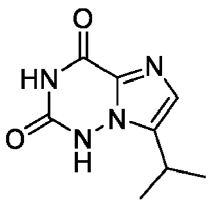

To a solution of **Intermediate 18** (1.0 eq, 17.5 mmol, 5.97 g) in iPrOH (30 mL), was added $NH_4OH$ 30 % in water (0.17 M, 90 mL). The mixture was heated to 120 °C for 16 h in a sealed flask. The mixture was cooled down and concentrated to dryness. The residue was triturated into $Et_2O$/MeOH 10:1 (3 x 28 mL) to afford **Intermediate 20** as a white powder (3.48 g, 17.9 mmol, quant.).

Alternatively, the mixture can be stirred at 120 °C for 4 h in a microwaves device.

NMR $^1$H (400 MHz, DMSO-$d_6$) : δ (ppm) = 9.66 (br. s, 1H), 7.10 (br. s, 1H), 6.87 (s, 1H), 3.05 (hept., *J* = 6.9 Hz, 1H), 1.21 (d, *J* = 6.9 Hz, 6H).

HPLC : $t_R$ = 0.57 min ; 82 %

MS (ES+, m/z) : [M+H+] 195.0

**Compound (e)**

**6.10. Compound (e): *1H*-Imidazo[2,1-*f*][1,2,4]triazine-2,4-dione**

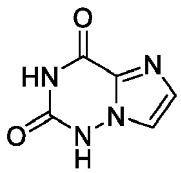

To a solution of **Compound (f)** (1.0 eq, 9.61 mmol, 2.88 g) in iPrOH (19.2 mL), was added $NH_4OH$ 30 % in water (0.17 M, 56.5 mL). It was split into 5 vials and stirred at 120 °C for 4 h under MW. The mixture was cooled down and concentrated to dryness. The residue was triturated into $Et_2O$/MeOH 10:1 (3 × 22 mL) to afford **Compound (e)** as a beige powder (1.67 g, 1.10 mmol, quant.).

Alternatively, the mixture can be stirred at 120 °C for 4 h in a microwaves device.

NMR $^1H$ (400 MHz, DMSO-$d_6$) : δ (ppm) = 9.86 (br. s, 1H), 7.22 (s, 1H), 7.13 (br. s, 1H), 7.06 (s, 1H).

HPLC : $t_R$ = 0.24 min; 79 %

MS (ES+, m/z) : [M+H+] 153.0

### 6.11. Intermediate 21: 2,4-Dichloro-7-isopropyl-imidazo[2,1-*f*][1,2,4]triazine

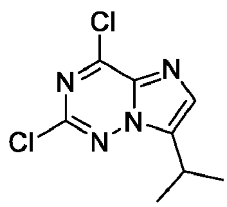

To **intermediate 20** (1.0 eq, 0.515 mmol, 0.100 g) were added $POCl_3$ (11.0 eq, 5.67 mmol, 0.53 mL) and *N*,*N*-dimethylaniline (2.0 eq, 1.03 mmol, 0.13 mL). The mixture was heated to 120 °C for 1 h in a sealed vial. The mixture was concentrated to dryness. The residue was diluted into DCM (30 mL) and poured into cold sat. $NaHCO_3$ (100 mL). The layers were separated. The aqueous layer was extracted with DCM (2 × 20 mL). The combined organic layers were washed with brine, dried over $MgSO_4$, filtered and concentrated to afford **Intermediate 21** as a green oil. It was used without further purification.

### 6.12. Compound (d): 2,4-Dichloroimidazo[2,1-*f*][1,2,4]triazine

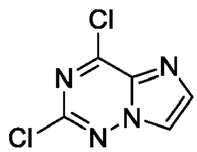

To **Compound (e)** (1.0 eq, 13.1 mmol, 2.00 g), were added $POCl_3$ (11.0 eq, 144 mmol, 13.5 mL) and *N,N*-dimethylaniline (2.0 eq, 26.3 mmol, 3.32 mL). The mixture was heated to 120 °C for 3 h in a sealed vial. The mixture was cooled down and concentrated to dryness. The residue was diluted into DCM (60 mL) and poured into cold sat. $NaHCO_3$. The aqueous layer was exctracted with DCM (2 × 120 mL). The combined organic layers were washed with brine, dried over $MgSO_4$, filtered and concentrated to afford **Compound (d)** as the crude product. It was used without further purification.

### 6.13. Intermediate 22: 7-bromo-*1H*-imidazo[2,1-*f*][1,2,4]triazine-2,4-dione

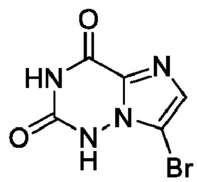

To a solution of **Compound (e)** (1.0 eq, 9.61 mmol, 2.88 g) in iPrOH (19.2 mL), was added $NH_4OH$ 30 % in water (0.17 M, 56.5 mL). It was split into 5 vials and stirred at 120 °C for 4 h under MW. The mixture was cooled down and concentrated to dryness. The residue was triturated into $Et_2O$/MeOH 10:1 (3 × 22 mL) to afford **Intermediate 22** as a beige powder (1.67 g, 1.10 mmol, quant.).

Alternatively, the mixture can be stirred at 120 °C for 4 h in a microwaves device.

NMR $^1H$ (400 MHz, DMSO-$d_6$) : δ (ppm) = 9.86 (br. s, 1H), 7.22 (s, 1H), 7.13 (br. s, 1H), 7.06 (s, 1H).

HPLC : $t_R$ = 0.24 min; 79 %

MS (ES+, m/z) : [M+H+] 153.0

**6.14. Intermediate 23: 7-bromo-2,4-dichloro-imidazo[2,1-*f*][1,2,4]triazine**

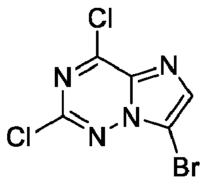

To a solution of **Intermediate 22** (1.0 eq, 9.61 mmol, 2.88 g) in iPrOH (19.2 mL), was added $NH_4OH$ 30 % in water (0.17 M, 56.5 mL). It was split into 5 vials and stirred at 120 °C for 4 h under MW. The mixture was cooled down and concentrated to dryness. The residue was triturated into $Et_2O$/MeOH 10:1 (3 × 22 mL) to afford **Intermediate 23** as a beige powder (1.67 g, 1.10 mmol, quant.).

Alternatively, the mixture can be stirred at 120 °C for 4 h in a microwaves device.

NMR $^1$H (400 MHz, DMSO-$d_6$) : δ (ppm) = 9.86 (br. s, 1H), 7.22 (s, 1H), 7.13 (br. s, 1H), 7.06 (s, 1H).

HPLC : $t_R$ = 0.24 min; 79 %

MS (ES+, m/z) : [M+H+] 153.0

**Example 7: Synthesis of compounds of formula (VIII) and for synthetic route 2a and 2b**

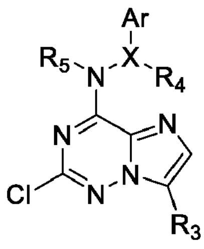
(VIII)

**GP5:** To a solution of **intermediate 21** or **compound (d)** in THF or *n*BuOH (0.5 M), was added corresponding amine (3.5 eq). The mixture was stirred at 70 °C for 16 h in a sealed vial.

A- The mixture was diluted into DCM and sat. $NH_4Cl$. The aqueous layer was extracted with DCM (2 x). The combined organic layers were washed with brine, dried over $MgSO_4$, filtered and concentrated. The crude product was purified by flash chromatography on silica gel to afford the desired product.

**B-** The mixture was concentrated to dryness. Pentane was added to the residue, the precipitate was filtered and washed with pentane and water to afford the desired product.

**7.1. Intermediate 24: *N*-benzyl-2-chloro-7-isopropyl-imidazo[2,1-*f*][1,2,4]triazin-4-amine**

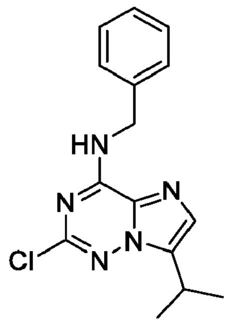

Fluffy slightly yellow solid (0.060 g, 0.199 mmol, 39 % yield over 2 steps) obtained from **Intermediate 21** (0.515 mmol) in THF according to the **GP5A,** purified by flash chromatography on silica gel with cHex/EtOAc (1:0 to 8:2 v/v).

NMR $^1$H (400 MHz, DMSO-$d_6$) : δ (ppm) = 9.76 (s, 1H), 7.43 (s, 1H), 7.34 (m, 4H), 7.25 (m, 1H), 4.68 (d, *J* = 4.6 Hz, 2H), 3.24 (hept, *J* = 6.9 Hz, 1H), 1.30 (d, *J* = 6.9 Hz, 6H). HPLC : $t_R$ = 3.23 min; 99 %

MS (ES+, m/z) : [M+H+] 302.2

**7.2. Intermediate 25: 2-Chloro-N-[(4-chlorophenyl)methyl]-7-isopropyl-imidazo[2,1-*f*][1,2,4]triazin-4-amine**

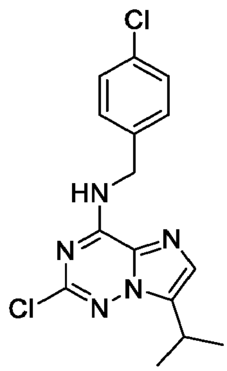

Fluffy slightly yellow solid (0.065 g, 0.193 mmol, 30 % yield over 2 steps) obtained from **Intermediate 21** (0.650 mmol) in *n*BuOH according to the **GP5B.**

NMR $^1$H (400 MHz, DMSO-$d_6$): δ (ppm) = 9.76 (s, 1H), 7.44 (s, 1H), 7.38 (m, 5H), 4.66 (s, 2H), 3.27 - 3.24 (hept, *J* = 7.0 Hz, 1H), 1.30 (d, *J* = 7.0 Hz, 6H).

HPLC : $t_R$ = 3.74 min; 96 %

MS (ES+, m/z) : [M+H+] 336.2

**7.3. Intermediate 26: 2-Chloro-7-isopropyl-N-(p-tolylmethyl)imidazo[2,1-*f*][1,2,4]triazin-4-amine**

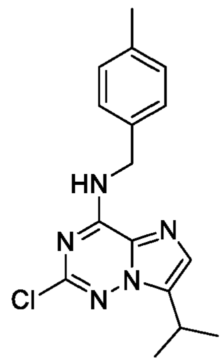

Fluffy slightly yellow solid (0.063 g, 0.199 mmol, 31 % yield over 2 steps) obtained from **Intermediate 21** (0.650 mmol) in *n*BuOH according to the **GP5B.**

NMR $^1H$ (400 MHz, DMSO-$d_6$: δ (ppm) = 9.72 (s, 1H), 7.42 (s, 1H), 7.23 (d, *J* = 7.6 Hz, 2H), 7.13 (d, *J* = 7.6 Hz, 2H), 4.63 (s, 2H), 3.23 (hept, *J* = 6.9 Hz, 1H), 2.26 (s, 3H), 1.30 (d, *J* = 6.9 Hz, 6H).

HPLC : $t_R$ = 3.72 min; 96 %

MS (ES+, m/z) : [M+H+] 316.2

### 7.4. Intermediate 27: 2-Chloro-7-isopropyl-*N*-[(4-methoxyphenyl)methyl]imidazo[2,1-*f*][1,2,4]triazin-4-amine

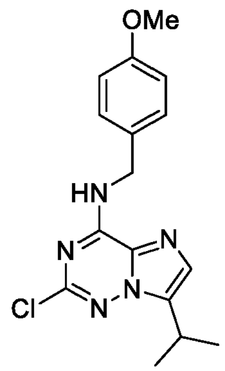

Fluffy slightly yellow solid (0.071 g, 0.214 mmol, 33 % yield over 2 steps) obtained from **Intermediate 21** (0.650 mmol) in *n*BuOH according to the **GP5B.**

NMR $^1H$ (400 MHz, DMSO-$d_6$): δ (ppm) = 9.71 (s, 1H), 7.41 (s, 1H), 7.29 (d, *J* = 8.3 Hz, 2H), 6.89 (d, *J* = 8.3 Hz, 2H), 4.60 (s, 2H), 3.72 (s, 3H), 3.23 (hept, *J* = 6.9 Hz, 1H), 1.30 (d, *J* = 6.9 Hz, 1H).

HPLC : $t_R$ = 3.55 min; 96 %

MS (ES+, m/z) : [M+H+] 332.2

### 7.5. Intermediate 28: 2-Chloro-*N*-[(3,4-dichlorophenyl)methyl]-7-isopropyl-imidazo[2,1-*f*][1,2,4]triazin-4-amine

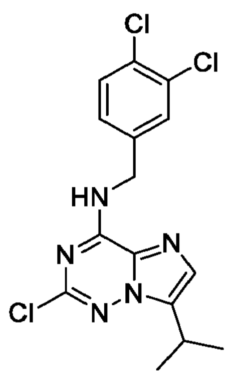

Flulffy white solid (0.071 g, 0.192 mmol, 29 % yield over 2 steps) obtained from **Intermediate 21** (0.650 mmol) in nBuOH according to the **GP5A,** purified by flash chromatography on silica gel with cHex/EtOAc (1:0 to 6:4 v/v).

NMR $^1H$ (400 MHz, DMSO-$d_6$) : δ (ppm) = 9.75 (s, 1H), 7.63 (s, 1H), 7.60 (d, *J* = 8.3 Hz, 1H), 7.44 (s, 1H), 7.34 (d, *J* = 8.3 Hz, 1H), 4.67 (s, 2H), 3.24 (hept, *J* = 6.9 Hz, 1H), 1.31 (d, *J* = 6.9 Hz, 6H).

HPLC : $t_R$ = 3.90 min; 97 % MS (ES+, m/z) : [M+H+] 370.1

### 7.6. Intermediate 29: 2-Chloro-*N*-[(4-fluorophenyl)methyl]-7-isopropyl-imidazo[2,1-*f*][1,2,4]triazin-4-amine

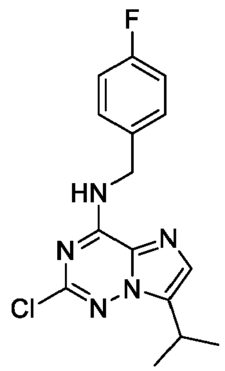

White solid (0.088 g, 0.275 mmol, 34 % yield over 2 steps) obtained from **Intermediate 21** (0.809 mmol) in THF according to the **GP5A,** purified by flash chromatography on silica gel with cHex/EtOAc (1:0 to 8:2 v/v).
NMR $^1$H (400 MHz, DMSO-$d_6$) : δ (ppm) = 9.75 (s, 1H), 7.43 (s, 1H), 7.42 - 7.34 (m, 2H), 7.20 - 7.09 (m, 2H), 4.66 (d, *J* = 4.1 Hz, 2H), 3.23 (hept, *J* = 6.9 Hz, 1H), 1.30 (d, *J* = 6.9 Hz, 6H).
HPLC : $t_R$ = 3.58 min; 90 %
MS (ES+, m/z) : [M+H+] 320.2

### 7.7. Intermediate 30: 2-Chloro-7-isopropyl-*N*-[[4-(trifluoromethyl)phenyl]methyl]imidazo[2,1-*f*][1,2,4]triazin-4-amine

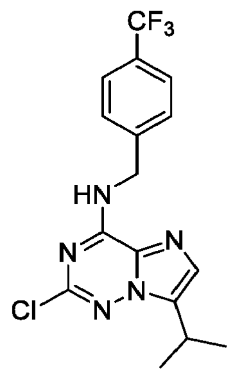

White solid (0.120 g, 0.325 mmol, 20 % over 2 steps) obtained from **Intermediate 21** (1.62 mmol) in THF according to the **GP5A,** purified by flash chromatography on silica gel with cHex/EtOAc (1:0 to 7:3 v/v).
NMR $^1$H (400 MHz, DMSO-$d_6$: δ (ppm) = 9.83 (t, *J* = 6.3 Hz, 1H), 7.70 (d, *J* = 8.1 Hz, 2H), 7.56 (d, *J* = 8.1 Hz, 2H), 7.45 (s, 1H), 4.77 (d, *J* = 6.3 Hz, 2H), 3.24 (hept, *J* = 6.9 Hz, 1H), 1.31 (d, *J* = 6.9 Hz, 6H).
HPLC : $t_R$ = 3.77 min; 87 %
MS (ES+, m/z) : [M+H+] 370.2

### 7.8. Intermediate 31: 2-Chloro-7-isopropyl-*N*-[(1*R*)-1-phenylethyl]imidazo[2,1-*f*][1,2,4]triazin-4-amine

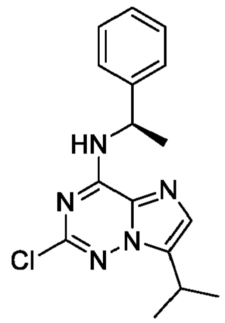

Yellow oil (0.136 g, 0.431 mmol, 27 % yield over 2 steps) obtained from **Intermediate 21** (1.62 mmol) in THF according to the **GP5A,** purified by flash chromatography on silica gel with cHex/DCM (1:0 to 0:1 v/v).
NMR $^1$H (400 MHz, DMSO-*d*$_6$): δ (ppm) = 9.75 (d, *J* = 8.5 Hz, 1H), 7.48 - 7.39 (m, 3H), 7.33 (t, *J* = 7.6 Hz, 2H), 7.27 - 7.19 (m, 1H), 5.45 (t, *J* = 7.7 Hz, 1H), 3.22 (hept, *J* = 6.7 Hz, 2H), 1.57 (d, *J* = 7.1 Hz, 3H), 1.28 (d, *J* = 6.7 Hz, 6H)

**7.9. Intermediate 32: 2-chloro-7-isopropyl-*N*-[(1S)-1-phenylethyl]imidazo[2,1-*f*][1,2,4]triazin-4-amine**

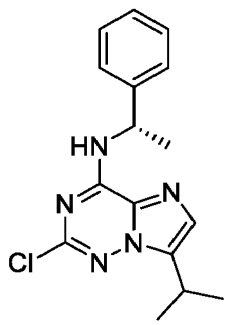

Yellow oil (0.128 g, 0.405 mmol, 25 % yield over 2 steps) obtained from **Intermediate 21** (1.62 mmol) in THF according to the **GP5A,** purified by flash chromatography on silica gel with cHex/EtOAc (1:0 to 9:1 v/v).
NMR $^1$H (400 MHz, DMSO-*d*$_6$) : δ (ppm) = 9.74 (d, *J* = 8.4 Hz, 1H), 7.47 - 7.41 (m, 3H), 7.33 (t, *J* = 7.4 Hz, 2H), 7.24 (t, *J* = 7.4 Hz, 1H), 5.45 (t, *J* = 7.6 Hz, 1H), 3.22 (hept, *J* = 6.9 Hz, 1H), 1.57 (d, *J* = 7.1 Hz, 3H), 1.29 (t, *J* = 7.4 Hz, 6H)

**7.10. Intermediate 33: 2-Chloro-7-isopropyl-*N*-(2-pyridylmethyl)methyl]-7-isopropyl-imidazo[2,1-*f*][1,2,4]triazin-4-amine**

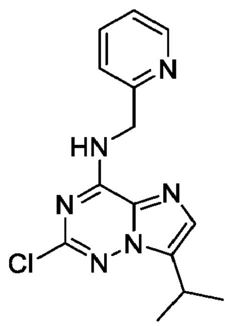

White solid (0.032 g, 0.106 mmol, 17 % yield over 2 steps) obtained from **Intermediate 21** (0.618 mmol) in *n*BuOH according to the **GP5A,** purified by flash chromatography on silica gel with cHex/EtOAc (1:0 to 0:1 v/v).
NMR $^1$H (400 MHz, DMSO-*d*$_6$) : δ (ppm) = 9.65 (s, 1H), 8.52 (d, *J* = 5.0 Hz, 1H), 7.76 (t, *J* = 7.5 Hz, 1H), 7.46

(s, 1H), 7.35 (d, *J* = 7.9 Hz, 1H), 7.29 (t, *J* = 6.2 Hz, 1H), 4.79 (d, *J* = 5.1 Hz, 2H), 3.25 (hept, *J* = 6.9 Hz, 2H), 1.32 (d, *J* = 6.9 Hz, 6H).

HPLC : $t_R$ = 2.70 min; 98 %

MS (ES+, m/z) : [M+H+] 303.2

### 7.11. Intermediate 34: 2-Chloro-7-isopropyl-*N*-(3-pyridylmethyl)methyl]-7-isopropyl-imidazo[2,1-*f*][1,2,4]triazin-4-amine

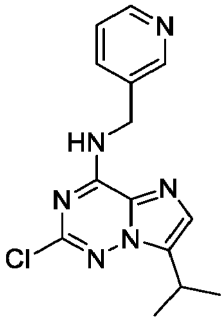

Beige solid (0.417 g, 1.38 mmol, 30 % yield over 2 steps) obtained from I**ntermediate 21** (4.64 mmol) in THF according to the **GP5A,** purified by flash chromatography on silica gel with DCM/MeOH (1:0 to 95:5 v/v).

NMR $^1$H (400 MHz, DMSO-*$d_6$*) : δ (ppm) = 9.78 (t, *J* = 6.1 Hz, 1H), 8.59 (s, 1H), 8.47 (dd, *J* = 4.8, 1.7 Hz, 1H), 7.76 (d, *J* = 7.9 Hz, 1H), 7.43 (s, 1H), 7.36 (dd, *J* = 7.9, 4.8 Hz, 1H), 4.70 (d, *J* = 6.1 Hz, 2H), 3.23 (hept, *J* = 6.9 Hz, 1H), 1.30 (d, *J* = 6.9 Hz, 6H).

HPLC : $t_R$ = 2.22 min; 66 %

MS (ES+, m/z) : [M+H+] 303.2

### 7.12. Intermediate 35: 2-Chloro-7-isopropyl-*N*-(4-pyridylmethyl)methyl]-7-isopropyl-imidazo[2,1-*f*][1,2,4]triazin-4-amine

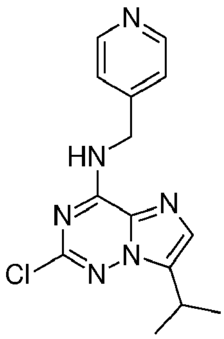

Beige solid (0.360 g, 1.19 mmol, 26 % yield over 2 steps) obtained from **Intermediate 21** (4.64 mmol) in THF according to the **GP5A**, purified by flash chromatography on silica gel with DCM/MeOH (1:0 to 92:8 v/v).

NMR $^1$H (400 MHz, DMSO-*$d_6$*): δ (ppm) = 9.79 (t, *J* = 6.1 Hz, 1H), 8.50 (d, *J* = 5.9 Hz, 2H), 7.46 (s, 1H), 7.33 (d, *J* = 5.9 Hz, 2H), 4.71 (d, *J* = 6.1 Hz, 2H), 3.25 (hept, *J* = 6.9 Hz, 1H), 1.31 (d, *J* = 6.9 Hz, 6H).

HPLC : $t_R$ = 2.06 min; 95 %

MS (ES+, m/z) : [M+H+] 303.2

### 7.13. Intermediate 36: 2-Chloro-7-isopropyl-*N*-(pyrazin-2-ylmethyl)imidazo[2,1-*f*][1,2,4]triazin-4-amine

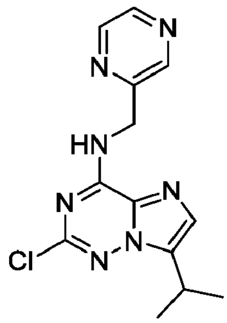

Beige solid (0.065 g, 0.214 mmol, 26 % yield over 2 steps) obtained from **Intermediate 21** (0.824 mmol) in THF according to the **GP5A,** purified by flash chromatography on silica gel with cHex/EtOAc (1:0 to 0:1 v/v).
NMR $^1$H (400 MHz, DMSO-*d*$_6$) : $\delta$ (ppm) = 9.73 (s, 1H), 8.69 (s, 1H), 8.59 (s, 1H), 8.55 (d, *J* = 2.6 Hz, 1H), 7.46 (s, 1H), 4.85 (s, 2H), 3.28 - 3.20 (m, 1H), 1.31 (d, *J* = 6.9 Hz, 6H).
UPLC : $t_R$ = 2.88 min; 89 %
MS (ES+, m/z) : [M+H+] 304.2

**7.14. Intermediate 37: *N*-Benzyl-2-chloro-7-isopropyl-*N*-methyl-imidazo[2,1-*f*][1,2,4]triazin-4-amine**

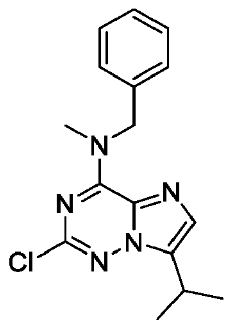

Colorless oil (0.173 g, 0.548 mmol, 30 % yield over 2 steps) obtained from **Intermediate 21** (1.84 mmol) in THF according to the **GP5A,** purified by flash chromatography on silica gel with cHex/DCM (1:0 to 8:2 v/v).
Alternatively, to a solution of **Intermediate 24** (1.0 eq, 0.497 mmol, 0.150 g) in dry THF (2.5 mL) at 0 °C, was added NaH 60 % in oil (1.2 eq, 0.990 mmol, 0.240 g). The mixture was allowed to reach r.t. over 15 min. MeI (1.2 eq, 0.990 mmol, 0.04 mL) was then added. The mixture was stirred at r.t. for 1 h. The mixture was diluted DCM (20 mL) and sat. $NH_4Cl$ (20 mL). The aqueous layer was extracted with DCM (2 $\times$ 20 mL). The combined organic layers were washed with brine, dried over $MgSO_4$, filtered and concentrated. The crude residue was purified by flash chromatography on silica gel with cHex/DCM (1:0 to 8:2 v/v) to afford a colorless oil (0.090 g, 0.285 mmol, 58 % yield).
NMR spectrum shows conformers, described below.
NMR $^1$H (400 MHz, DMSO-*d*$_6$) : $\delta$ (ppm) = 7.50 (s, 0.4H), 7.46 (s, 0.6H), 7.38 - 7.27 (m, 5H), 5.80 (s, 1.2H), 4.99 (s, 0.8H), 3.81 (s, 1.2H), 3.26 (hept, *J* = 6.9 Hz, 1H), 3.14 (s, 1.8H), 1.31 (d, *J* = 6.9 Hz, 6H).
HPLC : $t_R$ = 3.93 min; > 99 %
MS (ES+, m/z) : [M+H+] 316.3

**7.15. Intermediate 38: *N*-Benzyl-2-chloro-imidazo[2,1-*f*][1,2,4]triazin-4-amine**

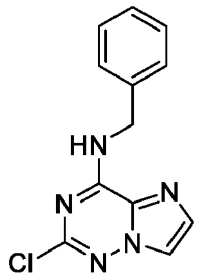

White solid (1.07 g, 4.12 mmol, 31 % yield over 2 steps) obtained from **Compound (d)** (13.1 mmol) in THF according to the **GP5A,** purified by flash chromatography on silica gel with cHex/DCM (1:0 to 6:4 v/v).
NMR $^1$H (400 MHz, DMSO-$d_6$) : δ (ppm) = 9.86 (s, 1H), 8.08 (s, 1H), 7.62 (s, 1H), 7.40 - 7.30 (m, 4H), 7.29 - 7.23 (m, 1H), 4.69 (d, *J* = 5.5 Hz, 2H).
HPLC : $t_R$ = 3.01 min; 90 %
MS (ES+, m/z) : [M+H+] 260.1

**Example 8: Synthesis of compounds of formula (XIV) for synthetic route 2**

**8.1. Intermediate 39: *N*-Benzyl-7-bromo-2-chloro-imidazo[2,1-*f*][1,2,4]triazin-4-amine**

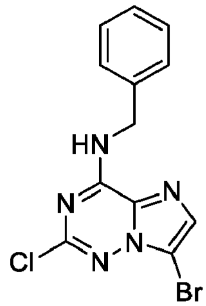

To a solution of **Intermediate 38** (1.0 eq, 0.820 mmol, 0.213 g) in THF (4.0 mL), was added *N*-bromosuccinimide (1.05 eq, 0.861 mmol, 0.153 g). The mixture was stirred at r.t. for 4 h. The mixture was diluted into EtOAc (30 mL) and $H_2O$ (30 mL). The aqueous layer was extracted with EtOAc (2 × 25 mL). The combined organic layers were washed with brine, dried over $MgSO_4$, filtered and concentrated. The crude residue was purified by flash chromatography on silica gel with cHex/DCM (1:0 to 0:1 v/v) to afford **Intermediate 39** as a yellow powder (0.225 g, 0.665 mmol, 81 % yield).
NMR $^1$H (400 MHz, DMSO-$d_6$): δ (ppm) = 9.98 (t, *J* = 6.3 Hz, 1H), 7.76 (s, 1H), 7.42 - 7.30 (m, 4H), 7.27 (m, 1H), 4.71 (d, *J* = 6.3 Hz, 2H).
HPLC : $t_R$ = 3.31 min; > 99 %
MS (ES+, m/z) : [M+H+] 338.0

**8.2. Intermediate 40: *N*-Benzyl-2-chloro-7-iodo-imidazo[2,1-*f*][1,2,4]triazin-4-amine**

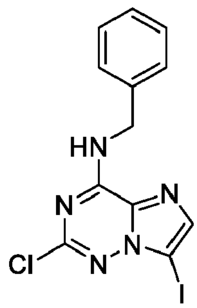

To a solution of **Intermediate 38** (1.0 eq, 1.16 mmol, 0.300 g) in DCM (5.8 mL), were added *N*-iodosuccinimide (1.5 eq, 1.73 mmol, 0.390 g) and trifluoroacetic acid (0.6 eq, 0.696 mmol, 0.05 mL) . The mixture was heated to reflux for 16 h. The mixture was diluted into DCM (20 mL) and sat. $NaHCO_3$ (20 mL). The organic layer was washed with sat. $Na_2S_2O_3$ (50 mL). The aqueous layers were extracted with DCM (2 × 25 mL). The combined organic layers were washed with brine, dried over $MgSO_4$, filtered and concentrated. The crude residue was purified by flash chromatography on silica gel with cHex/DCM (1:0 to 0:1 v/v) to afford **Intermediate 40** as a white powder (0.354 g, 0.918 mmol, 79 % yield).
NMR $^1$H (400 MHz, DMSO-$d_6$) : δ (ppm) = 9.89 (s, 1H), 7.72 (s, 1H), 7.39 - 7.29 (m, 4H), 7.29 - 7.20 (m, 1H), 4.69 (s, 2H).
HPLC : $t_R$ = 3.34 min; 96 %
MS (ES+, m/z) : [M+H+] 386.1

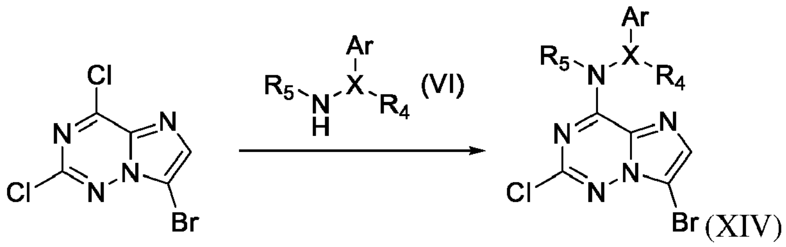

**GP6:** To the **Intermerdiate 23** (1.0 eq) in THF (0.2 M), was added base (1.5 eq.) and corresponding amine of formula (VI) (1.5 eq). The mixture was heated to T for t in a sealed vial. The mixture was diluted into $CH_2Cl_2$ (5 mL) and water (5 mL). The aqueous layer was extracted with $CH_2Cl_2$ (2 × 15 mL). The combined organic layers were washed with brine, dried over $MgSO_4$, filtered and concentrated. The crude residue was purified by flash chromatography on silica gel to afford the desired compound of formula (XIV).

**8.3. Intermediate 41: 7-bromo-2-chloro-*N*-(2-pyridylmethyl)imidazo[2,1-*f*][1,2,4]triazin-4-amine**

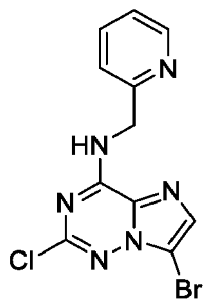

Off white solid (125 mg, 0.37 mmol, 82 % yield) obtained from **Intermerdiate 23** (120 mg, 0.45 mmol), triethylamine (94 μL, 1.5 eq.) and corresponding amine (73 mg, 1.5 eq.) according to the **GP6,** with T = 70 °C and t = 1.5 h, purified by flash chromatography on silica gel with DCM/MeOH + $NH_3$ 7N (100:0 to 97:3 v/v).
NMR $^1$H (400 MHz, $CDCl_3$) δ: 8.62 (d, *J* = 5.0 Hz, 1H), 8.16 (s, 1H), 7.77 - 7.69 (m, 1H), 7.55 (s, 1H), 7.33 (d, *J* = 7.8 Hz, 1H), 7.28 (d, *J* = 5.0 Hz, 1H), 4.93 (d, *J* = 4.7 Hz, 2H). MS (ES+, m/z): [M+H+] 339.0.

**8.4. Intermediate 42: 7-bromo-2-chloro-*N*-(3-pyridylmethyl)imidazo[2,1-*f*][1,2,4]triazin-4-amine**

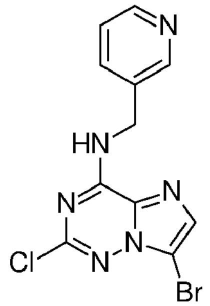

Off white solid (50 mg, 0.15 mmol, 33 % yield) obtained from **Intermerdiate 23** (120 mg, 0.45 mmol), triethylamine (94 μL, 1.5 eq.) and corresponding amine (73 mg, 1.5 eq.) according to the **GP6,** with T = 70 °C and t = 1.5 h, purified by flash chromatography on silica gel with DCM/MeOH + $NH_3$ 7N (100:0 to 97:3 v/v).

NMR $^1H$ (400 MHz, $CDCl_3$) δ: 8.71 - 8.66 (m, 1H), 8.63 - 8.56 (m, 1H), 7.74 (dt, *J* = 7.9, 2.0 Hz, 1H), 7.52 - 7.49 (m, 1H), 7.35 - 7.30 (m, 1H), 6.90 (s, 1H), 4.88 (d, *J* = 6.1 Hz, 2H). MS (ES+, m/z): [M+H+] 339.0.

**8.5. Intermediate 43: 7-bromo-2-chloro-*N*-(4-pyridylmethyl)imidazo[2,1-*f*][1,2,4]triazin-4-amine**

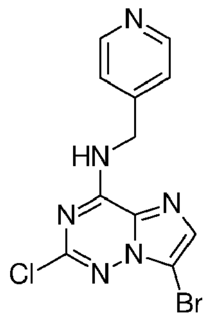

Off white solid (105 mg, 0.31 mmol, 69 % yield) obtained from **Intermerdiate 23** (120 mg, 0.45 mmol), triethylamine (94 μL, 1.5 eq.) and corresponding amine (73 mg, 1.5 eq.) according to the **GP6,** with T = 70 °C and t = 1.5 h, purified by flash chromatography on silica gel with DCM/MeOH + $NH_3$ 7N (100:0 to 97:3 v/v).

NMR $^1H$ (400 MHz, $CDCl_3$) δ: 8.67 - 8.57 (m, 2H), 7.53 (s, 1H), 7.29 (d, *J* = 5.8 Hz, 2H), 6.92 (s, 1H), 4.89 (d, *J* = 6.2 Hz, 2H).

MS (ES+, m/z): [M+H+] 339.0.

**8.6. Intermediate 44: 7-bromo-2-chloro-*N*-[2-(2-pyridyl)ethyl]imidazo[2,1-*f*][1,2,4]triazin-4-amine**

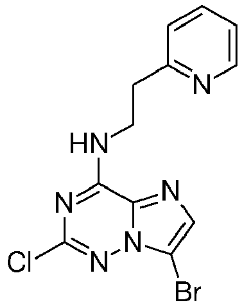

Off white solid (175 mg, 0.49 mmol, 74 % yield) obtained from **Intermediate 23** (180 mg, 0.68 mmol), triethylamine (141 μL, 1.5 eq.) and corresponding amine (73 mg, 1.5 eq.) according to the GP6, with T = 70 °C and t = 1.5 h, purified by flash chromatography on silica gel with DCM/MeOH (100:0 to 96:4 v/v).

NMR $^1$H (400 MHz, $CD_3OD$) δ: 8.47 (d, *J* = 5.1 Hz, 1H), 7.74 (td, *J* = 7.7,1.9 Hz, 1H), 7.53 (s, 1H), 7.36 (d, *J* = 7.8 Hz, 1H), 7.31 - 7.23 (m, 1H), 3.99 (t, *J* = 6.9 Hz, 2H), 3.18 (t, *J* = 6.9 Hz, 2H).
MS (ES+, m/z): [M+H+] 353.0.

**8.7. Intermediate 45: 7-bromo-2-chloro-*N*-[2-(3-pyridyl)ethyl]imidazo[2,1-*f*][1,2,4]triazin-4-amine**

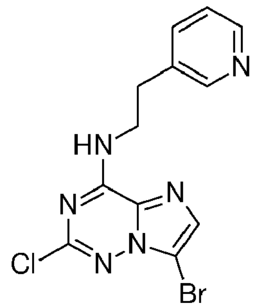

Off white solid (175 mg, 0.49 mmol, 74 % yield) obtained from **Intermediate 23** (190 mg, 0.71 mmol), triethylamine (140 μL, 1.5 eq.) and corresponding amine (130 mg, 1.5 eq.) according to the **GP6,** with T = 70 °C and t = 1.5 h, purified by flash chromatography on silica gel with DCM/MeOH (100:0 to 96:4 v/v).
NMR $^1$H (400 MHz, $CDCl_3$) δ: 8.56 - 8.47 (m, 2H), 7.59 (d, *J* = 7.8 Hz, 1H), 7.48 (s, 1H), 7.29 - 7.26 (m, 1H), 6.64 (s, 1H), 3.96 (q, *J* = 6.9 Hz, 2H), 3.04 (t, *J* = 7.1 Hz, 2H).
MS (ES+, m/z): [M+H+] 353.0.

**8.8. Intermediate 46: 7-bromo-2-chloro-*N*-[2-(4-pyridyl)ethyl]imidazo[2,1-*f*][1,2,4]triazin-4-amine**

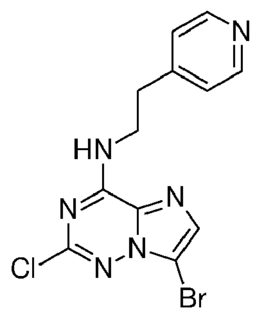

Off white solid (90 mg, 0.25 mmol, 68 % yield) obtained from **Intermediate 23** (100 mg, 0.37 mmol), triethylamine (77 μL, 1.5 eq.) and corresponding amine (68 mg, 1.5 eq.) according to the **GP6,** with T = 70 °C and t = 1.5 h, purified by flash chromatography on silica gel with DCM/MeOH + $NH_3$ 7N (100:0 to 97:3 v/v).
NMR $^1$H (400 MHz, $CD_3OD$) δ: 8.51 - 8.33 (m, 2H), 7.54 (s, 1H), 7.41 - 7.34 (m, 2H), 3.92 (t, *J* = 7.1 Hz, 2H), 3.08 (t, *J* = 7.1 Hz, 2H).
MS (ES+, m/z): [M+H+] 353.0.

**8.9. Intermediate 47: 7-bromo-2-chloro-*N*-[2-phenylethyl]imidazo[2,1-*f*][1,2,4]triazin-4-amine**

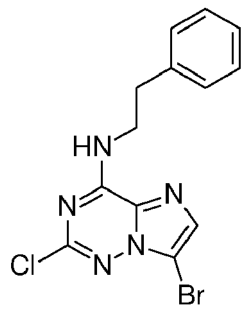

Off white solid (131 mg, 0.37 mmol, 80 % yield) obtained from **Intermediate 23** (125 mg, 0.47 mmol), triethylamine (97 μL, 1.5 eq.) and corresponding amine (85 mg, 1.5 eq.) according to the **GP6,** with T = 70 °C and t = 1.5 h, purified by flash chromatography on silica gel with cyclohexane/EtOAc (98:2 to 70:30 v/v).

NMR $^1$H (400 MHz, $CDCl_3$) δ: 7.38 (s, 1H), 7.33 - 7.26 (m, 2H), 7.25 - 7.16 (m, 3H), 7.04 (s, 1H), 3.92 (q, *J* = 6.8 Hz, 2H), 2.99 (t, *J* = 6.9 Hz, 2H).

MS (ES+, m/z): [M+H+] 352.0.

### 8.10. Intermediate 48: 7-bromo-2-chloro-*N*-phenyl-imidazo[2,1-*f*][1,2,4]triazin-4-amine

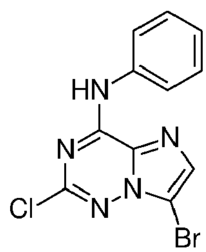

Off white solid (195 mg, 0.60 mmol, 94 % yield) obtained from **Intermediate 23** (175 mg, 0.64 mmol), triethylamine (132 μL, 1.5 eq.) and corresponding amine (110 μL, 1.5 eq.) according to the **GP6**, with T = 25 °C and t = 2 h. No purification needed.

NMR $^1$H (400 MHz, $CDCl_3$) δ: 8.31 (s, 1H), 7.85 - 7.77 (m, 2H), 7.59 (s, 1H), 7.48 - 7.42 (m, 2H), 7.26 - 7.22 (m, 1H).

MS (ES+, m/z): [M+H+] 324.0.

## Example 9: Synthesis of compounds of formula (VIII) for synthetic route 2b and 2c

### 9.1. Intermediate 49: *N*-Benzyl-2-chloro-7-phenyl-imidazo[2,1-*f*][1,2,4]triazin-4-amine

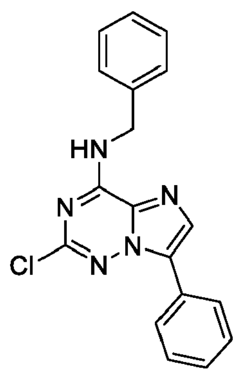

To a solution of **intermediate 39** (1.0 eq, 0.561 mmol, 0.190 g) in THF/$H_2O$ 9:1 (2.8 mL) under inert atmosphere, were added phenylboronic acid (1.5 eq, 0.842 mmol, 0.103 g), $Cs_2CO_3$ (0.6 eq, 0.337 mmol, 0.110 g), followed by

$Pd(dppf)Cl_2.CH_2Cl_2$ (5 %mol, 0.0281 mmol, 0.023 g). The mixture was heated to 90 °C for 19 h in a sealed vial. The mixture was diluted into EtOAc (20 mL) and $H_2O$ (20 mL) before filtration on Célite®. The aqueous layer was extracted with EtOAc (2 × 20 mL). The combined organic layers were washed with brine, dried over $MgSO_4$, filtered and concentrated. The crude residue was purified by flash chromatography on silica gel with cHex/EtOAc (1:0 to 72:28 v/v) to afford **intermediate 49** as a slightly yellow solid (0.152 g, 0.372 mmol, 81 % yield).

NMR $^1H$ (400 MHz, DMSO-$d_6$) : δ (ppm) = 9.90 (t, *J* = 6.3 Hz, 1H), 8.06 (s, 1H), 8.04 (d, *J* = 7.9 Hz, 2H), 7.54 (t, *J* = 7.6 Hz, 2H), 7.39 (m, 5H), 7.28 (m, 1H), 4.74 (d, *J* = 6.3 Hz, 2H).

HPLC : $t_R$ = 3.66 min; 97 %

MS (ES+, m/z) : [M+H+] 336.1

**9.2. Intermediate 50: *N*-Benzyl-2-chloro-7-vinyl-imidazo[2,1-*f*][1,2,4]triazin-4-amine**

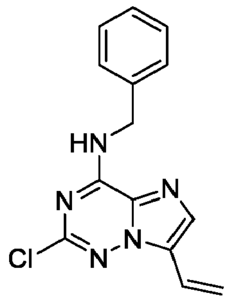

To a solution of **intermediate 40** (1.0 eq, 0.918 mmol, 0.354 g) in dioxane/$H_2O$ 9:1 (9.2 mL) under inert atmosphere, were added potassium vinyltrifluoroborate (1.2 eq, 0.950 mmol, 0.127 g), $Cs_2CO_3$ (0.6 eq, 0.551 mmol, 0.179 g), followed by $Pd(dppf)Cl_2.CH_2Cl_2$ (20 %mol, 0.184 mmol, 0.150 g). The mixture was heated to 110 °C for 16 h in a sealed vial. The mixture was diluted into DCM (50 mL) and $H_2O$ (50 mL). The aqueous layer was extracted with DCM (2 × 50 mL). The combined organic layers were washed with brine, dried over $MgSO_4$, filtered and concentrated. The crude residue was purified by flash chromatography on silica gel with cHex/EtOAc (1:0 to 7:3 v/v) to afford **intermediate 50** as a white solid (0.158 g, 0.553 mmol, 60 % yield).

NMR $^1H$ (400 MHz, DMSO-$d_6$): δ (ppm) = 9.84 (s, 1H), 7.86 (s, 1H), 7.40 - 7.29 (m, 5H), 7.29 - 7.22 (m, 1H), 6.87 (dd, *J* = 17.9, 11.8 Hz, 1H), 6.15 (d, *J* = 17.9 Hz, 1H), 5.48 (d, *J* = 11.8 Hz, 1H), 4.70 (d, *J* = 4.8 Hz, 2H).

HPLC : $t_R$ = 3.41 min; 98 %

MS (ES+, m/z) : [M+H+] 286.2

**9.3. Intermediate 51: *N*-Benzyl-2-chloro-7-isopropenyl-imidazo[2,1-*f*][1,2,4]triazin-4-amine**

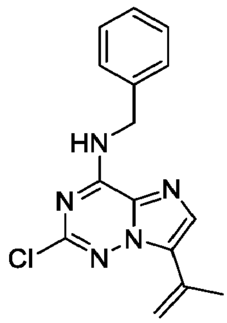

To a solution of **intermediate 39** (1.0 eq, 5.30 mmol, 1.80 g) in THF/$H_2O$ 9:1 (26.7 mL) under inert atmosphere, were added potassium trifluoro(prop-1-en-2-yl)borate (1.5 eq, 7.96 mmol, 1.18 g), $Cs_2CO_3$ (0.6 eq, 3.18 mmol, 1.04 g), followed by $Pd(dppf)Cl_2.CH_2Cl_2$ (5 %mol, 0.265 mmol, 0.217 g). The mixture was heated to 80 °C for 16 h in a sealed vial. The mixture was diluted into EtOAc (20 mL) and $H_2O$ (20 mL) before filtration on Célite®. The aqueous layer was extracted with EtOAc (2 × 25 mL). The combined organic layers were washed with brine, dried over $MgSO_4$, filtered

and concentrated. The crude residue was purified by flash chromatography on silica gel with cHex/EtOAc (1:0 to 8:2 v/v). The resulting solid was triturated into pentane to afford **intermediate 51** as a fluffy pale yellow solid (1.29 g, 4.30 mmol, 81 % yield).

NMR $^1$H (400 MHz, DMSO-$d_6$) : δ (ppm) = 9.85 (t, *J* = 6.3 Hz, 1H), 7.72 (s, 1H), 7.39 - 7.29 (m, 4H), 7.29 - 7.23 (m, 1H), 6.20 (s, 1H), 5.32 (s, 1H), 4.70 (d, *J* = 6.3 Hz, 2H), 2.17 (s, 3H).

HPLC : $t_R$ = 3.60 min; > 99 %

MS (ES+, m/z) : [M+H+] 300.2

*General procedure for Negishi couplings*

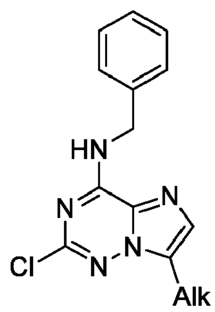

**GP7:** To a solution of **intermediate 39** (1.0 eq) and CPhos-Pd G4 (5 %mol) in dry THF (0.25 M) under inert atmosphere, was added dropwise a solution of alkylzinc bromide in THF (3.0 eq). The mixture was stirred at r.t. for 22 h. HCl 1 M was added to quench the mixture. It was then diluted into DCM (50 mL) and sat. $NaHCO_3$ (50 mL). The aqueous layer was extracted with DCM (2 × 50 mL). The combined organic layers were washed with brine, dried over $MgSO_4$, filtered and concentrated. The crude residue was purified by flash chromatography on silica gel to afford the desired products as white solids.

**9.4. Intermediate 52: *N*-Benzyl-2-chloro-7-cyclopropyl-imidazo[2,1-*f*][1,2,4]triazin-4-amine**

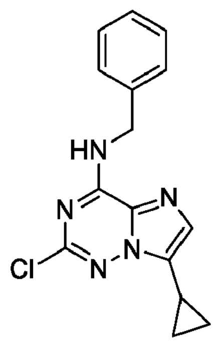

White solid (0.030 g, 0.164 mmol, 37 % yield) obtained from **intermediate 39** (0.443 mmol, 0.150 g) according to the **GP7,** purified by flash chromatography on silica gel with 100 % DCM.

NMR $^1$H (400 MHz, DMSO-$d_6$): δ (ppm) = 9.71 (s, 1H), 7.38 - 7.29 (m, 5H), 7.26 (dt, *J* = 8.6, 3.8 Hz, 1H), 4.68 (s, 2H), 2.05 (tt, *J* = 8.6, 4.1 Hz, 1H), 1.05 - 0.96 (m, 2H), 0.84 - 0.75 (m, 2H).

HPLC : $t_R$ = 3.37 min; 85 %

MS (ES+, m/z) : [M+H+] 300.2

**9.5. Intermediate 51: *N*-Benzyl-2-chloro-7-cyclohexyl-imidazo[2,1-*f*][1,2,4]triazin-4-amine**

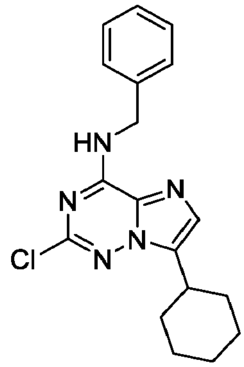

White solid (0.040 g, 0.133 mmol, 30 % yield) obtained from **intermediate 39** (0.443 mmol, 0.150 g) according to the **GP7,** purified by flash chromatography on silica gel with cHex/EtOAc (1:0 to 8:2 v/v).
NMR $^1$H (400 MHz, DMSO-$d_6$): δ (ppm) = 9.75 (t, *J* = 6.2 Hz, 1H), 7.41 (s, 1H), 7.40 - 7.21 (m, 5H), 4.68 (d, *J* = 6.2 Hz, 2H), 3.03 - 2.86 (m, 1H), 2.08 - 1.95 (m, 2H), 1.89 - 1.64 (m, 4H), 1.50 - 1.32 (m, 4H).
HPLC : $t_R$ = 3.93 min; 65 %
MS (ES+, m/z) : [M+H+] 342.3

**9.6. Intermediate 52: *N*-Benzyl-2-chloro-7-methyl-imidazo[2,1-*f*][1,2,4]triazin-4-amine**

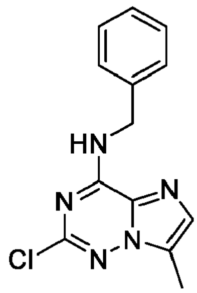

White solid (0.105 g, 0.448 mmol, 38 % yield) obtained from **intermediate 39** (1.18 mmol, 0.400 g) according to the **GP7,** purified by flash chromatography on silica gel with DCM/MeOH (1:0 to 99:1 v/v).
NMR $^1$H (400 MHz, DMSO-$d_6$): δ (ppm) = 9.72 (s, 1H), 7.42 (d, *J* = 1.1 Hz, 1H), 7.39 - 7.30 (m, 4H), 7.30 - 7.20 (m, 1H), 4.69 (s, 2H), 2.41 - 2.35 (m, 3H).
HPLC : $t_R$ = 3.18 min; 94 %
MS (ES+, m/z) : [M+H+] 274.2

*General procedure for Suzuki-Miyaura couplings following reduction by hydrogenation to introduce isopropyl group*

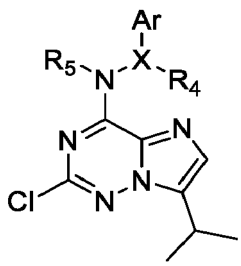

**GP8:** To the bromine starting material (1.0 eq) in degazed THF/$H_2O$ 95/5 (0.2 M), was added Cesium Carbonate (2.5 eq.), potassium allyltrifluoroborate (1.5 eq.) and $PdCl_2$(dppf) (0.1 eq). The mixture was heated to 100 °C for 16 h

in a sealed vial. After cooled down to room temperature, the mixture was diluted into EtOAc (5 mL) filtrated over a pad of celite® and the filtrate was evaporated. The corresponding crude residue was dissolved in a mixture THF/EtOH 1/1 (0.1 M) and $Pd(OH)_2$ (0.1 eq.) was added. If necessary, 2 drops of AcOH were added and the resulting solution was stirred under H2 atmosphere for 5 hours. The solution was filtrated through a pad of celite® and the filtrate was concentrated. The crude residue was purified by flash chromatography on silica gel to afford the desired isopropylated compound.

**9.7. Intermediate 53: 2-chloro-7-isopropyl-*N*-(2-pyridylmethyl)imidazo[2,1-*f*][1,2,4]triazin-4-amine**

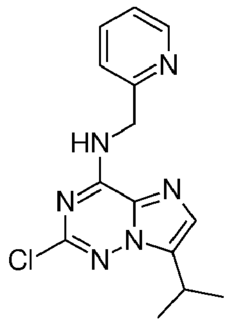

Off white solid (64 mg, 0.21 mmol, 49 % yield) obtained from **Intermediate 41** (120 mg, 0.35 mmol), cesium carbonate (288 mg, 2.5 eq.), potassium allyltrifluoroborate (78 mg, 1.5 eq.) and $PdCl_2$(dppf) (26 mg, 0.1 eq) followed by $Pd(OH)_2$ (5 mg, 0.1 eq.) according to the **GP8,** purified by flash chromatography on silica gel with DCM/MeOH + $NH_3$ 7N (100:0 to 98:2 v/v).

NMR $^1H$ (400 MHz, $CDCl_3$) δ: 8.64 - 8.57 (m, 1H), 8.01 (s, 1H), 7.70 (td, *J* = 7.7, 1.8 Hz, 1H), 7.35 - 7.29 (m, 2H), 7.26 - 7.22 (m, 1H), 4.92 (d, *J* = 4.8 Hz, 2H), 3.38 (p, *J* = 6.9 Hz, 1H), 1.38 (d, *J* = 6.9 Hz, 6H).

MS (ES+, m/z): [M+H+] 303.4

**9.8. Intermediate 54: 2-chloro-7-isopropyl-*N*-(3-pyridylmethyl)imidazo[2,1-*f*][1,2,4]triazin-4-amine**

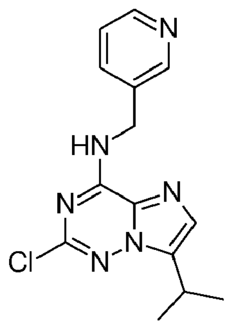

Off white solid (24 mg, 0.08 mmol, 32 % yield) obtained from **Intermediate 42** (70 mg, 0.21 mmol), cesium carbonate (168 mg, 2.5 eq.), potassium allyltrifluoroborate (37 mg, 1.5 eq.) and $PdCl_2$(dppf) (15 mg, 0.1 eq) followed by $Pd(OH)_2$ (3 mg, 0.1 eq.) according to the **GP8,** purified by flash chromatography on silica gel with DCM/MeOH + $NH_3$ 7N (100:0 to 98:2 v/v).

NMR $^1H$ (400 MHz, $CDCl_3$) δ: 8.69 - 8.64 (m, 1H), 8.58 (dd, *J* = 4.8, 1.7 Hz, 1H), 7.72 (dt, *J* = 7.8, 2.1 Hz, 1H), 7.31 - 7.27 (m, 1H), 7.00 (s, 1H), 4.85 (d, *J* = 6.0 Hz, 2H), 3.39 (dq, *J* = 13.8, 6.8 Hz, 1H), 1.37 (d, *J* = 6.9 Hz, 6H).

MS (ES+, m/z): [M+H+] 303.1.

**9.9. Intermediate 55: 2-chloro-7-isopropyl-*N*-(4-pyridylmethyl)imidazo[2,1-*f*][1,2,4]triazin-4-amine**

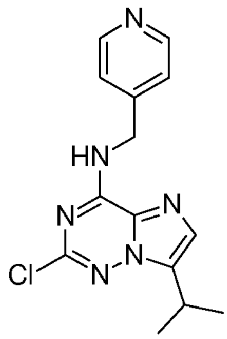

Off white solid (30 mg, 0.10 mmol, 35 % yield) obtained from **Intermediate 43** (95 mg, 0.28 mmol), cesium carbonate (227 mg, 2.5 eq.), potassium allyltrifluoroborate (62 mg, 1.5 eq.) and $PdCl_2$(dppf) (20 mg, 0.1 eq) followed by $Pd(OH)_2$(3.5 mg, 0.1 eq.) according to the **GP8,** purified by flash chromatography on silica gel with DCM/MeOH + $NH_3$ 7N (100:0 to 97:3 v/v).

NMR $^1$H (400 MHz, $CDCl_3$) δ: 8.61 - 8.56 (m, 2H), 7.55 - 7.46 (m, 1H), 7.29 - 7.27 (m, 2H), 7.24 (s, 1H), 4.86 (d, *J* = 6.1 Hz, 2H), 3.38 (hept, *J* = 7.0 Hz, 1H), 1.37 (d, *J* = 6.9 Hz, 6H).

MS (ES+, m/z) : [M+H+] 303.10

**9.10. Intermediate 56: 2-chloro-7-isopropyl-*N*-[2-(2-pyridyl)ethyl]imidazo[2,1-*f*][1,2,4]triazin-4-amine**

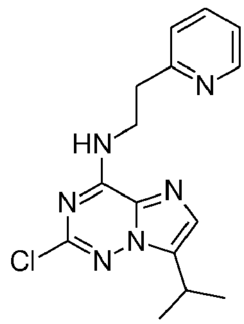

Off white solid (33 mg, 0.10 mmol, 33 % yield) obtained from **Intermediate 44** (100 mg, 0.28 mmol), cesium carbonate (230 mg, 2.5 eq.), potassium allyltrifluoroborate (63 mg, 1.5 eq.) and $PdCl_2$(dppf) (21 mg, 0.1 eq) followed by $Pd(OH)_2$ (4 mg, 0.1 eq.) according to the **GP8,** purified by flash chromatography on silica gel with DCM/MeOH + $NH_3$ 7N (100:0 to 97:3 v/v).

NMR $^1$H (400 MHz, $CDCl_3$) δ : 8.61 (d, *J* = 4.8 Hz, 1H), 7.74 (s, 1H), 7.62 (td, *J* = 7.6, 1.9 Hz, 1H), 7.25 (s, 1H), 7.18 - 7.15 (m, 1H), 4.09 (q, *J* = 6.1 Hz, 2H), 3.36 (p, *J* = 6.8 Hz, 1H), 3.17 (t, *J* = 6.3 Hz, 2H), 1.36 (d, *J* = 7.0 Hz, 6H).

MS (ES+, m/z) : [M+H+] 317.1.

**9.11. Intermediate 57: 2-chloro-7-isopropyl-*N*-[2-(3-pyridyl)ethyl]imidazo[2,1-*f*][1,2,4]triazin-4-amine**

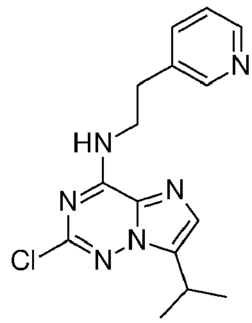

Off white solid (36 mg, 0.11 mmol, 49 % yield) obtained from **Intermediate 45** (77 mg, 0.21 mmol), cesium carbonate (177 mg, 2.5 eq.), potassium allyltrifluoroborate (48 mg, 1.5 eq.) and $PdCl_2$(dppf) (16 mg, 0.1 eq) followed by $Pd(OH)_2$(2.8 mg, 0.1 eq.) according to the **GP8,** purified by flash chromatography on silica gel with DCM/MeOH + $NH_3$ 7N (100:0 to 97:3 v/v).

NMR $^1H$ (400 MHz, $CDCl_3$) δ: 8.49 (d, *J* = 2.3 Hz, 1H), 8.47 (dd, *J* = 4.8, 1.7 Hz, 1H), 7.73 - 7.68 (m, 1H), 7.57 (dt, *J* = 7.8, 2.0 Hz, 1H), 7.24 - 7.19 (m, 2H), 3.96 - 3.88 (m, 2H), 3.44 - 3.31 (m, 1H), 3.02 (t, *J* = 7.2 Hz, 2H), 1.36 (d, *J* = 7.0 Hz, 6H).

MS (ES+, m/z) : [M+H+] 317.1.

**9.12. Intermediate 58: 2-chloro-7-isopropyl-*N*-[2-(4-pyridyl)ethyl]imidazo[2,1-*f*][1,2,4]triazin-4-amine**

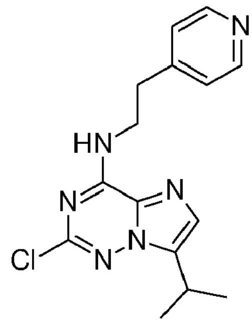

Off white solid (30 mg, 0.10 mmol, 37 % yield) obtained from **Intermediate 46** (80 mg, 0.23 mmol), cesium carbonate (184 mg, 2.5 eq.), potassium allyltrifluoroborate (50 mg, 1.5 eq.) and $PdCl_2$(dppf) (16 mg, 0.1 eq) followed by $Pd(OH)_2$(3.1 mg, 0.1 eq.) according to the **GP8,** purified by flash chromatography on silica gel with DCM/MeOH + $NH_3$ 7N (100:0 to 98:2 v/v).

NMR $^1H$ (400 MHz, $CDCl_3$) δ : 8.55 (d, *J* = 5.8 Hz, 2H), 7.22 - 7.17 (m, 3H), 6.56 (s, 1H), 4.01 - 3.93 (m, 2H), 3.37 (dt, *J* = 13.8, 6.7 Hz, 1H), 3.02 (t, *J* = 7.1 Hz, 2H), 1.37 (d, *J* = 6.9 Hz, 6H).

MS (ES+, m/z) : [M+H+] 317.4.

**9.13. Intermediate 59: 2-chloro-7-isopropyl-*N*-[2-phenylethyl]imidazo[2,1-*f*][1,2,4]triazin-4-amine**

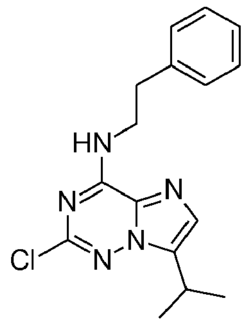

Off white solid (70 mg, 0.20 mmol, 42 % yield) obtained from **Intermediate 47** (125 mg, 0.35 mmol), cesium carbonate (289 mg, 2.5 eq.), potassium allyltrifluoroborate (79 mg, 1.5 eq.) and $PdCl_2$(dppf) (26 mg, 0.1 eq) followed by $Pd(OH)_2$ (5.9 mg, 0.1 eq.) according to the **GP8,** purified by flash chromatography on silica gel with Cyclohexane/EtOAc (98:2 to 75:25 v/v).

NMR $^1$H (400 MHz, $CDCl_3$) $\delta$: 7.40 (t, *J* = 6.4 Hz, 1H), 7.28 - 7.24 (m, 2H), 7.22 - 7.16 (m, 3H), 7.15 (s, 1H), 3.88 (q, *J* = 6.7 Hz, 2H), 3.37 (dq, *J* = 13.8, 6.9, 6.5 Hz, 1H), 2.96 (t, *J* = 6.8 Hz, 2H), 1.37 (d, *J* = 6.9 Hz, 6H).

MS (ES+, m/z) : [M+H+] 316.1.

**9.14. Intermediate 60: 2-chloro-7-isopropyl-*N*-phenyl-imidazo[2,1-*f*][1,2,4]triazin-4-amine**

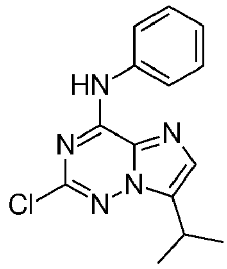

Off white solid (80 mg, 0.28 mmol, 57 % yield) obtained from **Intermediate** 46 (150 mg, 0.46 mmol), cesium carbonate (376 mg, 2.5 eq.), potassium allyltrifluoroborate (82 mg, 1.5 eq.) and $PdCl_2$(dppf) (34 mg, 0.1 eq) followed by $Pd(OH)_2$ (6 mg, 0.1 eq.) according to the **GP8,** purified by flash chromatography on silica gel with Cyclohexane/EtOAc (98:2 to 75:25 v/v).

NMR $^1$H (400 MHz, $CDCl_3$) $\delta$: 8.47 (s, 1H), 7.85 - 7.75 (m, 2H), 7.48 - 7.39 (m, 2H), 7.35 (d, *J* = 0.8 Hz, 1H), 7.24 - 7.17 (m, 1H), 3.41 (pd, *J* = 7.0, 0.7 Hz, 1H), 1.40 (d, *J* = 7.0 Hz, 6H).

MS (ES+, m/z) : [M+H+] 288.1.

Example 10: Synthesis of compounds of formula (XVI) for synthetic route 2c

**10.1. Intermediate 61: 7-bromo-*N2*-(1-ethylpropyl)-*N4*-[2-(3-pyridyl)ethyl]imidazo[2,1-*f*][1,2,4]triazine-2,4-diamine**

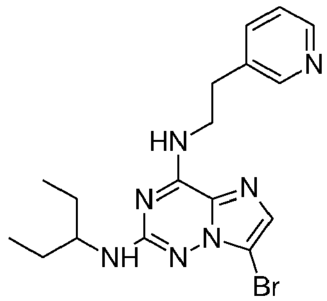

To the starting material **Intermediate 43** (150 mg, 0.42 mmol) in NMP (2.1 mL, 0.2 M) was added 1-ethylpropylamine (500 μL, 10 eq.). The solution was stirred 20 hours at 150 °C. After cooled down to room temperature, the mixture was diluted in EtOAc (1 mL) and washed with water (5 × 5 mL). Organic layer was dried over $Na_2SO_4$, filtered and concentrated. The crude residue was purified by flash chromatography on silica gel to afford **Intermediate 61** (137 mg, 0.34 mmol, 80%) as an off white solid.

NMR $^1H$ (400 MHz, $CDCl_3$) δ : 8.51 (d, *J* = 2.3 Hz, 1H), 8.48 (dd, *J* = 4.8, 1.6 Hz, 1H), 7.53 (dt, *J* = 7.8, 2.0 Hz, 1H), 7.25 (s, 1H), 7.21 (dd, *J* = 7.7, 4.8 Hz, 1H), 6.45 (s, 1H), 4.44 (d, *J* = 8.8 Hz, 1H), 3.81 (p, *J* = 8.6, 7.5 Hz, 3H), 2.98 (t, *J* = 7.2 Hz, 2H), 1.72 - 1.45 (m, 4H), 0.96 (t, *J* = 7.4 Hz, 6H).

MS (ES+, m/z): [M+H+] 404.1.

**10.2. Intermediate 62: 7-bromo-*N2*-(1-ethylpropyl)-*N4*-[2-(4-pyridyl)ethyl]imidazo[2,1-*f*][1,2,4]triazine-2,4-diamine**

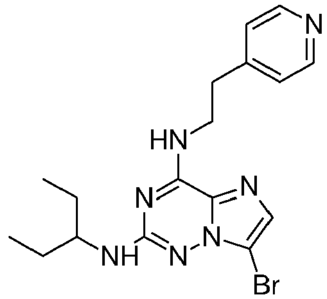

To the starting material **Intermediate 44** (60 mg, 0.17 mmol) in NMP (0.8 mL, 0.2 M) was added 1-ethylpropylamine (200 μL, 10 eq.). The solution was stirred 20 hours at 150 °C. After cooled down to room temperature, the mixture was diluted in EtOAc (1 mL) and washed with water (5 × 5 mL). Organic layer was dried over $Na_2SO_4$, filtered and concentrated. The crude residue was purified by flash chromatography on silica gel to afford **Intermediate 62** (34 mg, 0.08 mmol, 49%) as an off white solid.

NMR $^1H$ (400 MHz, $CDCl_3$) δ : 8.61 - 8.42 (m, 2H), 7.19 - 7.10 (m, 2H), 6.32 (s, 1H), 4.44 (d, *J* = 8.8 Hz, 1H), 3.90 - 3.75 (m, 3H), 2.98 (t, *J* = 7.2 Hz, 2H), 1.74 - 1.44 (m, 5H), 0.97 (t, *J* = 7.4 Hz, 6H).

MS (ES+, m/z) : [M+H+] 404.1.

**Example 11: Selection of representative compounds synthesis of formula (I) for each A classes of synthetic route 2a and 2b**

*General procedure of nucleophilic aromatic substituion in position 2*

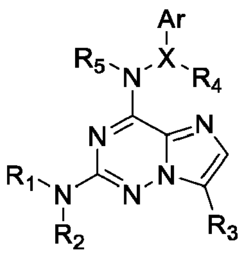

(I)

**GP7:** To a compound as obtained in **Example 7** or **Example 9** above (1.0 eq), was added $R_2R_3NH$ (15.0 eq) (with DIPEA (16.0 eq) in the case of a hydrochloride salt of $R_2R_3NH$). The mixture was heated to T for t in a sealed vial. The mixture was diluted into DCM (15 mL) and sat. $NH_4Cl$ (15 mL). The aqueous layer was extracted with DCM (2 × 15 mL). The combined organic layers were washed with brine, dried over $MgSO_4$, filtered and concentrated. The crude residue was purified by flash chromatography on silica gel and a second time with TLC prep if necessary to afford compound of formula **(I).** When needed, the product was triturated into a mixture of DCM and pentane.

Purifications conditions established following TLC reaction analysis with pure solvent or mixture thereof define before **Table 2.** All analytical data are in **Table 3** for each final compound.

### 11.1. *N*-Benzyl-7-isopropyl-2-morpholino-imidazo[2,1-*f*][1,2,4]triazin-4-amine (73)

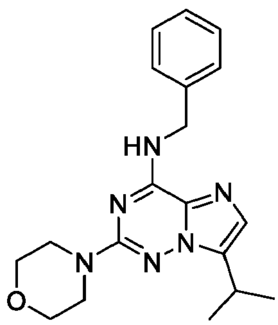

White solid (0.087 g, 0.247 mmol, 62 % yield) obtained from **intermediate 22** (0.11 g, 0.398 mmol) according to the **GP7,** with T = 160 °C and t = 16 h, purified by flash chromatography on silica gel with cHex/EtOAc (1:0 to 1:1 v/v).

### 11.2. *N4*-Benzyl-7-isorpopyl-*N2*-tetrahydropyran-4-yl-imidazo[2,1-*f*][1,2,4]diamine (44)

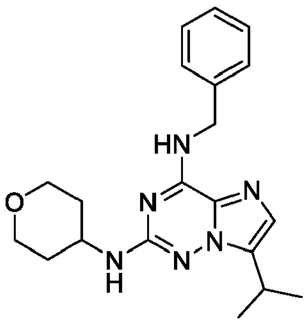

White powder (0.092 g, 0.251 mmol, 95 % yield) obtained from **intermediate 22** (0.080 g, 0.265 mmol) according to the **GP7,** with T = 160 °C and t = 16 h, purified by flash chromatography on silica gel with cHex/EtOAc (1:0 to 1:1 v/v).

**11.3. *N4*-Benzyl-*N2*-(1-ethylpropyl)-7-isopropyl-imidazo[2,1-*f*][1,2,4]triazine-2,4-diamine (7)**

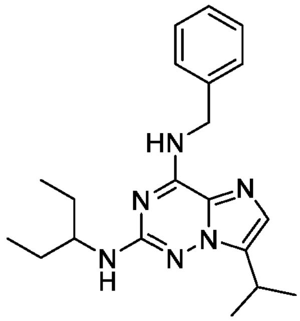

Beige solid (0.022 g, 0.0624 mmol, 32 % yield) obtained from **intermediate 22** (0.060 g, 0.198 mmol) according to the **GP7,** with T = 160 °C and t = 96 h, purified by flash chromatography on silica gel with cHex/EtOAc (1:0 to 9:1 v/v).

**11.4. *N2*-(1-ethylpropyl)-7-isopropyl-*N4*-(2-phenylethyl)imidazo[2,1-*f*][1,2,4]triazine-2,4-diamine (12)**

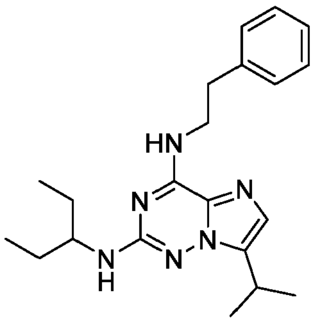

Beige solid (0.022 g, 0.0624 mmol, 32 % yield) obtained from **intermediate 22** (0.060 g, 0.198 mmol) according to the **GP7,** with T = 160 °C and t = 96 h, purified by flash chromatography on C18 reversed phase with ACN/$NH_4OH$.

**11.5. *N4*-Benzyl-*N2*-[3-(dimethylamino)propyl]-7-isopropyl-imidazo[2,1-*f*][1,2,4]triazine-2,4-diamine (5)**

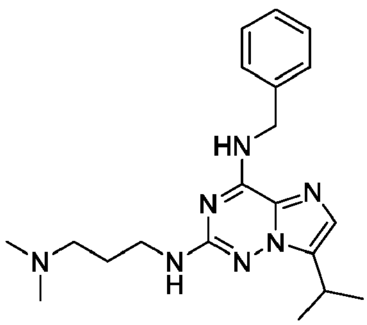

Reaction carried out according to the **GP7** in NMP (0.2M) on a 166 μmol scale of **intermediate 22,** with 10 eq of 3-(Dimethylamino)-1-propylamine at 150 °C for 16h. Purification by TLC-Prep (DCM/MeOH, 90:10 v/v). 49 % yield white solid.

**11.6. *N4*-Benzyl-*N2*-cyclohexyl-7-isopropyl-imidazo[2,1-*f*][1,2,4]triazine-2,4-diamine (59)**

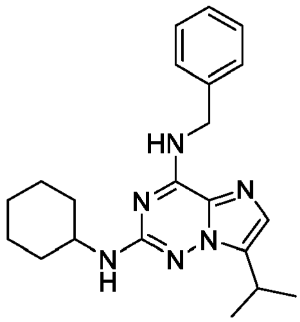

Reaction carried out according to the **GP7** in NMP (0.2M) on a 166 μmol scale of **intermediate 22,** with 10 eq of cyclohexylamine at 150 °C for 25h. Purification by flash chromatography on silica gel (DCM/EtOAc, 100:0 to 80:20 v/v). 47 % yield yellow solid.

**11.7. (1*S*)-2-[[4-(Benzylamino)-7-isopropyl-imidazo[2,1-*f*][1,2,4]triazin-2-yl]amino]cyclohexanol (62)**

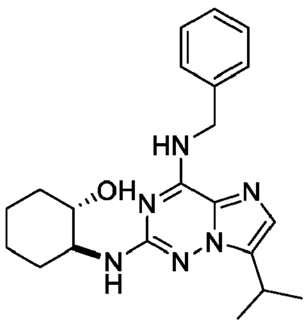

Reaction carried out according to the **GP7** in NMP (0.25M) on 166 μmol of **intermediate 22,** with 10 eq of (1S*,2S*)-2-aminocyclohexan-1-ol at 150 °C for 24h. Purification by flash chromatography on silica gel (cHex/EtOAc, 95:5 to 50:50 v/v). 86 % yield as beige solid.

**11.8. *N4*-Benzyl-7-isopropyl-*N2*-(4-methoxycyclohexyl)imidazo[2,1-*f*][1,2,4]triazine-2,4-diamine (64)**

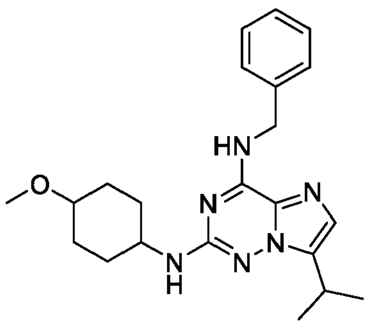

Reaction carried out according to the **GP7** in NMP (0.2M) on a 166 μmol scale of **intermediate 22,** with 10 eq of 4-methoxycyclohexylamine (mixture of diastereomers) at 150 °C for 25h. Purification by flash chromatography on

silica gel (DCM/EtOAc, 97:3 to 50:50 v/v) and PTLC (cHex/EtOAc, 80:20 v/v). 62 % yield as light pink solid.

**11.9. 7-Isopropyl-2-morpholino-*N*-(3-pyridylmethyl)imidazo[2,1-*f*][1,2,4]triazin-4-amine (74)**

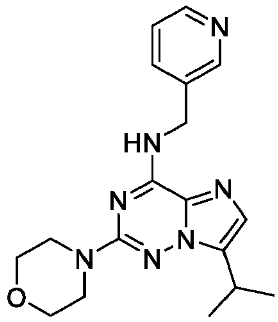

Beige solid (0.060 g, 0.170 mmol, 86 % yield) obtained from **intermediate 32** (0.060 g, 0.198 mmol) according to the **GP7,** with T = 140 °C and t = 16 h, purified by flash chromatography on silica gel with DCM/MeOH (1:0 to 95:5 v/v).

**11.10. *N*-Benzyl-7-isopropyl-2-(4-methoxy-1-piperidyl)imidazo[2,1-*f*][1,2,4]triazin-4-amine (81)**

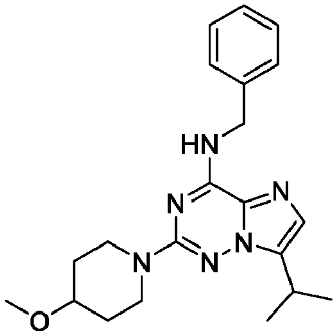

Reaction carried out according to the **GP7** in NMP (0.2M) on a 133 μmol scale of **intermediate 22,** with 10 eq of 4-Methoxypiperidine at 150 °C for 16h. Purification by flash chromatography on silica gel (CycH/EtOAc, 100:0 to 60:40 v/v). 75 % yield as white solid.

**11.11. *N4*-Benzyl-7-isopropyl-*N2*-phenyl-imidazo[2,1-*f*][1,2,4]triazine-2,4-diamine (114)**

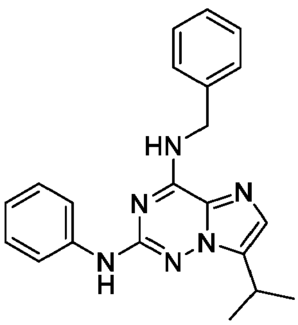

Reaction carried out according to the **GP7** in NMP (0.2M) on a 166 μmol scale of **intermediate 22,** with 10 eq

of aniline and 2 eq of DIPEA at 150 °C for 96h. Purification by flash chromatography on silica gel (DCM/EtOA, 97:3 to 70:30 v/v). 24 % yield off-white solid.

**11.12. *N2*,*N4*-Dibenzyl-7-isopropyl-imidazo[2,1-*f*][1,2,4]triazine-2,4-diamine (121)**

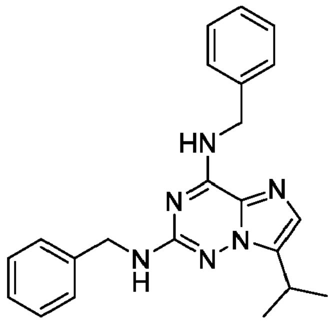

Reaction carried out according to the **GP7** in NMP (0.25M) on 166 μmol of **intermediate 22,** with 10 eq of benzylamine at 150 °C for 7h. Purification by flash chromatography on silica gel (cHex/EtOAc, 97:3 to 70:30 v/v). 88% yield as glass-like solid.

**11.13. 7-Isopropyl-*N4*-(2-pyridylmethyl)-*N2*-[(3*R*)-tetrahydrofuran-3-yl]imidazo[2,1-*f*][1,2,4]triazine-2,4-diamine (26)**

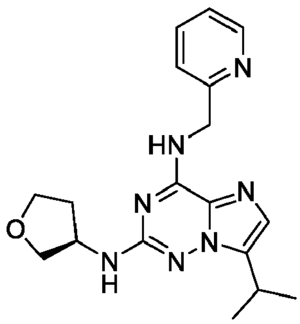

Light brown solid (0.016 g, 0.0453 mmol, 43 % yield) obtained from **intermediate 31** (0.032 g, 0.106 mmol) according to the **GP7,** with T = 120 °C and t = 32 h, purified by flash chromatography on silica gel with DCM/MeOH (1:0 to 9:1 v/v).

**11.14. *N4*-Benzyl-7-cyclopropyl-*N2*-[(3*R*)-tetrahydrofuran-3-yl]imidazo[2,1-*f*][1,2,4]triazine-2,4-diamine (37)**

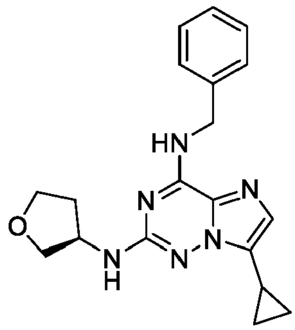

White solid (0.022 g, 0.0625 mmol, 47 % yield) obtained from **intermediate 42** (0.040 g, 0.133 mmol) according to the **GP7,** with T = 140 °C and t = 22 h, purified by flash chromatography on silica gel with DCM/MeOH (1:0 to 98:2 v/v).

**11.15. *N4*-Benzyl-7-isopropyl-*N2*-(2-methylpyrazol-3-yl)imidazo[2,1-*f*][1,2,4]triazine-2,4-diamine (107)**

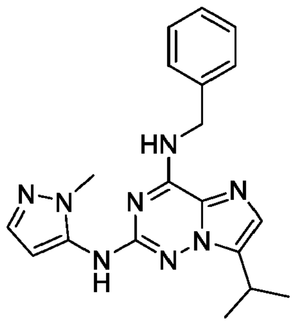

Reaction carried out according to the **GP7** in NMP (0.2M) on a 166 μmol scale of **intermediate 22,** with 10 eq of 2-Methyl-2H-pyrazol-3-ylamine and 3 eq of KHMDS at 150 °C for 36h. Purification by flash chromatography on silica gel (DCM/MeOH, 100:0 to 80:20) and PTLC (cHex/EtOAc, 20:80 v/v). 36 % yield as orange powder.

**11.16. 7-Isopropyl-*N4*-(4-pyridylmethyl)-*N2*-[(3*R*)-tetrahydrofuran-3-yl]imidazo[2,1-*f*][1,2,4]triazine-2,4-diamine (32)**

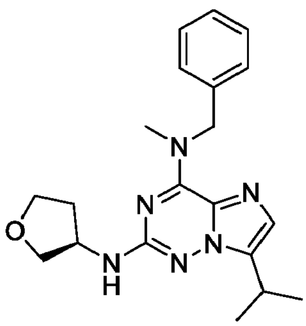

Slightly yellow paste (0.031 g, 0.0846 mmol, 65 % yield) obtained from **intermediate 35** (0.041 g, 0.130 mmol) according to the **GP7,** with T = 140 °C and t = 22 h, purified by flash chromatography on silica gel with DCM/MeOH (1:0 to 97:3 v/v).

**11.17. *N4*-Benzyl-7-isorpopyl-*N2*-tetrahydropyran-4-yl-imidazo[2,1-*f*][1,2,4]diamine (49)**

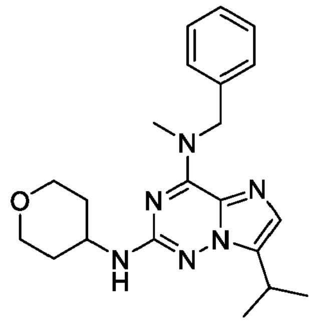

Brown paste (0.016 g, 0.0420 mmol, 30 % yield) obtained from **intermediate 35** (0.045 g, 0.143 mmol) according to the general procedure, with T = 140 °C and t = 22 h, purified by flash chromatography on silica gel with DCM/MeOH (1:0 to 97:3 v/v).

**11.18. *N4*-Benzyl-7-isopropyl-*N4*-methyl-*N2*-(2-methylpyrazol-3-yl)imidazo[2,1-*f*][1,2,4]triazine-2,4-diamine (108)**

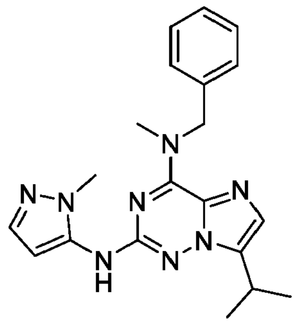

Yellow solid (0.009 g, 0.0239 mmol, 8 % yield) obtained from **intermediate 35** (0.090 g, 0.285 mmol) with tBuOK (0.128 g, 0.133 mmol) according to the **GP7,** with T = 130 °C and t = 2 h, purified by flash chromatography on silica gel with DCM/EtOAc (95:5 to 85:15 v/v).

**11.19. *N4*-Benzyl-7-isopropyl-*N2*-[(2-methylpyrazol-3-yl)methyl]imidazo[2,1-*f*][1,2,4]triazine-2,4-diamine (117)**

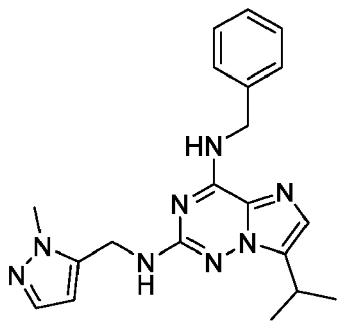

Reaction carried out according to the **GP7** in NMP (0.25M) on a 199 μmol scale of **intermediate 22,** with 10

eq of (1-methyl-1H-pyrazol-5-yl)methanamine at 150 °C for 24h. Purification by flash chromatography on silica gel (c-Hex/EtOAc, 90:10 to 0:100) and PTLC (DCM/MeOH, 97:3 v/v). 57 % yield as white solid.

**11.20. *N4*-Benzyl-*N2*-(1,3-dimethylpyrazol-4-yl)-7-isopropyl-imidazo[2,1-*f*][1,2,4]triazine-2,4-diamine (112)**

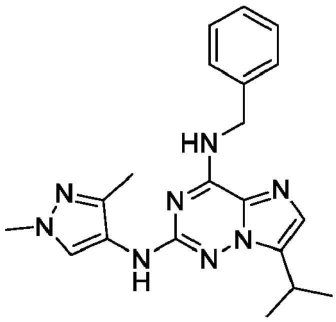

Reaction carried out according to the **GP7** in Dioxane (0.25M) on a 330 μmol scale of **intermediate 22,** with 1.2 eq of 1,3-dimethylpyrazol-4-amine, 2 eq of $Cs_2CO_3$ and 0.1 eq of XantPhos-Pd-G3 at 120 °C for 24 h. Purification by flash chromatography on silica gel (DCM/EtOAc 95:5 to 0:100 v/v) and PTLC (DCM/MeOH, 98:2 v/v). 35 % yield as white solid.

**11.21. *N4*-Benzyl-*N2*-(2,5-dimethylpyrazol-3-yl)-7-isopropyl-imidazo[2,1-*f*][1,2,4]triazine-2,4-diamine (113)**

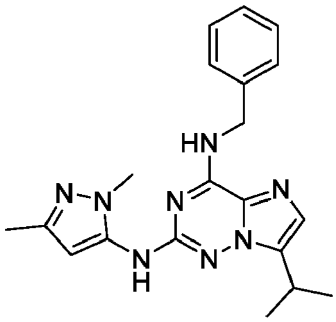

Reaction carried out according to the **GP7** in NMP (0.25M) on a 199 μmol scale of **intermediate 22**, with 8 eq of 1,3-dimethyl-1H-pyrazol-5-amine and 4 eq of $Cs_2CO_3$ at 150 °C for 48h. Purification by flash chromatography on silica gel (DCM/EtOAc, 95:5 to 0:100 v/v) and PTLC. 27 % yield as yellow solid.

**11.22. *N4*-Benzyl-7-isopropyl-*N2*-(2-pyridylmethyl)imidazo[2,1-*f*][1,2,4]triazine-2,4-diamine (123)**

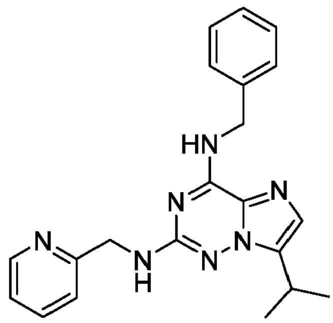

Reaction carried out according to the **GP7** in NMP (0.2M) on 166 μmol of **intermediate 22**, with 10 eq of 2-picolylamine at 150 °C for 16h. Purification by flash chromatography on silica gel (DCM/MeOH, 100:0 to 97:7 v/v). 49 % yield as yellow oil.

**Example 12: Selection of representative compounds synthesis of formula (I) for synthetic route 2C**

**12.1. 7-cyclopropyl-*N2*-(1-ethylpropyl)-*N4*-[2-(4-pyridyl)ethyl]imidazo[2,1-*f*][1,2,4]triazine-2,4-diamine (16)**

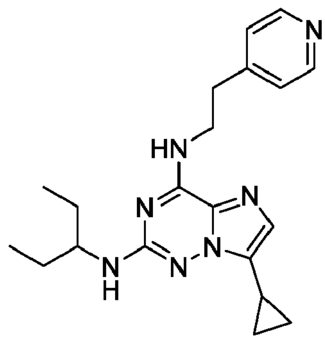

Reaction carried out in THF (0.25M) on a 75 μmol scale of **Intermediate 62** with 3 eq of cyclopropylzinc bromide and 5 mol% of CPhos-Pd-G3 at 40 °C for 16h. Purification by FC (DCM/MeOH + $NH_3$ 7N gradient) followed by TLC prep (DCM/MeOH/THF). 29 % yield as white solid.

**12.2. 7-cyclopropyl-*N2*-(1-ethylpropyl)-*N4*-[2-(3-pyridyl)ethyl]imidazo[2,1-*f*][1,2,4]triazine-2,4-diamine (15)**

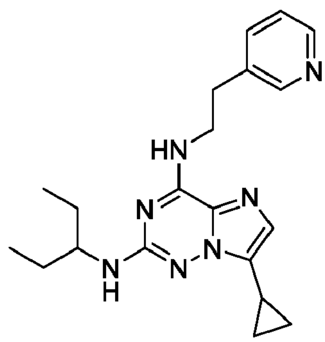

Reaction carried out in THF (0.25M) on a 125 μmol scale of **Intermediate 61** with 3 eq of cyclopropylzinc bromide and 5 mol% of CPhos-Pd-G3 at 40 °C for 4h. Purification by FC (DCM/MeOH) followed by TLC prep (THF/DCM/MeOH). 18 % yield as yellowish oil.

## Example 13: Biological activity

### MATERIAL AND METHODS

### Biological reagents

F508del-CFTR expressing cells used in this present study were treated with CFTR modulators 24 h prior the experiments. The following CFTR modulators were used: Vx770 (Ivacaftor) (Selleckchem, S 1144) (Selleckchem, 24, rue des Tuileries, BP684, 67460 Souffelweyersheim, France), Vx445 (Elexacaftor) (Selleckchem, SE-S8851), Vx661 (Tezacaftor) (TargetMol, HY-15448) (TargetMol, Tebu-bio, 39 rue de Houdan, BP 15, 78612 Le-Perray-en-Yvelines cedex, France). Trikafta® is the combination of 1 μM Vx770, 3 μM Vx 445 and 18 μM Vx661.
Amiloride (A7410), Forskolin (F6886), UTP (U6875) were purchased from Sigma Aldrich. CFTR Inh172 (219670) was obtained from Millipore Corp. Dimethyl sulfoxide (DMSO) was used as solvent for all the compounds.

### Cell culture

CFBE cell line stably expressing F508del-CFTR or WT-CFTR (CFBE WT-CFTR) were grown at 37°C in 5% CO2 - 95% air and media was replaced every 2 days. CFBE41o- cells overexpressing F508del-CFTR (CFBE F508del-CFTR) and CFBE WT-CFTR were grown on TPP® culture flasks in Eagle's Minimum Essential Medium (EMEM) containing non-essential amino acids (NEAA) (Gibco 10370) (Gibco/Thermo, Parc d'innovation, Boulevard Sebastien Brant, 67403 Illkirch cedex, France) supplemented with 10% fetal bovine serum (FBS) (Eurobio, CVFSVF00-01) (EuroBio, Technopolis Bat. 3, 17 Avenue du Parc 91380 Chilly-Mazarin, France, 2 mM L-glutamine (Gibco, G7513), 50 IU.mL-1 penicillin (Sigma Aldrich, 80 rue de Luzais BP 701 38070 Saint Quentin Fallavier) (Millipore-Sigma-Aldrich, 80 Rue de Luzais, BP701, Saint Quentin Fallavier, France), 50 μg/mL of streptomycin (Sigma Aldrich, P0781) and were selected using 5 μg/mL puromycin (Gibco, A11138-03) (Kunzelmann *et a.l,* 1993. *An immortalized cystic fibrosis tracheal epithelial cell line homozygous for the delta F508 CFTR mutation.* Am. J. Respir. Cell Mol. Biol. 8, 522-529. Froux *et al.,* 2019. *The short-term consequences of F508del-CFTR thermal instability on CFTR-dependent transepithelial currents in human airway epithelial cells.* Sci. Rep. 9, 13729. Becq *et al.,* 2021. *The rescue of F508del-CFTR by elexacaftor/tezacaftor/ivacaftor (Trikafta®) in human airway epithelial cells is underestimated due to the presence of ivacaftor.* Eur. Respir. J. 2021 Jul 15:2100671).

### CFTR activation assay: short-circuit current measurements

CBFE F508del-CFTR and CFBE WT-CFTR cells were seeded at a density of 5×105 cells on Snapwell permeable inserts (Corning, 3407, USA) coated with 5 μg/cm2 human fibronectin (Sigma Aldrich). After 2 days at liquid/liquid interface, cells were cultured at air/liquid interface during at least 2 weeks. The transepithelial resistances reached a minimum of ≈400-500 Ω·cm-2, as measured with a Millicell-ERS voltmeter-ohmmeter (Millipore).
Inserts containing CFBE cells were mounted in an EM-CSYS-6 Ussing chamber system (Physiologic Instruments Inc., 5875 Tyrone Rd, Reno, NV 89502, USA) composed of two hemi-chambers, each containing a different solution to create a basal to apical Cl-gradient to enhance Cl- currents detection. Their compositions were: 1.2 mM NaCl, 115 mM Na-Gluconate, 25 mM $NaHCO_3$, 1.2 mM $MgCl_2$, 4 mM $CaCl_2$, 2.4 mM $KH_2PO_4$, 1.24 mM $K_2HPU_4$, 10 mM mannitol (pH 7.4 and 300 mosm/L) for apical solution and 115 mM NaCl, 25 mM $NaHCO_3$, 1.2 mM $MgCl_2$, 1.2 mM $CaCl_2$, 2.4 mM $KH_2PO_4$, 1.24 mM $K_2HPO_4$, 10 mM glucose (pH 7.4 and 300 mosm/L) for basal solution. Both of the solutions keep maintained at 37°C with a circulating temperature water bath and gassed with 95% 02 - 5% CO2. Transepithelial potential difference and short-circuit currents (clamp at 0 mV) were measured/injected through 3 M KCl filled Ag/AgCl electrodes connected to a VCC MC2 voltage/current clamp (Physiologic Instruments Inc.). The current injected by the system to short-circuit cells (Isc) was visualized and recorded at a frequency of 0.1 Hz on a personal computer using Acquire and Analyse hardware and software (Physiologic Instruments Inc.). Transepithelial potential difference values were corrected for the junction potential between apical and basal solutions and for empty insert resistance. In our conditions, an apical anion secretion was indicated by an increase in Isc. All experiments were done in the presence of 100 μM amiloride in the apical solution to prevent ENaC currents, 1 μM Forskolin was added to apical solution to activate CFTR currents; 1 μM Vx770 was used in apical solution to potentiate CFTR current; 10 μM CFTR Inh172 in the apical solution was used as a reference to calculate ΔIsc values and 100 μM UTP to prove cell viability (Froux *et al.,* 2019. *The short-term consequences of F508del-CFTR thermal instability on CFTR-dependent transepithelial currents in human airway epithelial cells.* Sci. Rep. 9, 13729).

**Data Analysis and statistics**

Data are presented as Mean ± SEM of at least 3 observations. Statistical comparisons were made using nonparametric or parametric tests with a significance level of 0.05. Before using a parametric test, samples were checked for normality using the Shapiro-Wilk normality test. Statistical significance was determined using the Ordinary One-Way Anova test following by a Fisher's LSD test using GraphPad Prism version 6.0 (GraphPad software, San Diego, CA, USA) software. Differences were considered statistically significant at $p<0.05$.

**RESULTS**

**Table 4:** Classification of compounds according to their ability to enhance F508del-CFTR activation by Trikafta®. Compounds were tested according to Figure 1, and the F508del-CFTR activity was quantified by its sensitivity ('inhibition' on Fig. 1) to CFTR-172 and is reported as related to the activity of Trikafta® alone (100%). Compounds were tested at three doses (0.1, 1, 10 μM) and classified in four groups: **I** (110-120% activity), **II** (120-140%), **III** (140-160%), **IV** (160-200%).

| Dose | Group I 110-120% | Group II 120-140% | Group III 140-160% | Group IV 160-200% |
|---|---|---|---|---|
| **0.1 μM** | 3, 19, 20, 32, 46, 48, 49, 50, 52, 57, 58, 71, 91, 97, 101, 102, 103, 105, 110, 111, 115, 119, 124, 132 | 24, 27, 28, 30, 41, 42, 43, 44, 51, 56, 73, 93, 98, 100, 109, 117,125,133 | 96, 107, 113 | - |
| **1.0 μM** | 3, 21, 31, 32, 33, 38, 47, 60, 61, 62, 88, 92, 99,105,109,114,129, 130 | 6, 19, 20, 22, 23, 27, 37, 39, 41, 43, 44, 45, 49, 56, 64, 74, 91, 95, 98, 100, 101, 103, 110, 112, 115, 119, 120, 121, 122, 123, 124, 125 | 28, 42, 46, 48, 51, 52, 57, 58, 63, 94, 97, 102, 117 | 25, 93, 96, 107, 113, 133 |
| **10 μM** | 26, 30, 40, 92 | 6, 22, 28, 32, 45, 50, 73, 88, 89, 90, 93, 96, 98, 100, 101, 111, 132 | 4, 19, 21, 23, 24, 25, 39, 46, 47, 49, 56, 57, 69, 91, 94, 95, 99, 105, 109, 110 | 20, 31, 38, 41, 43, 44, 48, 58, 59, 70, 97, 102, 103, 107, 113, 115, 124, 125 |

Figure 2 represents the dose-response curves for compounds (44) and (107) tested in combination with Trikafta®. Dose-response curves were built from the activation and inhibition data (see FIG. 1). 100% represents the activity of Trikafta® alone.

In addition, figure 3 more particularly represents the results of compound (107) in combination with Trikafta®.

Finally, compound (107) was also tested according to Figure 1, i.e. according to the same protocol as described above in combination with other CFTR modulators than Trikafta® and namely Galapagos compounds GLPG 222 **(G1),** GLPG 1837 **(G2)** and GLPG 2451 **(G3),** as described above. It can be stated than an activating effect may similarly be observed in presence of compound (107).

**CONCLUSION**

Based on the previous results, it can be concluded that the compounds of formula (I) are suitable chemical compounds useful as activators or boostors in the prevention and/or treatment of genetic diseases associated with ABC transporters trafficking defects, and in particular in the prevention and/or treatment of Cystic Fibrosis.

**Example 14: Elimination of kinase activity of compounds of formula (I)**

CFE cells were treated with Trikafta® in the presence of increasing concentrations of various compounds and their corresponding compounds having a $\mathbf{R_5}$ group being a methyl group according to the protocol as set forth in example 13. Their F508del-CFTR activity is reported as percentage of F508del-CFTR activity in the presence of Trikafta® alone (100%). It was calculated on the basis of the inhibitory effect of the CFTR inhibitor CFTR-172, as shown in Fig. 1 (see

Table 5 below).

All compounds were additionally tested over a range of concentrations for their ability to inhibit a series of 14 kinases. $IC_{50}$ values were calculated from the dose-response curves and are reported in μM. > 10, no inhibition at 10 μM, the highest dose tested (see table 6 below).

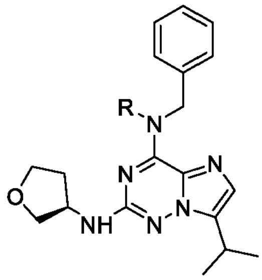

**19: R=H**
**32: R=Me**

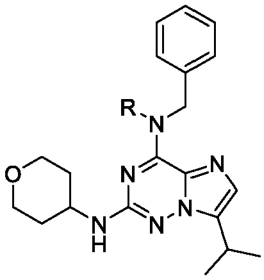

**44: R=H**
**49: R=Me**

**Table 5:** CFE cells were treated with Trikafta in the presence of increasing concentrations of various compounds and their N7-methylated counterparts. Their F508del-CFTR activity is reported as percentage of F508del-CFTR activity in the presence of Trikafta alone (100%). It was calculated on the basis of the inhibitory effect of the CFTR inhibitor CFTR-172, as shown in Fig. 1.

| | | concentration (μM) | | |
|---|---|---|---|---|
| **Compound** | **R** | **0.1** | **1** | **10** |
| **19** | **H** | 112.0 | **122.2** | **155.6** |
| **32** | **Methyl** | 117.9 | 113.7 | **135.1** |
| **44** | **H** | 127.2 | **133.8** | **161.3** |
| **49** | **Methyl** | 113.0 | **125.9** | **147.1** |

**Table 6:** All compounds were tested over a range of concentrations for their ability to inhibit a series of 14 kinases. $IC_{50}$ values were calculated from the dose-response curves and are reported in μM. > 10, no inhibition at 10 μM, the highest dose tested.

| | **19** | **32** | **44** | **49** |
|---|---|---|---|---|
| **CDK2** | 1,278 | >10 | 0,579 | >10 |
| **CDK7** | 0,496 | >10 | 0,207 | >10 |
| **CDK9** | 3,181 | >10 | 1,170 | >10 |
| **CK1α1** | >10 | >10 | >10 | >10 |
| **CK1δ** | 0,594 | >10 | 0,626 | >10 |
| **CK1ε** | 1,324 | >10 | 1,731 | >10 |
| **CK1γ1** | >10 | >10 | >10 | >10 |
| **CK1γ2** | >10 | >10 | >10 | >10 |
| **CK1γ3** | >10 | >10 | >10 | >10 |
| **CLK1** | 1,174 | >10 | 0,537 | >10 |
| **CLK2** | 1,047 | >10 | 0,493 | >10 |
| **CLK3** | >10 | >10 | >10 | >10 |
| **CLK4** | 1,840 | >10 | 0,580 | >10 |

(continued)

| | 19 | 32 | 44 | 49 |
|---|---|---|---|---|
| DYRK1A | >10 | >10 | 6,527 | >10 |

Results show that indeed the methylated compounds, i.e. having $\mathbf{R_5}$ being a methyl group, loose kinase inhibitory activity but maintain essentially all their ability to enhance Trikafta®'s activity on F508del-CFTR. This methylation offers the possibility to remove the kinase inhibitory activity of all compounds of formula (I) according to the present invention, in particular being Trikafta® activators or boosters, thereby removing side effects that might be linked to kinase inhibition.

The present invention further relates to a pharmaceutical composition comprising at least one compound of formula (I) as defined above or any of its pharmaceutically acceptable salts or at least any of compounds (1) to (133) as defined above or any of its pharmaceutically acceptable salts and also at least one pharmaceutically acceptable excipient.

Pharmaceutical compositions of the invention can contain one or more compound(s) of the invention in any form described herein.

Still a further object of the present invention consists of the use of at least one compound of formula (I) as defined above, and compounds (1) to (133) as defined above, or one of their pharmaceutically acceptable salts according to the present invention for preparing a drug for preventing and/or treating genetic diseases associated with ABC transporters trafficking defects, and in particular in the prevention and/or treatment of Cystic Fibrosis, in particular in combination with other CFTR modulators or combinations thereof,. According to a particular embodiment, the treatment is continuous or non-continuous.

A "continuous treatment" means a long-term treatment which can be implemented with various administration frequencies, such as once every day, every three days, once a week, or once every two weeks or once every month or by transdermal patch delivery.

According to one embodiment, the compound of formula (I), or any of its pharmaceutically acceptable salts, is administered at a dose varying from 0.1 to 1000 mg. in particular varying from 1 to 500 mg, or for example varying from 5 to 100 mg.

Another object of the invention relates to a therapeutic method for the treatment and/or for the prevention of genetic diseases associated with ABC transporters trafficking defects, and in particular for the treatment and/or for the prevention of Cystic Fibrosis, in particular in combination with other CFTR modulators or combinations thereof, consisting in administering to a patient in need thereof of a compound of formula (I) or any of its pharmaceutically acceptable salt or of at least any of compounds (1) to (133) as defined above, of any of its pharamecutically acceptable salt. In some embodiment of said method for the treatment and/or for the prevention, the CFTR modulator is selected from GLPG 2222, GLPG 1837, GLPG 2451, their combinations such as GLPG 2222 and GLPG 1837, GLPG 2222 and GLPG 2451 and GLPG 2222, GLPG 1837 and GLPG 2451, lumacaftor, galicaftor, navocaftor, dirocaftor, nesolicaftor, posenacaftor, ivacaftor, tezacaftor, elexacaftor, and their combinations, in particular is selected from ivacaftor, tezacaftor and elexacaftor and more particularly is a mixture of ivacaftor, tezacaftor and elexacaftor.

In a specific embodiment, the invention provides the use of a compound of formula (I) according to the invention or a pharmaceutically acceptable salt thereof or a pharmaceutically active derivative thereof or a method according to the invention wherein the compound of formula (I) is to be administered in combination with a ABC modulator, including a CFTR modulator, or combination of CFTR modulators or a pharmaceutically acceptable salt thereof useful in anyone of the hereabove mentioned diseases.

All combinations of doses, frequencies and treatment period are encompassed within the scope of the present invention.

Administration of the compound of formula (I) as defined herein above, a pharmaceutically acceptable salt thereof, and an ABC modulator, including the CFTR modulator or combination of CFTR modulators or a pharmaceutically acceptable salt thereof may be simultaneous, separate or spread out over time.

The combination can be administered repeatedly over the course of several sequences or cycles according to a protocol which depends on the nature of the disease and intensity of the disease to be treated and also on the patient to be treated (age, weight, previous treatment(s), etc.). The protocol can be determined by any specialized practitioner.

Various sequences or cycles of administration respectively of the compound of formula (I) as defined herein above, a pharmaceutically acceptable salt thereof, prodrug thereof and the ABC modulator, including a CFTR modulator or combination of CFTR modulators or a pharmaceutically acceptable salt thereof may be implemented within the framework of the present invention.

According to a particular embodiment, and to one of the possible sequences of administration, the compound of formula (I) as defined herein above, a pharmaceutically acceptable salt thereof, is administered to the patient during a first phase of treatment and the patient is then treated with the ABC modulator, including a CFTR modulator or

combination of CFTR modulators or a pharmaceutically acceptable salt thereof in a second phase of the treatment or inversely. Said both phases may overlap or not.

As another alternative of the sequential administration as described above, the compound of formula (I) as defined herein above, a pharmaceutically acceptable salt thereof, and an ABC modulator, including a CFTR modulator or combination of CFTR modulators or a pharmaceutically acceptable salt thereof may be administered in a unique dosage form or unit pharmaceutical preparation.

The compounds of formula (I) as defined above can be administered through any mode of administration such as, for example, intramuscular, intravenous. intranasal or oral route, transdermal patch, etc.

The inventive composition can further include one or more additives such as diluents, excipients, stabilizers and preservatives. Such additives are well known to those skilled in the art and are described notably in *"Ullmann's Encyclopedia of Industrial Chemistry, 6th Ed."* (various editors, 1989-1998, Marcel Dekker) and in *"Pharmaceutical Dosage Forms and Drug Delivery Systems"* (ANSEL *et al,* 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) according to the present invention can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.

Oral route of administration is in particular preferred in the prophylaxis or treatment aspect of the invention.

## Claims

1. A combination of a compound of formula (I) or any of its pharmaceutically acceptable salt with an ABC transporter modulator, including a CFTR modulator or a combination of CFTR modulators, for use in the prevention and/or treatment of genetic diseases associated with ABC transporters trafficking defects, in particular cystic fibrosis

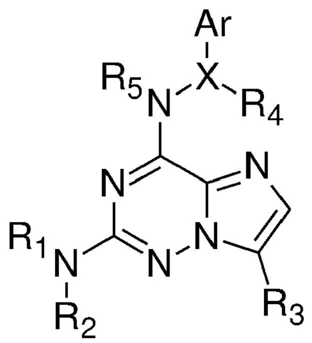

(I)

wherein

Ar represents

- a phenyl group, optionally substituted by one to three groups selected from a $(C_1-C_3)$alkyl group, a $(C_1-C_3)$alkoxy group, a $(C_1-C_3)$fluoroalkyl group and a halogen atom, or
- a 6-membered heteroaryl group comprising one to three nitrogen atoms, optionally substituted by one to three groups selected from a $(C_1-C_3)$alkyl group, a $(C_1-C_3)$alkoxy group and a halogen atom,

**-X-** represents a -CH-group, a bond, a -CHCH2- group or a -CH2CH group-,

**$R_1$** represents

- a $(C_1\text{-}C_6)$alkyl group, optionally substituted by a group selected from a hydroxy group, a halogen atom, a $NR_6R_7$ group, a $(C_1\text{-}C_4)$alkylthio group, a phenoxy group and a $(C_1\text{-}C_3)$alkoxy group,
- a **-R-L-** group, wherein

**L** is either a single bond, a $(C_1\text{-}C_3)$alkanediyl group, a -O-$(C_1\text{-}C_3)$alkanediyl group, said $(C_1\text{-}C_3)$alkanediyl group being optionally substituted by a group selected from a hydroxy group and a $(C_1\text{-}C_3)$alkoxy group or a -$SO_2$- group, and
**R** represents:

- a $(C_3\text{-}C_8)$cycloalkyl group, optionally substituted by one to three groups selected from a hydroxy group, a halogen atom, a $(C_1\text{-}C_4)$alkyl group and a $(C_1\text{-}C_3)$alkoxy group,
- a $(C_3\text{-}C_8)$heterocycloalkyl group, optionally substituted by one or two groups selected from a hydroxy group, a halogen atom, an oxo group, a $(C_1\text{-}C_4)$alkyl group and a $(C_1\text{-}C_3)$alkoxy group,
- a $(C_5\text{-}C_6)$heteroaryl group, optionally substituted by one to three groups selected from a ($C_1$ C4)alkyl group, a hydroxy group, a halogen atom and a $(C_1\text{-}C_3)$alkoxy group,
- a phenyl group, optionally substituted by one or two groups selected from a hydroxy group, a halogen atom, and a $(C_1\text{-}C_3)$alkoxy group,

$\mathbf{R_2}$ represents a hydrogen atom or a $(C_1\text{-}C_3)$alkyl group, or alternatively $\mathbf{R_1}$ and $\mathbf{R_2}$ together form with the nitrogen atom to which they are attached, a $(C_3\text{-}C_8)$heterocycloalkyl group or a spiro$(C_5\text{-}C_{11})$heterobicycloalkyl group, said groups being optionally substituted by one or two groups selected from a hydroxy group, a $(C_1\text{-}C_3)$alkoxy group, a hydroxy$(C_{1\text{-}}C_4)$alkyl group, an oxo group and a $(C_1\text{-}C_4)$alkyl group,
$\mathbf{R_3}$ represents

- a hydrogen atom,
a $(C_1\text{-}C_6)$alkyl group,
- a $(C_3\text{-}C_8)$cycloalkyl group,
- a halogen atom, or
- a phenyl group optionally substituted by one to three groups selected from a $(C_1\text{-}C_3)$alkyl group, a $(C_1\text{-}C_3)$alkoxy group, a $(C_1\text{-}C_3)$fluoroalkyl group and a halogen atom.

$\mathbf{R_4}$ represents a hydrogen atom or a $(C_1\text{-}C_4)$alkyl group or is absent when **-X-** is a bond,
$\mathbf{R_5}$ represents a hydrogen atom or a $(C_1\text{-}C_4)$alkyl group, and
$\mathbf{R_6}$ and $\mathbf{R_7}$ independently represent a hydrogen atom or a $(C_1\text{-}C_4)$alkyl group.

2. A combination for use according to claim 1, wherein genetic diseases associated with ABC transporters trafficking defects are Tangier Disease, Familial hypoapoproteinemia (ABCA1), Stargardt/fundus flavimaculatis, Retinitis pigmentosa & Cone-rod dystrophy & Age-related macular degeneration (ABCA4), Stargardt Macular disease (ABCA4), Harlequin ichthyosis (ABCA12), biliary diseases (such as Progressive familial intrahepatic cholestasis type 2 or 3 (PFIC2, PIFC3) (ABCB4, ABCB11), Dubin-Johnson Syndrome (ABCC2), Pseudoxanthoma elasticum (ABCC6), Cystic Fibrosis (ABCC7 = CFTR), idiopathic chronic cholestasis (ABCC12), Adrenoleukodystrophy (ABCD1), low-phospholipid associated cholelithiasis syndrome, intrahepatic cholestasis of pregnancy), Sitosteroliemia and Familial Hypercholesterolemia (ABCG5, ABCG8), and in particular Cystic Fibrosis.

3. A combination for use according to claim 1 or 2, wherein **Ar** represents

- a phenyl group, optionally substituted by one to three groups selected from a $(C_1\text{-}C_3)$alkyl group, a $(C_1\text{-}C_3)$alkoxy group, a $(C_1\text{-}C_3)$fluoroalkyl group and a halogen atom,
- a pyridyl group, optionally substituted by one to three groups selected from a (C1-C3)alkyl group, a (C1-C3)alkoxy group and a halogen atom,
- a pyrimidinyl group, optionally substituted by one to three groups selected from a (C1-C3)alkyl group, a (C1-C3)alkoxy group and a halogen atom, or
- a pyrazinyl group, optionally substituted by one to three groups selected from a (C1-C3)alkyl group, a (C1-C3)alkoxy group and a halogen atom, and more particularly represents
- a phenyl group, optionally substituted by one to three groups selected from a $(C_1\text{-}C_3)$alkyl group, a $(C_1\text{-}C_3)$alkoxy group, a $(C_1\text{-}C_3)$fluoroalkyl group and a halogen atom,
- a pyridyl group, or
- a pyrazinyl group.

**4.** A combination for use according to anyone of claims 1 to 3, wherein

**$R_1$** represents

- a $(C_1-C_6)$alkyl group, optionally substituted by a group selected from a hydroxy group, a NR6R7 group, a $(C_1-C_4)$alkylthio group, a phenoxy group and a $(C_1-C_3)$alkoxy group,
- a **-R-L-** group, wherein

**L** is either a single bond, a -CH2- group, a -CH(CH3)- group, a -CH2-CH2- group, or a $-SO_2-$ group, and **R** represents:

- a $(C_3-C_8)$cycloalkyl group, optionally substituted by one to three groups selected from a hydroxy group, a halogen atom, and a $(C_1-C_3)$alkoxy group,
- a $(C_3-C_8)$heterocycloalkyl group, optionally substituted by one or two groups selected from a hydroxy group, a $(C_1-C_4)$alkyl group and a $(C_1-C_3)$alkoxy group,
- a $(C_5-C_6)$heteroaryl group, optionally substituted by one to three groups selected from a $(C_1\ C_4)$alkyl group and a halogen atom, or
- a phenyl group, optionally substituted by one or two halogen atoms, and

**R2** represents a hydrogen atom or a $(C_1-C_3)$alkyl group, or alternatively **$R_1$** and **$R_2$** together form with the nitrogen atom to which they are attached, a $(C_3-C_8)$heterocycloalkyl group, optionally substituted by one or two groups selected from a hydroxy group, a $(C_1-C_3)$alkoxy group, a hydroxy$(C_{1-}C_4)$alkyl group or a spiro$(C_5-C_{11})$heterobicycloalkyl group, and **$R_6$** and **$R_7$** independently represent a hydrogen atom or a methyl group.

**5.** A combination for use according to anyone of claims 1 to 4, wherein **$R_3$** represents

- a hydrogen atom,
- a methyl group, an ethyl group or an isopropyl group,
- a cyclopropyl group, a cyclopentyl group or a cyclohexyl group,
- a bromine atom, or
- a phenyl group.

**6.** A combination for use according to anyone of claims 1 to 5, wherein **$R_4$** represents a hydrogen atom or a methyl group or is absent when **-X-** is a bond.

**7.** A combination for use according to anyone of claims 1 to 6, wherein **$R_5$** is a hydrogen atom.

**8.** A combination for use according to anyone of claims 1 to 6, wherein **$R_5$** is a $(C_1-C_4)$alkyl group, in particular a methyl group.

**9.** A combination for use according to anyone of claims 1 to 8, wherein the ABC transporter modulator, in particular the CFTR modulator, is selected from GLPG 2222, GLPG 1837, GLPG 2451, their combinations such as GLPG 2222 and GLPG 1837, GLPG 2222 and GLPG 2451 and GLPG 2222, GLPG 1837 and GLPG 2451, lumacaftor, galicaftor, navocaftor, dirocaftor, nesolicaftor, posenacaftor, ivacaftor, tezacaftor, elexacaftor, and their combinations, in particular is selected from ivacaftor, tezacaftor and elexacaftor and more particularly is a mixture of ivacaftor, tezacaftor and elexacaftor.

**10.** A compound of formula (I) or any of its pharmaceutically acceptable salt,

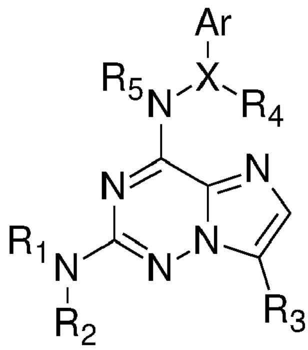
(I)

wherein

Ar represents

- a phenyl group, optionally substituted by one to three groups selected from a $(C_1-C_3)$alkyl group, a $(C_1-C_3)$alkoxy group, a $(C_1-C_3)$fluoroalkyl group and a halogen atom,
- a 6-membered heteroaryl group comprising one to three nitrogen atoms, optionally substituted by one to three groups selected from a $(C_1-C_3)$alkyl group, a $(C_1-C_3)$alkoxy group and a halogen atom, and in particular a pyridyl, a pyrimidinyl or a pyrazinyl group, more particularly a pyridyl or a pyrazinyl group, optionally substituted by one to three groups selected from a $(C_1-C_3)$alkyl group, a $(C_1-C_3)$alkoxy group and a halogen atom,

**-X-** represents a -CH-group, a bond, a -CHCH2- group or a -CH2CH group-,
**$R_1$** represents

- a $(C_1-C_6)$alkyl group, optionally substituted by a group selected from a hydroxy group, a halogen atom, a NR6R7 group, a $(C_1-C_4)$alkylthio group, a phenoxy group and a $(C_1-C_3)$alkoxy group,
- a **-R-L-** group, wherein

**L** is either a single bond, a $(C_1-C_3)$alkanediyl group, a -O-$(C_1-C_3)$alkanediyl group, said $(C_1-C_3)$alkanediyl group being optionally substituted by a group selected from a hydroxy group and a $(C_1-C_3)$alkoxy group or a -$SO_2$- group, and
**R** represents:

- a $(C_3-C_8)$cycloalkyl group, optionally substituted by one to three groups selected from a hydroxy group, a halogen atom, a $(C_1-C_4)$alkyl group and a $(C_1-C_3)$alkoxy group,
- a $(C_3-C_8)$heterocycloalkyl group, optionally substituted by one or two groups selected from a hydroxy group, a halogen atom, an oxo group, a $(C_1-C_4)$alkyl group and a $(C_1-C_3)$alkoxy group,
- a $(C_5-C_6)$heteroaryl group, optionally substituted by one to three groups selected from a $(C_1$ C4)alkyl group, a hydroxy group, a halogen atom and a $(C_1-C_3)$alkoxy group,
- a phenyl group, optionally substituted by one or two groups selected from a hydroxy group, a halogen atom, and a $(C_1-C_3)$alkoxy group,

**R2** represents a hydrogen atom or a $(C_1-C_3)$alkyl group, or alternatively **$R_1$** and **$R_2$** together form with the nitrogen atom to which they are attached, a $(C_3-C_8)$heterocycloalkyl group or a spiro$(C_5-C_{11})$heterobicycloalkyl group, said groups being optionally substituted by one or two groups selected from a hydroxy group, a $(C_1-C_3)$alkoxy group, a hydroxy$(C_{1-}C_4)$alkyl group, an oxo group and a $(C_1-C_4)$alkyl group,
**$R_3$** represents -

- a hydrogen atom, a $(C_1-C_6)$alkyl group,
- a $(C_3-C_8)$cycloalkyl group,
- a halogen atom, or
- a phenyl group.

**$R_4$** represents a hydrogen atom or a $(C_1-C_4)$alkyl group or is absent when **-X-** is a bond,

**$R_5$** represents a hydrogen atom or a ($C_1$-$C_4$)alkyl group, and
**$R_6$** and **$R_7$** independently represent a hydrogen atom or a ($C_1$-$C_4$)alkyl group, wherein when **Ar** is a phenyl group and **$R_3$** is an isopropyl group, then **$R_1$** is different from a ($C_1$-$C_6$)alkyl group substituted by a hydroxy group.

**11.** A compound of formula (I) or any of its pharmaceutically acceptable salt, as defined in claim 10, wherein

**$R_1$** represents a ($C_1$-$C_6$)alkyl group, optionally substituted by a group selected from a hydroxy group, a halogen atom, a NR6R7 group, a ($C_1$-$C_4$)alkylthio group, a phenoxy group and a ($C_1$-$C_3$)alkoxy group, and
**R2** represents a hydrogen atom or a ($C_1$-$C_3$)alkyl group.

**12.** A compound of formula (I) or any of its pharmaceutically acceptable salt, as defined in claim 10, wherein

**$R_1$** represents a **-R-L-** group, wherein

**L** is either a single bond, a ($C_1$-$C_3$)alkanediyl group, a -O-($C_1$-$C_3$)alkanediyl group, said ($C_1$-$C_3$)alkanediyl group being optionally substituted by a group selected from a hydroxy group and a ($C_1$-$C_3$)alkoxy group or a -$SO_2$- group, and
**R** represents a ($C_3$-$C_8$)cycloalkyl group, optionally substituted by one to three groups selected from a hydroxy group, a halogen atom, a ($C_1$-$C_4$)alkyl group and a ($C_1$-$C_3$)alkoxy group, and

**R2** represents a hydrogen atom or a ($C_1$-$C_3$)alkyl group.

**13.** A compound of formula (I) or any of its pharmaceutically acceptable salt, as defined in claim 10, wherein **$R_1$** and **$R_2$** together form with the nitrogen atom to which they are attached, a ($C_3$-$C_8$)heterocycloalkyl group or a spiro($C_5$-$C_{11}$)heterobicycloalkyl group, said groups being optionally substituted by one or two groups selected from a hydroxy group, a ($C_1$-$C_3$)alkoxy group, a hydroxy($C_1$-$C_4$)alkyl group, an oxo group and a ($C_1$-$C_4$)alkyl group.

**14.** A compound of formula (I) or any of its pharmaceutically acceptable salt, as defined in claim 10, wherein

**$R_1$** represents a **-R-L-** group, wherein
**L** is either a single bond, a ($C_1$-$C_3$)alkanediyl group, a -O-($C_1$-$C_3$)alkanediyl group, said ($C_1$-$C_3$)alkanediyl group being optionally substituted by a group selected from a hydroxy group and a ($C_1$-$C_3$)alkoxy group or a -$SO_2$- group, and
**R** represents a phenyl group, optionally substituted by one or two groups selected from a hydroxy group, a halogen atom, and a ($C_1$-$C_3$)alkoxy group, and
**R2** represents a hydrogen atom or a ($C_1$-$C_3$)alkyl group.

**15.** A compound of formula (I) or any of its pharmaceutically acceptable salt, as defined in claim 10, wherein

**$R_1$** represents a **-R-L-** group, wherein

**L** is either a single bond, a ($C_1$-$C_3$)alkanediyl group, a -O-($C_1$-$C_3$)alkanediyl group, said ($C_1$-$C_3$)alkanediyl group being optionally substituted by a group selected from a hydroxy group and a ($C_1$-$C_3$)alkoxy group or a -$SO_2$- group, and
**R** represents a ($C_3$-$C_8$)heterocycloalkyl group, optionally substituted by one or two groups selected from a hydroxy group, a halogen atom, an oxo group, a ($C_1$-$C_4$)alkyl group and a ($C_1$-$C_3$)alkoxy group, and

**R2** represents a hydrogen atom or a ($C_1$-$C_3$)alkyl group.

**16.** A compound of formula (I) or any of its pharmaceutically acceptable salt, as defined in claim 10, wherein

**$R_1$** represents a **-R-L-** group, wherein

**L** is either a single bond, a ($C_1$-$C_3$)alkanediyl group, a -O-($C_1$-$C_3$)alkanediyl group, said ($C_1$-$C_3$)alkanediyl group being optionally substituted by a group selected from a hydroxy group and a ($C_1$-$C_3$)alkoxy group or a -$SO_2$- group, and
**R** represents a ($C_5$-$C_6$)heteroaryl group, such as a pyridyl, a pyrazinyl, a pyrimidyl, a pyrazolyl or an imidazolyl group, optionally substituted by one to three groups selected from a ($C_1$ $C_4$)alkyl group, a hydroxy group,

a halogen atom and a ($C_1$-$C_3$)alkoxy group, and

**R2** represents a hydrogen atom or a ($C_1$-$C_3$)alkyl group.

**17.** A compound of formula (I) as defined in claim 10, or any of its pharmaceutically acceptable salt, selected from *N*4-Benzyl-7-isopropyl-*N2*-(2-methoxyethyl)imidazo[2,1-

(1) *f*][1,2,4]triazine-2,4-diamine *N4*-Benzyl-7-isopropyl-*N2*-(2-phenoxyethyl)imidazo[2,1-
(2) *f*][1,2,4]triazine-2,4-diamine *N4*-Benzyl-7-isopropyl-*N2*-(2-methylsulfanylethyl)imidazo[2,1-
(3) *f*][1,2,4]triazine-2,4-diamine *N4*-Benzyl-7-isopropyl-*N2*-(3-methoxypropyl)imidazo[2,1-
(4) *f*][1,2,4]triazine-2,4-diamine *N4*-Benzyl-*N2*-[3-(dimethylamino)propyl]-7-isopropyl-imidazo[2,1-
(5) *f*][1,2,4]triazine-2,4-diamine *N4*-Benzyl-*N2*-(1-ethylpropyl)-7-isopropyl-imidazo[2,1-*f*][1,2,4]triazine-
(7) 2,4-diamine *N2*-(1-ethylpropyl)-7-isopropyl-*N4*-(2-pyridylmethyl)imidazo[2,1-
(8) *f*][1,2,4]triazine-2,4-diamine *N2*-(1-ethylpropyl)-7-isopropyl-*N4*-(3-pyridylmethyl)imidazo[2,1-
(9) *f*][1,2,4]triazine-2,4-diamine *N2*-(1-ethylpropyl)-7-isopropyl-*N4*-(4-pyridylmethyl)imidazo[2,1-
(10) *f*][1,2,4]triazine-2,4-diamine *N2*-(1-ethylpropyl)-7-isopropyl-*N4*-phenyl-imidazo[2,1-*f*][1,2,4]triazine-
(11) 2,4-diamine *N2*-(1-ethylpropyl)-7-isopropyl-*N4*-(2-phenylethyl)imidazo[2,1-
(12) *f*][1,2,4]triazine-2,4-diamine *N2*-(1-ethylpropyl)-7-isopropyl-*N4*-[2-(2-pyridyl)ethyl]imidazo[2,1-
(13) *f*][1,2,4]triazine-2,4-diamine *N2*-(1-ethylpropyl)-7-isopropyl-*N4*-[2-(3-pyridyl)ethyl]imidazo[2,1-
(14) *f*][1,2,4]triazine-2,4-diamine *N2*-(1-ethylpropyl)-7-isopropyl-*N4*-[2-(4-pyridyl)ethyl]imidazo[2,1-
(15) *f*][1,2,4]triazine-2,4-diamine 7-cyclopropyl-*N2*-(1-ethylpropyl)-*N4*-[2-(3-pyridyl)ethyl]imidazo[2,1-
(16) *f*][1,2,4]triazine-2,4-diamine 7-cyclopropyl-*N2*-(1-ethylpropyl)-*N4*-[2-(4-pyridyl)ethyl]imidazo[2,1-
(17) *f*][1,2,4]triazine-2,4-diamine 2-[[4-(Benzylamino)-7-isopropyl-imidazo[2,1-*f*][1,2,4]triazin-2-
(18) yl]amino]-2-methyl-propan-1-ol *N4*-Benzyl-7-isopropyl-*N2*-[(*3R*)-tetrahydrofuran-3-yl]imidazo[2,1-
(19) *f*][1,2,4]triazine-2,4-diamine *N4*-[(4-Chlorophenyl)methyl]-7-isopropyl-*N2*-[(*3R*)-tetrahydrofuran-3-
(20) yl]imidazo[2,1-*f*][1,2,4]triazine-2,4-diamine 7-Isopropyl-*N4*-(p-tolylmethyl)-*N2*-[(*3R*)-tetrahydrofuran-3-
(21) yl]imidazo[2,1-*f*][1,2,4]triazine-2,4-diamine 7-Isopropyl-*N4*-[(4-methoxyphenyl)methyl]-*N2*-[(*3R*)-tetrahydrofuran-3-
(22) yl]imidazo[2,1-*f*][1,2,4]triazine-2,4-diamine *N4*-[(3,4-Dichlorophenyl)methyl]-7-isopropyl-*N2*-[(*3R*)-tetrahydrofuran-
(23) 3-yl]imidazo[2,1-*f*][1,2,4]triazine-2,4-diamine *N4*-[(4-Fluorophenyl)methyl]-7-isopropyl-*N2*-[(*3R*)-tetrahydrofuran-3-
(24) yl]imidazo[2,1-*f*][1,2,4]triazine-2,4-diamine 7-Isopropyl-*N2*-[(*3R*)-tetrahydrofuran-3-yl]-*N4*-[[4-
(25) (trifluoromethyl)phenyl] methyl]imidazo[2,1-*f*][1,2,4]triazine-2,4-diamine 7-Isopropyl-*N4*-(2-pyridylmethyl)-*N2*-[(*3R*)-tetrahydrofuran-3-
(26) yl]imidazo[2,1-*f*][1,2,4]triazine-2,4-diamine 7-Isopropyl-*N4*-(3-pyridylmethyl)-*N2*-[(*3R*)-tetrahydrofuran-3-
(27) yl]imidazo[2,1-*f*][1,2,4]triazine-2,4-diamine 7-Isopropyl-*N4*-(4-pyridylmethyl)-*N2*-[(*3R*)-tetrahydrofuran-3-
(28) yl]imidazo[2,1-*f*][1,2,4]triazine-2,4-diamine 7-Isopropyl-*N4*-(pyrazin-2-ylmethyl)-*N2*-[(*3R*)-tetrahydrofuran-3-
(29) yl]imidazo[2,1-*f*][1,2,4]triazine-2,4-diamine 7-Isopropyl-*N4*-[(*1R*)-1-phenylethyl]-*N2*-[(*3R*)-tetrahydrofuran-3-
(30) yl]imidazo[2,1-*f*][1,2,4]triazine-2,4-diamine 7-Isopropyl-*N4*-[(*1S*)-1-phenylethyl]-*N2*-[(*3R*)-tetrahydrofuran-3-
(31) yl]imidazo[2,1-*f*][1,2,4]triazine-2,4-diamine *N4*-Benzyl-7-isopropyl-*N4*-methyl-*N2*-[(*3R*)-tetrahydrofuran-3-
(32) yl]imidazo[2,1-*f*][1,2,4]triazine-2,4-diamine *N4*-Benzyl-*N2*-[(*3R*)-tetrahydrofuran-3-yl]imidazo[2,1-*f*][1,2,4]triazine-
(33) 2,4-diamine *N4*-Benzyl-7-bromo-*N2*-[(*3R*)-tetrahydrofuran-3-yl]imidazo[2,1-
(34) *f*][1,2,4]triazine-2,4-diamine *N4*-Benzyl-7-methyl-*N2*-[(*3R*)-tetrahydrofuran-3-yl]imidazo[2,1-
(35) *f*][1,2,4]triazine-2,4-diamine *N4*-Benzyl-7-ethyl-*N2*-[(*3R*)-tetrahydrofuran-3-yl]imidazo[2,1-
(36) *f*][1,2,4]triazine-2,4-diamine *N4*-Benzyl-7-cyclopropyl-*N2*-[(*3R*)-tetrahydrofuran-3-yl]imidazo[2,1-
(37) *f*][1,2,4]triazine-2,4-diamine *N4*-Benzyl-7-cyclopentyl-*N2*-[(*3R*)-tetrahydrofuran-3-yl]imidazo[2,1-
(38) *f*][1,2,4]triazine-2,4-diamine *N4*-Benzyl-7-cyclohexyl-*N2*-[(*3R*)-tetrahydrofuran-3-yl]imidazo[2,1-
(39) *f*][1,2,4]triazine-2,4-diamine *N4*-Benzyl-7-phenyl-*N2*-[(*3R*)-tetrahydrofuran-3-yl]imidazo[2,1-
(40) *f*][1,2,4]triazine-2,4-diamine N4-Benzyl-7-isopropyl-*N2*-[(*3R*)-tetrahydropyran-3-yl]imidazo[2,1-
(41) *f*][1,2,4]triazine-2,4-diamine 7-Isopropyl-*N4*-(3-pyridylmethyl)-*N2*-[(*3R*)-tetrahydropyran-3-
(42) yl]imidazo[2,1-*f*][1,2,4]triazine-2,4-diamine 7-Isopropyl-*N4*-(4-pyridylmethyl)-*N2*-[(*3R*)-tetrahydropyran-3-
(43) yl]imidazo[2,1-*f*][1,2,4]triazine-2,4-diamine *N4*-Benzyl-7-isorpopyl-*N2*-tetrahydropyran-4-yl-imidazo[2,1-
(44) *f*][1,2,4]diamine *N4*-[(4-Fluorophenyl)methyl]-7-isopropyl-*N2*-tetrahydropyran-4-yl-

(45) imidazo[2,1-*f*][1,2,4]triazine-2,4-diamine 7-Isopropyl-*N2*-tetrahydropyran-4-yl-*N4*-[[4-
(46) (trifluoromethyl)phenyl] methyl]imidazo[2,1-*f*][1,2,4]triazine-2,4-diamine 7-Isopropyl-*N4*-[(*1R*)-1-phenylethyl]-*N2*-tetrahydropyran-4-yl-
(47) imidazo[2,1-*f*][1,2,4]triazine-2,4-diamine 7-Isopropyl-*N4*-[(*1S*)-1-phenylethyl]-*N2*-tetrahydropyran-4-yl-
(48) imidazo[2,1-*f*][1,2,4]triazine-2,4-diamine *N4*-Benzyl-7-isopropyl-*N4*-methyl-*N2*-tetrahydropyran-4-yl-imidazo[2,1-
(49) *f*][1,2,4]triazine-2,4-diamine 7-Isopropyl-*N4*-(2-pyridylmethyl)-*N2*-tetrahydropyran-4-yl-imidazo[2,1-
(50) *f*][1,2,4]triazine-2,4-diamine 7-Isopropyl-*N4*-(3-pyridylmethyl)-*N2*-tetrahydropyran-4-yl-imidazo[2,1-
(51) *f*][1,2,4]triazine-2,4-diamine 7-Isopropyl-*N4*-(4-pyridylmethyl)-*N2*-tetrahydropyran-4-yl-imidazo[2,1-
(52) *f*][1,2,4]triazine-2,4-diamine 7-Isopropyl-*N4*-(pyrazin-2-ylmethyl)-*N2*-tetrahydropyran-4-yl-
(53) imidazo[2,1-*f*][1,2,4]triazine-2,4-diamine *N4*-Benzyl-7-methyl-*N2*-tetrahydropyran-4-yl-imidazo[2,1-
(54) *f*][1,2,4]triazine-2,4-diamine *N4*-Benzyl-7-ethyl-*N2*-tetrahydropyran-4-yl-imidazo[2,1-
(55) *f*][1,2,4]triazine-2,4-diamine *N4*-Benzyl-7-cyclopropyl-*N2*-tetrahydropyran-4-yl-imidazo[2,1-
(56) *f*][1,2,4]triazine-2,4-diamine *N4*-Benzyl-7-cyclohexyl-*N2*-tetrahydropyran-4-yl-imidazo[2,1-
(57) *f*][1,2,4]triazine-2,4-diamine *N4*-Benzyl-7-isopropyl-*N2*-(oxepan-4-yl)imidazo[2,1-*f*][1,2,4]triazine-
(58) 2,4-diamine *N4*-Benzyl-*N2*-cyclohexyl-7-isopropyl-imidazo[2,1-*f*][1,2,4]triazine-2,4-
(59) diamine *N4*-Benzyl-*N2*-(4,4-difluorocyclohexyl)-7-isopropyl-imidazo[2,1-
(60) *f*][1,2,4]triazine-2,4-diamine *N4*-Benzyl-7-isopropyl-*N2*-(1-methyl-4-piperidyl)imidazo[2,1-
(61) *f*][1,2,4]triazine-2,4-diamine (*1S*)-2-[[4-(Benzylamino)-7-isopropyl-imidazo[2,1-*f*][1,2,4]triazin-2-
(62) yl]amino]cyclohexanol 4-[[4-(Benzylamino)-7-isopropyl-imidazo[2,1-*f*][1,2,4]triazin-2-
(63) yl]amino]cyclohexanol *N4*-Benzyl-7-isopropyl-*N2*-(4-methoxycyclohexyl)imidazo[2,1-
(64) *f*][1,2,4]triazine-2,4-diamine (*1S*)-2-[[4-(Benzylamino)-7-isopropyl-imidazo[2,1-*f*][1,2,4]triazin-2-
(65) yl]amino]cyclopentanol (*1S*)-2-[[7-Isopropyl-4-(3-pyridylmethylamino)imidazo[2,1-
(66) *f*][1,2,4]triazin-2-yl]amino]cyclopentanol *N4*-Benzyl-7-isopropyl-*N2*-[(*2S*)-2-methoxycyclopentyl]imidazo[2,1-
(67) *f*][1,2,4]triazine-2,4-diamine 7-Isopropyl-*N2*-[(*2S*)-2-methoxycyclopentyl]-*N4*-(3-
(68) pyridylmethyl)imidazo[2,1-*f*][1,2,4]triazine-2,4-diamine *N4*-Benzyl-7-isopropyl-*N2*-methyl-*N2*-[(*3R*)-tetrahydrofuran-3-
(69) yl]imidazo[2,1-*f*][1,2,4]triazine-2,4-diamine *N4*-Benzyl-7-isopropyl-*N2*-methyl-*N2*-tetrahydropyran-4-yl-imidazo[2,1-
(70) *f*][1,2,4]triazine-2,4-diamine 3-Isopropyl-*N6*-methyl-*N8*-(4-pyridylmethyl)-*N6*-tetrahydropyran-4-yl-
(71) [1,2,4]triazolo[4,3-*b*]pyridazine-6,8-diamine *N*-Benzyl-7-isopropyl-2-oxazolidin-3-yl-imidazo[2,1-*f*][1,2,4]triazin-4-
(72) amine
(73) *N*-Benzyl-7-isopropyl-2-morpholino-imidazo[2,1-*f*][1,2,4]triazin-4-amine 7-Isopropyl-2-morpholino-*N*-(3-pyridylmethyl)imidazo[2,1-
(74) *f*][1,2,4]triazin-4-amine 7-Isopropyl-2-morpholino-*N*-(4-pyridylmethyl)imidazo[2,1-
(75) *f*][1,2,4]triazin-4-amine 1-[4-(Benzylamino)-7-isopropyl-imidazo[2,1-*f*][1,2,4]triazin-2-
(76) yl]pyrrolidin-3-ol 1-[7-Isopropyl-4-(3-pyridylmethylamino)imidazo[2,1-*f*][1,2,4]triazin-2-
(77) yl]pyrrolidin-3-ol *N*-Benzyl-7-isopropyl-2-(2-oxa-6-azaspiro[3.3]heptan-6-yl)imidazo[2,1-
(78) *f*][1,2,4]triazin-4-amine *N*-Benzyl-7-isopropyl-2-(3-methoxypyrrolidin-1-yl)imidazo[2,1-
(79) *f*][1,2,4]triazin-4-amine 7-Isopropyl-2-(3-methoxypyrrolidin-1-yl)-*N*-(3-
(80) pyridylmethyl)imidazo[2,1-*f*][1,2,4]triazin-4-amine *N*-Benzyl-7-isopropyl-2-(4-methoxy-1-piperidyl)imidazo[2,1-
(81) *f*][1,2,4]triazin-4-amine 7-Isopropyl-2-(4-methoxy-1-piperidyl)-*N*-(3-pyridylmethyl)imidazo[2,1-
(82) *f*][1,2,4]triazin-4-amine *N*-Benzyl-7-isopropyl-2-thiomorpholino-imidazo[2,1-*f*][1,2,4]triazin-4-
(83) amine 7-Isopropyl-*N*-(3-pyridylmethyl)-2-thiomorpholino-imidazo[2,1-
(84) *f*][1,2,4]triazin-4-amine 1-[4-(Benzylamino)-7-isopropyl-imidazo[2,1-*f*][1,2,4]triazin-2-
(85) yl]piperidin-4-ol 1-[7-Isopropyl-4-(3-pyridylmethylamino)imidazo[2,1-*f*][1,2,4]triazin-2-
(86) yl]piperidin-4-ol 2-[1-[4-(Benzylamino)-7-isopropyl-imidazo[2,1-*f*][1,2,4]triazin-2-yl]-2-
(87) piperidyl]ethanol *N4*-Benzyl-7-isopropyl-*N2*-(tetrahydrofuran-3-ylmethyl)imidazo[2,1-
(88) *f*][1,2,4]triazine-2,4-diamine *N4*-Benzyl-7-isopropyl-*N2*-(tetrahydrofuran-2-ylmethyl)imidazo[2,1-
(89) *f*][1,2,4]triazine-2,4-diamine *N4*-Benzyl-7-isopropyl-*N2*-(tetrahydropyran-4-ylmethyl)imidazo[2,1-
(90) *f*][1,2,4]triazine-2,4-amine *N4*-Benzyl-7-isopropyl-*N2*-(tetrahydropyran-3-ylmethyl)imidazo[2,1-
(91) *f*][1,2,4]triazine-2,4-amine 7-Isopropyl-*N4*-(2-pyridylmethyl)-*N2*-(tetrahydropyran-3-
(92) ylmethyl)imidazo[2,1-*f*][1,2,4]triazine-2,4-diamine 7-Isopropyl-*N4*-(3-pyridylmethyl)-*N2*-(tetrahydropyran-3-
(93) ylmethyl)imidazo[2,1-*f*][1,2,4]triazine-2,4-diamine 7-Isopropyl-*N4*-(4-pyridylmethyl)-*N2*-(tetrahydropyran-3-
(94) ylmethyl)imidazo[2,1-*f*][1,2,4]triazine-2,4-diamine *N4*-Benzyl-7-isopropyl-*N2*-[[(3*S*)-tetrahydropyran-3-
(95) yl]methyl]imidazo[2,1-*f*][1,2,4]triazine-2,4-diamine 7-Isopropyl-*N4*-(3-pyridylmethyl)-*N2*-[[(3*S*)-tetrahydropyran-3-
(96) yl]methyl]imidazo[2,1-*f*][1,2,4]triazine-2,4-diamine *N4*-Benzyl-7-isopropyl-*N2*-[[(3*R*)-tetrahydropyran-3-
(97) yl]methyl]imidazo[2,1-*f*][1,2,4]triazine-2,4-diamine 7-Isopropyl-*N4*-(3-pyridylmethyl)-*N2*-[[(3*R*)-tetrahydropyran-3-
(98) yl]methyl]imidazo[2,1-*f*][1,2,4]triazine-2,4-diamine *N4*-Benzyl-7-isopropyl-*N2*-(tetrahydropyran-2-ylmethyl)imidazo[2,1-
(99) *f*][1,2,4]triazine-2,4-amine 7-Isopropyl-*N4*-(3-pyridylmethyl)-*N2*-(tetrahydropyran-2-
(100) ylmethyl)imidazo[2,1-*f*][1,2,4]triazine-2,4-diamine 7-Isopropyl-*N4*-(4-pyridylmethyl)-*N2*-(tetrahydropyran-2-
(101) ylmethyl)imidazo[2,1-*f*][1,2,4]triazine-2,4-diamine *N4*-Benzyl-7-isopropyl-*N2*-(1-tetrahydropyran-3-ylethyl)imidazo[2,1-
(102) *f*][1,2,4]triazine-2,4-diamine *N4*-Benzyl-7-isopropyl-*N2*-methyl-*N2*-(tetrahydropyran-3-
(103) ylmethyl)imidazo[2,1-*f*][1,2,4]triazine-2,4-diamine *N4*-Benzyl-7-isopropyl-*N2*-[(1-methyl-4-piperidyl)methyl]imidazo[2,1-
(104) *f*][1,2,4]triazine-2,4-diamine *N4*-Benzyl-*N2*-(cyclohexylmethyl)-7-isopropyl-imidazo[2,1-
(105) *f*][1,2,4]triazine-2,4-diamine *N4*-Benzyl-7-isopropyl-*N2*-[2-(1-methylpyrrolidin-2-
(106) yl)ethyl]imidazo[2,1-*f*][1,2,4]triazine-2,4-diamine *N4*-Benzyl-7-isopropyl-*N2*-(2-methylpyrazol-3-yl)imidazo[2,1-
(107) *f*][1,2,4]triazine-2,4-diamine *N4*-Benzyl-7-isopropyl-*N4*-methyl-*N2*-(2-methylpyrazol-3-
(108) yl)imidazo[2,1-*f*][1,2,4]triazine-2,4-diamine *N4*-Benzyl-7-isopropyl-*N2*-(1-methylimidazol-2-yl)imidazo[2,1-
(109) *f*][1,2,4]triazine-2,4-diamine *N4*-Benzyl-7-isopropyl-*N2*-(1-methylpyrazol-4-yl)imidazo[2,1-
(110) *f*][1,2,4]triazine-2,4-diamine *N4*-Benzyl-7-isopropyl-*N2*-(1-methylpyrazol-3-yl)imidazo[2,1-
(111) *f*][1,2,4]triazine-2,4-diamine *N4*-Benzyl-*N2*-(1,3-dimethylpyrazol-4-yl)-7-isopropyl-imidazo[2,1-
(112) *f*][1,2,4]triazine-2,4-diamine *N4*-Benzyl-*N2*-(2,5-dimethylpyrazol-3-yl)-7-isopropyl-imidazo[2,1-
(113) *f*][1,2,4]triazine-2,4-diamine *N4*-Benzyl-7-isopropyl-*N2*-phenyl-imidazo[2,1-*f*][1,2,4]triazine-2,4-
(114) diamine *N4*-Benzyl-7-isopropyl-*N2*-[(1-methylpyrazol-4-yl)methyl]imidazo[2,1-
(115) *f*][1,2,4]triazine-2,4-diamine *N4*-Benzyl-7-isopropyl-*N2*-[(1-methylimidazol-2-yl)methyl]imidazo[2,1-
(116) *f*][1,2,4]triazine-2,4-diamine *N4*-Benzyl-7-isopropyl-*N2*-[(2-methylpyrazol-3-yl)methyl]imidazo[2,1-
(117) *f*][1,2,4]triazine-2,4-diamine *N4*-Benzyl-7-isopropyl-*N2*-[(1-methylpyrazol-3-yl)methyl]imidazo[2,1-
(118) *f*][1,2,4]triazine-2,4-diamine *N4*-Benzyl-7-isopropyl-*N2*-[(3-methylimidazol-4-yl)methyl]imidazo[2,1-
(119) *f*][1,2,4]triazine-2,4-diamine *N4*-Benzyl-7-isopropyl-*N2*-[(1-methylimidazol-4-yl)methyl]imidazo[2,1-
(120) *f*][1,2,4]triazine-2,4-diamine
(121) *N2*,*N4*-Dibenzyl-7-isopropyl-imidazo[2,1-*f*][1,2,4]triazine-2,4-diamine *N4*-Benzyl-*N2*-[(4-Fluorophenyl)methyl]-7-isopropyl-imidazo[2,1-
(122) *f*][1,2,4]triazine-2,4-diamine *N4*-Benzyl-7-isopropyl-*N2*-(2-pyridylmethyl)imidazo[2,1-
(123) *f*][1,2,4]triazine-2,4-diamine *N4*-Benzyl-7-isopropyl-*N2*-(3-pyridylmethyl)imidazo[2,1-
(124) *f*][1,2,4]triazine-2,4-diamine *N4*-Benzyl-7-isopropyl-*N2*-(4-pyridylmethyl)imidazo[2,1-
(125) *f*][1,2,4]triazine-2,4-diamine *N4*-Benzyl-7-isopropyl-*N2*-[(5-methylpyrazin-2-yl)methyl]imidazo[2,1-
(126) *f*][1,2,4]triazine-2,4-diamine *N4*-Benzyl-*N2*-[(4,6-dimethylpyrimidin-2-yl)methyl]-7-isopropyl-
(127) imidazo[2,1-*f*][1,2,4]triazine-2,4-diamine *N*-[4-(benzylamino)-7-isopropyl-imidazo[2,1-*f*][1,2,4]triazin-2-yl]-2-
(128) methyl-pyrazole-3-sulfonamide *N*-[4-(benzylamino)-7-isopropyl-imidazo[2,1-*f*][1,2,4]triazin-2-yl]-1,3-
(129) dimethyl-pyrazole-4-sulfonamide *N*-[4-(benzylamino)-7-isopropyl-imidazo[2,1-*f*][1,2,4]triazin-2-yl]-2,5-
(130) dimethyl-pyrazole-3-sulfonamide *N*-[4-(Benzylamino)-7-isopropyl-imidazo[2,1-*f*][1,2,4]triazin-2-yl]-2,4-
(131) dimethyl-pyrazole-3-sulfonamide *N4*-benzyl-7-isopropyl-*N2*-[(3*S*)-tetrahydrofuran-3-yl]imidazo[2,1-
(132) *f*][1,2,4]triazine-2,4-diamine *N4*-benzyl-*N2*-(2,4-dimethylpyrazol-3-yl)-7-isopropyl-imidazo[2,1-
(133) *f*][1,2,4]triazine-2,4-diamine

and in particular selected from: (19), (20), (22), (23), (25), (27), (28), (37), (39), (41), (42), (43), (44), (45), (46), (48), (49), (51), (52), (56), (57), (58), (63), (64), (74), (91), (93), (94), (95), (96), (97), (98), (100), (101), (102), (103), (107), (110), (112), (113), (115), (117), (119), (120), (121), (122), (123), (124), (125), (133) and their pharmaceutically acceptable salts, in particular from compounds (25), (28), (42), (46), (48), (51), (52), (57), (58), (63), (93), (94), (96), (97), (102), (107), (113), (117), (133) and their pharmaceutically acceptable salts, and even more partiucarly from (25), (93), (96), (107), (113), (133) and their pharmaceutically acceptable salts.

**18.** Synthesis process for manufacturing a compound of formula (I) as defined in anyone of claim 10 to 16 or any of its pharmaceutically acceptable salts, or at least any of compounds (1) to (5) and (7) to (133) as defined in claim 17, comprising at least a step of reacting a compound of formula (II)

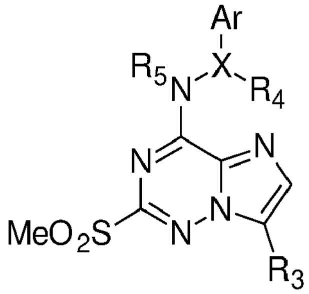

(II)

wherein **Ar, X, $R_3$, $R_4$** and **$R_5$** are as defined in claim 10 with an amine of formula (V) $R_{1_{NH}}R_2$ (V) wherein **$R_1$** and **$R_2$** are as defined in anyone of claims 10 to 16, for example in a molar ratio ranging from 1 to 20, in particular of 10, with respect to the compound of formula (II), in an aprotic solvent such as Et3N, THF 2-MeTHF, dioxane, cineol or NMP or a mixture thereof or without solvent.

**19.** Synthesis process for manufacturing a compound of formula (I) as defined in anyone of claim 10 to 16 or any of its pharmaceutically acceptable salts, or at least any of compounds (1) to (5) and (7) to (133) as defined in claim 17, comprising at least a step of reacting a compound of formula (VIII)

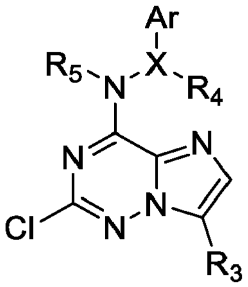

(VIII)

wherein **X, $R_3$, $R_4$, $R_5$** and **Ar** are as defined in claim 10, with an amine of formula (V)

$R_{1_{NH}}R_2$ (V)

wherein **$R_1$** and **$R_2$** are as defined in anyone of claims 8 to 14, for example in a molar ratio ranging from 1 to 20, in particular of 15, with respect to the compound of formula (VIII), in an aprotic solvent such as Et3N, THF, dioxane, cineol or NMP or a mixture thereof, or without solvent.

**20.** Synthesis process for manufacturing a compound of formula (I) as defined in anyone of claim 10 to 16 or any of its pharmaceutically acceptable salts, or at least any of compounds (1) to (5) and (7) to (133) as defined in claim 17, comprising at least a step of reacting a compound of formula (XVI)

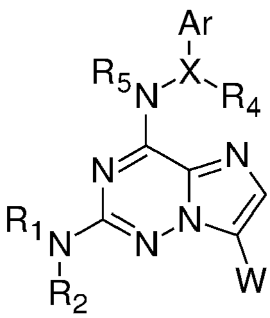

(XVI), wherein **$R_1$, $R_2$, $R_4$, $R_5$, X,** and **Ar** are as defined in claim 10 and **W** is a bromine or a iodine atom, in palladium-catalyzed cross-coupling conditions, with a compound of formula R3-ZnHal compound, wherein $R_3$ is as defined in claim 10 and Hal is a chlorine or bromine atom, or with a compound of formula R3-BF3K followed by a hydrogenation reaction step.

**21.** Compound of formula (XIV), in particular as in intermediate compound

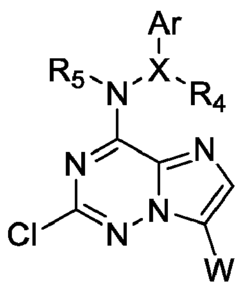

(XIV)

wherein

**Ar, X** and **$R_4$** are as defined in claim 10 and **$R_5$** represents a ($C_1$-$C_4$)alkyl group and **W** represents a iodine or bromine atom, or alternatively
**X, W, $R_4$** and **$R_5$** are as defined in claim 10 and **Ar** represents

- a 6-membered heteroaryl group comprising one to three nitrogen atoms, optionally substituted by one to three groups selected from a ($C_1$-$C_3$)alkyl group, a ($C_1$-$C_3$)alkoxy group and a halogen atom, and in particular a pyridyl, a pyrimidinyl or a pyrazinyl group, more particularly a pyridyl or a pyrazinyl group, optionally substituted by one to three groups selected from a ($C_1$-$C_3$)alkyl group, a ($C_1$-$C_3$)alkoxy group and a halogen atom.

**22.** Compound of formula (VIII), in particular as in intermediate compound

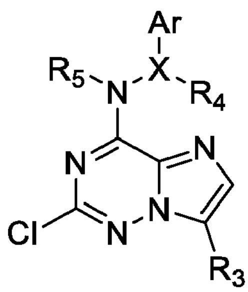

(VIII)

wherein
**Ar, X, $R_3$, $R_4$** and **$R_5$** are as defined in claim 10.

**23.** Compound of formula (XVI), in particular as in intermediate compound

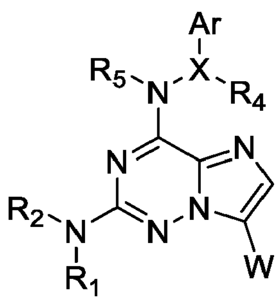

(XVI)

wherein

**Ar, X, $R_4$, $R_5$** are as defined in claim 10 and **W** is a bromine or a iodine atom,
**$R_1$** and **$R_2$** are as defined in anyone of claim 10 to 15.

**24.** A compound of formula (I) as defined in anyone of claim 10 to 16 or any of its pharmaceutically acceptable salts, or at least any of compounds (1) to (5) and (7) to (133) as defined in claim 17 or any of its pharmaceutically acceptable salts, for use as a medicament.

**25.** A pharmaceutical composition comprising at least one compound of formula (I) as defined in anyone of claim 10 to 16 or or any of its pharmaceutically acceptable salt or at least any of compounds (1) to (5) and (7) to (133) as defined in claim 17.

**26.** A pharmaceutical combination of a compound of formula (I) as defined in anyone of claims 10 to 16 or any of its pharmaceutically acceptable salt or at least any of compounds (1) to (5) and (7) to (133) as defined in claim 17, with an ABC modulator, including a CFTR modulator, or a combination of CFTR modulators, or a pharmaceutically acceptable salt thereof.

**27.** A pharmaceutical combination according to claim 26, wherein the ABC modulator is a CFTR modulator selected from GLPG 2222, GLPG 1837, GLPG 2451, their combinations such as GLPG 2222 and GLPG 1837, GLPG 2222 and GLPG 2451 and GLPG 2222, GLPG 1837 and GLPG 2451, lumacaftor, galicaftor, navocaftor, dirocaftor, nesolicaftor, posenacaftor, ivacaftor, tezacaftor, elexacaftor, and their combinations, in particular is selected from ivacaftor, tezacaftor and elexacaftor and more particularly is a mixture of ivacaftor, tezacaftor and elexacaftor.

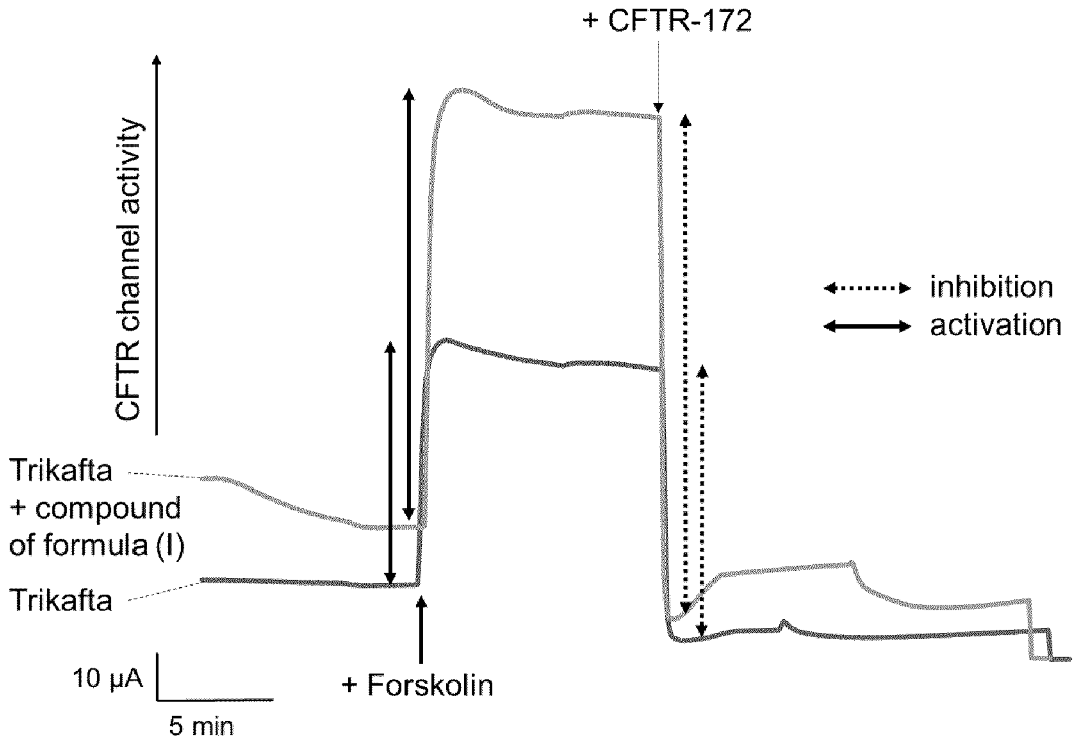
+ CFTR-172
CFTR channel activity
inhibition
activation
Trikafta
+ compound
of formula (I)
Trikafta
10 µA
5 min
+ Forskolin

FIG 1.

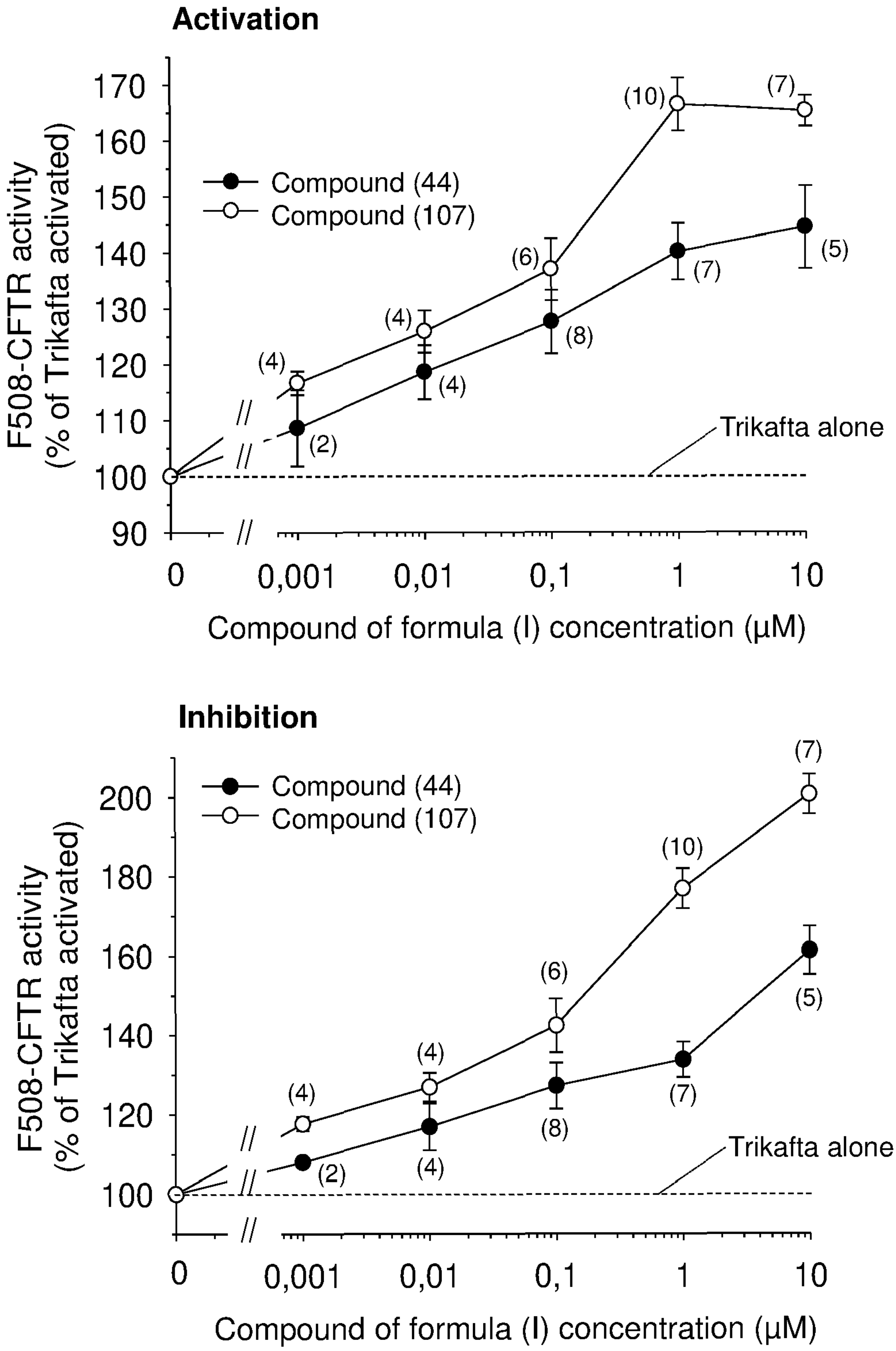
Activation
F508-CFTR activity
(% of Trikafta activated)
170
160
150
140
130
120
110
100
90
Compound (44)
Compound (107)
(10)
(7)
(6)
(4)
(4)
(2)
(4)
(8)
(7)
(5)
Trikafta alone
0
0,001
0,01
0,1
1
10
Compound of formula (I) concentration (μM)
Inhibition
F508-CFTR activity
(% of Trikafta activated)
200
180
160
140
120
100
Compound (44)
Compound (107)
(7)
(10)
(6)
(4)
(4)
(2)
(4)
(8)
(7)
(5)
Trikafta alone
0
0,001
0,01
0,1
1
10
Compound of formula (I) concentration (μM)

FIG 2.

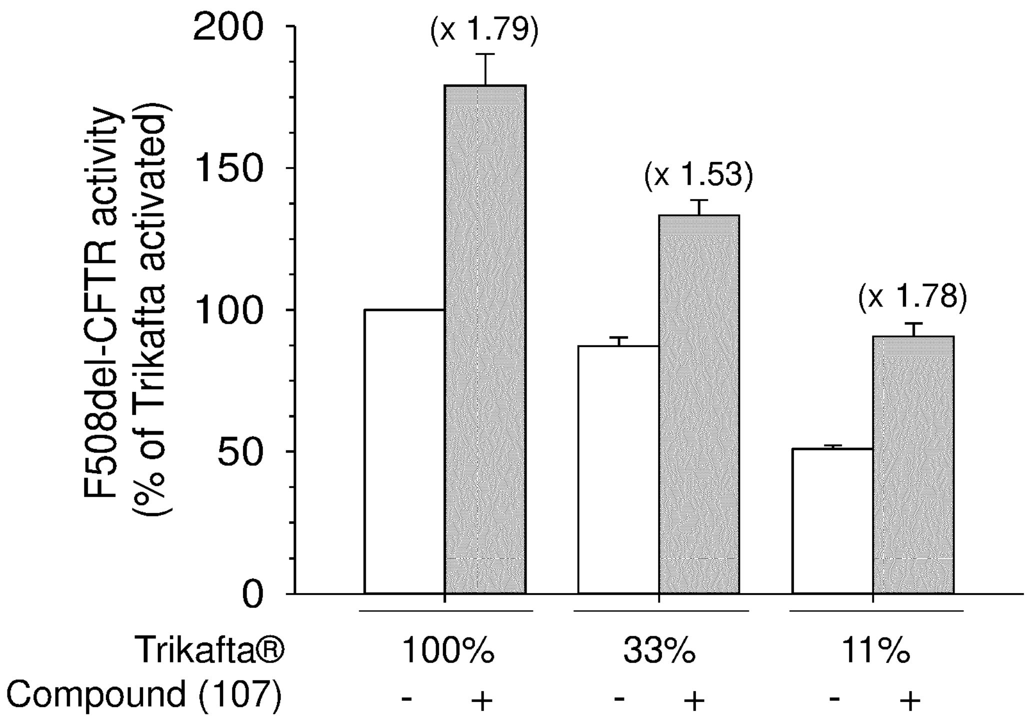

200
150
100
50
0
F508del-CFTR activity
(% of Trikafta activated)
(x 1.79)
(x 1.53)
(x 1.78)
Trikafta®
Compound (107)
100%
33%
11%
- +
- +
- +


FIG 3.

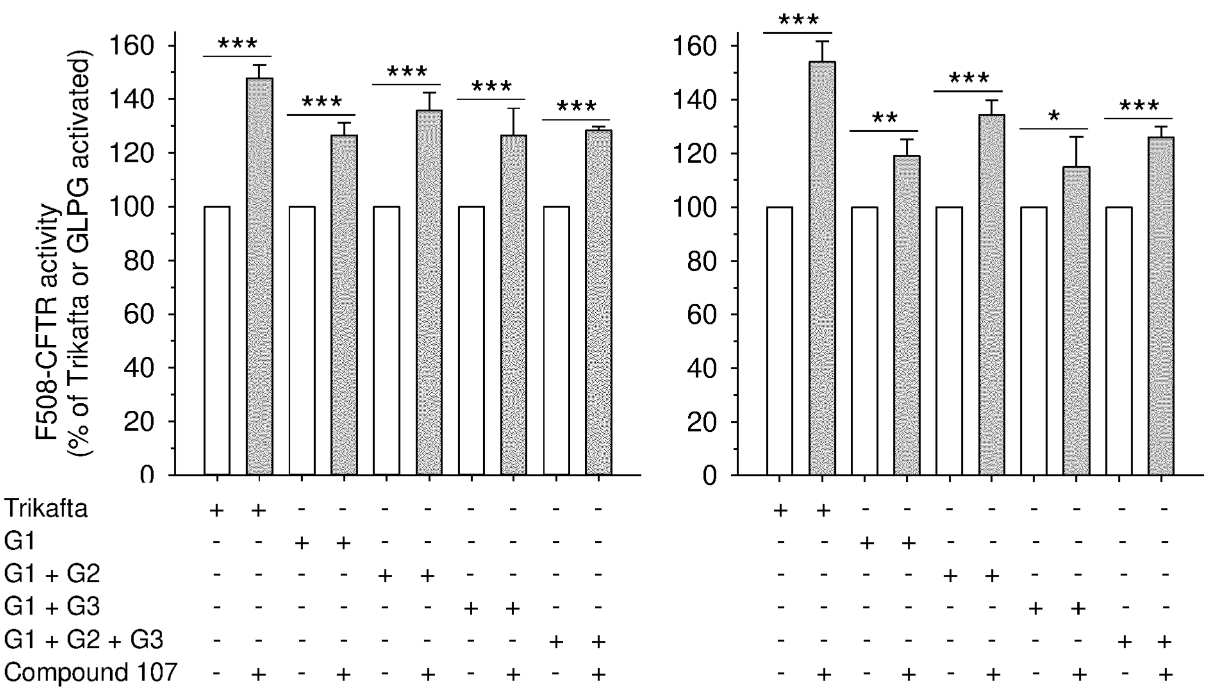

F508-CFTR activity
(% of Trikafta or GLPG activated)
160
140
120
100
80
60
40
20
0
***
**
*
Trikafta + + - - - - - - - -
G1 - - + + - - - - - -
G1 + G2 - - - - + + - - - -
G1 + G3 - - - - - - + + - -
G1 + G2 + G3 - - - - - - - - + +
Compound 107 - + - + - + - + - +


FIG 4.

Europäisches Patentamt
European Patent Office
Office européen des brevets

# EUROPEAN SEARCH REPORT

Application Number
EP 22 30 5621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOVERING FRANK ET AL: "Imidazotriazines: Spleen Tyrosine Kinase (Syk) Inhibitors Identified by Free-Energy Perturbation (FEP)", CHEMMEDCHEM COMMUNICATIONS, vol. 11, no. 2, 18 September 2015 (2015-09-18), pages 217-233, XP055877038, DE ISSN: 1860-7179, DOI: 10.1002/cmdc.201500333 * intermediate in the formatiom of compound 18; page 220; table 1 * | 22 | INV. A61K31/53 A61K31/4745 A61K31/5025 A61K31/519 A61K31/52 A61K31/5377 A61K45/06 A61P43/00 A61K31/404 A61K31/4439 A61K31/47 C07D403/14 |
| X | WO 2017/070135 A1 (BRISTOL MYERS SQUIBB CO [US]) 27 April 2017 (2017-04-27) * page 40, lines 5-19 * | 21,22 | |
| X,D | POPOWYCZ FLORENCE ET AL: "Pyrazolo[1,5-a]-1,3,5-triazine as a Purine Bioisostere: Access to Potent Cyclin-Dependent Kinase Inhibitor (R)-Roscovitine Analogue", JOURNAL OF MEDICINAL CHEMISTRY, AMERICAN CHEMICAL SOCIETY, US, vol. 52, no. 3, 1 January 2009 (2009-01-01), pages 655-663, XP002548292, ISSN: 0022-2623, DOI: 10.1021/JM801340Z [retrieved on 2009-01-07] | 1-27 | TECHNICAL FIELDS SEARCHED (IPC) |
| Y | * whole document, in particular scheme 1, compounds 7a-7b * | 1-27 | A61K A61P C07D |
| X | US 2007/078136 A1 (VACCARO WAYNE [US] ET AL) 5 April 2007 (2007-04-05) | 1-27 | |
| Y | * page 50; compound XIII * * the whole document * | 1-27 | |

-/--

The present search report has been drawn up for all claims

1

| Place of search | Date of completion of the search | Examiner |
|---|---|---|
| Munich | 7 October 2022 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 30 5621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2021/176045 A1 (BAYER AG [DE]; DEUTSCHES KREBSFORSCH [DE]) 10 September 2021 (2021-09-10)<br>* examples 1-23 *<br>* the whole document *<br>----- | 1-27<br><br><br>1-27 | |
| Y | GHIGO ALESSANDRA ET AL: "Dysfunctional Inflammation in Cystic Fibrosis Airways: From Mechanisms to Novel Therapeutic Approaches", INTERNATIONAL JOURNAL OF MOLECULAR SCIENCES, vol. 22, no. 4, 16 February 2021 (2021-02-16), page 1952, XP055967031, DOI: 10.3390/ijms22041952 Retrieved from the Internet: URL:https://www.mdpi.com/1422-0067/22/4/1952><br>* whole document, disclosure of trikafta and roscovitine *<br>----- | 1-27 | TECHNICAL FIELDS SEARCHED (IPC) |
| X<br><br>Y | WO 2022/040447 A2 (UNIV LELAND STANFORD JUNIOR [US]) 24 February 2022 (2022-02-24)<br>* the combination of trikafta (CFTR modulator) with semagacestat or MK-0752 (=MK-04752 in the text, GSI modulators); claims 14-16, 22-24; figures 28, 32; examples 11, 15 *<br>----- | 1-27<br><br>1-27 | |

The present search report has been drawn up for all claims

1

| Place of search | Date of completion of the search | Examiner |
|---|---|---|
| Munich | 7 October 2022 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 22 30 5621

This annex lists the patent family members relating to the patent documents cited in the above-mentioned European search report. The members are as contained in the European Patent Office EDP file on
The European Patent Office is in no way liable for these particulars which are merely given for the purpose of information.

07-10-2022

| Patent document cited in search report | Publication date | Patent family member(s) | Publication date |
|---|---|---|---|
| WO 2017070135 A1 | 27-04-2017 | US 2018305364 A1 | 25-10-2018 |
| | | WO 2017070135 A1 | 27-04-2017 |
| US 2007078136 A1 | 05-04-2007 | AR 055177 A1 | 08-08-2007 |
| | | AU 2006295439 A1 | 05-04-2007 |
| | | BR PI0616393 A2 | 21-06-2011 |
| | | CA 2623369 A1 | 05-04-2007 |
| | | CY 1114001 T1 | 27-07-2016 |
| | | DK 1928879 T3 | 21-05-2013 |
| | | EA 200800873 A1 | 29-08-2008 |
| | | EP 1928879 A2 | 11-06-2008 |
| | | ES 2402664 T3 | 07-05-2013 |
| | | GE P20104943 B | 12-04-2010 |
| | | HR P20130155 T1 | 31-03-2013 |
| | | IL 190280 A | 29-08-2013 |
| | | JP 5241498 B2 | 17-07-2013 |
| | | JP 2009509961 A | 12-03-2009 |
| | | KR 20080063344 A | 03-07-2008 |
| | | KR 20140058645 A | 14-05-2014 |
| | | NZ 566663 A | 31-03-2011 |
| | | PE 20070520 A1 | 29-08-2007 |
| | | PH 12013501329 A1 | 27-08-2014 |
| | | PL 1928879 T3 | 31-07-2013 |
| | | PT 1928879 E | 15-04-2013 |
| | | SI 1928879 T1 | 28-06-2013 |
| | | TW 200804385 A | 16-01-2008 |
| | | TW 201350488 A | 16-12-2013 |
| | | US 2007078136 A1 | 05-04-2007 |
| | | WO 2007038314 A2 | 05-04-2007 |
| WO 2021176045 A1 | 10-09-2021 | AU 2021231312 A1 | 25-08-2022 |
| | | WO 2021176045 A1 | 10-09-2021 |
| WO 2022040447 A2 | 24-02-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GUO et al.** Worldwide rates of diagnosis and effective treatment for cystic fibrosis. *J. Cystic Fibrosis,* 2022
- **KIDD, T.J. et al.** Defining antimicrobial resistance in cystic fibrosis. *J. Cyst. Fibros.,* 2018, vol. 17, 696-704
- **WATERS, V.J. et al.** Reconciling antimicrobial susceptibility testing and clinical response in antimicrobial treatment of chronic cystic fibrosis lung infections. *Clin. Infect. Dis.,* 2019, vol. 69, 1812-1816
- **K. BETTAYEB et al.** N-&-N, a new class of cell death-inducing kinase inhibitors derived from the purine roscovitine. *Mol. Cancer Ther.,* 2008, vol. 7, 2713-2724
- **F. POPOWYCZ et al.** Pyrazolo[1,5-a]-1,3,5-triazine as purine bioisostere: access to potent CDK inhibitor (R)-roscovitine analogue. *J. Med. Chem.,* 2009, vol. 52, 655-663
- **C. NOREZ et al.** Roscovitine is a proteostasis regulator that corrects the trafficking defect of F508del-CFTR by a CDK-independent mechanism. *Br. J. Pharmacol.,* 2014, vol. 171, 4831-4849
- **RIAZANSKI et al.** TRPC6 channel translocation into phagosomal membrane augments phagosomal function. *Proc. Natl. Acad. Sci. USA,* 2015, vol. 112, E6486-6495
- **KUNZELMANN, K. et al.** An immortalized cystic fibrosis tracheal epithelial cell line homozygous for the F508del CFTR mutation. *Am. J. Respir. Cell Mol. Biol.,* 1993, vol. 8, 522-529
- **BORST P ; ELFERINK RO.** Mammalian ABC transporters in health and disease. *Annu Rev Biochem.,* 2002, vol. 71, 537-592
- **LOPES-PACHECO M.** CFTR Modulators: The Changing Face of Cystic Fibrosis in the Era of Precision Medicine. *Front. Pharmacol.,* 2020, vol. 10, 1662